(19) Europäisches Patentamt / European Patent Office / Office européen des brevets

(11) EP 4 700 025 A1

(12) # EUROPEAN PATENT APPLICATION
published in accordance with Art. 153(4) EPC

(43) Date of publication:
**25.02.2026 Bulletin 2026/09**

(21) Application number: **24796041.2**

(22) Date of filing: **23.04.2024**

(51) International Patent Classification (IPC):
***C07D 471/04*** (2006.01) ***C07D 401/12*** (2006.01)
***C07D 413/14*** (2006.01) ***C07D 405/14*** (2006.01)
***A61K 31/407*** (2006.01) ***A61P 35/00*** (2006.01)
***A61P 35/02*** (2006.01)

(52) Cooperative Patent Classification (CPC):
**A61K 31/407; A61P 35/00; A61P 35/02; C07D 401/12; C07D 405/14; C07D 413/14; C07D 471/04**

(86) International application number:
**PCT/CN2024/089253**

(87) International publication number:
**WO 2024/222660 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.04.2023 CN 202310440810**

(71) Applicant: **Suzhou Genhouse Bio Co., Ltd.**
**Suzhou,Jiangsu 215123 (CN)**

(72) Inventors:
- **ZHANG, Guiping**
  **Suzhou, Jiangsu 215123 (CN)**
- **LI, Jiapeng**
  **Suzhou, Jiangsu 215123 (CN)**
- **WANG, Kuifeng**
  **Suzhou, Jiangsu 215123 (CN)**
- **ZHENG, Jiyue**
  **Suzhou, Jiangsu 215123 (CN)**
- **ZHANG, Tao**
  **Suzhou, Jiangsu 215123 (CN)**
- **FARIDOON**
  **Suzhou, Jiangsu 215123 (CN)**
- **XU, Haojie**
  **Suzhou, Jiangsu 215123 (CN)**
- **DONG, Xue**
  **Suzhou, Jiangsu 215123 (CN)**

(74) Representative: **Turner, Craig Robert**
**Venner Shipley LLP**
**200 Aldersgate**
**London EC1A 4HD (GB)**

(54) **AMIDE COMPOUND, PHARMACEUTICAL COMPOSITION COMPRISING SAME, AND USE THEREOF**

(57)

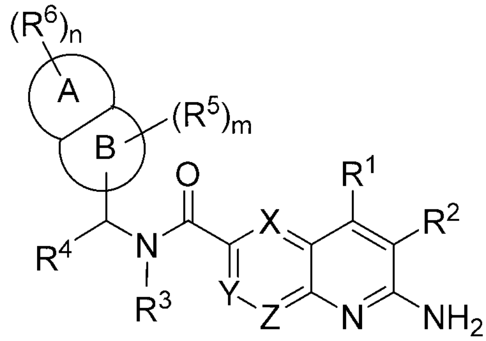

(I)

The present invention relates to a compound of for-



EP 4 700 025 A1

mula (I), a pharmaceutical composition comprising same, and a use thereof for preventing or treating a disease.

## Description

### TECHNICAL FIELD

The present disclosure relates to an amide compound, a pharmaceutical composition comprising same, and use thereof in preventing or treating a disease.

### BACKGROUND

Arginine methylation, a type of histone methylation, is one of the most common post-translational modifications in mammals and is primarily regulated by the PRMT (protein arginine methyltransferase) gene family. PRMTs can transfer the methyl group on S-adenosylmethionine (AdoMet/SAM) to the guanidyl nitrogen atom of the arginine side chain of proteins, resulting in methylated arginine. PRMTs can regulate arginine methylation via a variety of different mechanisms and play important roles in biological processes such as gene expression, splicing, and DNA damage repair. Alterations in PRMT enzymatic activity and mutations or deletions in PRMT genes are usually closely associated with abnormal development in individual animals and the initiation and progression of cancer.

In the PRMT gene family, PRMT5, as an epigenetic enzyme, is involved in a variety of physiological processes such as transcription regulation, RNA metabolism, ribosome biosynthesis, and cell cycle regulation, and is particularly upregulated in a variety of cancers such as lymphoma, lung cancer, breast cancer, and ovarian cancer, indicating its critical role in the formation and development of tumors. Codeletion of MTAP (methylthioadenosine phosphorylase) and CDKN2A, the common tumor suppressor gene in the body, is frequent, and the codeletion rate can be up to 9% to 15% in tumors. Other studies have reported that deletion of MTAP results in accumulation of intracellular MTA (methylthioadenosine, a substrate of MTAP), and excessive MTA binds to and inhibits partial activity of PRMT5, thereby exhibiting an MTAP-PRMT5 lethal effect. Currently, several PRMT5 inhibitors have entered clinical trials, but most of them are non-selective for PRMT5·MTA and exhibit poor selectivity for MTAP-deficient tumors, with considerable toxicity.

### BRIEF SUMMARY

The present application provides a compound as a PRMT5 inhibitor, which exhibits excellent inhibitory activity against PRMT5 (particularly in MTAP-deficient cells). In addition, the compound of the present disclosure also has good physicochemical properties (e.g., solubility and physical and/or chemical stability), good pharmacokinetic properties (e.g., improved bioavailability, good metabolic stability, and suitable half-life and duration of action), good safety (less toxicity (e.g., reduced cardiotoxicity), and/or fewer side effects), and lower propensity to induce drug resistance.

One aspect of the present disclosure provides a compound, or a pharmaceutically acceptable salt, an ester, a stereoisomer, an atropisomer, a tautomer, a polymorph, a solvate, a metabolite, an isotopically labeled compound, or a prodrug thereof, wherein the compound has a structure of formula (I):

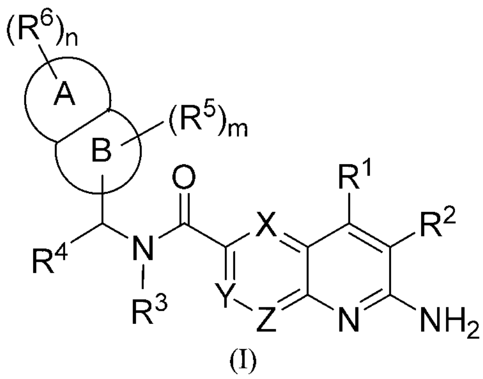

(I)

wherein:

- ring A is a $C_{3-10}$ hydrocarbon ring, a 3- to 10-membered heterocyclic ring, a $C_{6-10}$ aromatic ring, or a 5- to 14-membered heteroaromatic ring;
- ring B is a benzene ring;
- X is $CR^X$ or N;
- Y is $CR^Y$ or N;
- Z is $CR^Z$ or N;

$R^X$, $R^Y$, $R^Z$, $R^1$, $R^2$, $R^3$, $R^4$, $R^5$, and $R^6$, at each occurrence, are each independently selected from H, halogen, -OH, $-NH_2$, -CN, $-NO_2$, $C_{1-6}$ alkyl, deuterated $C_{1-6}$ alkyl, $C_{2-6}$ alkenyl, $C_{2-6}$ alkynyl, $C_{3-6}$ cyclohydrocarbyl, 3-to 10-membered heterocyclyl, $C_{6-10}$ aryl, 5- to 14-membered heteroaryl, $C_{6-12}$ aralkyl, $-C(=O)R^a$, $-OC(=O)R^a$, $-C(=O)OR^a$, $-OR^a$, $-SR^a$, $-S(=O)R^a$, $-S(=O)_2R^a$, $-S(=O)_2NR^aR^b$, $-NR^aR^b$, $-C(=O)NR^aR^b$, $-NR^a-C(=O)R^b$, $-NR^a-C(=O)OR^b$, $-NR^a-S(=O)_2-R^b$, $-NR^a-C(=O)-NR^aR^b$, $-C_{1-6}$ alkylene-$OR^a$, $-C_{1-6}$ alkylene-$NR^aR^b$, and $-O-C_{1-6}$ alkylene-$NR^aR^b$; or $R^1$ and $R^2$, together with the groups to which they are attached, optionally form a $C_{3-10}$ hydrocarbon ring, a 3- to 10-membered heterocyclic ring, a $C_{6-10}$ aromatic ring, or a 5- to 14-membered heteroaromatic ring; and/or, $R^3$ and $R^4$, together with the groups to which they are attached, optionally form a 3- to 10-membered heterocyclic ring;

$R^a$ and $R^b$, at each occurrence, are each independently selected from H, $C_{1-6}$ alkyl, $C_{3-10}$ cyclohydrocarbyl, 3-to 10-membered heterocyclyl, $C_{6-10}$ aryl, 5- to 14-membered heteroaryl, and $C_{6-12}$ aralkyl;

the above alkyl, alkylene, alkenyl, alkynyl, cyclohydrocarbyl, hydrocarbon ring, heterocyclyl, heterocyclic ring, aryl, aromatic ring, heteroaryl, heteroaromatic ring, and aralkyl, at each occurrence, are each optionally substituted with one or more substituents independently selected from the following: halogen, -OH, =O, $-NH_2$, -CN, $-NO_2$, $C_{1-6}$ alkyl, $C_{3-6}$ cyclohydrocarbyl, 3- to 10-membered heterocyclyl, $C_{6-10}$ aryl, 5- to 14-membered heteroaryl, $C_{6-12}$ aralkyl, $-C(=O)R^c$, $-OC(=O)R^c$, $-C(=O)OR^c$, $-OR^c$, $-SR^c$, $-S(=O)R^c$, $-S(=O)_2R^c$, $-S(=O)_2NR^cR^d$, $-NR^cR^d$, $-C(=O)NR^cR^d$, $-NR^c-C(=O)R^d$, $-NR^c-C(=O)OR^d$, $-NR^c-S(=O)_2-R^d$, $-NR^c-C(=O)-NR^cR^d$, $-C_{1-6}$ alkylene-$OR^c$, $-C_{1-6}$ alkylene-$NR^cR^d$, and $-O-C_{1-6}$ alkylene-$NR^cR^d$; the alkyl, cyclohydrocarbyl, heterocyclyl, aryl, heteroaryl, and aralkyl are further optionally substituted with one or more substituents independently selected from the following: halogen, -OH, =O, -C(=O)O-tert-butyl, $-NH_2$, -CN, $-NO_2$, $C_{1-6}$ alkyl, halogenated $C_{1-6}$ alkyl, $C_{3-6}$ cyclohydrocarbyl, 3- to 10-membered heterocyclyl, $C_{6-10}$ aryl, 5- to 14-membered heteroaryl, $C_{6-12}$ aralkyl, $-O-C_{1-6}$ alkyl, and $-C_{1-6}$ alkylene-$O-C_{1-6}$ alkyl;

$R^c$ and $R^d$, at each occurrence, are each independently selected from: H, $C_{1-6}$ alkyl, $C_{3-10}$ cyclohydrocarbyl, 3- to 10-membered heterocyclyl, $C_{6-10}$ aryl, 5- to 14-membered heteroaryl, and $C_{6-12}$ aralkyl; the alkyl, cyclohydrocarbyl, heterocyclyl, aryl, heteroaryl, and aralkyl are further optionally substituted with one or more substituents independently selected from the following: halogen, -OH, =O, -C(=O)O-tert-butyl, $-NH_2$, -CN, $-NO_2$, $C_{1-6}$ alkyl, halogenated $C_{1-6}$ alkyl, $C_{3-6}$ cyclohydrocarbyl, 3- to 10-membered heterocyclyl, $C_{6-10}$ aryl, 5- to 14-membered heteroaryl, $C_{6-12}$ aralkyl, and $-C_{1-6}$ alkylene-$O-C_{1-6}$ alkyl; and

m and n are each independently an integer selected from 1, 2, 3, or 4, preferably an integer selected from 1, 2, or 3.

Another aspect of the present disclosure provides a pharmaceutical composition comprising a prophylactically or therapeutically effective amount of the compound, or the pharmaceutically acceptable salt, the ester, the stereoisomer, the tautomer, the polymorph, the solvate, the metabolite, the isotopically labeled compound, or the prodrug thereof of the present disclosure, and one or more pharmaceutically acceptable carriers.

Another aspect of the present disclosure provides use of the compound, or the pharmaceutically acceptable salt, the ester, the stereoisomer, the tautomer, the polymorph, the solvate, the metabolite, the isotopically labeled compound, or the prodrug thereof of the present disclosure, or of the pharmaceutical composition of the present disclosure in preparing a medicament as a PRMT5 inhibitor.

Another aspect of the present disclosure provides the compound, or the pharmaceutically acceptable salt, the ester, the stereoisomer, the tautomer, the polymorph, the solvate, the metabolite, the isotopically labeled compound, or the prodrug thereof of the present disclosure, or the pharmaceutical composition of the present disclosure, for use as a PRMT5 inhibitor.

Another aspect of the present disclosure provides a method for preventing or treating a cancer (preferably an MTAP-deficient cancer), comprising administering to a subject in need thereof an effective amount of the compound, or the pharmaceutically acceptable salt, the ester, the stereoisomer, the tautomer, the polymorph, the solvate, the metabolite, the isotopically labeled compound, or the prodrug thereof of the present disclosure, or the pharmaceutical composition of the present disclosure.

## DETAILED DESCRIPTION OF THE INVENTION

### Definitions

Unless otherwise defined below, all technical and scientific terms used herein are intended to have the same meaning as commonly understood by those skilled in the art. Reference to the techniques used herein is intended to refer to techniques commonly understood in the art, including those variations of or equivalent alternatives to the techniques that are apparent to those skilled in the art. Although the following terms are believed to be well understood by those skilled in the art, the following definitions are set forth to better explain the present disclosure.

The terms "include", "comprise", "have", "contain", or "involve", and other variant forms thereof herein, are inclusive or open-ended and do not exclude other unrecited elements or method steps.

As used herein, the term "alkylene" refers to saturated divalent hydrocarbyl, preferably saturated divalent

hydrocarbyl having 1, 2, 3, 4, 5, or 6 carbon atoms, e.g., methylene, ethylene, propylene, or butylene.

As used herein, the term "alkyl" is defined as a linear or branched saturated aliphatic hydrocarbon. In some embodiments, the alkyl has 1 to 12, e.g., 1 to 6, carbon atoms. For example, as used herein, the term "$C_{1-6}$ alkyl" refers to a linear or branched group of 1 to 6 carbon atoms (e.g., methyl, ethyl, *n*-propyl, isopropyl, *n*-butyl, isobutyl, *sec*-butyl, *tert*-butyl, *n*-pentyl, or *n*-hexyl) that is optionally substituted with 1 or more (such as 1 to 3) suitable substituents such as halogen (in this case the group is referred to as "halogenated alkyl") (e.g., $CF_3$, $C_2F_5$, $CHF_2$, $CH_2F$, $CH_2CF_3$, $CH_2Cl$, $-CH_2CH_2CF_3$, or the like). The term "$C_{1-4}$ alkyl" refers to a linear or branched aliphatic hydrocarbon chain of 1 to 4 carbon atoms (i.e., methyl, ethyl, *n*-propyl, isopropyl, *n*-butyl, isobutyl, *sec*-butyl, or *tert*-butyl).

As used herein, the term "alkenyl" refers to linear or branched monovalent hydrocarbyl containing one or more double bonds and having 2 to 6 carbon atoms ("$C_{2-6}$ alkenyl"). The alkenyl is, for example, $-CH=CH_2$, $-CH_2CH=CH_2$, $-C(CH_3)=CH_2$, $-CH_2-CH=CH-CH_3$, 2-pentenyl, 3-pentenyl, 4-pentenyl, 2-hexenyl, 3-hexenyl, 4-hexenyl, 5-hexenyl, 2-methyl-2-propenyl, or 4-methyl-3-pentenyl. When the compound of the present disclosure contains the alkenyl, the compound may be present in a pure E (entgegen) form, a pure Z (zusammen) form, or any mixture thereof. The term "alkenylene" is a corresponding divalent group, including, for example, "$C_{2-6}$ alkenylene", "$C_{2-4}$ alkenylene", and the like, specific examples of which include, but are not limited to: $-CH=CH-$, $-CH_2CH=CH-$, $-C(CH_3)=CH-$, butenylene, pentenylene, hexenylene, and the like.

As used herein, the term "alkynyl" refers to monovalent hydrocarbyl containing one or more triple bonds and preferably having 2, 3, 4, 5, or 6 carbon atoms, e.g., ethynyl, 2-propynyl, 2-butynyl, 1,3-butadiynyl, and the like. The alkynyl is optionally substituted with one or more (such as 1 to 3) identical or different substituents. The term "alkynylene" is a corresponding divalent group, including, for example, "$C_{2-8}$ alkynylene", "$C_{2-6}$ alkynylene", "$C_{2-4}$ alkynylene", and the like, examples of which include, but are not limited to,

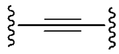,

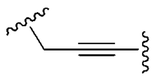, 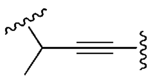, 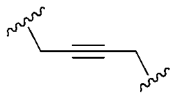,

and the like. The alkynylene is optionally substituted with one or more (such as 1 to 3) identical or different substituents.

As used herein, the term "fused ring" refers to a ring system formed by two or more cyclic structures that share two adjacent atoms.

As used herein, the term "spiro ring" refers to a ring system formed by two or more cyclic structures that share one ring atom.

As used herein, the term "bridged ring" refers to a ring system formed by two or more cyclic structures that share two atoms not directly connected.

As used herein, the terms "cyclohydrocarbylene", "cyclohydrocarbyl", and "hydrocarbon ring" refer to a saturated (i.e., "cycloalkylene" and "cycloalkyl") or partially unsaturated (i.e., having one or more double and/or triple bonds within the ring) monocyclic or polycyclic hydrocarbon ring (including a spiro ring, fused ring, or bridged ring system) having, for example, 3 to 10 (suitably 3 to 8, and more suitably 3 to 6) ring carbon atoms, including, but not limited to, cyclopropyl(ene) (cyclopropane ring), cyclobutyl(ene) (cyclobutane ring), cyclopentyl(ene)(cyclopentane ring), cyclohexyl(ene) (cyclohexane ring), cycloheptyl(ene) (cycloheptane ring), cyclooctyl(ene) (cyclooctane ring), cyclononyl(ene) (cyclononane ring), cyclohexenyl(ene) (cyclohexene ring), and the like.

As used herein, the term "cycloalkyl" refers to a saturated monocyclic or polycyclic (such as bicyclic) hydrocarbon ring (e.g., a monocyclic ring, such as cyclopropyl, cyclobutyl, cyclopentyl, cyclohexyl, cycloheptyl, cyclooctyl, or cyclononyl, or a bicyclic ring, including a spiro ring, fused ring, or bridged ring system (such as bicyclo[1.1.1]pentyl, bicyclo[2.2.1]heptyl, bicyclo[3.2.1]octyl, bicyclo[5.2.0]nonyl, or decahydronaphthyl)) optionally substituted with 1 or more (such as 1 to 3) suitable substituents. The cycloalkyl has 3 to 15 carbon atoms. For example, the term "$C_{3-6}$ cycloalkyl" refers to a saturated monocyclic or polycyclic (such as bicyclic) hydrocarbon ring of 3 to 6 ring-forming carbon atoms (e.g., cyclopropyl, cyclobutyl, cyclopentyl, or cyclohexyl) optionally substituted with 1 or more (such as 1 to 3) suitable substituents, for example, cyclopropyl substituted with methyl.

As used herein, the term "heterocyclyl" (or "heterocyclic ring") refers to a saturated or partially unsaturated monocyclic or bicyclic group having 2, 3, 4, 5, 6, 7, 8, 9, or 10 carbon atoms and one or more (e.g., one, two, three, or four) heteroatoms selected from O, S, N, and P in the ring, and the "heterocyclyl" (or "heterocyclic ring") may contain -C(=O)- as a ring member. The heterocyclyl may be connected to the rest of the molecule by the carbon atom and/or the heteroatom (if present). In particular, 3- to 10-membered heterocyclyl is a group having 3 to 10 carbon atoms and heteroatoms in the ring, for example, but not limited to, oxiranyl, aziridinyl, azetidinyl, oxetanyl, tetrahydrofuranyl, dioxolinyl, pyrrolidinyl, pyrrolidinonyl, imidazolidinyl, pyrazolidinyl, pyrrolinyl, tetrahydropyranyl, piperidinyl, morpholinyl, dithianyl, thiomorpholinyl, piperazinyl, or trithianyl.

As used herein, the term "heterocyclyl" (or "heterocyclic ring") encompasses fused ring structures, the point of attachment of which to other groups may be on any ring of the fused ring structures. Thus, the heterocyclyl of the present disclosure further includes, but is not limited to, heterocyclyl fused with heterocyclyl, heterocyclyl fused with cycloalkyl, monoheterocyclyl fused with monoheterocyclyl, monoheterocyclyl fused with monocycloalkyl, aryl fused with heterocyclyl, and heteroaryl fused with heterocyclyl, e.g., 3- to 7-membered (mono)heterocyclyl fused with 3- to 7-membered (mono)heterocyclyl, 3- to 7-membered (mono)heterocyclyl fused with (mono)cycloalkyl, 3- to 7-membered (mono) heterocyclyl fused with $C_{4-6}$ (mono)cycloalkyl, $C_{6-10}$ aryl fused with 3- to 7-membered heterocyclyl, and 5- to 6-membered heteroaryl fused with 3- to 7-membered heterocyclyl, examples of which include, but are not limited to, pyrrolidinyl fused with cyclopropyl, cyclopentyl fused with aziridinyl, pyrrolidinyl fused with cyclobutyl, pyrrolidinyl fused with pyrrolidinyl, pyrrolidinyl fused with piperidinyl, pyrrolidinyl fused with piperazinyl, piperidinyl fused with morpholinyl,

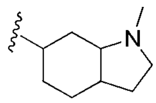, 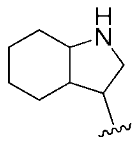, or 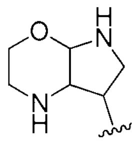.

As used herein, the term "heterocyclyl" (or "heterocyclic ring") encompasses bridged heterocyclyl (bridged heterocyclic ring) and spiro heterocyclyl (spiro heterocyclic ring).

As used herein, the term "bridged heterocyclic ring" refers to a cyclic structure containing one or more (e.g., 1, 2, 3, or 4) heteroatoms (e.g., oxygen atoms, nitrogen atoms, and/or sulfur atoms) formed by two rings that share two ring atoms not directly connected, including, but not limited to, a 7- to 10-membered bridged heterocyclic ring, a 8- to 10-membered bridged heterocyclic ring, a 7- to 10-membered nitrogen-containing bridged heterocyclic ring, a 7- to 10-membered oxygen-containing bridged heterocyclic ring, a 7- to 10-membered sulfur-containing bridged heterocyclic ring, and the like, e.g.,

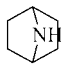, 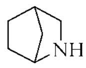, 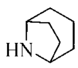, 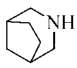,

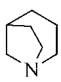, 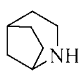, 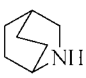, 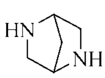, 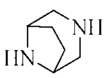, 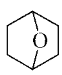, 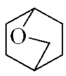, 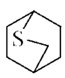, 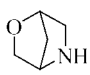, 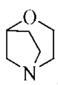, 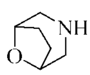, 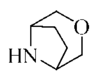, 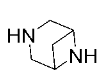,

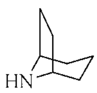, 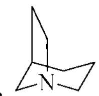, 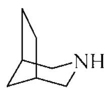, 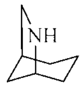, and 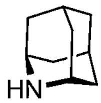.

The "nitrogen-containing bridged heterocyclic ring", "oxygen-containing bridged heterocyclic ring", or "sulfur-containing bridged heterocyclic ring" optionally further contains one or more other heteroatoms selected from oxygen, nitrogen, and sulfur.

As used herein, the term "spiro heterocyclic ring" refers to a cyclic structure containing one or more (e.g., 1, 2, 3, or 4) heteroatoms (e.g., oxygen atoms, nitrogen atoms, sulfur atoms) formed by two or more rings that share one ring atom, including, but not limited to, a 5- to 10-membered spiro heterocyclic ring, a 6- to 10-membered spiro heterocyclic ring, a 6- to 10-membered nitrogen-containing spiro heterocyclic ring, a 6- to 10-membered oxygen-containing spiro heterocyclic ring, a 6- to 10-membered sulfur-containing spiro heterocyclic ring, and the like, e.g.,

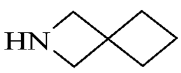, 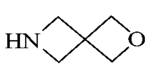, 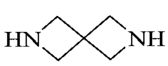, 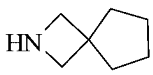,

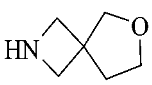, 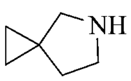, 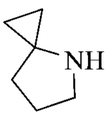, 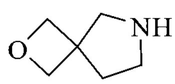, 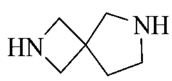, 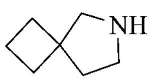, 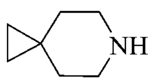,

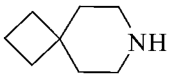, 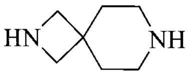, 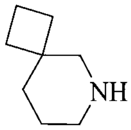, 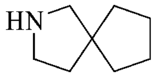, 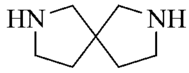, 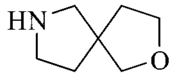,

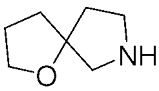, 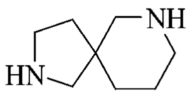, and 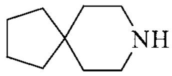.

The "nitrogen-containing spiro heterocyclic ring", "oxygen-containing spiro heterocyclic ring", or "sulfur-containing spiro heterocyclic ring" optionally further contains one or more other heteroatoms selected from oxygen, nitrogen, and sulfur. The term "6- to 10-membered nitrogen-containing spiro heterocyclyl" refers to spiro heterocyclyl containing a total of 6 to 10 ring atoms, and at least one of which is a nitrogen atom.

As used herein, the terms "aryl(ene)" and "aromatic ring" refer to an all-carbon monocyclic or fused polycyclic aromatic group having a conjugated $\pi$-electron system. For example, as used herein, the terms "$C_{6-10}$ aryl(ene)" and "$C_{6-10}$ aromatic ring" refer to an aromatic group containing 6 to 10 carbon atoms, such as phenyl(ene) (benzene ring) or naphthyl(ene) (naphthalene ring). The aryl(ene) and aromatic ring are optionally substituted with 1 or more (such as 1 to 3) suitable substituents (e.g., halogen, -OH, -CN, $-NO_2$, $C_{1-6}$ alkyl, and the like).

The term "aralkyl" refers to alkyl substituted with aryl, wherein the aryl and the alkyl are as defined herein. Generally, the aryl may have 6 to 14 carbon atoms and the alkyl may have 1 to 6 carbon atoms. Exemplary aralkyl includes, but is not limited to, benzyl, phenylethyl, phenylpropyl, and phenylbutyl.

As used herein, the terms "heteroaryl(ene)" and "heteroaromatic ring" refer to a monocyclic, bicyclic, or tricyclic aromatic ring system, which has 5, 6, 7, 8, 9, 10, 11, 12, 13, or 14 ring atoms, in particular 1 or 2 or 3 or 4 or 5 or 6 or 9 or 10 carbon atoms, and contains at least one heteroatom which may be identical or different (the heteroatom is, for example, oxygen, nitrogen, or sulfur), and may be benzo-fused in each case. In particular, "heteroaryl(ene)" or "heteroaromatic ring" is selected from thienyl(ene) (thiophene ring), furyl(ene) (furan ring), pyrrolyl(ene) (pyrrole ring), oxazolyl(ene) (oxazole ring), thiazolyl(ene) (thiazole ring), imidazolyl(ene) (imidazole ring), pyrazolyl(ene) (pyrazole ring), isoxazolyl(ene) (isoxazole ring), isothiazolyl(ene) (isothiazole ring), oxadiazolyl(ene) (oxadiazole ring), triazolyl(ene) (triazole ring), thiadiazolyl(ene) (thiadiazole ring) and the like, as well as benzo derivatives thereof; or pyridinyl(ene) (pyridine ring), pyridazinyl(ene) (pyridazine ring), pyrimidinyl(ene) (pyrimidine ring), pyrazinyl(ene) (pyrazine ring), triazinyl(ene) (triazine ring), and the like, as well as benzo derivatives thereof.

As used herein, the term "halogenated" or "halogen" group is defined to include F, Cl, Br, or I.

As used herein, the term "alkylthio" refers to alkyl as defined above that is attached to a parent molecular moiety via a sulfur atom. Representative examples of $C_{1-6}$ alkylthio include, but are not limited to, methylthio, ethylthio, tert-butylthio, and hexylthio.

As used herein, the term "nitrogen-containing heterocyclic ring" refers to a saturated or partially unsaturated monocyclic or bicyclic group having 2, 3, 4, 5, 6, 7, 8, 9, 10, 11, 12, or 13 carbon atoms and at least one nitrogen atom in the ring, and may also optionally contain one or more (e.g., one, two, three, or four) ring members selected from N, O, S, S=O, and $S(=O)_2$. The nitrogen-containing heterocyclic ring is attached to the rest of the molecule via any one of the ring members. The nitrogen-containing heterocyclic ring is preferably a saturated nitrogen-containing monocyclic ring. In particular, a 3- to 14-membered nitrogen-containing heterocyclic ring is a group having 3 to 14 carbon atoms and heteroatoms (at least one of which is a nitrogen atom) in the ring, including, but not limited to, a three-membered nitrogen-containing heterocyclic ring (e.g., aziridinyl), a four-membered nitrogen-containing heterocyclic ring (e.g., azetidinyl), a five-membered nitrogen-containing heterocyclic ring (e.g., pyrrolyl, pyrrolidinyl (pyrrolidine ring), pyrrolinyl, pyrrolidinonyl, imidazolyl, imidazolidinyl, imidazolinyl, pyrazolyl, or pyrazolinyl), a six-membered nitrogen-containing heterocyclic ring (e.g., piperidinyl (piperidine ring), morpholinyl, thiomorpholinyl, or piperazinyl), a seven-membered nitrogen-containing heterocyclic ring, and the like.

The term "substitution" means that one or more (e.g., one, two, three, or four) hydrogen atoms on a specified atom are replaced with a selection from the designated group, provided that the normal valency of the atom specified under the present circumstances is not exceeded and that the replacement results in a stable compound. A combination of substituents and/or variables is permissible only if the combination results in a stable compound.

If a substituent is described as "optionally substituted with...", the substituent may be (1) unsubstituted or (2) substituted. If a carbon of a substituent is described as being optionally substituted with one or more of a list of substituents, then one or more hydrogen atoms on the carbon (to the extent of any hydrogen atoms present) may be replaced individually and/or together with an independently selected optional substituent. If a nitrogen of a substituent is described as being optionally substituted with one or more of a list of substituents, then one or more hydrogen atoms on the nitrogen (to the extent any hydrogen atoms present) may each be replaced with an independently selected optional substituent.

If a substituent is described as being "independently selected from" one group, each substituent is selected independently of the other. Thus, each substituent may be identical to or different from another (other) substituent(s).

As used herein, the term "one or more" refers to 1 or more than 1, such as 2, 3, 4, 5, or 10, under reasonable conditions.

Unless otherwise indicated, as used herein, the point of attachment of a substituent may be from any suitable position of the substituent.

When a bond of a substituent is shown to pass through a bond connecting two atoms in the ring, the substituent may be bonded to any one of the ring-forming atoms in the substitutable ring.

The present disclosure also includes all pharmaceutically acceptable isotopically labeled compounds, which are identical to the compounds of the present disclosure, except that one or more atoms are replaced by an atom having the same atomic number, but an atomic mass or mass number different from the atomic mass or mass number predominant in nature. Examples of isotopes suitable for incorporation into the compounds of the present disclosure include, but are not limited to, isotopes of hydrogen (e.g., deuterium (D, $^{2}H$), tritium (T, $^{3}H$)); isotopes of carbon (e.g., $^{11}C$, $^{13}C$, and $^{14}C$); isotopes of chlorine (e.g., $^{36}Cl$); isotopes of fluorine (e.g., $^{18}F$); isotopes of iodine (e.g., $^{123}I$ and $^{125}I$); isotopes of nitrogen (e.g., $^{13}N$ and $^{15}N$); isotopes of oxygen (e.g., $^{15}O$, $^{17}O$, and $^{18}O$); isotopes of phosphorus (e.g., $^{32}P$); and isotopes of sulfur (e.g., $^{35}S$). Certain isotopically-labeled compounds of the present disclosure (e.g., those into which a radioactive isotope is incorporated) can be used in drug and/or substrate tissue distribution studies (e.g., assays). The radioactive isotopes tritium (i.e., $^{3}H$) and carbon-14 (i.e., $^{14}C$) are particularly useful for this purpose because of their ease of incorporation and detection. Substitution with positron emitting isotopes (e.g., $^{11}C$, $^{18}F$, $^{15}O$, and $^{13}N$) can be used to examine substrate receptor occupancy in positron emission tomography (PET) studies. The isotopically labeled compounds of the present disclosure can be prepared by processes analogous to those described in the accompanying routes and/or in the examples and preparations by using an appropriate isotopically labeled reagent in place of the non-labeled reagent previously employed. The pharmaceutically acceptable solvates of the present disclosure include those in which the crystallization solvent may be isotopically substituted, e.g., $D_2O$, acetone-$d_6$, or DMSO-$d_6$.

The term "stereoisomer" refers to an isomer formed due to at least one asymmetric center. In compounds having one or more (e.g., one, two, three, or four) asymmetric centers, racemic mixtures, single enantiomers, mixtures of diastereomers, and individual diastereomers may be generated. Certain individual molecules may also present as geometric isomers (cis/trans). Similarly, the compound of the present disclosure may present as a mixture of two or more structurally different forms in rapid equilibrium (commonly referred to as tautomers). Representative examples of tautomers include keto-enol tautomers, phenol-keto tautomers, nitroso-oxime tautomers, imine-enamine tautomers, and the like. It should be understood that the scope of the present application encompasses all such isomers or mixtures thereof in any proportion (e.g., 60%, 65%, 70%, 75%, 80%, 85%, 90%, 95%, 96%, 97%, 98%, or 99%).

A solid line ( ), a solid wedge ( ), or a dashed wedge ( ) may be used herein to depict carbon-carbon bonds of the compounds of the present disclosure. Depiction of a bond bonded to an asymmetric carbon atom using a solid line is intended to indicate that all possible stereoisomers (e.g., specific enantiomers and racemic mixtures) at the carbon atom are included. Depiction of a bond bonded to an asymmetric carbon atom using a solid or dashed wedge is intended to indicate that the shown stereoisomer is present. When a bond is present in a racemic mixture, solid and dashed wedges are used to define relative stereochemistry, rather than absolute stereochemistry. Unless otherwise indicated, the compounds of the present disclosure are intended to be present in the forms of stereoisomers (including cis and trans isomers, optical isomers (e.g., R and S enantiomers), diastereomers, geometric isomers, rotamers, conformers, atropisomers, and mixtures thereof). The compounds of the present disclosure may exhibit one or more types of isomerism, and consist of mixtures thereof (e.g., racemic mixtures and diastereomeric pairs).

Atropisomers refer to compounds that can be separated into rotationally restricted isomers.

It should be understood that certain compounds of the present disclosure may be present in free form for use in therapy or, where appropriate, in the form of a pharmaceutically acceptable derivative thereof. In the present disclosure, the pharmaceutically acceptable derivatives include, but are not limited to, pharmaceutically acceptable salts, esters, solvates, metabolites, or prodrugs, which upon administration to a patient in need thereof are capable of providing, directly or indirectly, the compound of the present disclosure or a metabolite or residue thereof. Thus, when reference is made herein to "the compound of the present disclosure", it is also intended to encompass the various derivative forms of the compound described above. The pharmaceutically acceptable salts of the compounds of the present disclosure include acid addition salts and base addition salts thereof.

A review of suitable salts is found in Stahl and Wermuth, "Handbook of Pharmaceutical Salts: Properties, Selection, and Use" (Wiley-VCH, 2002). Methods for preparing the pharmaceutically acceptable salts of the compounds of the present disclosure are known to those skilled in the art.

As used herein, the term "ester" refers to an ester derived from each formula compound in the present application, including physiologically hydrolyzable esters (which can be hydrolyzed under physiological conditions to release the compounds of the present disclosure in the form of free acids or alcohols). The compounds of the present disclosure *per se* may also be esters.

The compounds of the present disclosure may be present in the form of solvates (preferably hydrates), and the compounds of the present disclosure contain a polar solvent as a structural element of the crystal lattice of the compound, particularly, for example, water, methanol, or ethanol. The amount of the polar solvent, particularly water, may be present in a stoichiometric or non-stoichiometric ratio.

Also included within the scope of the present disclosure are metabolites of the compounds of the present disclosure, i.e., substances formed *in vivo* upon administration of the compounds of the present disclosure. Such products may result, for example, from oxidation, reduction, hydrolysis, amidation, deamidation, esterification, defatting, enzymatic digestion, and the like of the administered compound. Thus, the present disclosure includes metabolites of the compounds of the present disclosure, including compounds prepared by the process of contacting the compounds of the present disclosure with a mammal for a time sufficient to produce metabolites thereof.

Further included within the scope of the present disclosure are prodrugs of the compounds of the present disclosure, which are certain derivatives of the compounds of the present disclosure that may themselves have relatively weak or no pharmacological activity and, upon administration into or onto the body, are converted to the compounds of the present disclosure having the desired activity by, for example, hydrolysis. Generally, such prodrugs will be functional group derivatives of the compounds, which are readily converted into desired therapeutically active compounds *in vivo.* Additional information on the use of prodrugs can be found in "Pro-drugs as Novel Delivery Systems", volume 14, ACS Symposium Series (T. Higuchi and V. Stella) and "Bioreversible Carriers in Drug Design", Pergamon Press, 1987 (E. B. Roche eds., American Pharmaceutical Association). The prodrugs of the present disclosure may be prepared by, for example, replacing appropriate functional groups present in the compounds of the present disclosure with certain moieties known to those skilled in the art as "pro-moieties", for example, as described in "Design of Prodrugs", H. Bundgaard (Elsevier, 1985).

The present disclosure further encompasses the compounds of the present disclosure containing a protective group. In any process of preparing the compounds of the present disclosure, it may be necessary and/or desirable to protect sensitive or reactive groups on any of the molecules concerned, thereby forming a chemically protected form of the compounds of the present disclosure. This can be achieved by conventional protective groups, as described, for example, in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, which are incorporated herein by reference. The protective groups may be removed at an appropriate subsequent stage by methods known in the art.

As used herein, the term "about" means within $\pm$ 10%, preferably within $\pm$ 5%, and more preferably within $\pm$ 2% of the stated numerical value.

<u>**Compound**</u>

In some embodiments, the present disclosure provides a compound, or a pharmaceutically acceptable salt, an ester, a stereoisomer, an atropisomer, a tautomer, a polymorph, a solvate, a metabolite, an isotopically labeled compound, or a prodrug thereof, wherein the compound has a structure of formula (I):

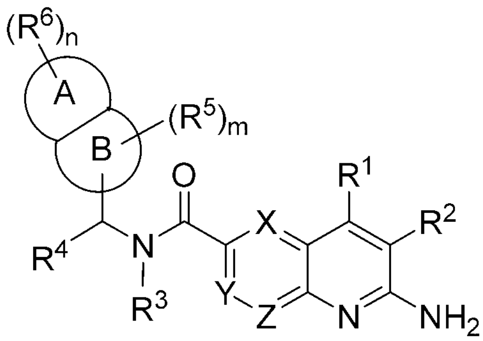

(I)

wherein:

ring A is a $C_{3-10}$ hydrocarbon ring, a 3- to 10-membered heterocyclic ring, a $C_{6-10}$ aromatic ring, or a 5- to 14-membered heteroaromatic ring;
ring B is a benzene ring;
X is $CR^X$ or N;
Y is $CR^Y$ or N;
Z is $CR^Z$ or N;

$R^X$, $R^Y$, $R^Z$, $R^1$, $R^2$, $R^3$, $R^4$, $R^5$, and $R^6$, at each occurrence, are each independently selected from H, halogen, -OH, $-NH_2$, -CN, $-NO_2$, $C_{1-6}$ alkyl, deuterated $C_{1-6}$ alkyl, $C_{2-6}$ alkenyl, $C_{2-6}$ alkynyl, $C_{3-6}$ cyclohydrocarbyl, 3-to 10-membered heterocyclyl, $C_{6-10}$ aryl, 5- to 14-membered heteroaryl, $C_{6-12}$ aralkyl, $-C(=O)R^a$, $-OC(=O)R^a$, $-C(=O)OR^a$, $-OR^a$, $-SR^a$, $-S(=O)R^a$, $-S(=O)_2R^a$, $-S(=O)_2NR^aR^b$, $-NR^aR^b$, $-C(=O)NR^aR^b$, $-NR^a-C(=O)R^b$, $-NR^a-C(=O)OR^b$, $-NR^a-S(=O)_2-R^b$, $-NR^a-C(=O)-NR^aR^b$, $-C_{1-6}$ alkylene-$OR^a$, $-C_{1-6}$ alkylene-$NR^aR^b$, and $-O-C_{1-6}$ alkylene-$NR^aR^b$; or $R^1$ and $R^2$, together with the groups to which they are attached, optionally form a $C_{3-10}$ hydrocarbon ring, a 3- to 10-membered heterocyclic ring, a $C_{6-10}$ aromatic ring, or a 5- to 14-membered heteroaromatic ring; and/or, $R^3$ and $R^4$, together with the groups to which they are attached, optionally form a 3- to 10-membered heterocyclic ring;

$R^a$ and $R^b$, at each occurrence, are each independently selected from H, $C_{1-6}$ alkyl, $C_{3-10}$ cyclohydrocarbyl, 3-to 10-membered heterocyclyl, $C_{6-10}$ aryl, 5- to 14-membered heteroaryl, and $C_{6-12}$ aralkyl;

the above alkyl, alkylene, alkenyl, alkynyl, cyclohydrocarbyl, hydrocarbon ring, heterocyclyl, heterocyclic ring, aryl, aromatic ring, heteroaryl, heteroaromatic ring, and aralkyl, at each occurrence, are each optionally substituted with one or more substituents independently selected from the following: halogen, -OH, =O, $-NH_2$, -CN, $-NO_2$, $C_{1-6}$ alkyl, $C_{3-6}$ cyclohydrocarbyl, 3- to 10-membered heterocyclyl, $C_{6-10}$ aryl, 5- to 14-membered heteroaryl, $C_{6-12}$ aralkyl, $-C(=O)R^c$, $-OC(=O)R^c$, $-C(=O)OR^c$, $-OR^c$, $-SR^c$, $-S(=O)R^c$, $-S(=O)_2R^c$, $-S(=O)_2NR^cR^d$, $-NR^cR^d$, $-C(=O)NR^cR^d$, $-NR^c-C(=O)R^d$, $-NR^c-C(=O)OR^d$, $-NR^c-S(=O)_2-R^d$, $-NR^c-C(=O)-NR^cR^d$, $-C_{1-6}$ alkylene-$OR^c$, $-C_{1-6}$ alkylene-$NR^cR^d$, and $-O-C_{1-6}$ alkylene-$NR^cR^d$; the alkyl, cyclohydrocarbyl, heterocyclyl, aryl, heteroaryl, and aralkyl are further optionally substituted with one or more substituents independently selected from the following: halogen, -OH, =O, -C(=O)O-tert-butyl, $-NH_2$, -CN, $-NO_2$, $C_{1-6}$ alkyl, halogenated $C_{1-6}$ alkyl, $C_{3-6}$ cyclohydrocarbyl, 3- to 10-membered heterocyclyl, $C_{6-10}$ aryl, 5- to 14-membered heteroaryl, $C_{6-12}$ aralkyl, $-O-C_{1-6}$ alkyl, and $-C_{1-6}$ alkylene-$O-C_{1-6}$ alkyl;

$R^c$ and $R^d$, at each occurrence, are each independently selected from: H, $C_{1-6}$ alkyl, $C_{3-10}$ cyclohydrocarbyl, 3- to 10-membered heterocyclyl, $C_{6-10}$ aryl, 5- to 14-membered heteroaryl, and $C_{6-12}$ aralkyl; the alkyl, cyclohydrocarbyl, heterocyclyl, aryl, heteroaryl, and aralkyl are further optionally substituted with one or more substituents independently selected from the following: halogen, -OH, =O, -C(=O)O-tert-butyl, $-NH_2$, -CN, $-NO_2$, $C_{1-6}$ alkyl, halogenated $C_{1-6}$ alkyl, $C_{3-6}$ cyclohydrocarbyl, 3- to 10-membered heterocyclyl, $C_{6-10}$ aryl, 5- to 14-membered heteroaryl, $C_{6-12}$ aralkyl, and $-C_{1-6}$ alkylene-$O-C_{1-6}$ alkyl; and

m and n are each independently an integer selected from 1, 2, 3, or 4, preferably an integer selected from 1, 2, or 3.

In some embodiments, the present disclosure provides a compound, or a pharmaceutically acceptable salt, an ester, a stereoisomer, an atropisomer, a tautomer, a polymorph, a solvate, a metabolite, an isotopically labeled compound, or a prodrug thereof, wherein the compound has the following structures:

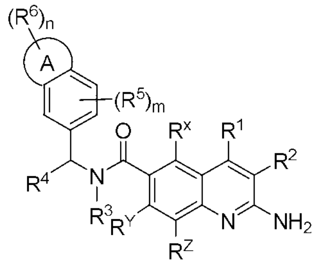
(II)

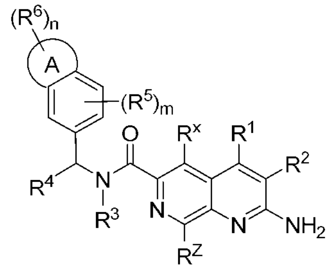
(II)'

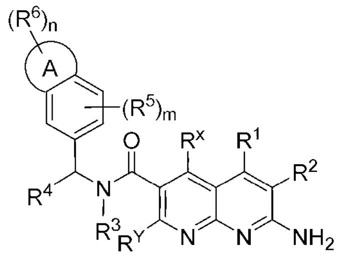
(II)''

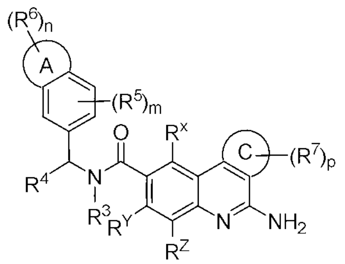
(III)

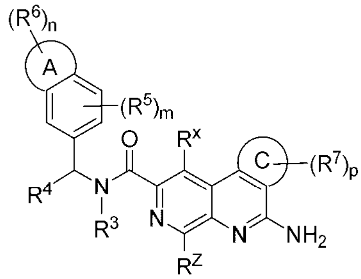
(III)'

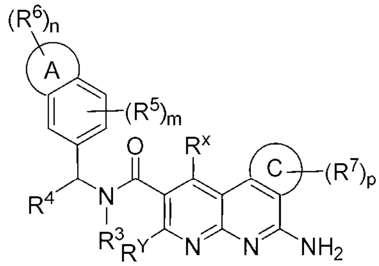
(III)''

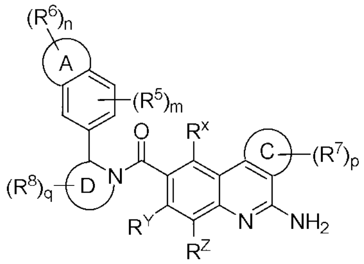

(IV)

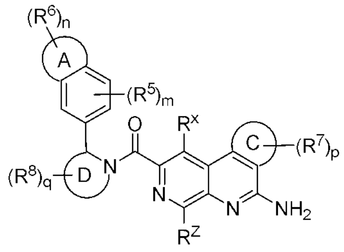

(IV)’

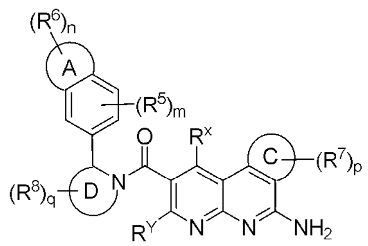

(IV)”

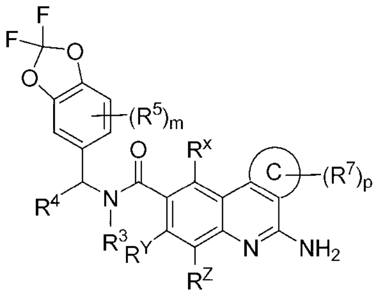

(V)

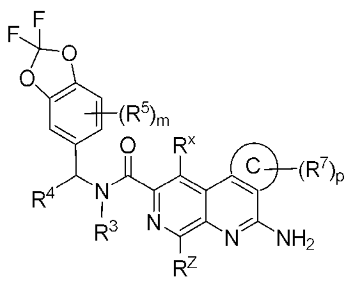

(V)’

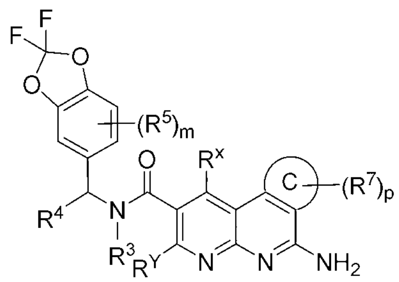

(V)”

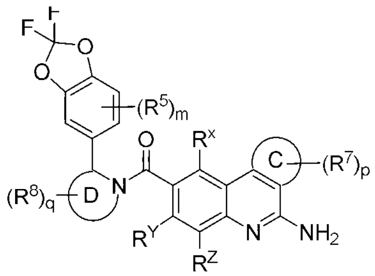

(VI)

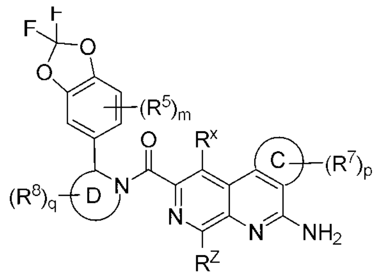

(VI)’

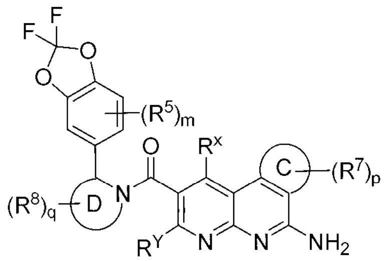

(VI)”

wherein:

ring C is selected from a $C_{5-6}$ hydrocarbon ring, a 5- to 6-membered heterocyclic ring, and a 5- to 6-membered heteroaromatic ring;

ring D is a 3- to 10-membered heterocyclic ring;

$R^7$ and $R^8$, at each occurrence, are each independently selected from H, $C_{1-6}$ alkyl, and $C_{3-6}$ cyclohydrocarbyl; preferably, $R^7$ and $R^8$, at each occurrence, are each independently selected from H and $C_{1-6}$ alkyl;

p and q are each independently an integer selected from 1 or 2; and

the other groups are as defined herein.

In some embodiments, in the general formulas as disclosed herein, $R^X$, $R^Y$, and $R^Z$ are each independently selected from H, halogen, -CN, $C_{1-6}$ alkyl, halogenated $C_{1-6}$ alkyl, and -O-($C_{1-6}$ alkyl).

In a preferred embodiment, $R^X$, $R^Y$, and $R^Z$ are each independently selected from H, F, Cl, -CN, methyl, trifluoromethyl, and methoxy.

In some embodiments, in the general formulas as disclosed herein,

X is CH or C-$CH_3$;

Y is $CR^Y$ or N, wherein $R^Y$ is selected from H, halogen, -CN, $C_{1-6}$ alkyl, halogenated $C_{1-6}$ alkyl, and -O-($C_{1-6}$ alkyl), preferably selected from H, F, Cl, -CN, methyl, trifluoromethyl, and methoxy; and

Z is CH or N.

In some embodiments, in the general formulas as disclosed herein, $R^1$ and $R^2$ are each independently selected from H, halogen, $C_{1-6}$ alkyl, and deuterated $C_{1-6}$ alkyl; preferably, $R^1$ and $R^2$ are each independently selected from H, Cl, Br, I, methyl, -$CD_3$, and ethyl.

In some embodiments, in the general formulas as disclosed herein, $R^1$ and $R^2$, together with the groups to which they are attached, optionally form a $C_{5-6}$ hydrocarbon ring, a 5- to 6-membered heterocyclic ring, or a 5- to 6-membered heteroaromatic ring; preferably, $R^1$ and $R^2$, together with the groups to which they are attached, optionally form

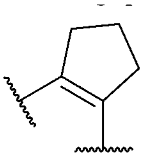 , 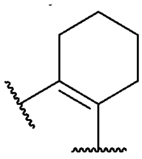 , 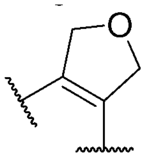 , 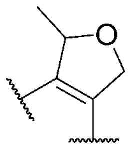 , 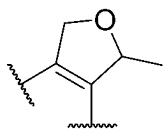 ,

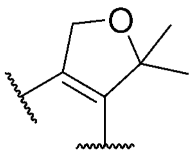 , 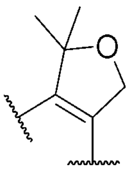 , 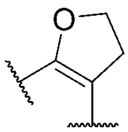 , 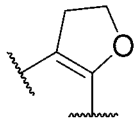 , 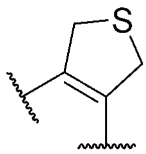 , 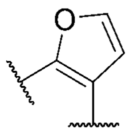 , 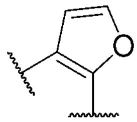 ,

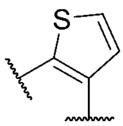 , 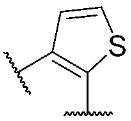 , 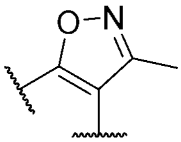 , 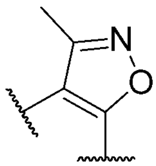 , 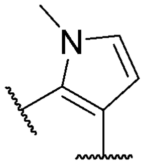 , 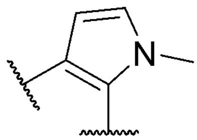 , 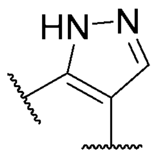 ,

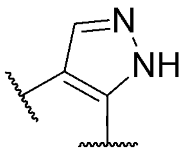 , 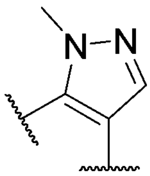 , 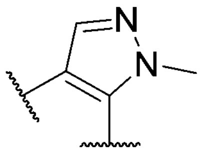 , 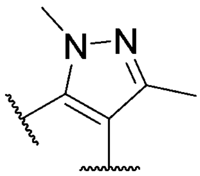 , 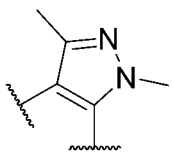 , 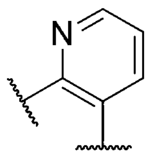 ,

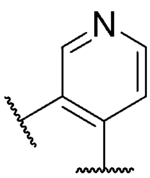 , 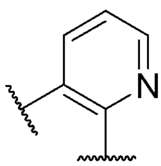 , 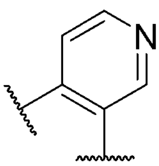 , 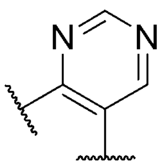 , or 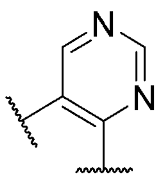 .

In some embodiments, in the general formulas as disclosed herein,

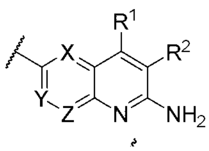

is selected from

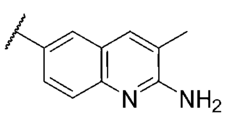 , 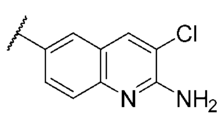 , 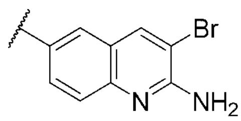 , 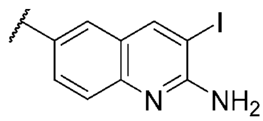 ,

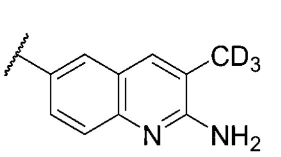, 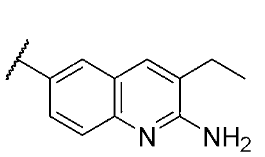, 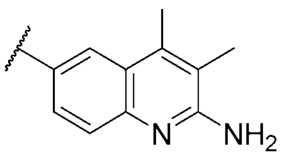, 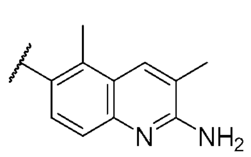,

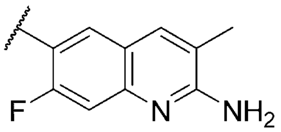, 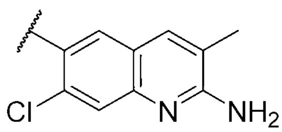, 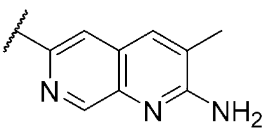, 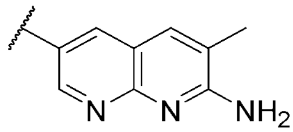,

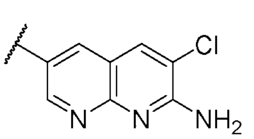, 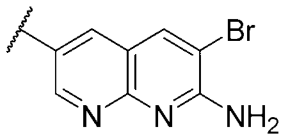, 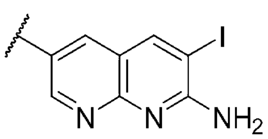, 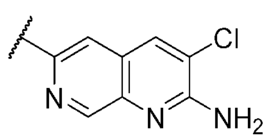,

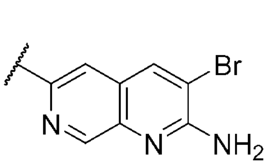, 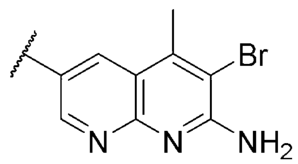, 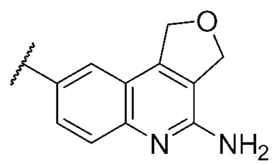, 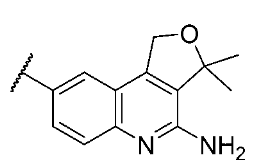,

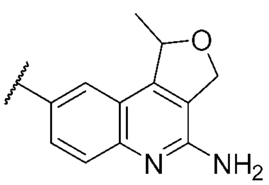, 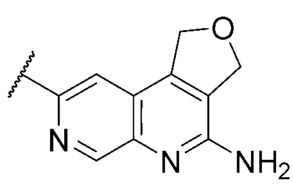, 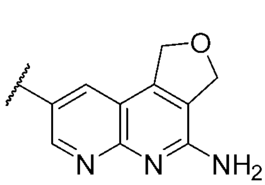, 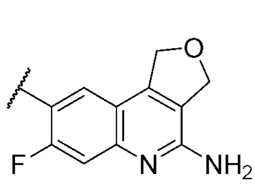,

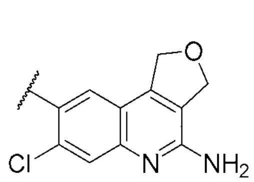, 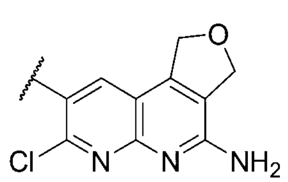, 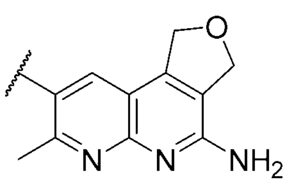, 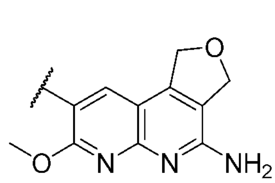,

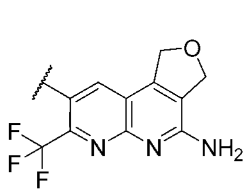, 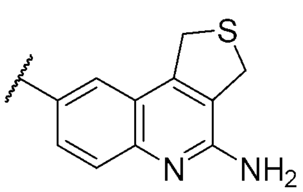, 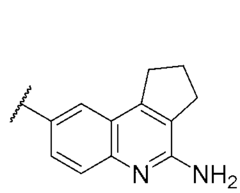, 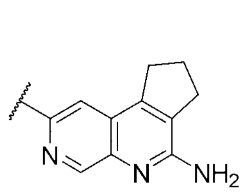,

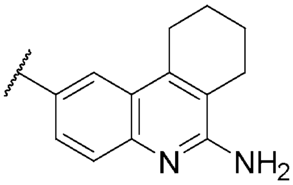, 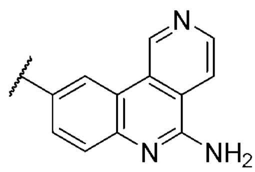, 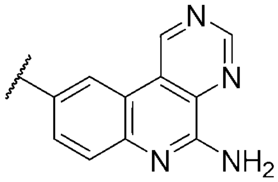, 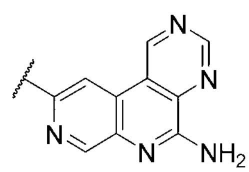,

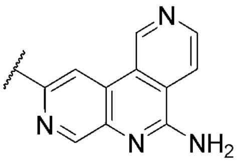, 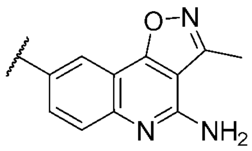, 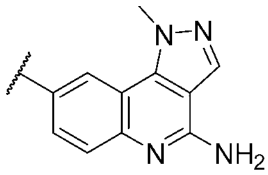, 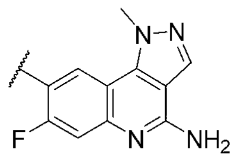,

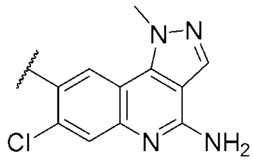, 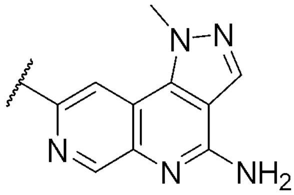, 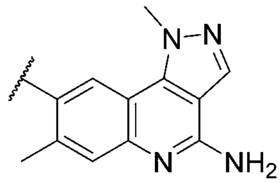, 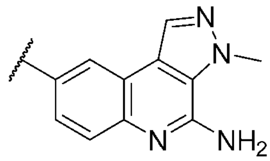,

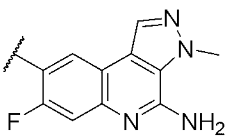, 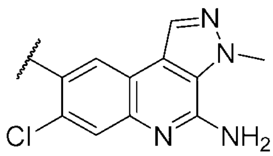, 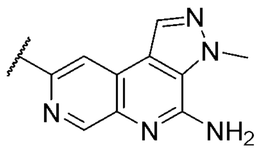, 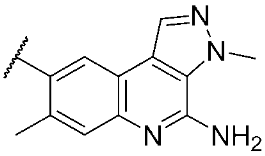,

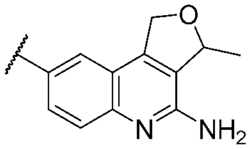, 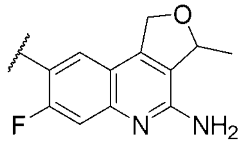, 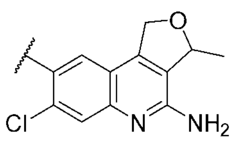, 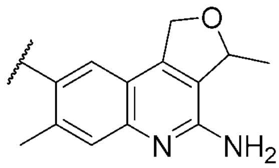,

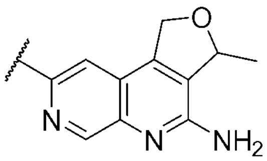, 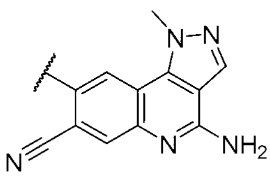, 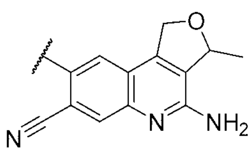, 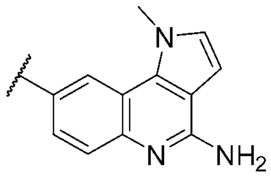,

and

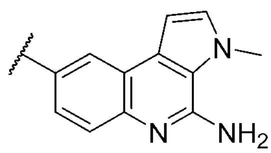.

In some embodiments, in the general formulas as disclosed herein, ring A is a 5- to 6-membered heterocyclic ring or a 5- to 6-membered heteroaromatic ring.

In some embodiments, in the general formulas as disclosed herein, ring A is an oxygen-containing 5-membered heterocyclic ring (e.g., a 5-membered heterocyclic ring containing one or two oxygen atoms).

In some embodiments, ring A is

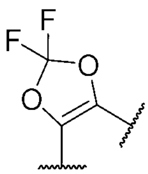, 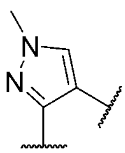, or 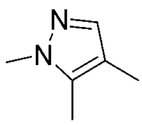;

and in some embodiments,

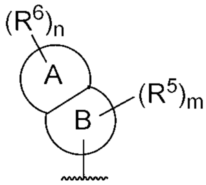

is

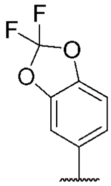, 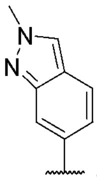, 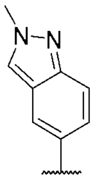, 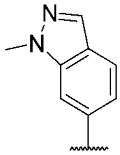, or 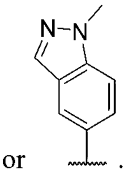.

In some embodiments, in the general formulas as disclosed herein, $R^3$ and $R^4$ are each independently selected from H, $C_{1-6}$ alkyl, $C_{3-6}$ cyclohydrocarbyl, 3- to 10-membered heterocyclyl, -$C_{1-6}$ alkylene-OH, and -$C_{1-6}$ alkylene-O-$C_{1-6}$ alkyl; the alkylene, alkyl, cyclohydrocarbyl, and heterocyclyl are optionally further substituted with $C_{3-6}$ cyclohydrocarbyl, 3- to 10-membered heterocyclyl, or 5- to 6-membered heteroaryl; the cyclohydrocarbyl, heterocyclyl, or heteroaryl is further optionally substituted with one or more $C_{1-6}$ alkyl groups.

In a preferred embodiment, $R^3$ and $R^4$ are each independently selected from H, methyl, ethyl, isopropyl, cyclopropyl,

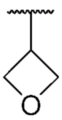, 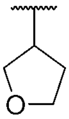, 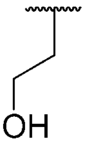, 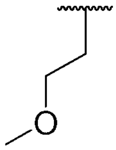, 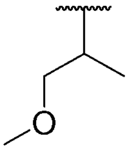, 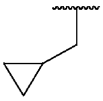, 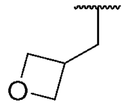, 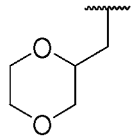, 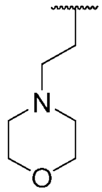,

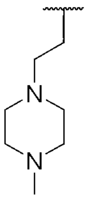, 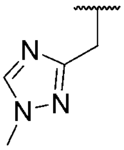, 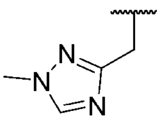, 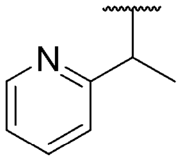, 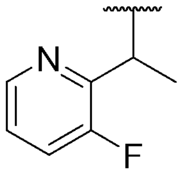, 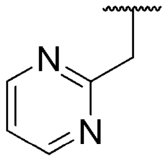, and 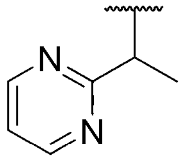.

In some embodiments, in the general formulas as disclosed herein, $R^3$ and $R^4$, together with the groups to which they are attached, optionally form a group selected from the following:

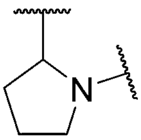, 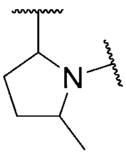, 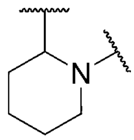, 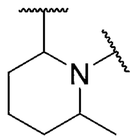, 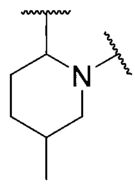, 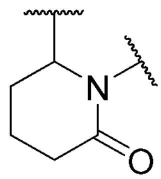, 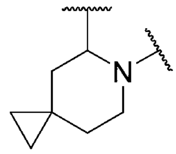,

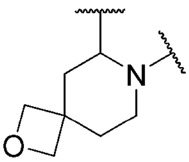, 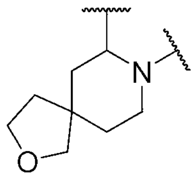, 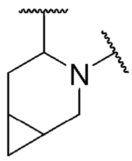, 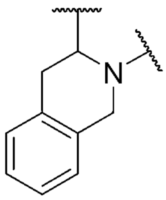, 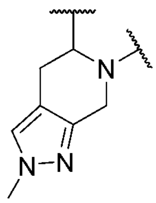, 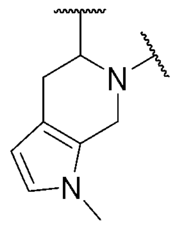,

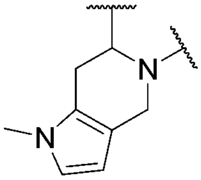, 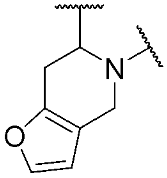, 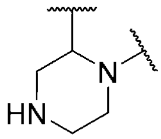, 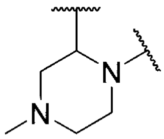, 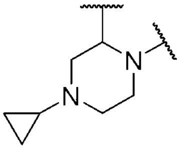, 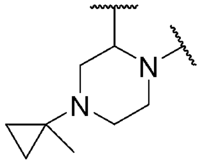,

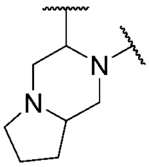, 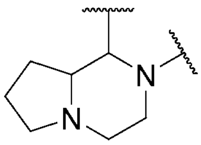, 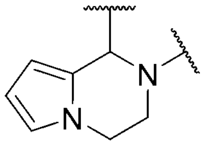, 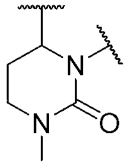, 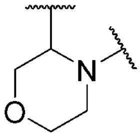, 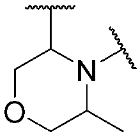, 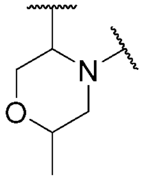,

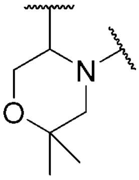, 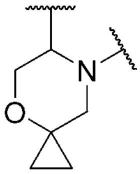, 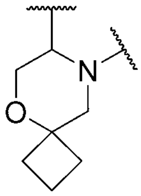, 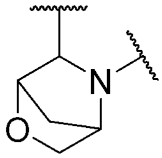, 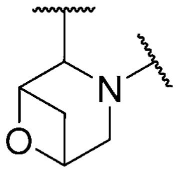, and 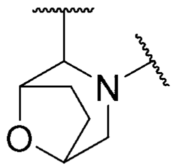.

In some embodiments, in the general formulas as disclosed herein, $R^3$ and $R^4$, together with the groups to which they are attached, optionally form a group selected from the following:

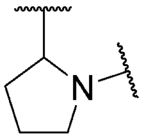, 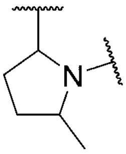, 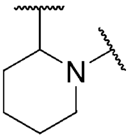, 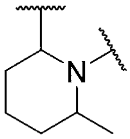, 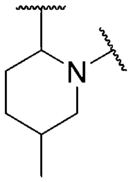, 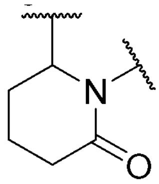, 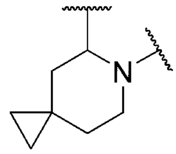,

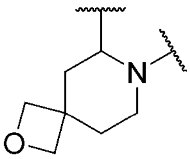, 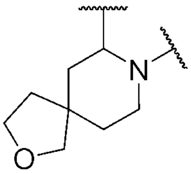, 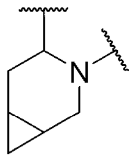, 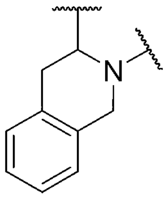, 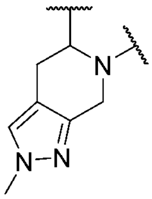, 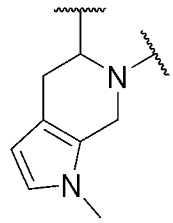,

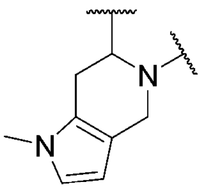, 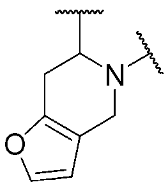, 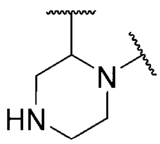, 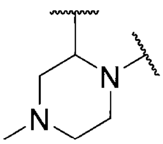, 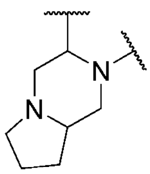, 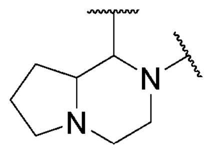,

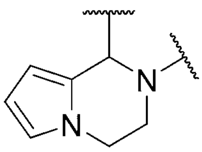, 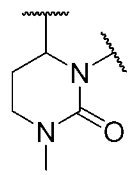, , , , , ,

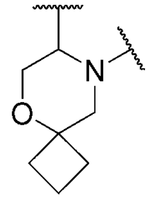, 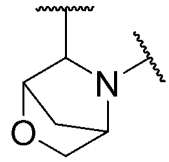, 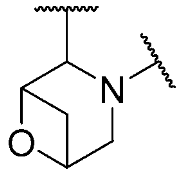, and 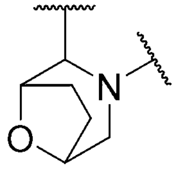.

In some embodiments, in the general formulas as disclosed herein,

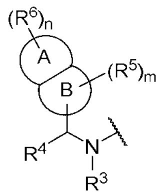

is selected from

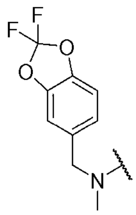, 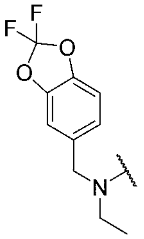, 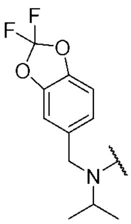, 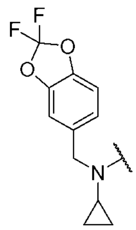, 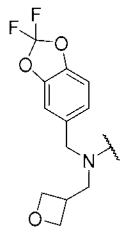, 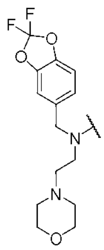, 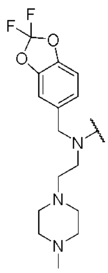, 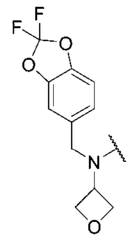,

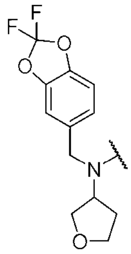, 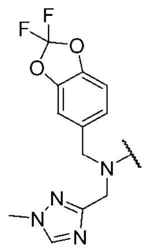, 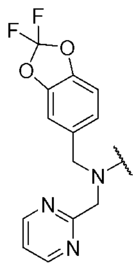, 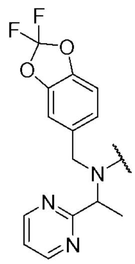, 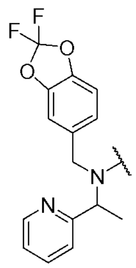, 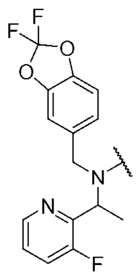, 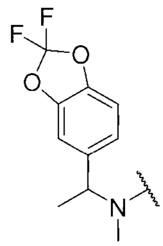, 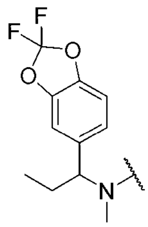,

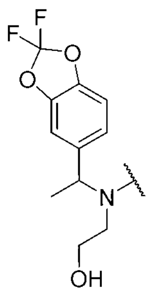, 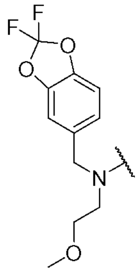, 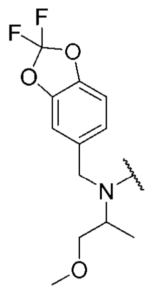, 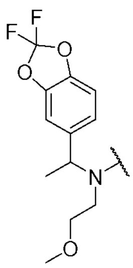, 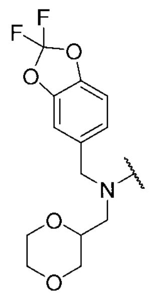, 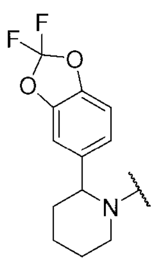, 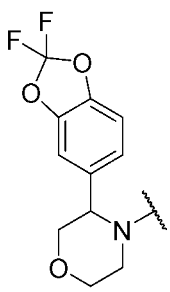,

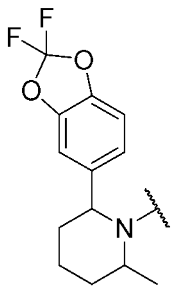, 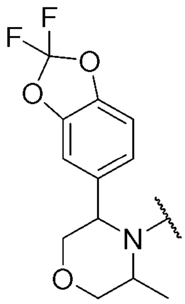, 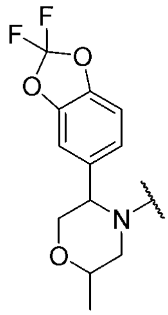, 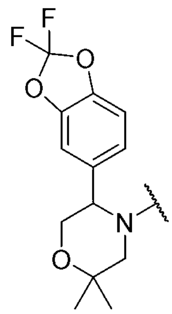, 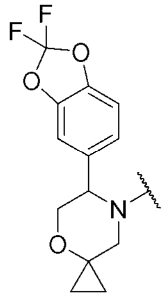, 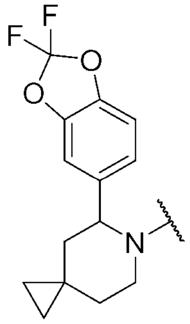,

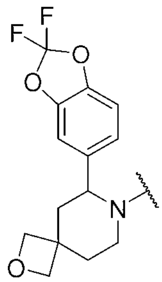, 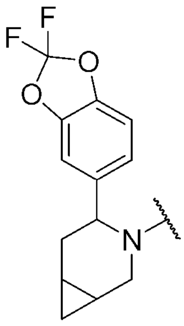, 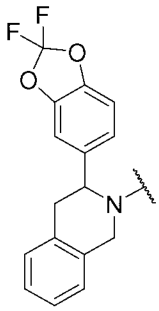, 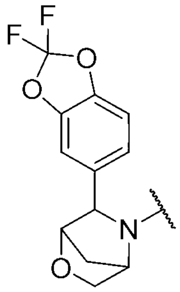, 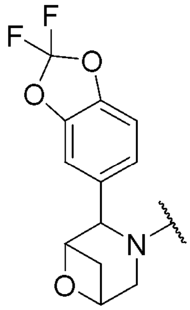, 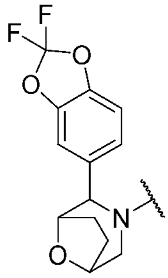,

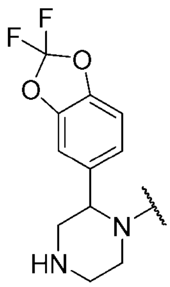, 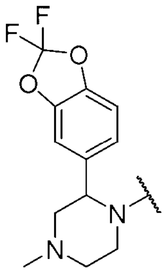, 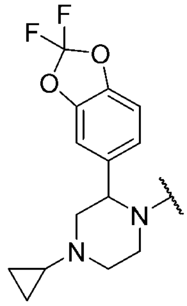, 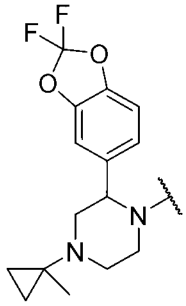, 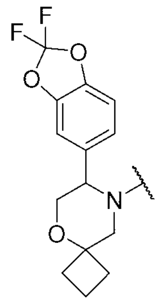, 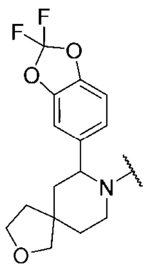,

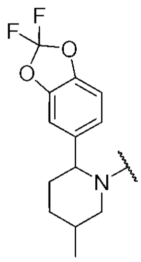, 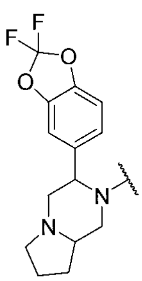, 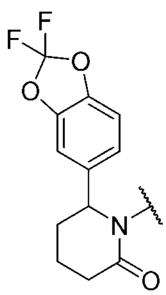, 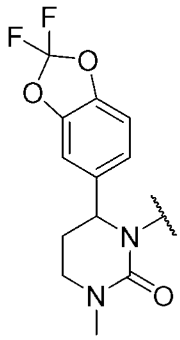, 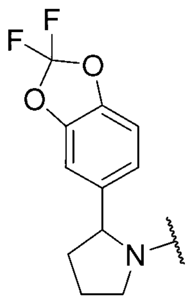, 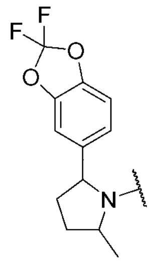, 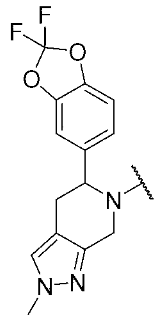,

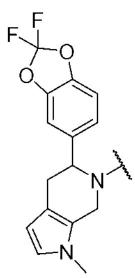, 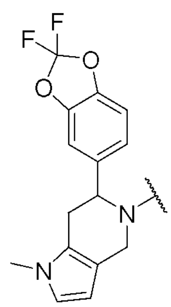, 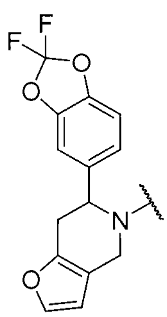, 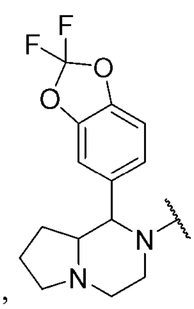, 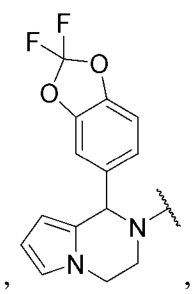, 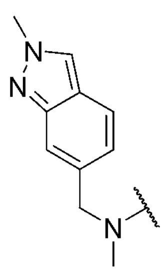, 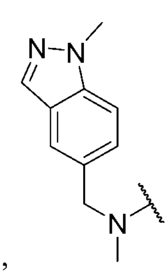,

and

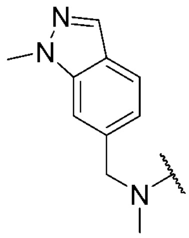

.

In some embodiments, in the general formulas as disclosed herein,

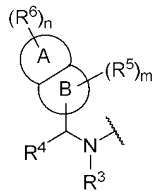

is selected from

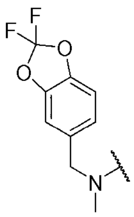 , 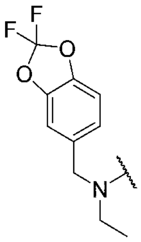 , 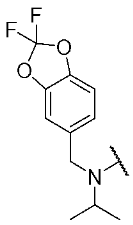 , 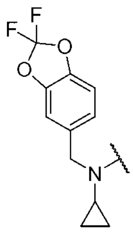 , 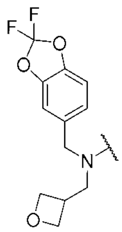 , 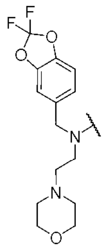 , 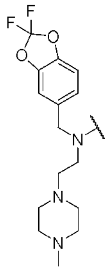 , 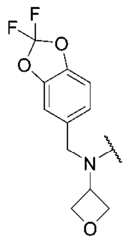 ,

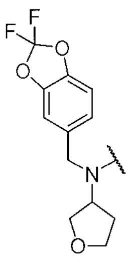 , 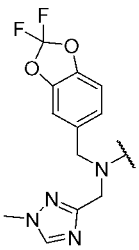 , 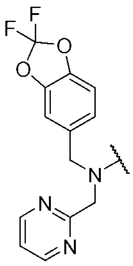 , 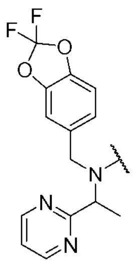 , 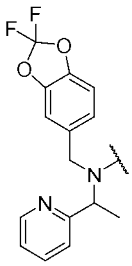 , 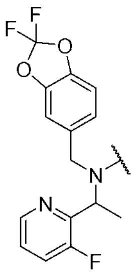 , 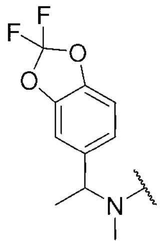 , 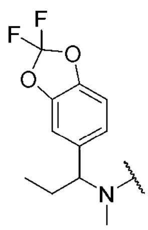 ,

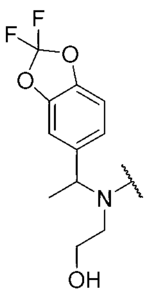 , 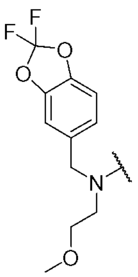 , 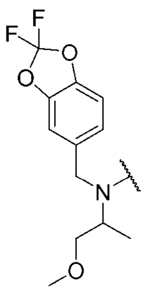 , 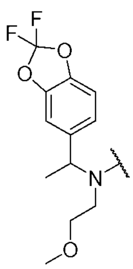 , 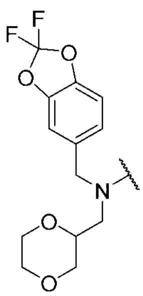 , 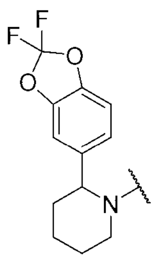 , 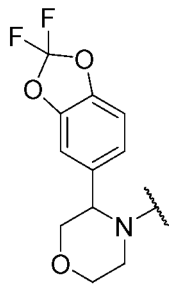 ,

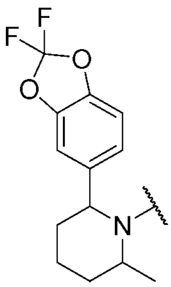, 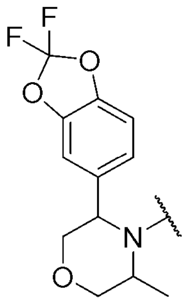, 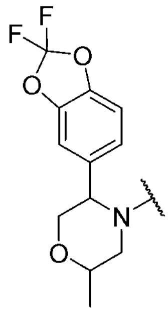, 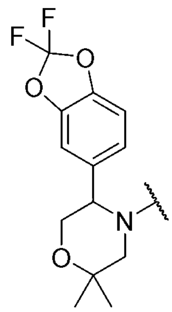, 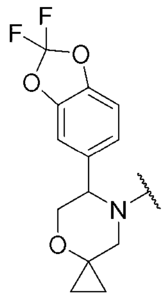, 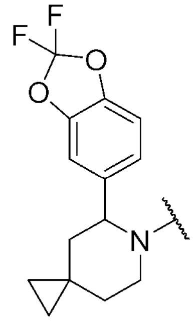,

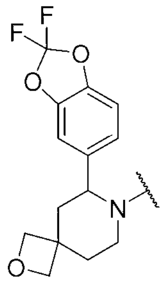, 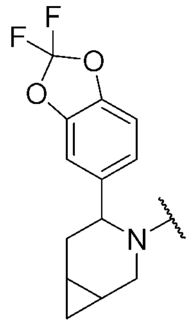, 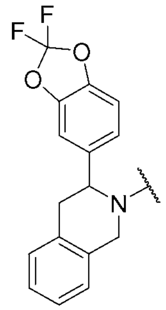, 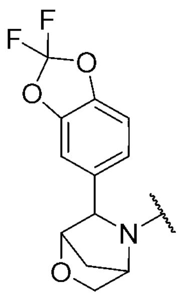, 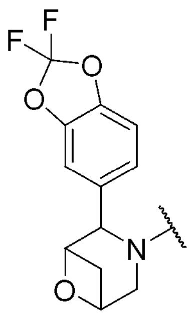, 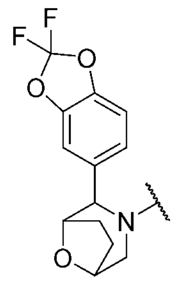,

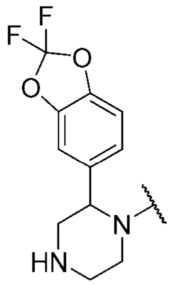, 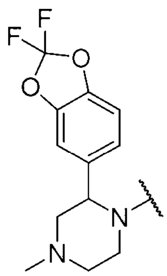, 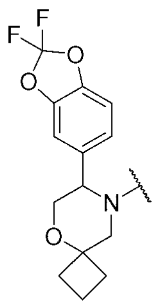, 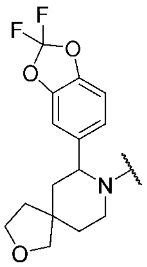, 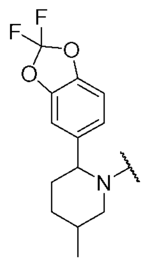, 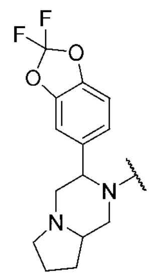, 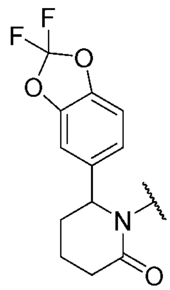,

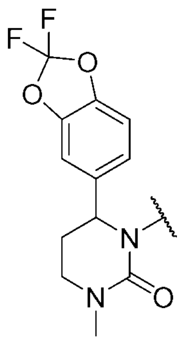, 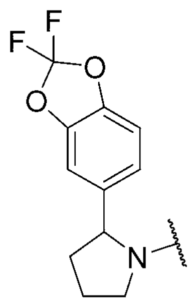, 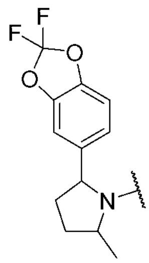, 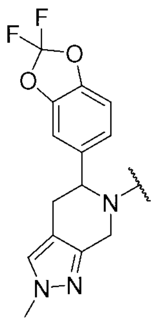, 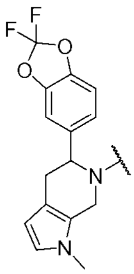, 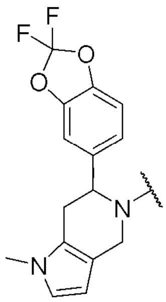, 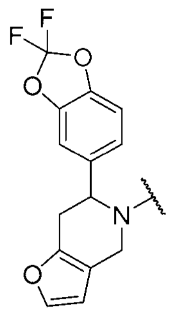,

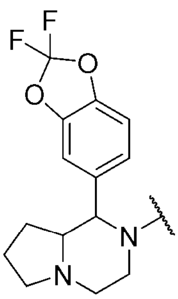, 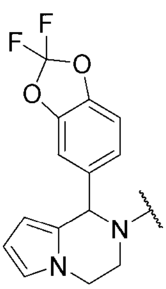, 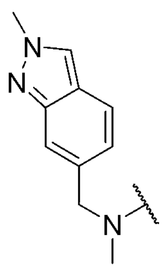, 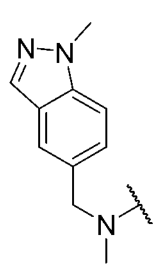, and 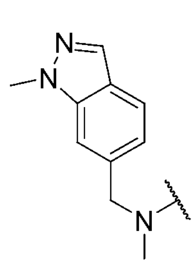.

In some embodiments, the present disclosure provides a compound, or a pharmaceutically acceptable salt, an ester, a stereoisomer, an atropisomer, a tautomer, a polymorph, a solvate, a metabolite, an isotopically labeled compound, or a prodrug thereof, wherein the compound is selected from:

| | | | | | |
|---|---|---|---|---|---|
| **I-1** | 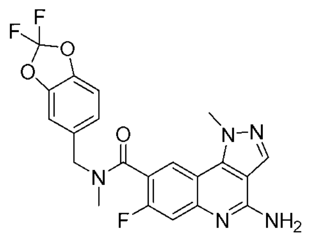 | **I-2** | 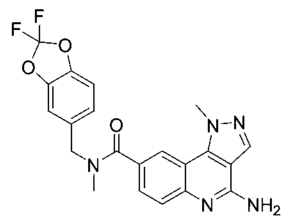 | **I-3** | 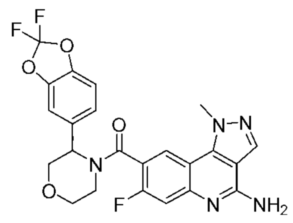 |
| **I-4** | 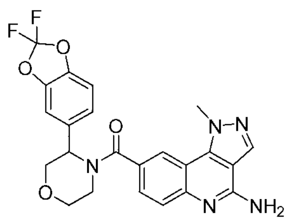 | **I-5** | 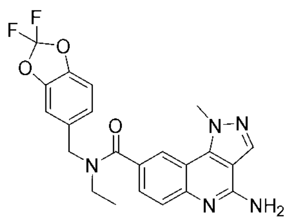 | **I-6** | 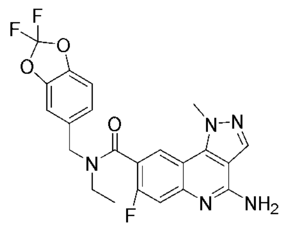 |
| **I-7** | 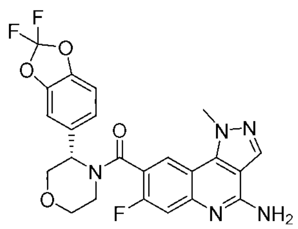 | **I-8** | 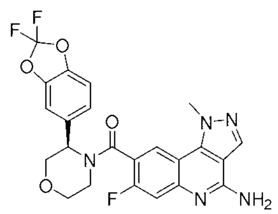 | **I-9** | 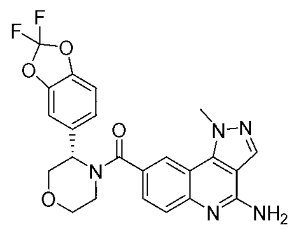 |
| **I-10** | 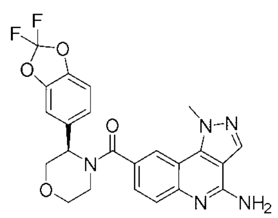 | **I-11** | 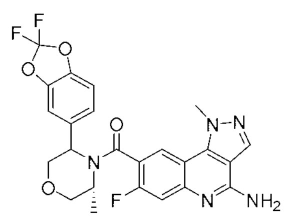 | **I-12** | 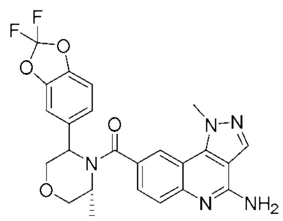 |
| **I-13** | 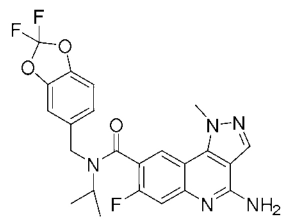 | **I-14** | 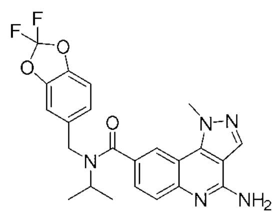 | **I-15** | 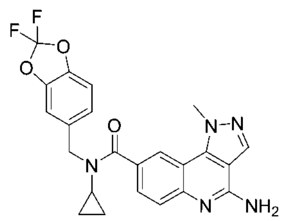 |
| **I-16** | 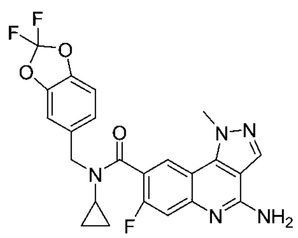 | **I-17** | 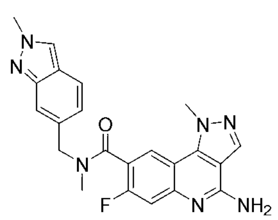 | **I-18** | 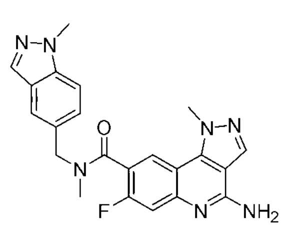 |

(continued)

| | | | | | |
|---|---|---|---|---|---|
| **I-19** | 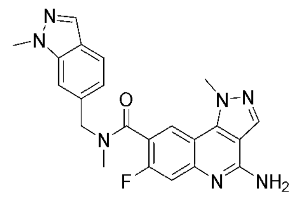 | **I-20** | 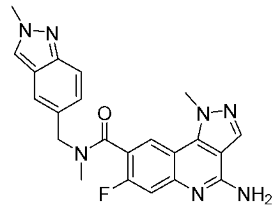 | **I-21** | 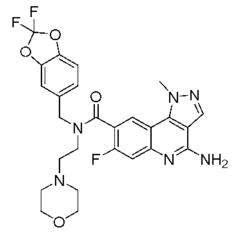 |
| **I-22** | 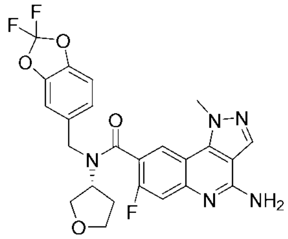 | **I-23** | 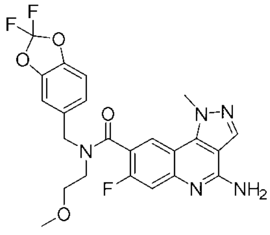 | **I-24** | 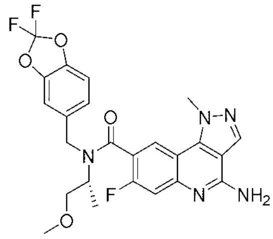 |
| **I-25** | 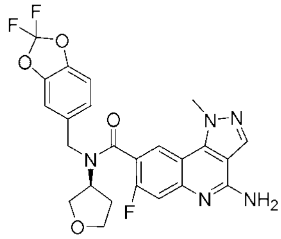 | **I-26** | 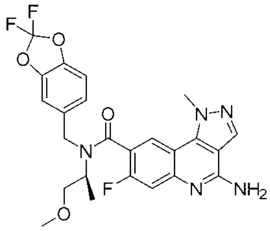 | **I-27** | 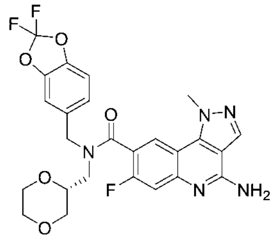 |
| **I-28** | 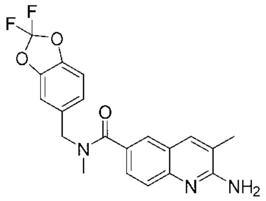 | **I-29** | 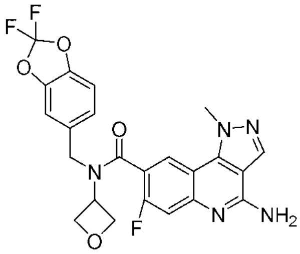 | **I-30** | 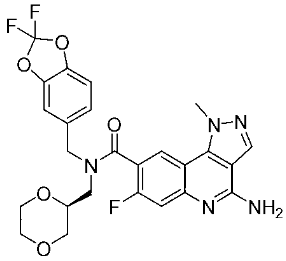 |
| **I-31** | 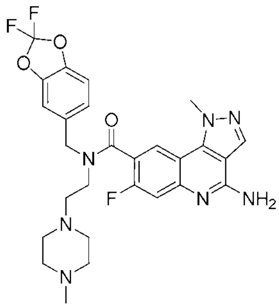 | **I-32** | 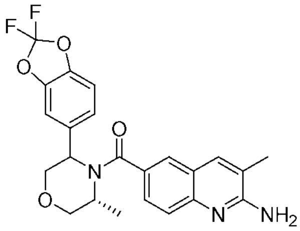 | **I-33** | 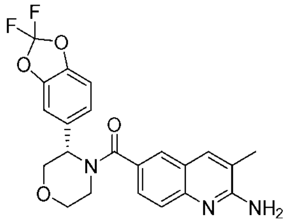 |
| **I-34** | 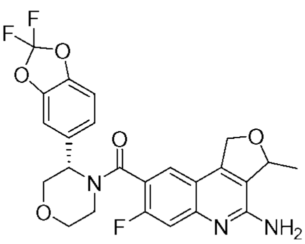 | **I-35** | 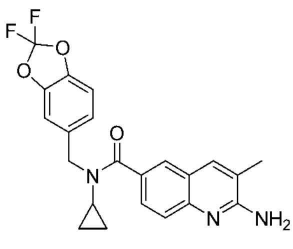 | **I-36** | 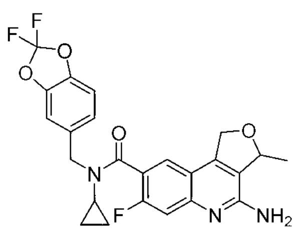 |

(continued)

| | | | | | |
|---|---|---|---|---|---|
| **I-37** | 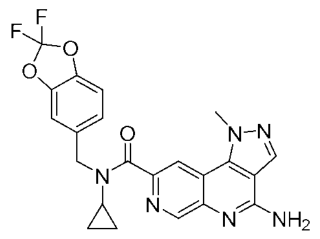 | **I-38** | 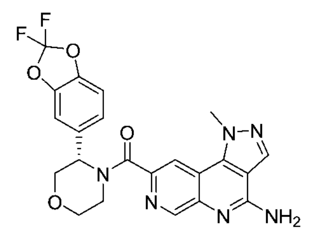 | **I-39** | 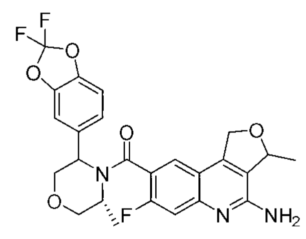 |
| **I-40** | 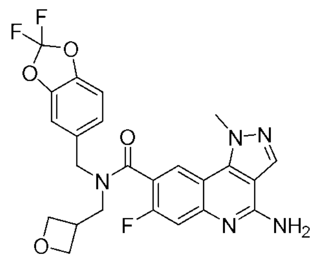 | **I-41** | 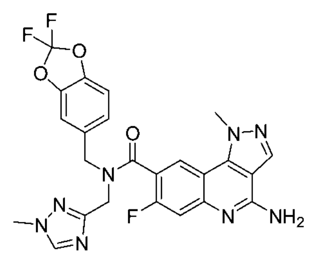 | **I-42** | 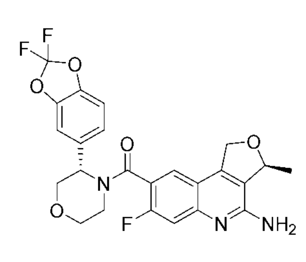 |
| **I-43** | 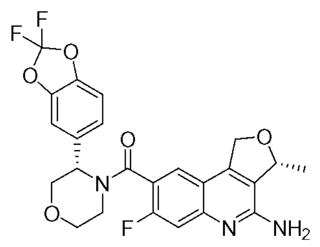 | **I-44** | 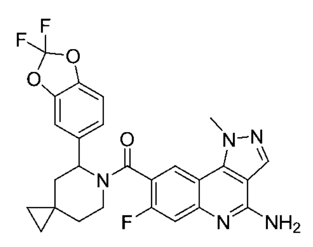 | **I-45** | 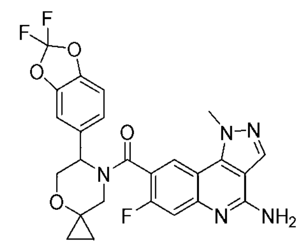 |
| **I-46** | 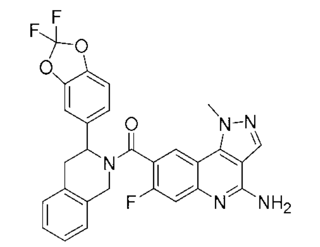 | **I-47** | 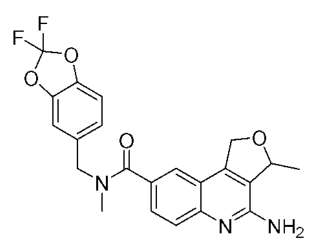 | **I-48** | 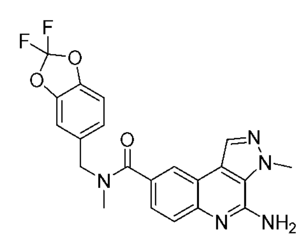 |
| **I-49** | 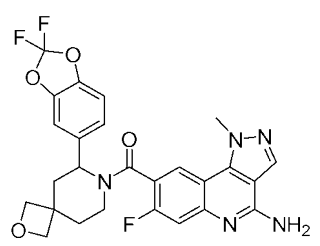 | **I-50** | 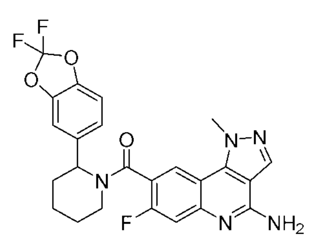 | **I-51** | 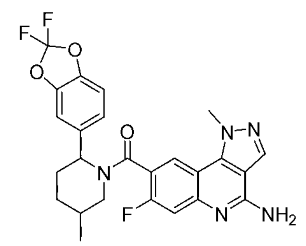 |
| **I-52** | 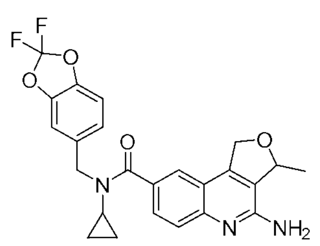 | **I-53** | 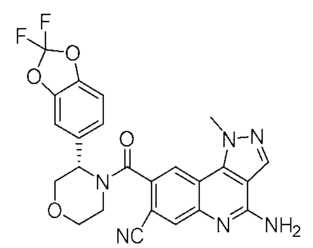 | **I-54** | 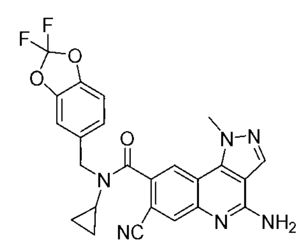 |

(continued)

| | | | | | |
|---|---|---|---|---|---|
| **I-55** | 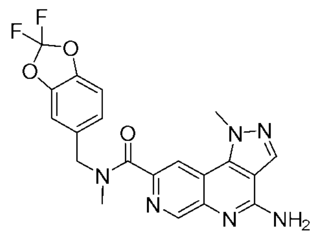 | **I-56** | 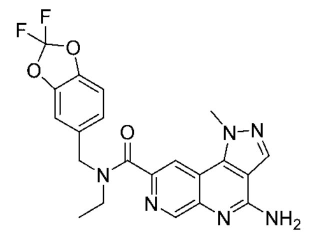 | **I-57** | 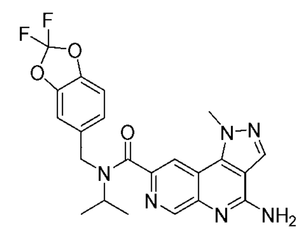 |
| **I-58** | 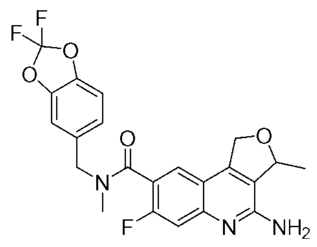 | **I-59** | 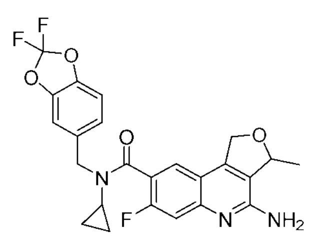 | **I-60** | 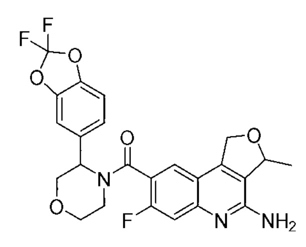 |
| **I-61** | 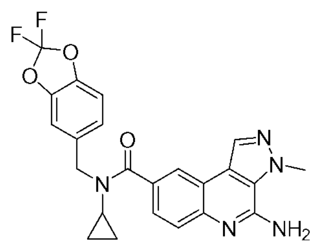 | **I-62** | 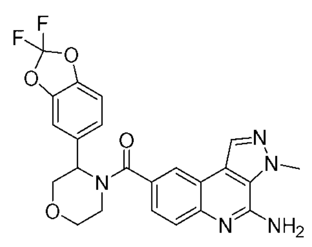 | **I-63** | 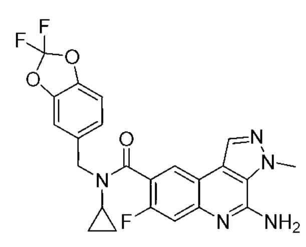 |
| **I-64** | 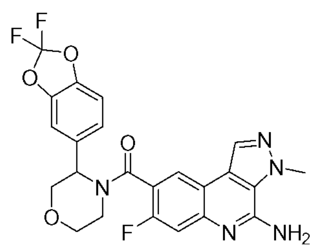 | **I-65** | 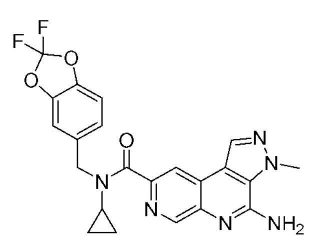 | **I-66** | 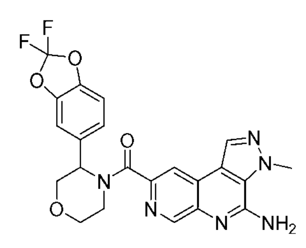 |
| **I-67** | 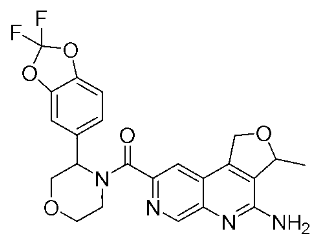 | **I-68** | 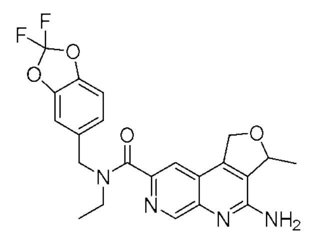 | **I-69** | 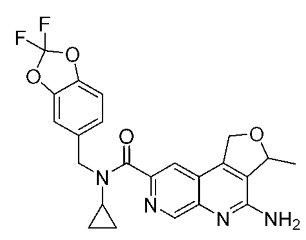 |
| **I-70** | 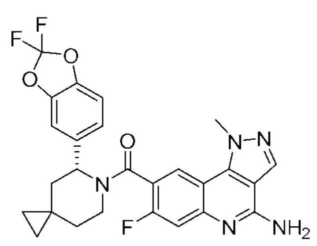 | **I-71** | 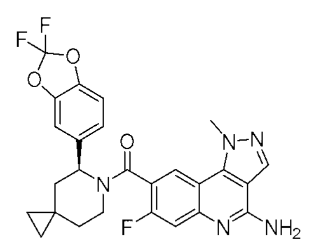 | **I-72** | 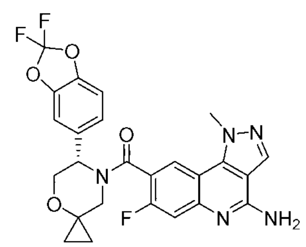 |

(continued)

| | | | | | |
|---|---|---|---|---|---|
| **I-73** | 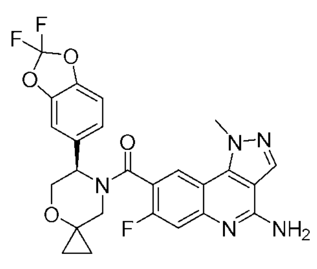 | **I-74** | 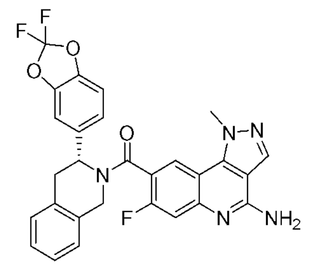 | **I-75** | 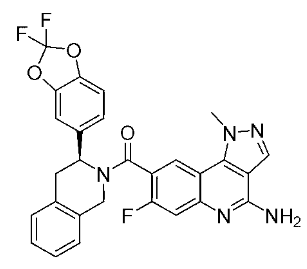 |
| **I-76** | 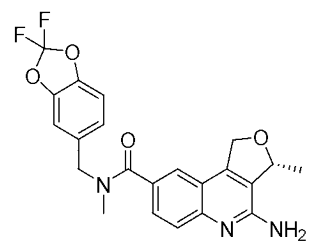 | **I-77** | 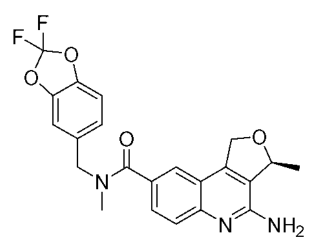 | **I-78** | 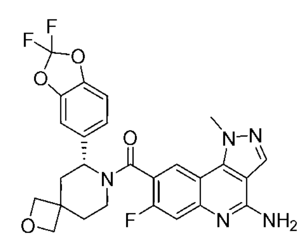 |
| **I-79** | 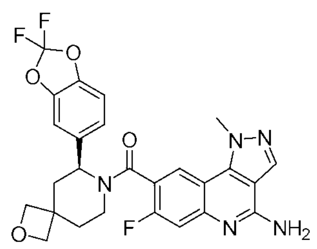 | **I-80** | 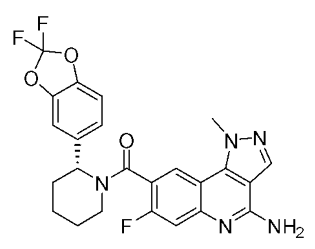 | **I-81** | 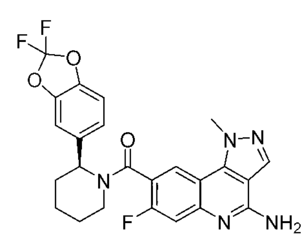 |
| **I-82** | 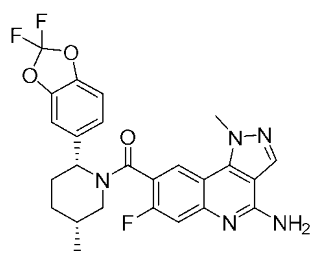 | **I-83** | 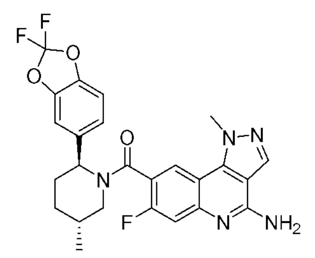 | **I-84** | 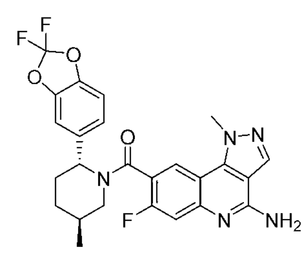 |
| **I-85** | 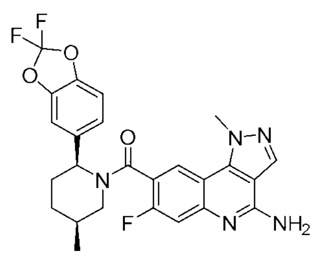 | **I-86** | 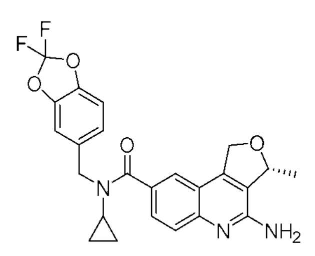 | **I-87** | 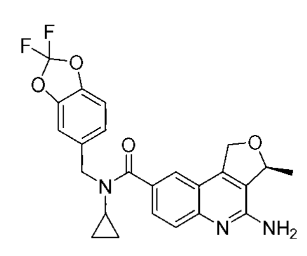 |
| **I-88** | 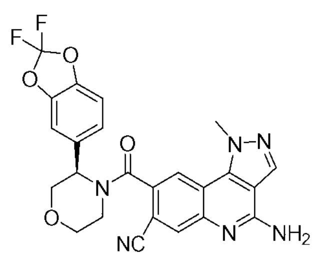 | **I-89** | 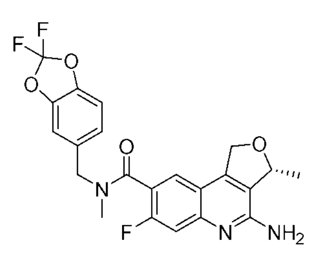 | **I-90** | 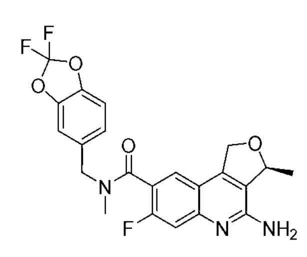 |

(continued)

| | | | | | |
|---|---|---|---|---|---|
| **I-91** | 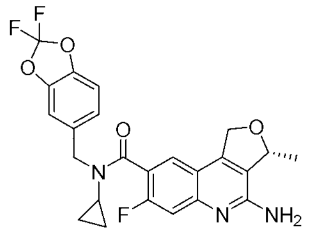 | **I-92** | 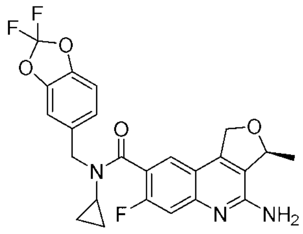 | **I-93** | 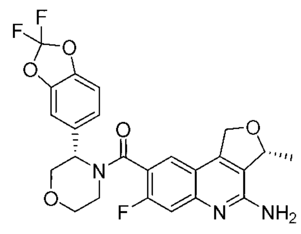 |
| **I-94** | 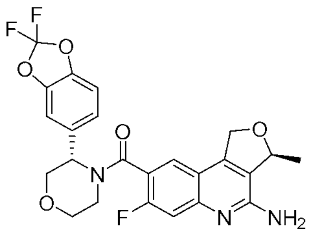 | **I-95** | 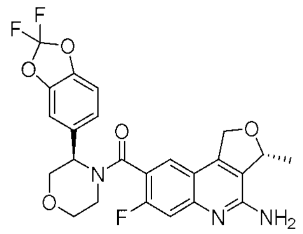 | **I-96** | 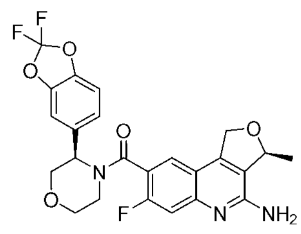 |
| **I-97** | 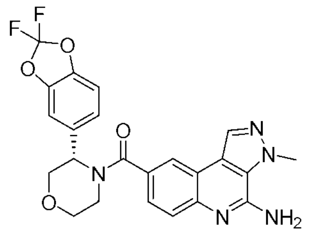 | **I-98** | 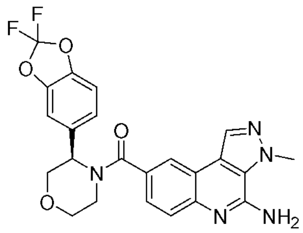 | **I-99** | 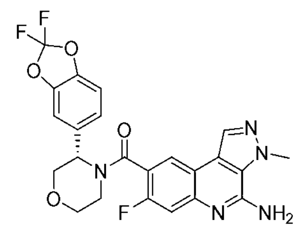 |
| **I-100** | 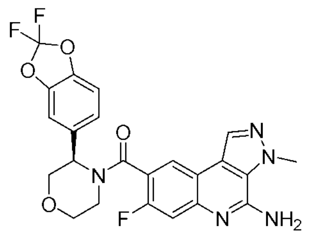 | **I-101** | 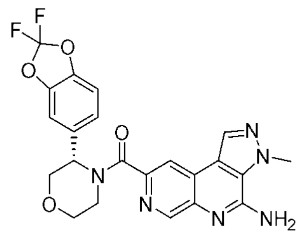 | **I-102** | 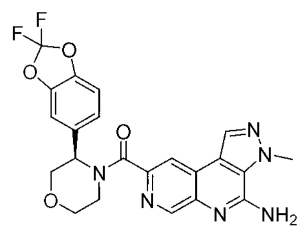 |
| **I-103** | 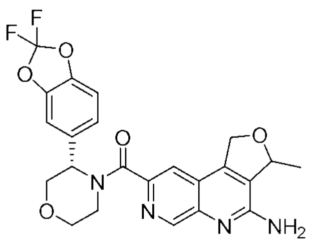 | **I-104** | 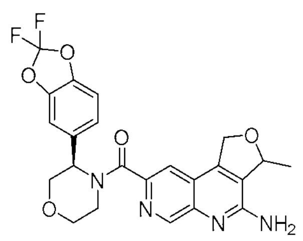 | **I-105** | 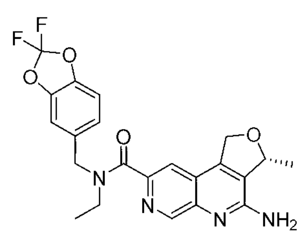 |
| **I-106** | 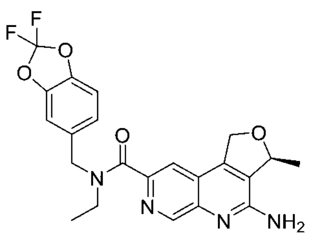 | **I-107** | 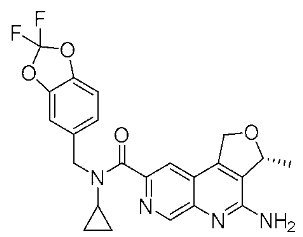 | **I-108** | 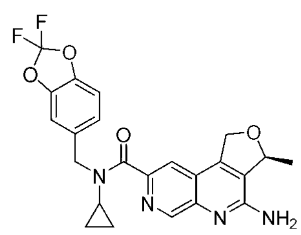 |
| **I-109** | 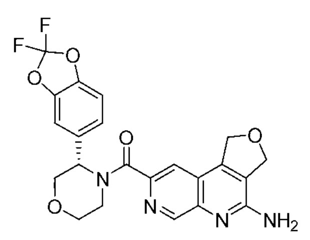 | **I-110** | 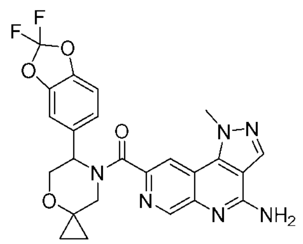 | **I-111** | 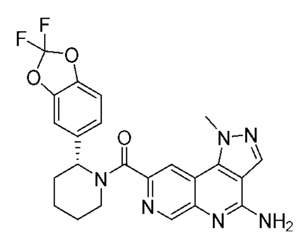 |

(continued)

| | | | | | |
|---|---|---|---|---|---|
| **I-112** | 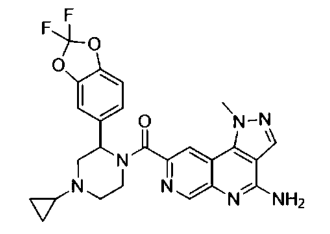 | **I-113** | 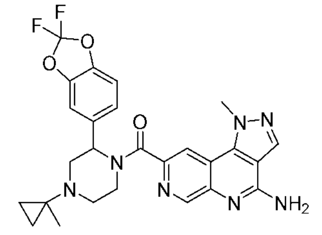 | **I-114** | 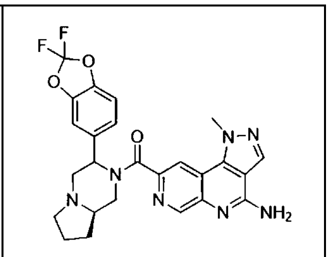 |
| **I-115** | 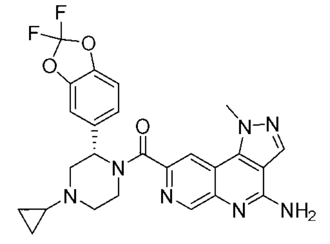 | **I-116** | 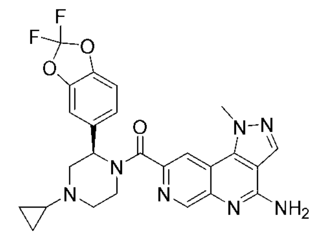 | **I-117** | 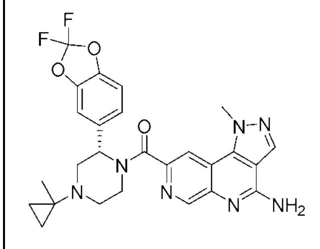 |
| **I-118** | 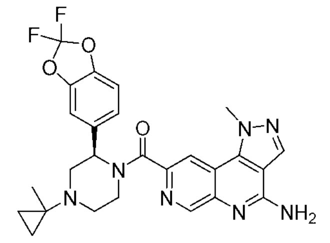 | | | | |

**Pharmaceutical Composition and Treatment Method**

In some embodiments, the present disclosure provides a pharmaceutical composition comprising a prophylactically or therapeutically effective amount of the compound, or the pharmaceutically acceptable salt, the ester, the stereoisomer, the tautomer, the polymorph, the solvate, the metabolite, the isotopically labeled compound, or the prodrug thereof of the present disclosure, and one or more pharmaceutically acceptable carriers. The pharmaceutical composition is preferably a solid formulation, a semisolid formulation, a liquid formulation, or a gaseous formulation. In some embodiments, the pharmaceutical composition may further comprise one or more additional therapeutic agents.

In some embodiments, the present disclosure provides use of the compound, or the pharmaceutically acceptable salt, the ester, the stereoisomer, the tautomer, the polymorph, the solvate, the metabolite, the isotopically labeled compound, or the prodrug thereof of the present disclosure, or of the pharmaceutical composition of the present disclosure in preparing a medicament as a PRMT5 inhibitor.

In some embodiments, the present disclosure provides the compound, or the pharmaceutically acceptable salt, the ester, the stereoisomer, the tautomer, the polymorph, the solvate, the metabolite, the isotopically labeled compound, or the prodrug thereof of the present disclosure, or the pharmaceutical composition of the present disclosure, for use as a PRMT5 inhibitor.

In some embodiments, the present disclosure provides a method for preventing or treating a cancer (preferably an MTAP-deficient cancer), comprising administering to a subject in need thereof an effective amount of the compound, or the pharmaceutically acceptable salt, the ester, the stereoisomer, the tautomer, the polymorph, the solvate, the metabolite, the isotopically labeled compound, or the prodrug thereof of the present disclosure, or the pharmaceutical composition of the present disclosure.

In some embodiments, the cancer includes pancreatic cancer, lung cancer, colorectal cancer, cholangiocarcinoma, multiple myeloma, melanoma, uterine cancer, endometrial cancer, thyroid cancer, acute myeloid leukemia, bladder cancer, urothelial cancer, gastric cancer, cervical cancer, head and neck cancer, head and neck squamous cell carcinoma, lymphoma, diffuse large B-cell lymphoma, esophageal cancer, chronic lymphocytic leukemia, hepatocellular carcinoma, skin cancer, breast cancer, ovarian cancer, prostate cancer, glioblastoma, renal cancer, and sarcoma.

The term "pharmaceutically acceptable carrier" in the present disclosure refers to a diluent, an adjuvant, an excipient, or a vehicle administered together with a therapeutic agent, which is suitable, within the scope of sound medical judgment, for contact with the tissues of humans and/or other animals without undue toxicity, irritation, allergic response, or other problems or complications relative to a reasonable benefit/risk ratio.

Unless otherwise stated, the term "treat", "treating", or "treatment" as used herein refers to reversing, alleviating,

or inhibiting the progression of a disorder or condition to which such term applies or one or more symptoms of such a disorder or condition, or preventing such a disorder or condition or one or more symptoms of such a disorder or condition.

As used herein, an "individual" includes a human or a non-human animal. Exemplary human individuals include human individuals suffering from a disease (e.g., a disease described herein), which are referred to as patients, or normal individuals. In the present disclosure, "non-human animals" include all vertebrates, e.g., non-mammals (e.g., birds, amphibians, reptiles) and mammals, e.g., non-human primates, livestock, and/or domesticated animals (e.g., sheep, dogs, cats, cows, pigs, etc.).

In another type of embodiment, the pharmaceutical composition of the present disclosure may further comprise one or more additional therapeutic or prophylactic agents.

## Examples

The present disclosure is further described below with reference to examples, but these provided examples are not intended to limit the scope of the present disclosure.

The abbreviations in the present disclosure have the following meanings:

| **Abbreviation** | **Full version** |
|---|---|
| ACN | Acetonitrile |
| Boc | *tert*-Butyloxycarbonyl |
| CMPI | 2-Chloro-1-methylpyridinium iodide |
| $Cu(OTf)_2$ | Copper(II) trifluoromethanesulfonate |
| DCM | Dichloromethane |
| DEA | Diethylamine |
| Dess-Martin | Dess-Martin reagent |
| DIBAL-H | Diisobutylaluminium hydride |
| DIEA/DIPEA | N,N-diisopropylethylamine |
| dioxane | 1,4-Dioxane |
| DMAC | *N,N*-dimethylacetamide |
| DMF | *N,N*-dimethylformamide |
| DMSO | Dimethyl sulfoxide |
| dppf | 1,1'-Bis(diphenylphosphino)ferrocene |
| EA | Ethyl acetate |
| Fmoc | Fluorenylmethoxycarbonyl |
| h/hrs | Hour |
| HATU | *O*-(7-azabenzotriazol-1-yl)-*N*,*N*,*N'*,*N'*-tetramethyluronium hexafluorophosphate |
| HCl | Hydrochloric acid |
| HFIP | Hexafluoroisopropanol |
| $H_2O$ | Water |
| i-PrMgCl | Isopropylmagnesium chloride |
| $K_2CO_3$ | Potassium carbonate |
| KOAc | Potassium acetate |
| LCMS | Liquid chromatography-mass spectrometry |
| $LiAlH_4$ | Lithium aluminium hydride |
| LiOH | Lithium hydroxide |
| 2,6-Lutidine | 2,6-Dimethylpyridine |
| MeCN | Acetonitrile |

(continued)

| Abbreviation | Full version |
|---|---|
| MeI | Iodomethane |
| MeOH | Methanol |
| min | Minute |
| MS | Molecular sieve |
| NBS | *N*-bromosuccinimide |
| NCS | *N*-chlorosuccinimide |
| $NaBF_4$ | Sodium tetrafluoroborate |
| NMI | *N*-methylimidazole |
| $Pd(dppf)Cl_2$ | [1,1'-Bis(diphenylphosphino)ferrocene]palladium(II) dichloride |
| $Pd(PPh_3)_4$ | Tetrakis(triphenylphosphine)palladium(0) |
| PE | Petroleum ether |
| PhMe | Toluene |
| $PMB-NH_2$ | 4-Methoxybenzylamine |
| $SOCl_2$ | Thionyl chloride |
| tBuOK | Potassium *tert*-butoxide |
| TBTU | *O*-(Benzotriazol-1-yl)-*N,N,N',N'*-tetramethyluronium tetrafluoroborate |
| TCHF | *N,N,N',N'*-Tetramethylchloroformamidinium hexafluorophosphate |
| TEA | Triethylamine |
| TFA | Trifluoroacetic acid |
| $TF_2O$ | Trifluoromethanesulfonic anhydride |
| THF | Tetrahydrofuran |
| $TiOEt_4$ | Tetraethyl titanate |
| r.t./rt | Room temperature |
| X-Phos | 2-Dicyclohexylphosphino-2',4',6'-triisopropylbiphenyl |
| X-Phos Pd $G_3$ | (2-Dicyclohexylphosphino-2',4',6'-triisopropyl-1,1'-biphenyl)[2-(2'-amino-1,1'-biphenyl)]palladium(II) methanesulfonate |

The general synthetic route is as follows:

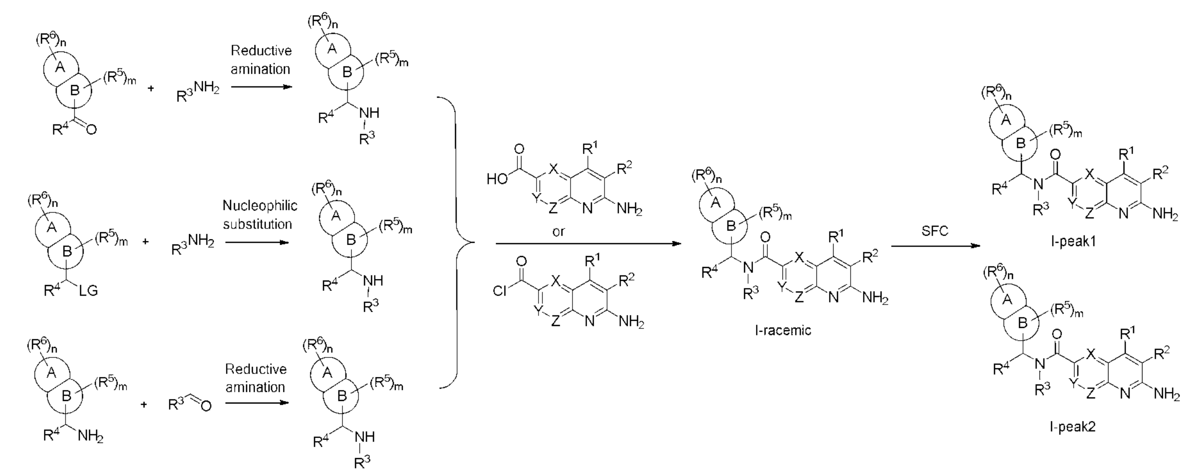

wherein LG is a leaving group, preferably halogen; and
the other groups are as defined above.

Example I-1:

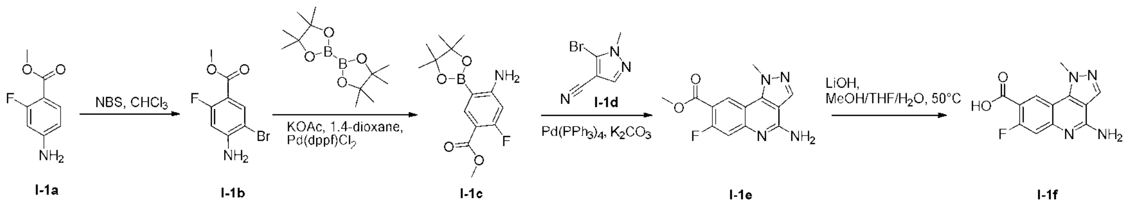


I-1a I-1b I-1c I-1d I-1e I-1f

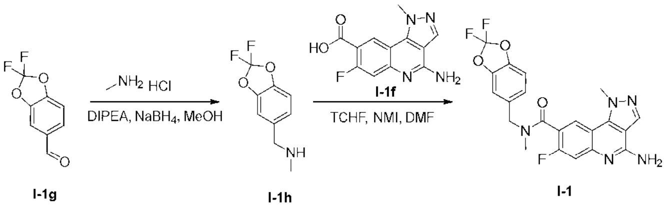


I-1g I-1h I-1f I-1

Step **1**

**I-1a** (20 g, 118 mmol) was dissolved in chloroform (500 mL), and NBS (21 g, 118 mmol) was added. The reaction liquid was stirred at room temperature for 5 hours for reaction. After the reaction was completed, the reaction mixture was diluted by adding water (200 mL) and extracted with dichloromethane (200 mL × 2). The dichloromethane phase was washed with saturated brine (200 mL × 2), dried, filtered, and concentrated. The residue was purified by column chromatography to give intermediate **I-1b** (25 g, yield: 85%). LCMS (ESI) $[M+H]^+$= 248.0.

**Step 2**

**I-1b** (11.5 g, 46 mmol), bis(pinacolato)diboron (14 g, 55.6 mmol), $Pd(dppf)Cl_2$ (4.22 g, 5.8 mmol), and potassium acetate (9.1 g, 93 mmol) were dissolved in 1,4-dioxane (100 mL). The reaction liquid was purged with nitrogen, and then stirred at 85 °C for 18 hours for reaction. After the reaction was completed, the reaction mixture was cooled to room temperature and filtered. The filtrate was concentrated, and the residue was purified by column chromatography to give intermediate **I-1c** (11.5 g, yield: 84%). $^1$H NMR (400 MHz, $CDCl_3$) δ 8.24 (d, *J* = 9.1 Hz, 1H), 6.23 (d, *J* = 13.4 Hz, 1H), 5.34 (brs, 2H), 3.85 (s, 3H), 1.34 (s, 12H). LCMS (ESI) $[M+H]^+$ = 296.1.

**Step 3**

**I-1c** (10 g, 33.9 mmol) was dissolved in 1,4-dioxane (120 mL), and **I-1d** (6.3 g, 33.9 mmol), $Pd(PPh_3)_4$ (4.7 g, 4.1 mmol), and potassium carbonate (9.4 g, 67.8 mmol) were added. The reaction liquid was purged with nitrogen, and then stirred at 100 °C for 18 hours for reaction. After the reaction was completed, the reaction mixture was cooled to room temperature. The reaction liquid was filtered, and the filtrate was diluted by adding water (200 mL) and extracted with dichloromethane (200 mL × 2). The dichloromethane phase was washed with saturated brine (100 mL × 2), dried, filtered, and concentrated. The residue was purified by column chromatography to give intermediate **I-1e** (7.7 g, yield: 83%).$^1$H NMR (400 MHz, DMSO-$d_6$) δ 8.71 (d, *J* = 7.9 Hz, 1H), 8.27 (s, 1H), 7.56 (s, 2H), 7.28 (d, *J* = 13.4 Hz, 1H), 4.39 (s, 3H), 3.89 (s, 3H). LCMS (ESI) $[M+H]^+$ = 275.0.

**Step 4**

**I-1e** (200 mg, 0.73 mmol) was dissolved in a mixed solution of methanol/tetrahydrofuran/water (5 mL/5 mL/5 mL), and lithium hydroxide (52 mg, 2.2 mmol) was added. The reaction liquid was stirred at 50 °C for 2 hours for reaction. After the reaction was completed, the reaction mixture was diluted by adding water (20 mL) and adjusted to pH 5 with 1 N hydrochloric acid. A solid was precipitated, and after filtration, the solid was dried to give intermediate **I-1f** (120 mg, yield: 63%).$^1$H NMR (400 MHz, DMSO-$d_6$) δ 8.59 (d, *J* = 8.1 Hz, 1H), 8.21 (s, 1H), 7.16 - 7.07 (m, 3H), 4.35 (s, 3H). LCMS (ESI) $[M+H]^+$ = 261.2.

**Step 5**

**I-1g** (200 mg, 1.1 mmol) was dissolved in methanol (10 mL), and methylamine hydrochloride (220 mg, 3.2 mmol) and triethylamine (320 mg, 3.2 mmol) were added. The mixture was stirred at room temperature for 1 hour for reaction, and sodium borohydride (40 mg, 1.1 mmol) was added at 0 °C. The resulting mixture was stirred at room temperature for another 1 hour. After the reaction was completed, the reaction liquid was diluted by adding water (50 mL) and extracted with ethyl acetate (50 mL × 2). The ethyl acetate phase was washed with saturated brine (50 mL × 2), dried, filtered, and concentrated. The residue was purified by column chromatography to give intermediate **I-1h** (200 mg, yield: 92%). $^1$H NMR (400 MHz, DMSO-$d_6$) δ 7.34 (d, *J* = 1.5 Hz, 1H), 7.31 (d, *J* = 8.2 Hz, 1H), 7.13 (dd, *J* = 8.3, 1.6 Hz, 1H), 3.63 (s, 2H), 2.22 (s, 3H). LCMS (ESI) $[M+H]^+$ = 202.1.

**Step 6**

**I-1h** (50 mg, 0.25 mmol), **I-1f** (65 mg, 0.25 mmol), *N,N,N',N'*-tetramethylchloroformamidinium hexafluorophosphate (84 mg, 0.3 mmol), and *N*-methylimidazole (62 mg, 0.75 mmol) were dissolved in DMF (5 mL). The reaction liquid was stirred at room temperature for 16 hours for reaction. After the reaction was completed, the reaction mixture was diluted by adding ethyl acetate (30 mL) and washed with saturated brine (30 mL × 3). The ethyl acetate phase was dried, filtered, and concentrated. The residue was purified by preparative column chromatography to give **I-1** (40 mg, yield: 36%). $^1$H NMR (400 MHz, DMSO-$d_6$) δ 8.28 - 8.13 (m, 2H), 7.48 - 7.03 (m, 6H), 4.64 (d, *J* = 96.4 Hz, 2H), 4.30 (d, *J* = 62.4 Hz, 3H), 2.92 (d, *J* = 52.0 Hz, 3H). LCMS (ESI) $[M+H]^+$ = 444.2.

**Example I-2:**

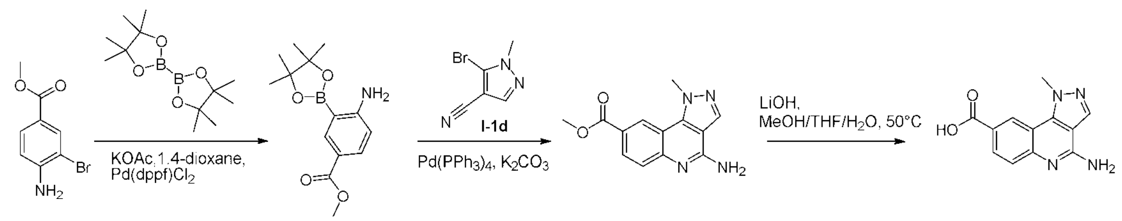


I-2a I-2b I-2c I-2d

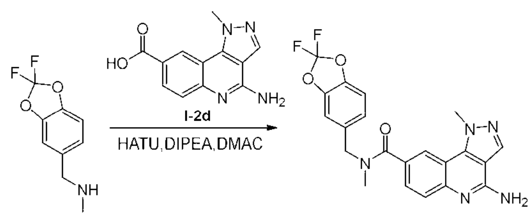


I-1h I-2

**Step 1**

**I-2a** (20 g, 86.9 mmol), bis(pinacolato)diboron (23.2 g, 91.3 mmol), Pd(dppf)$Cl_2$ (6.33 g, 8.7 mmol), and potassium acetate (17.1 g, 174 mmol) were dissolved in 1,4-dioxane (200 mL). The reaction liquid was purged with nitrogen, and then stirred at 90 °C for 18 hours for reaction. After the reaction was completed, the reaction mixture was cooled to room temperature and filtered. The filtrate was concentrated, and the residue was purified by column chromatography to give intermediate **I-2b** (21 g, yield: 87%). LCMS (ESI) $[M+H]^+$ = 278.2.

**Step 2**

**I-2b** (10.7 g, 38.7 mmol) was dissolved in 1,4-dioxane (50 mL), and **I-1d** (6 g, 32.2 mmol), Pd($PPh_3$)$_4$ (3.73 g, 3.23 mmol), and potassium carbonate (8.92 g, 64.5 mmol) were added. The reaction liquid was purged with nitrogen, and then stirred at 100 °C for 18 hours for reaction. After the reaction was completed, the reaction mixture was cooled to room temperature. The reaction liquid was filtered, and the filtrate was diluted by adding water (200 mL) and extracted with dichloromethane (200 mL × 2). The dichloromethane phase was washed with saturated brine (100 mL × 2), dried, filtered,

and concentrated. The residue was purified by column chromatography to give intermediate I-2c (5.1 g, yield: 62%).$^1$H NMR (400 MHz, DMSO-$d_6$) δ 8.81 (s, 1H), 8.28 (s, 1H), 8.02 (d, *J* = 8.8 Hz, 1H), 7.61 (d, *J* = 8.7 Hz, 1H), 7.38 (s, 2H), 4.42 (s, 3H), 3.89 (s, 3H). LCMS (ESI) $[M+H]^+$ = 257.1.

**Step 3**

**I-2c** (990 mg, 3.9 mmol) was dissolved in a mixed solution of methanol/tetrahydrofuran/water (10 mL/10 mL/5 mL), and lithium hydroxide (280 mg, 2.2 mmol) was added. The reaction liquid was stirred at 50 °C for 2 hours for reaction. After the reaction was completed, the reaction mixture was diluted by adding water (20 mL) and adjusted to pH 5 with 1 N hydrochloric acid. A solid was precipitated, and after filtration, the solid was dried to give intermediate **I-2d** (900 mg, yield: 96%).$^1$H NMR (400 MHz, DMSO-$d_6$) δ 13.57 (s, 1H), 8.86 (d, *J* = 1.7 Hz, 1H), 8.64 (s, 1H), 8.26 (dd, *J* = 8.7, 1.7 Hz, 1H), 7.87 (d, *J* = 8.7 Hz, 1H), 4.50 (s, 3H). LCMS (ESI) $[M+H]^+$ = 243.2.

**Step 4**

**I-1h** (52 mg, 0.26 mmol), **I-2d** (50 mg, 0.21 mmol), HATU (120 mg, 0.32 mmol), and DIPEA (81 mg, 0.63 mmol) were dissolved in DMAC (5 mL). The reaction liquid was stirred at 45 °C for 16 hours for reaction. After the reaction was completed, the reaction mixture was diluted by adding ethyl acetate (30 mL) and washed with saturated brine (30 mL × 3). The ethyl acetate phase was dried, filtered, and concentrated. The residue was purified by preparative column chromatography to give **I-2** (63 mg, yield: 71%).$^1$H NMR (400 MHz, DMSO-$d_6$) δ 8.27 (s, 2H), 7.60 (d, *J* = 8.4 Hz, 2H), 7.42 (d, *J* = 8.3 Hz, 2H), 7.28 (s, 3H), 4.68 (s, 2H), 4.49 - 4.04 (m, 3H), 2.97 (s, 3H). LCMS (ESI) $[M+H]^+$ = 426.2.

**Example I-3:**

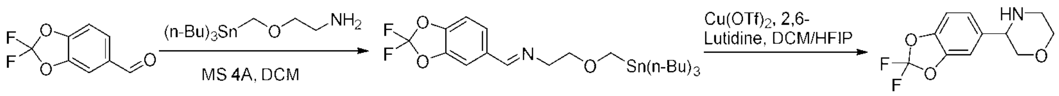


I-1g I-3a I-3b

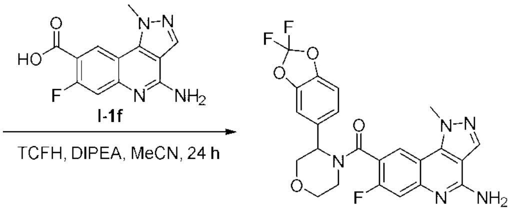


I-3

**Step 1**

**I-1g** (380 mg, 2.04 mmol) was dissolved in dichloromethane (10 mL), and then 2-((tributylstannyl)methoxy) ethan-1-amine (743 g, 2.04 mmol) and a molecular sieve (500 mg) were added. The mixture was reacted at room temperature for 16 hours under a nitrogen atmosphere. After the reaction was completed as monitored by TLC, the reaction liquid was filtered and concentrated under reduced pressure. The residue was directly used in the next step.

**Step 2**

Copper(II) trifluoromethanesulfonate (680 mg, 1.88 mmol) was dissolved in dichloromethane (9 mL) and hexafluoroisopropanol (3 mL), and 2,6-lutidine (201 mg, 1.89 mmol) was added. The mixture was stirred at room temperature for 1 hour. A solution of **I-3a** (crude product) in dichloromethane (3 mL) was added, and the resulting mixture was stirred at room temperature for 16 h. The resulting mixture was diluted with dichloromethane, and a mixed solution of 12% aqueous ammonia and a saturated aqueous sodium chloride solution (1:1, 30 mL) was added. The reaction mixture was stirred for 20 min. The aqueous phase was extracted with dichloromethane (10 mL × 2). The organic phases were combined, washed with saturated aqueous sodium chloride solution (10 mL), dried, filtered, and concentrated under reduced pressure. The crude product was separated and purified by column chromatography to give **I-3b** (150

mg, two-step yield: 26.2%). LCMS (ESI) $[M+H]^+$= 244.1.

**Step 3**

**I-1f** (86 mg, 0.33 mmol) and **I-3b** (80 mg, 0.33 mmol) were dissolved in acetonitrile (10 mL), and *N,N*-diisopropylethylamine (127.7 mg, 0.99 mmol) and *N,N,N',N'*-tetramethylchloroformamidinium hexafluorophosphate (185.2 mg, 0.66 mmol) were added. The mixture was reacted at 25 °C for 24 hours. After the reaction was completed as monitored by LCMS, the reaction liquid was concentrated under reduced pressure. The crude product was separated by prep-HPLC to give I-3 (16.3 mg, yield: 10%). $^1H$ NMR (400 MHz, DMSO-$d_6$) δ 8.22 (s, 1H), 8.14 (d, *J* = 7.4 Hz, 1H), 7.42 (s, 1H), 7.38 - 7.35 (m, 1H), 7.30 - 7.27 (m, 2H), 7.03 (s, 2H), 5.50 - 5.20 (m, 1H), 4.40 - 4.37 (m, 1H), 4.28 (s, 3H), 3.91 - 3.78 (m, 2H), 3.60 - 3.54 (m, 1H), 3.26 - 3.24 (m, 1H), 3.16 - 3.08 (m, 1H). LCMS (ESI) $[M+H]^+$ = 486.1.

**Example 1-4:**

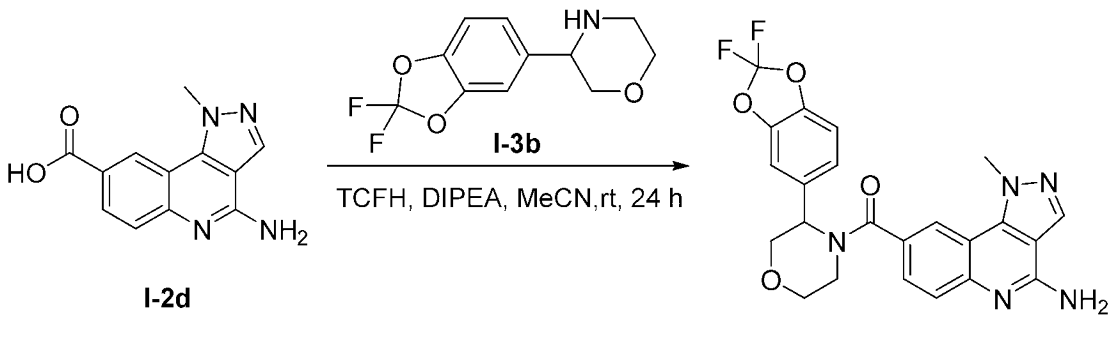


**I-2d**

**I-4**

**Step 1**

**I-3b** (50 mg, 0.21 mmol) was dissolved in anhydrous acetonitrile (3 mL), and then I-2d (50 mg, 0.21 mmol), *N,N*-diisopropylethylamine (64 mg, 0.495 mmol), and *N,N,N',N'*-tetramethylchloroformamidinium hexafluorophosphate (93 mg, 0.330 mmol) were added. The mixture was reacted at room temperature for 24 hours. After the reaction was completed as monitored by LCMS, water was added to the reaction liquid, and the resulting mixture was extracted several times with ethyl acetate. The organic phase was dried over anhydrous sodium sulfate, filtered, and concentrated under reduced pressure. The crude product was separated by prep-HPLC to give **I-4** (47.0 mg, yield: 48%). $^1H$ NMR (400 MHz, DMSO-$d_6$) δ 8.25 (s, 1H), 8.20 (d, *J* = 1.4 Hz, 1H), 7.61 (d, *J* = 8.5 Hz, 1H), 7.56 (dd, *J* = 8.5, 1.8 Hz, 1H), 7.45 (s, 1H), 7.36 (d, *J* = 8.4 Hz, 1H), 7.29 (d, *J* = 8.5 Hz, 1H), 7.04 (s, 2H), 5.40 - 5.35 (m, 1H), 4.38 - 4.34 (m, 1H), 4.26 (s, 3H), 3.94 - 3.79 (m, 3H), 3.66 - 3.57 (m, 1H), 3.30 - 3.20 (m, 1H). LCMS (ESI) $[M+H]^+$ = 468.2.

**Example I-5:**

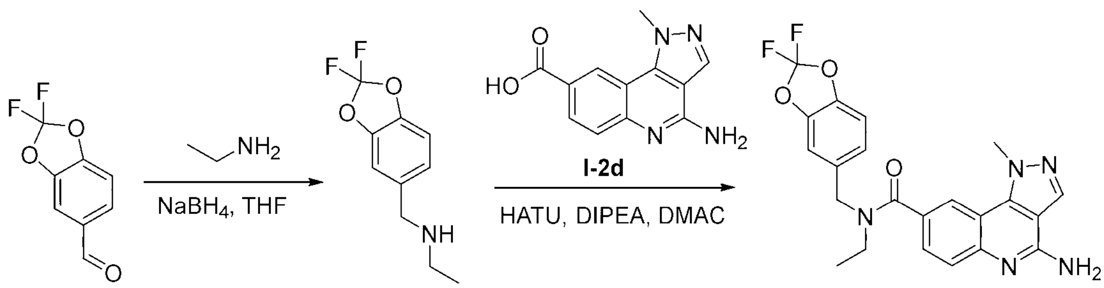


**I-1g** **I-5a** **I-5**

**Step 1**

**I-1g** (300 mg, 1.6 mmol) was dissolved in a solution of ethylamine/tetrahydrofuran (1.6 mL, 2 M). After the mixture was stirred at room temperature for 2 hours for reaction, sodium borohydride (73 mg, 1.9 mmol) was added at 0 °C, and the resulting mixture was stirred at room temperature for another 1 hour. After the reaction was completed, water (2 mL) was added to quench the reaction, and the mixture was filtered. The filtrate was concentrated, and the residue was purified by column chromatography to give **I-5a** (200 mg, yield: 57%). $^1$H NMR (400 MHz, DMSO-$d_6$) δ 7.37 (d, *J* = 1.5 Hz, 1H), 7.32 (d, *J* = 8.2 Hz, 1H), 7.15 (dd, *J* = 8.2, 1.6 Hz, 1H), 3.69 (s, 2H), 2.47 (d, *J* = 7.1 Hz, 2H), 1.01 (t, *J* = 7.1 Hz, 3H). LCMS (ESI) $[M+H]^+$ =216.1.

**Step 2**

**I-2d** (80 mg, 0.3 mmol), **I-5a** (88 mg, 0.4 mmol), HATU (190 mg, 0.5 mmol), and DIPEA (130 mg, 1 mmol) were dissolved in DMAC (5 mL), and the mixture was stirred at 45 °C for 16 hours for reaction. After the reaction was completed, water (2 mL) was added to quench the reaction, and the mixture was extracted with ethyl acetate (30 mL × 3). The ethyl acetate phase was washed with saturated brine (10 mL × 3), dried, filtered, and concentrated. The residue was purified by column chromatography to give **I-5** (63 mg, yield: 43%). $^1$H NMR (400 MHz, DMSO-$d_6$) δ 8.24 (s, 2H), 7.58 (s, 2H), 7.46 - 7.36 (m, 2H), 7.22 (s, 1H), 7.14 (s, 2H), 4.69 (s, 2H), 4.20 (d, *J* = 97.5 Hz, 3H), 2.46 (s, 2H), 1.14 (t, *J* = 6.6 Hz, 3H). LCMS (ESI) $[M+H]^+$ = 440.2.

**Example I-6:**

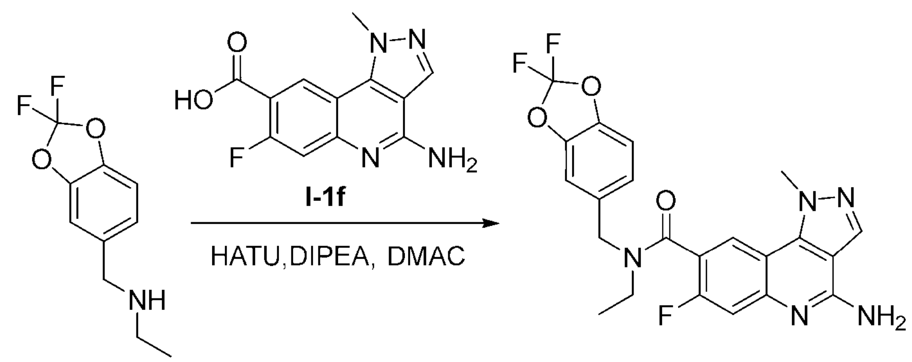


I-5a

I-6

**Step 1**

**I-1f** (80 mg, 0.3 mmol), **I-5a** (83 mg, 0.4 mmol), HATU (180 mg, 0.46 mmol), and DIPEA (120 mg, 0.93 mmol) were dissolved in DMAC (5 mL). The mixture was stirred at 45 °C for 16 hours for reaction. After the reaction was completed, water (2 mL) was added to quench the reaction, and the mixture was extracted with ethyl acetate (20 mL × 2). The ethyl acetate phase was washed with saturated brine (10 mL × 3), dried, filtered, and concentrated. The residue was purified by column chromatography to give **I-6** (77 mg, yield: 54%). $^1$H NMR (400 MHz, DMSO-$d_6$) δ 8.33 - 8.09 (m, 2H), 7.47 - 7.01 (m, 6H), 4.64 (d, *J* = 108.0 Hz, 2H), 4.30 (d, *J* = 68.3 Hz, 3H), 3.55 - 3.40 (m, 1H), 3.20 (*q*, *J* = 7.0 Hz, 1H), 1.16 (t, *J* = 7.0 Hz, 1H), 1.03 (t, *J* = 7.0 Hz, 2H). LCMS (ESI) $[M+H]^+$ = 458.2.

**Example I-3 (PEAK1) and I-3 (PEAK2):**

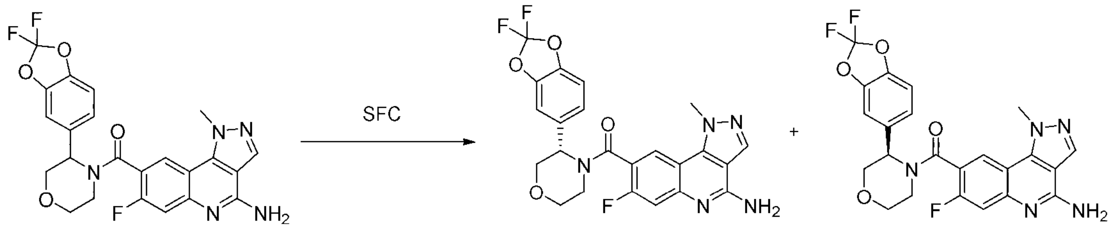


I-3

**Step 1**

**I-3** was purified by preparative SFC (IA, Hex:EtOH:TFA = 70:30:0.3, 25 mL/min, 230 nm, IA-3.0 column) to give **I-3 (PEAK1,** Rt = 8.298 min) and **I-3 (PEAK2,** Rt = 11.157 min).

**I-3 (PEAK1):** $^{1}$H NMR (400 MHz, DMSO-$d_6$) δ 8.25 (s, 2H), 7.63 - 7.23 (m, 6H), 5.71 (s, 1H), 4.44 (d, *J* = 39.6 Hz, 4H), 4.16 - 3.49 (m, 4H), 3.31 - 3.01 (m, 1H). LCMS (ESI) $[M+H]^+$ = 486.2.

**I-3 (PEAK2):** $^{1}$H NMR (400 MHz, DMSO-$d_6$) δ 8.25 (s, 2H), 7.63 - 7.23 (m, 6H), 5.71 (s, 1H), 4.44 (d, *J* = 39.6 Hz, 4H), 4.16 - 3.49 (m, 4H), 3.31 - 3.01 (m, 1H). LCMS (ESI) $[M+H]^+$= 486.2.

**Example I-4 (PEAK1) and I-4 (PEAK2):**

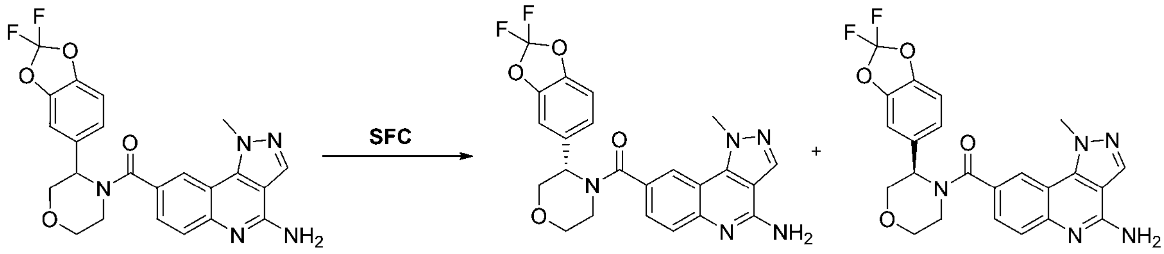


I-4

**Step 1**

**I-4** was purified by preparative SFC (IA, Hex:EtOH:TFA= 70:30:0.3, 25 mL/min, 230 nm, IA-3.0 column) to give **I-4 (PEAK1,** Rt = 6.841 min) and **I-4 (PEAK2,** Rt = 10.851 min).

**I-4 (PEAK1):** $^{1}$H NMR (400 MHz, DMSO-$d_6$) δ 8.25 (s, 1H), 8.20 (d, *J* = 1.4 Hz, 1H), 7.61 (d, *J* = 8.5 Hz, 1H), 7.56 (dd, *J* = 8.5, 1.8 Hz, 1H), 7.45 (s, 1H), 7.36 (d, *J* = 8.4 Hz, 1H), 7.29 (d, *J* = 8.5 Hz, 1H), 7.04 (s, 2H), 5.40 - 5.35 (m, 1H), 4.38 - 4.34 (m, 1H), 4.26 (s, 3H), 3.94 - 3.79 (m, 3H), 3.66 - 3.57 (m, 1H), 3.30 - 3.20 (m, 1H). LCMS (ESI) $[M+H]^+$ = 468.2.

**I-4 (PEAK2):** $^{1}$H NMR (400 MHz, DMSO-$d_6$) δ 8.25 (s, 1H), 8.20 (d, *J* = 1.4 Hz, 1H), 7.61 (d, *J* = 8.5 Hz, 1H), 7.56 (dd, *J* = 8.5, 1.8 Hz, 1H), 7.45 (s, 1H), 7.36 (d, *J* = 8.4 Hz, 1H), 7.29 (d, *J* = 8.5 Hz, 1H), 7.04 (s, 2H), 5.40 - 5.35 (m, 1H), 4.38 - 4.34 (m, 1H), 4.26 (s, 3H), 3.94 - 3.79 (m, 3H), 3.66 - 3.57 (m, 1H), 3.30 - 3.20 (m, 1H). LCMS (ESI) $[M+H]^+$ = 468.2.

**Example I-11:**

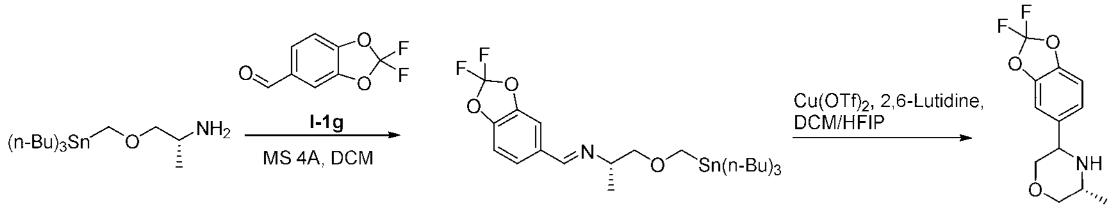


I-11a I-11b I-11c

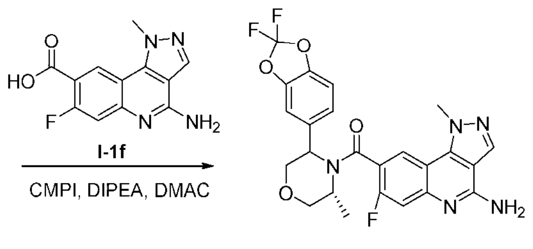


I-11

**Step 1**

**I-11a** (800 mg, 2.2 mmol) and **I-1g** (400 mg, 2.2 mmol) were dissolved in dichloromethane (5 mL), and a 4A molecular sieve (1 g) was added. The reaction liquid was purged with nitrogen and then stirred at room temperature for 16 hours for reaction. After the reaction was completed, the mixture was filtered, and the filtrate was concentrated to give a crude product **I-11b,** which was directly used in the next step without any purification.

**Step 2**

Copper(II) trifluoromethanesulfonate (660 mg, 1.8 mmol) was dissolved in a mixed solution of dichloromethane (30 mL)/hexafluoroisopropanol (10 mL), and 2,6-lutidine (200 mg, 1.8 mmol) was added. After the mixture was stirred at room temperature for 1 hour, the crude product **I-11b** was added. The resulting mixture was stirred at room temperature for another 16 hours for reaction. After the reaction was completed, the mixture was diluted by adding dichloromethane (20 mL), and a mixed solution of 12% aqueous ammonia and a saturated aqueous sodium chloride solution (1:1, 30 mL) was added. The mixture was stirred for another 20 min, and liquid separation was performed. The aqueous phase was extracted with dichloromethane (20 mL × 2). The organic phases were combined, washed with saturated brine (50 mL), dried, filtered, and concentrated. The residue was purified by column chromatography to give **I-11c** (400 mg, two-step yield: 70%).$^{1}$H NMR (400 MHz, DMSO-$d_6$) δ 7.44 (s, 1H), 7.35 (d, *J* = 8.2 Hz, 1H), 7.26 (d, *J* = 8.3 Hz, 1H), 3.88 (s, 1H), 3.68 (s, 2H), 3.05 - 2.90 (m, 3H), 0.93 (d, *J* = 4.7 Hz, 3H). LCMS (ESI) $[M+H]^+$ = 258.1.

**Step 3**

**I-1f** (100 mg, 0.4 mmol), **I-11c** (200 mg, 0.5 mmol), CMPI (120 mg, 0.5 mmol), and DIPEA (150 mg, 1.2 mmol) were dissolved in DMAC (5 mL). The reaction liquid was stirred at 45 °C for 16 hours for reaction. After the reaction was completed, water (2 mL) was added to quench the reaction. The mixture was extracted with ethyl acetate (20 mL × 2). The ethyl acetate phase was washed with saturated brine (10 mL × 3), dried, filtered, and concentrated. The residue was purified by column chromatography to give **I-11** (43 mg, yield: 21%).$^{1}$H NMR (400 MHz, DMSO-$d_6$) δ 8.24 (d, *J* = 8.9 Hz, 2H), 7.56 (s, 1H), 7.42 (d, *J* = 7.1 Hz, 2H), 7.36 (d, *J* = 11.8 Hz, 1H), 7.32 (s, 2H), 4.70 (d, *J* = 12.6 Hz, 1H), 4.38 (s, 3H), 3.78 (d, *J* = 44.2 Hz, 5H), 0.82 (d, *J* = 21.0 Hz, 3H). LCMS (ESI) $[M+H]^+$ = 500.1.

**Example I-12:**

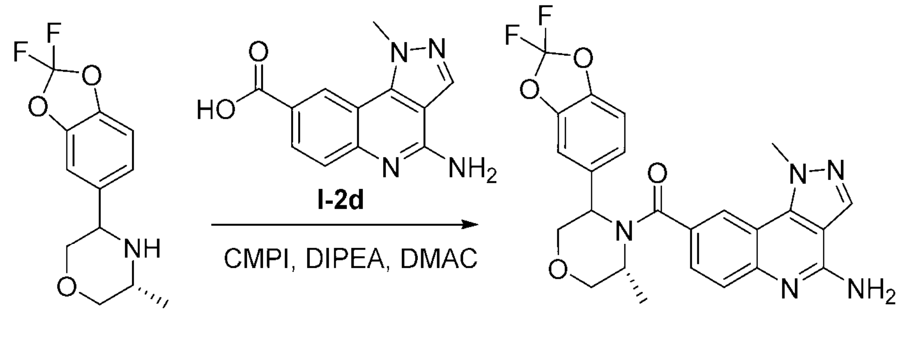


I-11c I-12

**I-2d** (100 mg, 0.41 mmol), **I-11c** (200 mg, 0.47 mmol), CMPI (130 mg, 0.49 mmol), and DIPEA (160 mg, 1.24 mmol) were dissolved in DMAC (5 mL). The mixture was stirred at 45 °C for 16 hours for reaction. After the reaction was completed, water (2 mL) was added to quench the reaction, and the mixture was extracted with ethyl acetate (20 mL × 2). The ethyl acetate phase was washed with saturated brine (10 mL × 3), dried, filtered, and concentrated. The residue was purified by column chromatography to give **I-12** (48 mg, yield: 25%).$^1$H NMR (400 MHz, $CDCl_3$) δ 8.46 (s, 1H), 8.22 (s, 1H), 7.92 (d, *J* = 8.4 Hz, 1H), 7.67 - 7.62 (m, 1H), 7.48 - 7.37 (m, 2H), 7.03 (d, *J* = 8.3 Hz, 1H), 5.73 (s, 1H), 4.65 (d, *J* = 12.6 Hz, 1H), 4.44 (s, 3H), 3.91 (dd, *J* = 12.6, 3.9 Hz, 1H), 3.84 (s, 1H), 3.70 (s, 2H), 0.98 (d, *J* = 6.6 Hz, 3H). LCMS (ESI) $[M+H]^+$ = 482.2.

**Example I-13:**

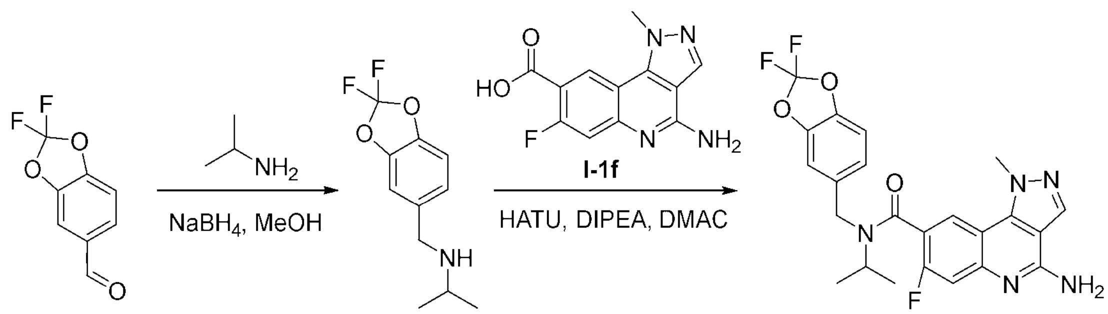


I-1g I-13a I-13

**Step 1**

**I-1g** (1 g, 5.4 mmol) was dissolved in methanol (20 mL), and isopropylamine (630 mg, 10.7 mmol) was added. After the mixture was stirred at room temperature for 16 hours, sodium borohydride (240 mg, 6.4 mmol) was added at 0 °C. The mixture was stirred at room temperature for another 1 hour for reaction. After the reaction was completed, water (2 mL) was added to quench the reaction, and the mixture was filtered. The filtrate was concentrated, and the residue was purified by column chromatography to give intermediate **I-13a** (1 g, 81%).$^1$H NMR (400 MHz, DMSO-$d_6$) δ 7.37 (d, *J* = 1.6 Hz, 1H), 7.30 (d, *J* = 8.2 Hz, 1H), 7.18 - 7.13 (m, 1H), 3.68 (s, 2H), 2.65 (hept, *J* = 6.2 Hz, 1H), 0.97 (d, *J* = 6.2 Hz, 6H). LCMS (ESI) $[M+H]^+$ = 230.1.

**Step 2**

**I-13a** (88 mg, 0.4 mmol), **I-1f** (80 mg, 0.3 mmol), HATU (180 mg, 0.46 mmol), and DIPEA (120 mg, 0.93 mmol) were dissolved in DMAC (5 mL). The mixture was stirred at 45 °C for 16 hours for reaction. After the reaction was completed, the reaction liquid was diluted by adding water (30 mL) and extracted with ethyl acetate (30 mL × 3). The ethyl acetate phase was washed with saturated brine (30 mL × 3), dried, filtered, and concentrated. The residue was purified by column chromatography to give **1-13** (71 mg, yield: 47%).$^1$H NMR (400 MHz, DMSO-$d_6$) δ 8.22 (d, *J* = 30.6 Hz, 2H), 7.49 - 6.93 (m, 6H), 4.67 (d, *J* = 23.5 Hz, 2H), 4.36 (s, 3H), 4.01 (s, 1H), 1.13 (s, 6H). LCMS (ESI) $[M+H]^+$ = 472.2.

**Example I-14:**

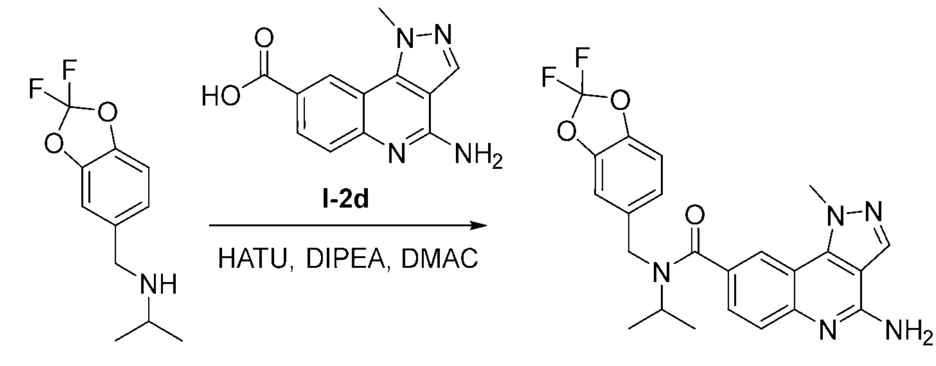


**I-13a** **I-14**

**I-13a** (80 mg, 0.3 mmol), **I-2d** (94 mg, 0.4 mmol), HATU (190 mg, 0.5 mmol), and DIPEA (130 mg, 1 mmol) were dissolved in DMAC (5 mL). The mixture was stirred at 45 °C for 5 hours for reaction. After the reaction was completed, the reaction liquid was diluted by adding water (30 mL) and extracted with ethyl acetate (30 mL × 3). The ethyl acetate phase was washed with saturated brine (30 mL × 3), dried, filtered, and concentrated. The residue was purified by column chromatography to give **I-14** (55 mg, yield: 35%).$^1$H NMR (400 MHz, DMSO-$d_6$) δ 8.25 (s, 1H), 8.22 (s, 1H), 7.60 (s, 2H), 7.40 (s, 1H), 7.36 (d, $J$ = 8.3 Hz, 1H), 7.23 (d, $J$ = 8.4 Hz, 1H), 7.16 (s, 2H), 4.63 (s, 2H), 4.33 (d, $J$ = 29.7 Hz, 4H), 1.16 (d, $J$ = 6.6 Hz, 6H). LCMS (ESI) $[M+H]^+$ = 454.2.

**Example I-15:**

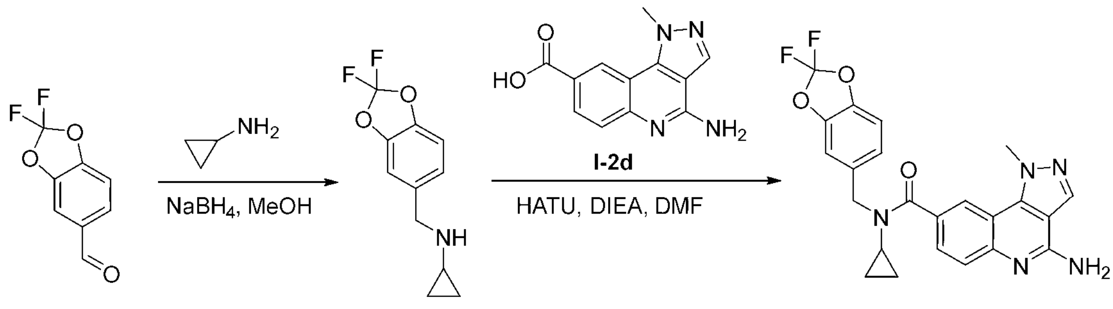


**I-1g** **I-15a** **I-15**

**Step 1**

Compound **I-1g** (1.2 g, 6.45 mmol) was dissolved in dry methanol (15 mL), and cyclopropylamine (0.74 g, 12.9 mmol) was added. The mixture was stirred at room temperature for 16 hours, and then sodium borohydride (490 mg, 12.9 mmol) was added batchwise. The reaction liquid was stirred at room temperature for 6 hours. After the reaction was completed, the reaction liquid was concentrated, diluted by adding water (20 mL), and extracted with ethyl acetate (20 mL × 3). The ethyl acetate phase was dried over anhydrous sodium sulfate, filtered, and concentrated. The residue was purified by column chromatography to give **I-15a** (1.2 g, yield: 82%). LCMS (ESI) $[M+H]^+$ = 228.1.

**Step 2**

**I-2d** (80 mg, 0.33 mmol) and **I-15a** (110 mg, 0.49 mmol) were dissolved in dry DMF (1.5 mL), and DIEA (130 mg, 0.99 mmol) and HATU (190 mg, 0.49 mmol) were added sequentially. The reaction liquid was stirred at room temperature for 16 hours. After the reaction was completed, the reaction liquid was diluted by adding water (20 mL) and extracted with ethyl acetate (20 mL × 3). The ethyl acetate phase was dried over anhydrous sodium sulfate, filtered, and concentrated. The residue was purified by column chromatography to give **I-15** (104 mg, yield: 70%).$^1$H NMR (400 MHz, DMSO-$d_6$) δ

8.57 - 8.37 (m, 2H), 7.88 (d, *J* = 8.4 Hz, 1H), 7.71 (d, *J* = 8.6 Hz, 1H), 7.49 - 7.38 (m, 2H), 7.25 (d, *J* = 8.2 Hz, 1H), 4.73 (s, 2H), 4.43 (s, 3H), 2.89 (m, 1 H), 0.50 (s, 4H). LCMS (ESI) $[M+H]^+$ = 452.2.

**Example I-16:**

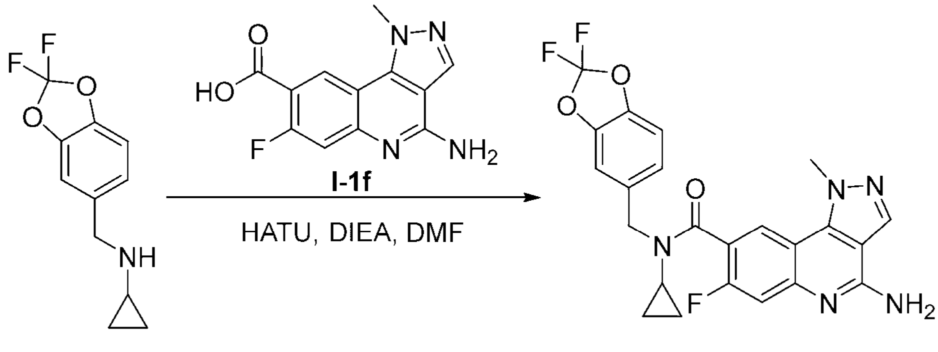


**I-15a** **I-16**

Compounds **I-1f** (80 mg, 0.31 mmol) and **I-15a** (110 mg, 0.46 mmol) were dissolved in dry DMF (1.5 mL), and DIEA (120 mg, 0.93 mmol) and HATU (180 mg, 0.46 mmol) were added sequentially. The reaction liquid was stirred at room temperature for 16 hours. After the reaction was completed, the reaction liquid was diluted by adding water (20 mL) and extracted with ethyl acetate (20 mL × 3). The ethyl acetate phase was dried over anhydrous sodium sulfate, filtered, and concentrated under reduced pressure. The residue was purified by column chromatography to give **I-16** (103 mg, yield: 71%). $^1$H NMR (400 MHz, DMSO-$d_6$) δ 8.24 (d, *J* = 17.4 Hz, 2H), 7.49 - 7.37 (m, 2H), 7.27 (dd, *J* = 26.3, 10.1 Hz, 4H), 4.76 (s, 2H), 4.37 (s, 3H), 2.69 (s, 1H), 0.48 (d, *J* = 50.5 Hz, 4H). LCMS (ESI) $[M+H]^+$ = 470.2.

**Example 1-17:**

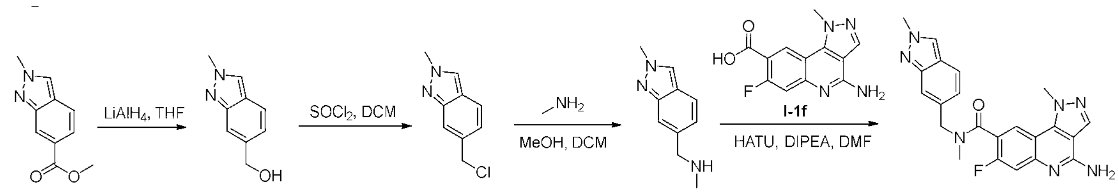


I-17a I-17b I-17c I-17d I-17

**Step 1**

**I-17a** (1 g, 5.3 mmol) was dissolved in tetrahydrofuran (30 mL), and lithium aluminium hydride (240 mg, 6.3 mmol) was added at 0 °C. The mixture was stirred at room temperature for another 2 hours for reaction. After the reaction was completed, water (5 mL) was added to quench the reaction, and the reaction liquid was filtered. The filtrate was concentrated, and the residue was purified by column chromatography to give **I-17b** (750 mg, 88%). $^1$H NMR (400 MHz, DMSO-$d_6$) δ 8.25 (s, 1H), 7.61 (dd, *J* = 8.6, 0.8 Hz, 1H), 7.47 (q, *J* = 1.1 Hz, 1H), 6.97 (dd, *J* = 8.5, 1.3 Hz, 1H), 5.21 (t, *J* = 5.8 Hz, 1H), 4.55 (dd, *J* = 5.8, 1.0 Hz, 2H), 4.13 (s, 3H).

**Step 2**

**I-17b** (250 mg, 1.54 mmol) was dissolved in dichloromethane (5 mL), and thionyl chloride (220 mg, 1.9 mmol) was slowly added dropwise at room temperature. The mixture was stirred at room temperature for another 16 hours for reaction. After the reaction was completed, the mixture was diluted by adding ethyl acetate (50 mL), washed with a saturated aqueous sodium bicarbonate solution (20 mL × 3), and washed with saturated brine (20 mL × 3). The ethyl acetate phase was dried, filtered, and concentrated. The residue was purified by column chromatography to give **I-17c** (250 mg, 90%). $^1$H NMR (400 MHz, DMSO-$d_6$) δ 8.33 (s, 1H), 7.70 (dd, *J* = 8.6, 0.9 Hz, 1H), 7.66 (s, 1H), 7.07 (dd, *J* = 8.6, 1.4

Hz, 1H), 4.85 (s, 2H), 4.16 (s, 3H).

**Step 3**

**I-17c** (130 mg, 0.7 mmol) was dissolved in dichloromethane (2 mL), and a methylamine/methanol solution (2 mL) was added. The mixture was stirred at room temperature for 16 hours for reaction. After the reaction was completed, the reaction liquid was concentrated to give **I-17d** (100 mg, 79%).$^1$H NMR (400 MHz, DMSO-$d_6$) δ 9.20 (s, 1H), 8.38 (s, 1H), 7.75 (dd, *J* = 7.0, 1.7 Hz, 2H), 7.17 (dd, *J* = 8.7, 1.2 Hz, 1H), 4.18 (s, 3H), 4.17 (s, 2H), 2.53 (s, 3H). LCMS (ESI) $[M+H]^+$ = 176.1.

**Step 4**

**I-1f** (60 mg, 0.23 mmol), **I-17d** (50 mg, 0.3 mmol), HATU (130 mg, 0.35 mmol), and DIPEA (90 mg, 0.7 mmol) were dissolved in DMF (5 mL). The mixture was stirred at 45 °C for 16 hours for reaction. After the reaction was completed, the mixture was diluted by adding water (5 mL). A solid was precipitated, and after filtration, the solid was purified by column chromatography to give **I-17** (36 mg, 37%).$^1$H NMR (400 MHz, DMSO-$d_6$) δ 8.35 - 8.12 (m, 3H), 7.68 (dd, *J* = 29.6, 8.6 Hz, 1H), 7.48 (d, *J* = 58.3 Hz, 1H), 7.37 - 7.21 (m, 3H), 6.94 (dd, *J* = 92.6, 8.6 Hz, 1H), 4.69 (d, *J* = 100.6 Hz, 2H), 4.27 (d, *J* = 89.0 Hz, 3H), 4.11 (d, *J* = 21.8 Hz, 3H), 2.93 (d, *J* = 67.1 Hz, 3H). LCMS (ESI) $[M+H]^+$ = 418.2.

**Example I-18:**

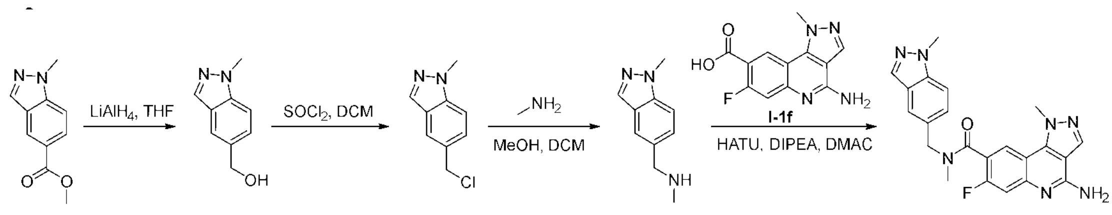


I-18a I-18b I-18c I-18d I-18

**Step 1**

**I-18a** (500 mg, 2.6 mmol) was dissolved in tetrahydrofuran (10 mL), and lithium aluminium hydride (120 mg, 3.2 mmol) was added at 0 °C. The mixture was stirred at room temperature for another 2 hours for reaction. After the reaction was completed, water (5 mL) was added to quench the reaction, and the reaction liquid was filtered. The filtrate was concentrated, and the residue was purified by column chromatography to give **I-18b** (400 mg, 94%).$^1$H NMR (400 MHz, DMSO-$d_6$) δ 8.02 (d, *J* = 0.9 Hz, 1H), 7.68 (dq, *J* = 1.7, 0.9 Hz, 1H), 7.60 (dt, *J* = 8.6, 0.9 Hz, 1H), 7.38 (dd, *J* = 8.6, 1.5 Hz, 1H), 5.21 (t, *J* = 5.7 Hz, 1H), 4.61 (d, *J* = 5.6 Hz, 2H), 4.05 (s, 3H). LCMS (ESI) $[M+H]^+$ = 163.0.

**Step 2**

**I-18b** (400 mg, 2.5 mmol) was dissolved in dichloromethane (10 mL), and thionyl chloride (590 mg, 4.9 mmol) was slowly added dropwise at room temperature. The mixture was stirred at room temperature for another 16 hours for reaction. After the reaction was completed, the mixture was diluted by adding ethyl acetate (50 mL), washed with a saturated aqueous sodium bicarbonate solution (20 mL × 3), and washed with saturated brine (20 mL × 3). The ethyl acetate phase was dried, filtered, and concentrated. The residue was purified by column chromatography to give **I-18c** (430 mg, 96%).$^1$H NMR (400 MHz, DMSO-$d_6$) δ 8.05 (s, 1H), 7.82 (s, 1H), 7.65 (d, *J* = 8.6 Hz, 1H), 7.45 (dd, *J* = 8.7, 1.6 Hz, 1H), 4.90 (s, 2H), 4.04 (s, 3H).

**Step 3**

**I-18c** (430 mg, 2.4 mmol) was dissolved in dichloromethane (5 mL), and a methylamine/methanol solution (5 mL) was added. The mixture was stirred at room temperature for 16 hours for reaction. After the reaction was completed, the reaction liquid was concentrated to give **I-18d** (400 mg, 76%).$^1$H NMR (400 MHz, DMSO-$d_6$) δ 8.11 (s, 1H), 7.90 (s, 1H), 7.70 *(d, J*= 8.7 Hz, 1H), 7.57 (dd, *J* = 8.7, 1.6 Hz, 1H), 4.19 (s, 2H), 4.06 (d, *J* = 1.9 Hz, 3H), 2.51 (d, *J* = 2.2 Hz, 3H).

**Step 4**

**I-1f** (100 mg, 0.38 mmol), **I-18d** (67 mg, 0.38 mmol), HATU (220 mg, 0.57 mmol), and DIPEA (150 mg, 1.1 mmol) were dissolved in DMAC (5 mL). The mixture was stirred at 45 °C for 16 hours for reaction. After the reaction was completed, the mixture was diluted by adding water (5 mL). A solid was precipitated, and after filtration, the solid was purified by column chromatography to give **I-18** (21 mg, 3%). $^1$H NMR (400 MHz, DMSO-$d_6$) δ 8.29 - 8.17 (m, 2H), 8.02 (d, *J* = 20.6 Hz, 1H), 7.79 - 7.56 (m, 2H), 7.47 - 7.15 (m, 4H), 4.72 (d, *J* = 99.2 Hz, 2H), 4.25 (d, *J* = 105.7 Hz, 3H), 4.03 (d, *J* = 16.9 Hz, 3H), 2.91 (d, *J* = 66.0 Hz, 3H). LCMS (ESI) [M+H]$^+$ = 418.2.

**Example 1-19:**

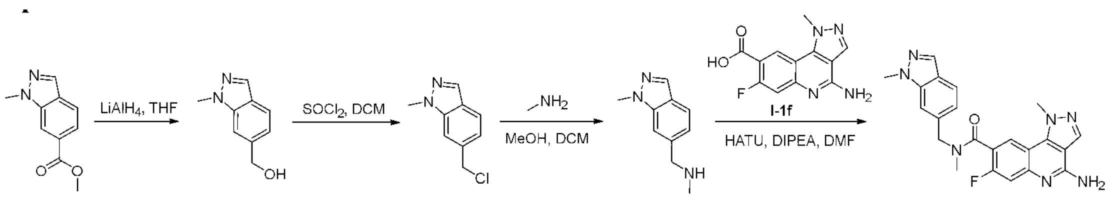


I-19a I-19b I-19c I-19d I-19

The synthesis in Example **I-19** was performed with reference to the synthesis method of Example 1-17 by using **I-19a** as a starting material to finally give **I-19** (67 mg, 52%). $^1$H NMR (400 MHz, DMSO-$d_6$) δ 8.22 (dd, *J* = 26.5, 7.3 Hz, 2H), 8.01 (d, *J* = 16.7 Hz, 1H), 7.74 (dd, *J* = 33.8, 8.3 Hz, 1H), 7.53 (d, *J* = 41.8 Hz, 1H), 7.38 - 7.22 (m, 3H), 7.06 (dd, *J* = 89.1, 8.4 Hz, 1H), 4.78 (d, *J* = 101.6 Hz, 2H), 4.23 (d, *J* = 136.2 Hz, 3H), 4.01 (d, *J* = 12.2 Hz, 3H), 2.97 (d, *J* = 72.4 Hz, 3H). LCMS (ESI) [M+H]$^+$ = 418.2.

**Example I-20:**

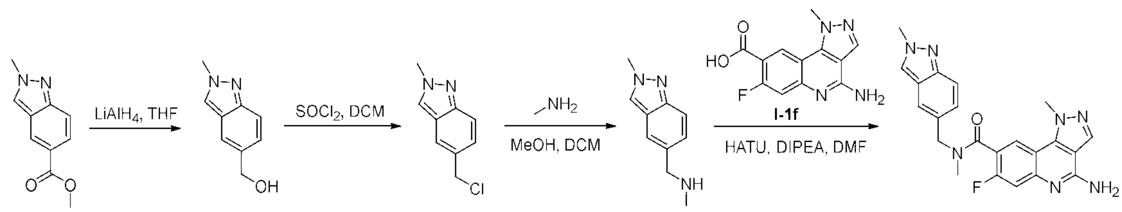


I-20a I-20b I-20c I-20d I-20

The synthesis in Example **I-20** was performed with reference to the synthesis method of Example **1-17** by using **I-20a** as a starting material to finally give **I-20** (16 mg, 16%). $^1$H NMR (400 MHz, DMSO-$d_6$) δ 8.35 - 8.13 (m, 3H), 7.68 - 6.99 (m, 5H), 4.66 (d, *J* = 99.9 Hz, 2H), 4.27 (d, *J* = 87.6 Hz, 3H), 4.11 (d, *J* = 25.0 Hz, 3H), 2.91 (d, *J* = 69.5 Hz, 3H). LCMS (ESI) [M+H]$^+$ = 418.2.

**Example I-21:**

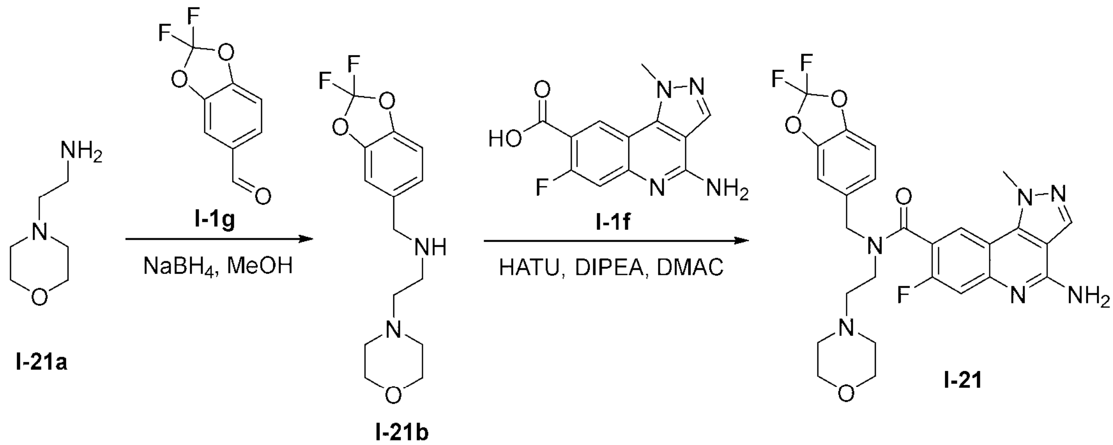


**Step 1**

**I-21a** (300 mg, 1.6 mmol) and **I-1g** (210 mg, 1.6 mmol) were dissolved in methanol (5 mL). After the mixture was stirred at room temperature for 16 hours, sodium borohydride (61 mg, 1.6 mmol) was added at 0 °C. The resulting mixture was reacted at room temperature for another 1 hour. After the reaction was completed, water (20 mL) was added to quench the reaction, and the mixture was extracted with ethyl acetate (30 mL × 3). The ethyl acetate phase was washed with saturated brine (30 mL × 3), dried, filtered, and concentrated. The residue was purified by column chromatography to give **I-21b** (400 mg, 82%). LCMS (ESI) $[M+H]^+$ =301.1.

**Step 2**

**I-1f** (50 mg, 0.2 mmol) and **I-21b** (70 mg, 0.23 mmol) were dissolved in DMAC (5 mL), and HATU (87 mg, 0.23 mmol) and DIPEA (74 mg, 0.6 mmol) were added. The mixture was stirred at 45 °C for 16 hours for reaction. After the reaction was completed, water (20 mL) was added for dilution, and the mixture was extracted with ethyl acetate (20 mL × 3). The ethyl acetate phase was dried, filtered, and concentrated. The residue was purified by column chromatography to give **I-21** (30 mg, 29%). $^1H$ NMR (400 MHz, DMSO-$d_6$) δ 8.30 - 8.08 (m, 2H), 7.49 - 7.02 (m, 6H), 4.82 (s, 1H), 4.56 (s, 1H), 4.30 (d, *J* = 67.9 Hz, 3H), 3.59 - 3.56 (m, 2H), 3.37 (d, *J* = 4.5 Hz, 2H), 3.34 - 3.29 (m, 2H), 2.55 (t, *J* = 6.6 Hz, 1H), 2.42 (s, 2H), 2.36 - 2.31 (m, 1H), 2.03 (t, *J* = 4.5 Hz, 2H). LCMS (ESI) $[M+H]^+$ =543.1.

**Example I-22:**

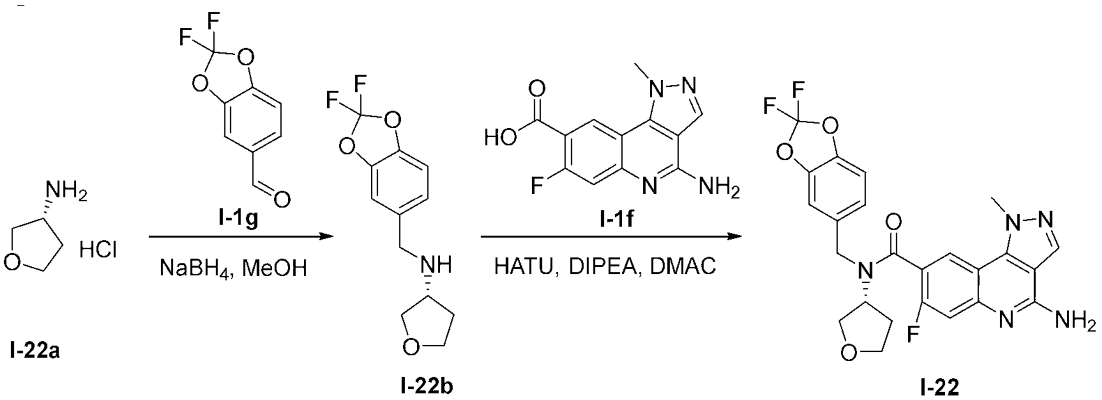


The synthesis in Example **I-22** was performed with reference to the synthesis method of Example **I-21** by using **I-22a** as a starting material to finally give **I-22** (12 mg, 9%). $^1H$ NMR (400 MHz, DMSO-$d_6$) δ9.82 (s, 1H), 8.66 (d, *J* = 22.6 Hz, 2H), 8.35 (dd, *J* = 76.5, 6.6 Hz, 1H), 7.71 (dd, *J* = 65.4, 10.3 Hz, 1H), 7.52 - 6.96 (m, 3H), 5.06 - 4.24 (m, 6H), 3.93 - 3.42 (m, 4H), 2.27 - 1.77 (m, 2H). LCMS (ESI) $[M+H]^+$ = 500.1

**Example I-23:**

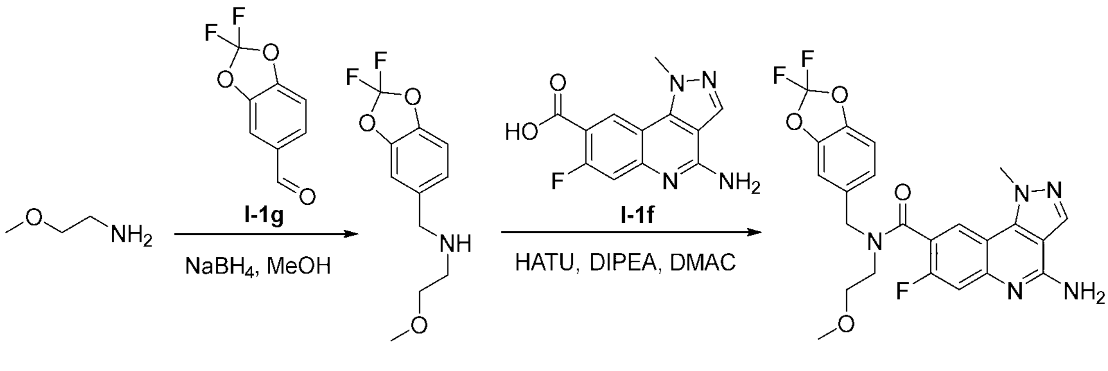


I-23a I-23b I-23

The synthesis in Example I-23 was performed with reference to the synthesis method of Example 1-21 by using I-23a as a starting material to finally give I-23 (80 mg, 50%). $^1$H NMR (400 MHz, DMSO-$d_6$) δ9.83 (s, 1H), 8.76 (s, 1H), 8.66 (d, *J* = 13.3 Hz, 1H), 8.35 (dd, *J* = 33.9, 6.6 Hz, 1H), 7.72 (*dd, J* = 21.6, 10.3 Hz, 1H), 7.47 - 6.99 (m, 3H), 4.68 (d, *J* = 114.6 Hz, 2H), 4.38 (d, *J* = 58.4 Hz, 3H), 3.60 (td, *J* = 6.6, 4.0 Hz, 1H), 3.36 (s, 3H), 3.29 (s, 1H), 3.12 (s, 2H). LCMS (ESI) $[M+H]^+$ = 488.2.

**Example I-24:**

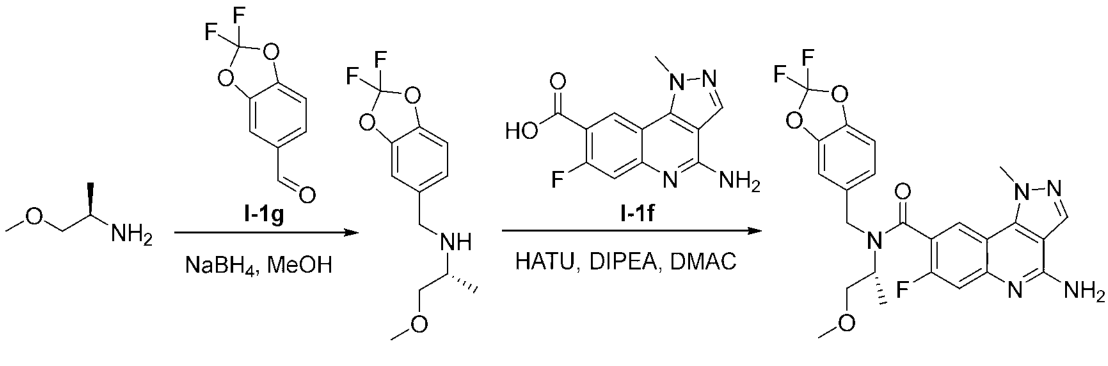


I-24a I-24b I-24

The synthesis in Example I-24 was performed with reference to the synthesis method of Example **I-21** by using **I-24a** as a starting material to finally give **I-24** (30 mg, 18%). $^1$H NMR (400 MHz, DMSO-$d_6$) δ9.77 (s, 1H), 8.75 (s, 1H), 8.64 (d, *J* = 18.5 Hz, 1H), 8.44 - 8.04 (m, 1H), 7.67 (dd, *J* = 68.3, 10.3 Hz, 1H), 7.47 - 6.94 (m, 3H), 4.91 - 4.57 (m, 2H), 4.37 (d, *J* = 54.0 Hz, 3H), 4.01 (s, 1H), 3.31 - 2.84 (m, 5H), 1.12 (d, *J* = 90.6 Hz, 3H). LCMS (ESI) $[M+H]^+$ = 502.2.

**Example** I-25:

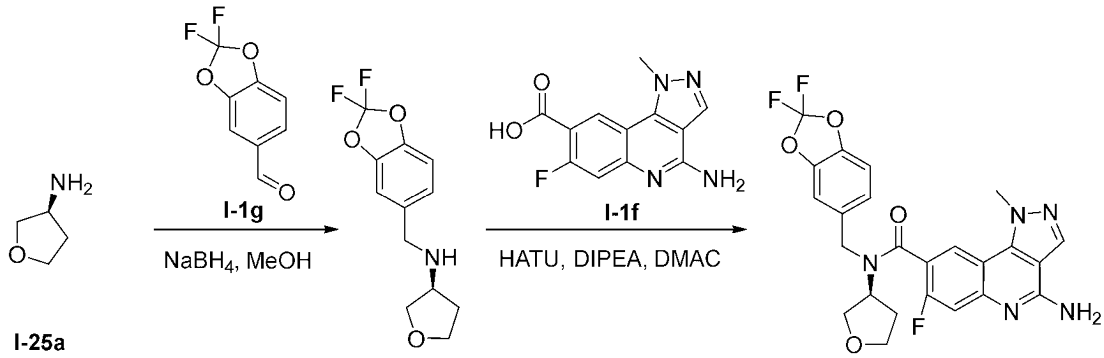


I-25a I-25b I-25

The synthesis in Example **I-25** was performed with reference to the synthesis method of Example 1-21 by using **I-25a** as a starting material to finally give **I-25** (30 mg, 24%). $^1$H NMR (400 MHz, DMSO-*d*$_6$) δ 14.00 (s, 1H), 9.92 (s, 1H), 8.79 (s, 1H), 8.69 (d, *J* = 22.9 Hz, 1H), 8.35 (dd, *J* = 77.7, 6.6 Hz, 1H), 7.74 (dd, *J* = 64.4, 10.3 Hz, 1H), 7.48 - 6.97 (m, 3H), 5.02 - 4.22 (m, 6H), 3.95 - 3.41 (m, 4H), 2.27 - 1.78 (m, 2H). LCMS (ESI) [M+H]$^+$ = 500.2.

**Example I-26:**

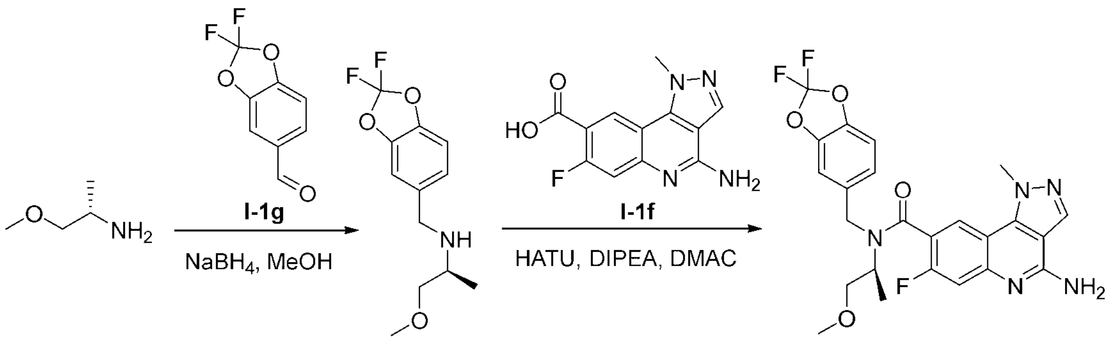


I-26a I-26b I-26

The synthesis in Example I-26 was performed with reference to the synthesis method of Example 1-21 by using **I-26a** as a starting material to finally give I-26 (40 mg, 32%). $^1$H NMR (400 MHz, DMSO-*d*$_6$) δ 9.91 (s, 1H), 8.79 (s, 1H), 8.69 (d, *J* = 19.4 Hz, 1H), 8.41 - 8.08 (m, 1H), 7.71 (dd, *J* = 67.8, 10.2 Hz, 1H), 7.47 - 6.94 (m, 3H), 4.88 - 4.53 (m, 2H), 4.37 (d, *J* = 54.8 Hz, 3H), 4.01 (s, 1H), 3.33 - 2.84 (m, 5H), 1.29 - 0.95 (m, 3H). LCMS (ESI) [M+H]$^+$= 502.2.

**Example I-27:**

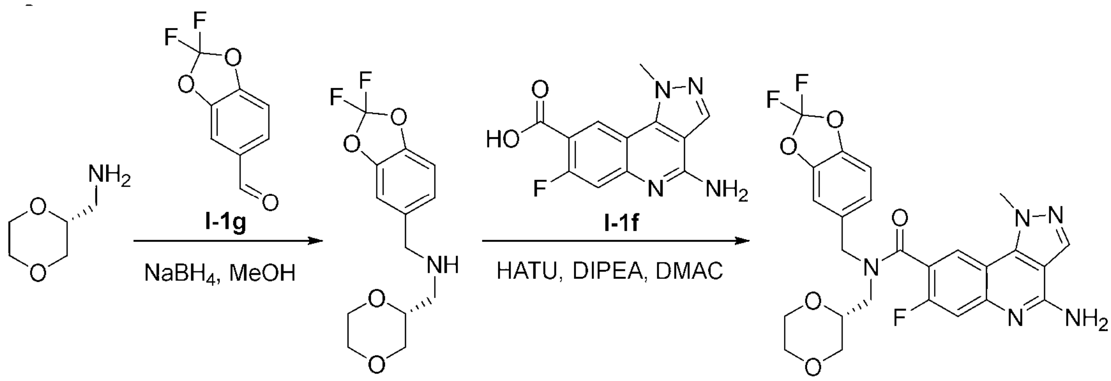


I-27a I-27b I-27

The synthesis in Example I-27 was performed with reference to the synthesis method of Example **I-21** by using I-27a as a starting material to finally give I-27 (80 mg, 60%).$^1$H NMR (400 MHz, DMSO-$d_6$) δ 9.91 (s, 1H), 8.77 (s, 1H), 8.70 (d, *J* = 15.5 Hz, 1H), 8.34 (dd, *J* = 34.6, 6.6 Hz, 1H), 7.75 (dd, *J* = 16.9, 10.3 Hz, 1H), 7.47 - 7.38 (m, 1H), 7.32 (d, *J* = 8.4 Hz, 1H), 7.14 (ddd, *J* = 88.3, 8.3, 1.7 Hz, 1H), 4.56 (s, 2H), 4.37 (d, *J* = 61.2 Hz, 3H), 3.94 - 3.40 (m, 7H), 3.30 - 2.90 (m, 2H). LCMS (ESI) $[M+H]^+$ = 530.1.

**Example I-28:**

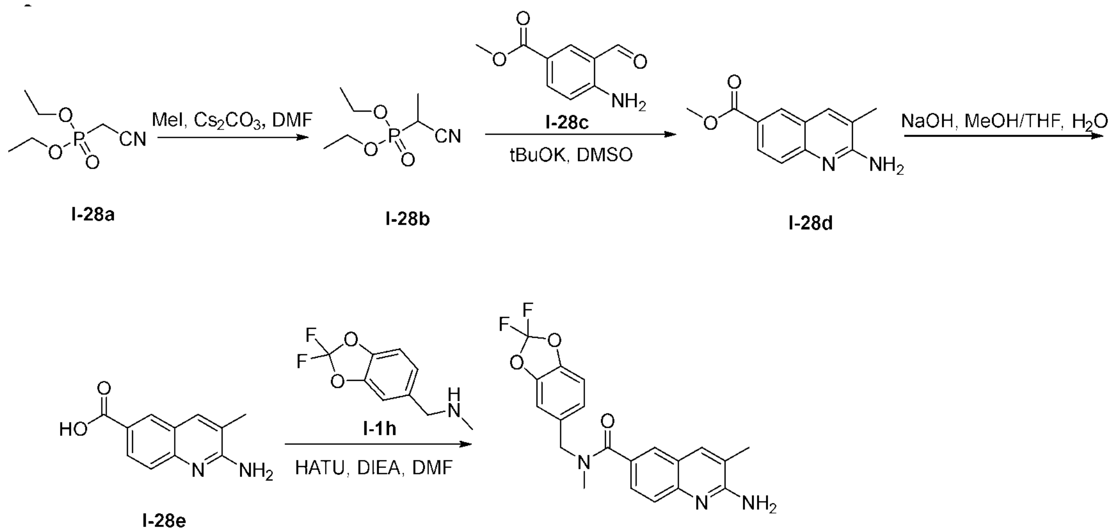


I-28a I-28b I-28d

I-28e

I-28

**Step 1**

**I-28a** (12 g, 67.7 mmol) was dissolved in DMF (60 mL), and cesium carbonate (44.1 g, 135.5 mmol) and iodomethane (12.5 g, 88.1 mmol) were added. After the mixture was stirred at room temperature for 72 hours for reaction, the mixture was diluted by adding water (300 mL) and extracted with ethyl acetate (200 mL × 3). The ethyl acetate phase was washed with saturated brine (200 mL), and dried over anhydrous sodium sulfate. The organic phase was filtered and concentrated, and the residue was purified by column chromatography to give **I-28b** (10 g, 77%).

**Step 2**

**I-28b** (7.3 g, 38.0 mmol) was dissolved in DMSO (40 mL), and potassium *tert*-butoxide (4.3 g, 38.0 mmol) was added. After the mixture was stirred at room temperature for 30 min, **I-28c** (4.0 g, 38.0 mmol) was added. The reaction liquid was heated to 65 °C and stirred for 16 hours for reaction, and then heated to 80 °C and stirred for another 8 hours. After the reaction was completed, the mixture was cooled to room temperature, and a saturated aqueous ammonium chloride solution (100 mL) was slowly added dropwise under an ice bath. The resulting mixture was extracted with

dichloromethane (200 mL × 3). The dichloromethane phase was washed with saturated brine (70 mL × 3), filtered, dried, and concentrated. The residue was purified by column chromatography to give **I-28d** (3 g, 62%).$^1$H NMR (400 MHz, DMSO-$d_6$) δ 8.27 (d, *J* = 2.0 Hz, 1H), 7.94 - 7.86 (m, 2H), 7.47 (d, *J* = 8.8 Hz, 1H), 6.72 (s, 2H), 3.85 (s, 3H), 2.21 (d, *J* = 1.0 Hz, 3H). LCMS (ESI) $[M+1]^+$ = 217.1.

**Step 3**

**I-28d** (2.9 g, 13.4 mmol) was dissolved in a mixed solution of methanol/tetrahydrofuran (24 mL/24 mL), and water (45 mL) and sodium hydroxide (1.1 g, 26.8 mmol) were added. The reaction liquid was stirred at 48 °C for 3 hours for reaction. After the reaction was completed, the mixture was cooled to room temperature and adjusted to pH 6.5 by adding a hydrochloric acid (2 M) solution. After the reaction liquid was concentrated, water (10 mL) was added, and the resulting mixture was stirred and filtered to give **I-28e** (2 g, 74%).$^1$H NMR (400 MHz, DMSO-$d_6$) δ 8.23 (d, J = 2.0 Hz, 1H), 7.91 (dd, *J* = 8.7, 2.0 Hz, 1H), 7.86 (s, 1H), 7.45 (d, *J* = 8.7 Hz, 1H), 6.67 (s, 2H), 2.21 (s, 3H).

**Step 4**

**I-28e** (60 mg, 0.3 mmol) and **I-1h** (86 mg, 0.36 mmol) were dissolved in DMF (1.5 mL), and DIPEA (120 mg, 0.9 mmol) was added. HATU (170 mg, 0.45 mmol) was slowly added while stirring at room temperature. The reaction liquid was stirred at room temperature for another 16 hours for reaction. After the reaction was completed, water (10 mL) was added, and the resulting mixture was extracted with ethyl acetate (15 mL × 2). The ethyl acetate phase was dried, filtered, and concentrated. The residue was purified by column chromatography to give **I-28** (103 mg, 90%). $^1$H NMR (400 MHz, DMSO-$d_6$) δ 7.77 (d, J = 19.9 Hz, 2H), 7.60 - 7.32 (m, 4H), 7.19 (s, 1H), 6.58 (s, 2H), 4.66 (s, 2H), 2.91 (s, 3H), 2.21 (s, 3H). LCMS (ESI) $[M+1]^+$ = 386.2.

**Example I-29:**

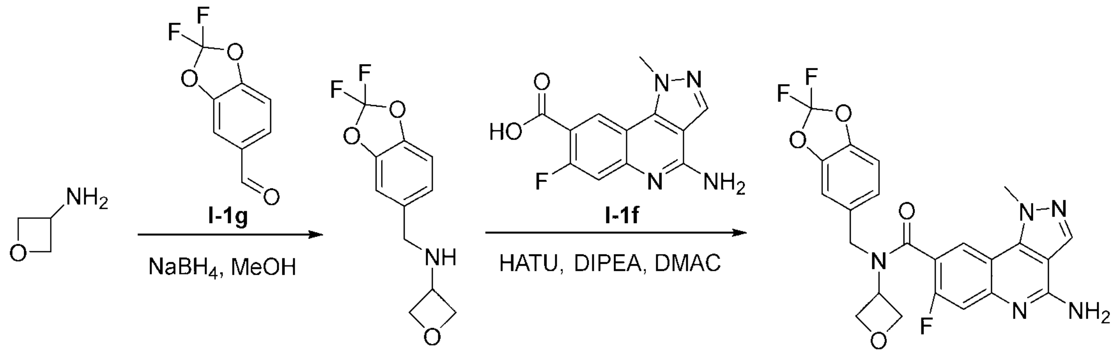


I-29a I-29b I-29

The synthesis in Example **I-29** was performed with reference to the synthesis method of Example **I-21** by using **I-29a** as a starting material to finally give **I-29** (74 mg, 50%).$^1$H NMR (400 MHz, DMSO-$d_6$) δ 8.25 (s, 1H), 8.20 (d, *J* = 7.4 Hz, 1H), 7.49 - 6.93 (m, 6H), 5.03 (dd, *J* = 14.8, 7.6 Hz, 2H), 4.81 - 4.44 (m, 5H), 4.30 (d, *J* = 54.3 Hz, 3H). LCMS (ESI) $[M+H]^+$ = 486.2.

**Example I-30:**

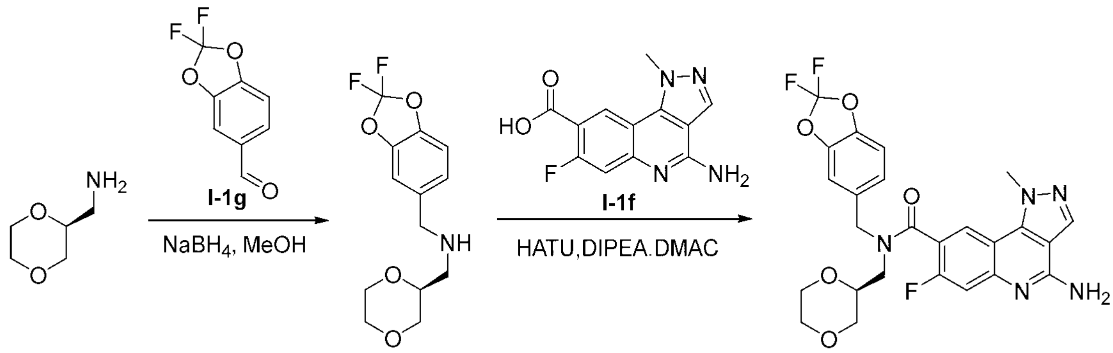


I-30a I-30b I-30

The synthesis in Example **I-30** was performed with reference to the synthesis method of Example **I-21** by using **I-30a** as a starting material to finally give **I-30** (72 mg, 41%).$^1$H NMR (400 MHz, DMSO-$d_6$) δ 9.86 (s, 1H), 8.77 (s, 1H), 8.67 (d, *J* = 21.7 Hz, 1H), 8.35 (dd, *J* = 34.3, 6.6 Hz, 1H), 7.74 (dd, *J* = 17.7, 9.7 Hz, 1H), 7.47 - 7.38 (m, 1H), 7.36 - 7.29 (m, 1H), 7.14 (ddd, *J* = 88.4, 8.3, 1.7 Hz, 1H), 5.07 - 4.51 (m, 2H), 4.37 (d, *J* = 60.9 Hz, 3H), 3.95 - 3.41 (m, 7H), 3.27 - 2.88 (m, 2H). LCMS (ESI) $[M+H]^+$ = 530.1.

**Example I-31:**

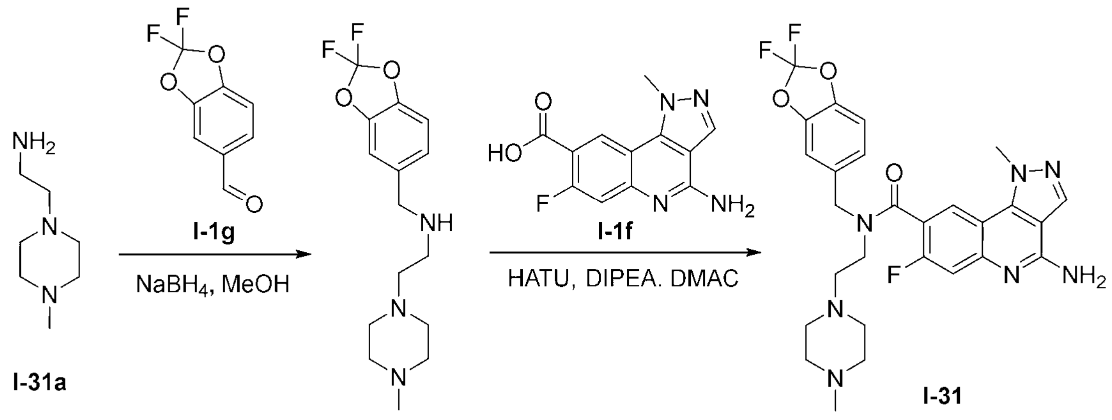


I-31a I-31b I-31

The synthesis in Example **I-31** was performed with reference to the synthesis method of Example **I-21** by using **I-31a** as a starting material to finally give **I-31** (76 mg, 41%).$^1$H NMR (400 MHz, DMSO-$d_6$) δ 10.02 (s, 1H), 8.96 - 8.45 (m, 3H), 7.77 (dd, *J* = 25.6, 10.3 Hz, 1H), 7.50 - 6.98 (m, 3H), 5.18 - 4.56 (m, 2H), 4.46 (d, *J* = 21.1 Hz, 3H), 3.79 (d, *J* = 75.6 Hz, 8H), 3.42 - 3.07 (m, 4H), 2.78 (d, *J* = 48.1 Hz, 3H). LCMS (ESI) $[M+H]^+$ = 556.3.

**Example I-32:**

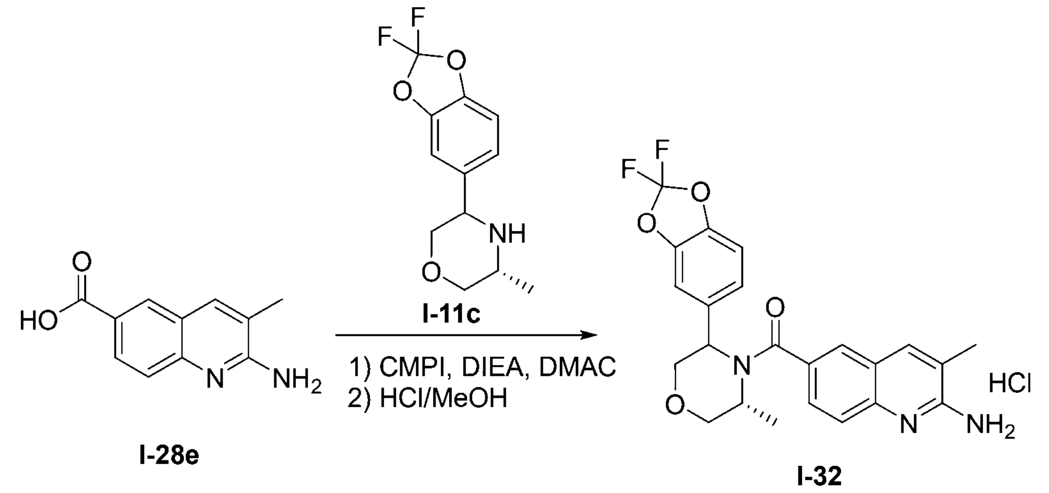


**I-28e** (60 mg, 0.30 mmol), **I-11c** (88 mg, 0.3 mmol), CMPI (92 mg, 0.36 mmol), and DIEA (117 mg, 0.91 mmol) were dissolved in DMAC (5 mL), and the mixture was stirred at 45 °C for 16 hours for reaction. After the reaction was completed, water (20 mL) was added to quench the reaction, and the mixture was extracted with ethyl acetate (30 mL × 3). The ethyl acetate phase was washed with saturated brine (10 mL × 3), dried, filtered, and concentrated. After the residue was purified by column chromatography to give an intermediate, hydrogen chloride in methanol (5 mL) was added, and the mixture was lyophilized to give **I-32** (hydrochloride form, 54 mg, 38%). $^1$H NMR (400 MHz, DMSO-$d_6$) δ 14.38 (s, 1H), 8.65 (s, 2H), 8.31 (s, 1H), 8.01 (d, *J* = 1.7 Hz, 1H), 7.89 - 7.73 (m, 2H), 7.51 (s, 1H), 7.41 (d, *J* = 3.1 Hz, 2H), 5.55 (s, 1H), 4.68 (d, *J* = 12.6 Hz, 1H), 3.84 (dt, *J* = 11.4, 5.7 Hz, 2H), 3.71 (dd, *J* = 11.5, 3.4 Hz, 1H), 3.60 (d, *J* = 11.4 Hz, 1H), 2.32 (s, 3H), 0.80 (d, *J* = 7.0 Hz, 3H). LCMS (ESI) $[M+H]^+$ = 442.2.

**Example I-33:**

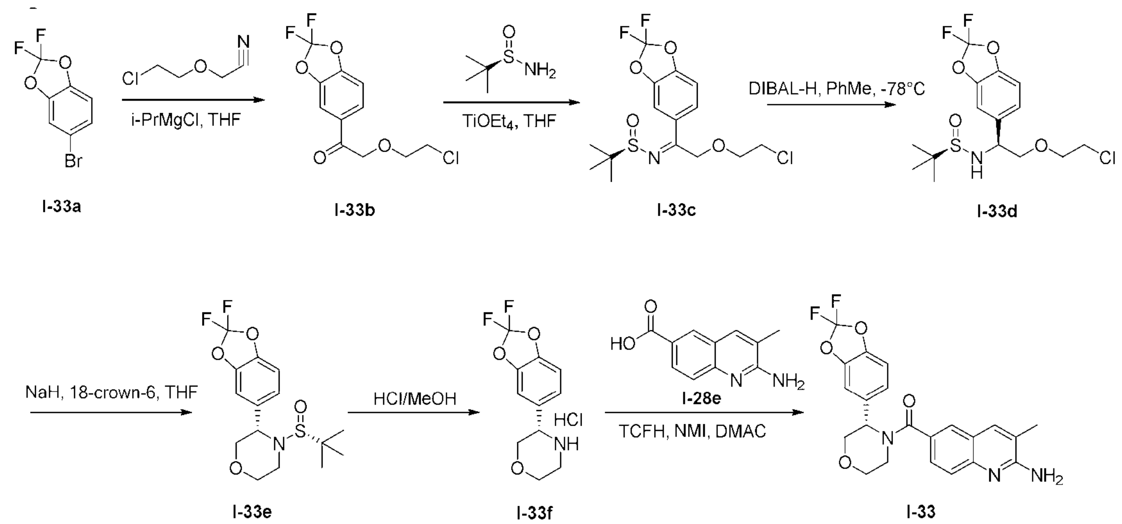


**Step 1**

**I-33a** (30 g, 126.6 mmol) was dissolved in tetrahydrofuran (100 mL). Nitrogen purging was performed before isopropylmagnesium chloride (63.3 mL, 126.6 mmol) was slowly added dropwise at 0 °C. After the dropwise addition was completed, the mixture was warmed to room temperature and stirred for 4 hours for reaction. The reaction mixture was added dropwise to a solution of 2-chloroethoxyacetonitrile (15.1 g, 126.6 mmol) in tetrahydrofuran (100 mL) at 0 °C. After the dropwise addition was completed, the resulting mixture was warmed to room temperature and stirred for another 1 hour for reaction. After the reaction was completed, the reaction liquid was poured into a saturated aqueous ammonium chloride solution (500 mL), and the resulting mixture was extracted with ethyl acetate (300 mL × 2). The ethyl acetate phase was

washed with saturated brine (100 mL × 3), dried, filtered, and concentrated. The residue was purified by column chromatography to give **I-33b** (10 g, 28%).$^{1}$H NMR (400 MHz, DMSO-$d_6$) δ 7.79 (dd, *J* = 8.4, 1.7 Hz, 1H), 7.72 (d, *J*= 1.6 Hz, 1H), 7.15 (d, *J* = 8.4 Hz, 1H), 4.77 (s, 2H), 3.86 (dd, *J* = 5.7, 2.1 Hz, 2H), 3.71 (dd, *J* = 6.1, 5.2 Hz, 2H).

**Step 2**

**I-33b** (10 g, 35.9 mmol) was dissolved in tetrahydrofuran (100 mL), and (R)-(+)-*tert*-butylsulfinamide (5 g, 41.25 mmol) and tetraethyl titanate (24.6 g, 107.7 mmol) were added. The mixture was stirred at 30 °C for 16 hours for reaction. After the reaction was completed, the reaction liquid was diluted by adding ethyl acetate (500 mL). Water (100 mL) was added dropwise to quench the reaction under rapid stirring. After stirring for another 30 min, the resulting mixture was filtered, and the filtrate was separated. The organic phase was washed with saturated brine (100 mL), and the ethyl acetate phase was dried, filtered, and concentrated. The residue was purified by column chromatography to give **I-33c** (7.6 g, 55%).$^{1}$H NMR (400 MHz, $CDCl_3$) δ 7.73 (d, *J* = 8.3 Hz, 1H), 7.65 (s, 1H), 7.10 (d, *J* = 8.5 Hz, 1H), 5.05 (q, *J* = 13.1 Hz, 2H), 3.84 - 3.74 (m, 2H), 3.62 (t, *J* = 5.5 Hz, 2H), 1.33 (s, 9H).

**Step 3**

A 500 mL three-necked flask was sealed and purged with nitrogen three times, and DIBAL-H (24.9 mL, 49.8 mmol) was added. The system was cooled to -78 °C, and a solution of **I-33c** (7.6 g, 19.9 mmol) in toluene (50 mL) was slowly added dropwise. After the dropwise addition was completed, the mixture was stirred at -78 °C for another 1 hour for reaction. After the reaction was completed, the reaction liquid was poured into a saturated aqueous ammonium chloride solution (300 mL), and the resulting mixture was extracted with ethyl acetate (100 mL × 2). The ethyl acetate phase was washed with saturated brine (100 mL × 3), dried, filtered, and concentrated. The residue was purified by column chromatography to give **I-33d** (5.6 g, yield: 73%).$^{1}$H NMR (400 MHz, $CDCl_3$) δ 7.22 (d, *J* = 1.7 Hz, 1H), 7.16 (dd, *J* = 8.2, 1.7 Hz, 1H), 7.07 (d, *J* = 8.2 Hz, 1H), 4.59 (q, *J* = 5.2 Hz, 1H), 4.07 (d, *J* = 5.2 Hz, 1H), 3.81 (d, *J* = 5.3 Hz, 2H), 3.77 (t, *J* = 5.5 Hz, 2H), 3.65 (dd, *J*= 6.2, 4.7 Hz, 2H), 1.26 (s, 9H). LCMS (ESI) $[M+H]^+$ = 384.1.

**Step 4**

**I-33d** (5.1 g, 13.29 mmol) and 18-crown-6 (1.8 g, 6.81 mmol) were dissolved in tetrahydrofuran (50 mL), and the mixture was purged with nitrogen three times. Sodium hydride (1.6 g, 40.4 mmol, 60%) was added batchwise at 0 °C, and the resulting mixture was stirred at room temperature for another 2 hours for reaction. After the reaction was completed, the reaction liquid was poured into a saturated aqueous ammonium chloride solution (200 mL), and the resulting mixture was extracted with ethyl acetate (100 mL × 2). The ethyl acetate phase was washed with saturated brine (100 mL × 3), dried, filtered, and concentrated. The residue was purified by column chromatography to give **I-33e** (3.6 g, 78%). $^{1}$H NMR (400 MHz, $CDCl_3$) δ 7.33 (d, *J* = 1.7 Hz, 1H), 7.26 - 7.22 (m, 1H), 7.06 (d, *J* = 8.3 Hz, 1H), 4.35 (t, *J* = 3.5 Hz, 1H), 4.08 - 3.95 (m, 2H), 3.90 - 3.79 (m, 2H), 3.45 (ddd, *J* = 13.2, 9.3, 3.7 Hz, 1H), 3.12 (dt, *J* = 13.7, 3.3 Hz, 1H), 1.19 (s, 9H). LCMS (ESI) $[M+H]^+$ = 348.1.

**Step 5**

**I-33e** (3.6 g, 10.4 mmol) was dissolved in methanol (20 mL), and a hydrochloric acid/methanol solution (20 mL, 4 mol/L) was added. The mixture was stirred at room temperature for 1 hour for reaction. After the reaction was completed, the reaction liquid was concentrated, and ethyl acetate (5 mL) and petroleum ether (10 mL) were added. The resulting mixture was stirred for 30 min and filtered, and the filter cake was dried to give **I-33f** (2.8 g, 96%).$^{1}$H NMR (400 MHz, DMSO-$d_6$) δ 10.16 (s, 1H), 9.83 (s, 1H), 7.81 (d, *J* = 1.5 Hz, 1H), 7.60 - 7.45 (m, 2H), 4.56 - 4.48 (m, 1H), 4.06 - 3.91 (m, 3H), 3.83 (dd, *J* = 12.3, 10.7 Hz, 1H), 3.34 - 3.28 (m, 1H), 3.25 - 3.15 (m, 1H). LCMS (ESI) $[M+H]^+$ = 244.1.

**Step 6**

**I-33f** (50 mg, 0.25 mmol), **I-28e** (100 mg, 0.36 mmol), TCFH (110 mg, 0.38 mmol), and NMI (62 mg, 0.75 mmol) were dissolved in DMAC (5 mL), and the mixture was stirred at 45 °C for 16 hours for reaction. After the reaction was completed, water (20 mL) was added to quench the reaction. The resulting mixture was extracted with ethyl acetate (30 mL × 3). The ethyl acetate phase was washed with saturated brine (10 mL × 3), dried, filtered, and concentrated. The residue was purified by reversed-phase preparative chromatography to give **I-33** (35 mg, 31%).$^{1}$H NMR (400 MHz, DMSO-$d_6$) δ 7.79 (s, 1H), 7.73 (d, *J* = 1.5 Hz, 1H), 7.48 (d, *J* = 2.2 Hz, 3H), 7.43 (d, *J* = 8.4 Hz, 1H), 7.28 (d, *J* = 8.5 Hz, 1H), 6.53 (s, 2H), 5.38 (d, *J* = 50.0 Hz, 1H), 4.42 (d, *J* = 12.3 Hz, 1H), 3.88 (dd, *J* = 12.3, 3.5 Hz, 1H), 3.81 - 3.77 (m, 1H), 3.57 (td, *J* = 11.6, 2.8 Hz, 2H), 3.24 (*d, J* = 12.9 Hz, 1H), 2.21 (d, *J* = 1.1 Hz, 3H). LCMS (ESI) $[M+H]^+$ = 428.2.

**Example I-7:**

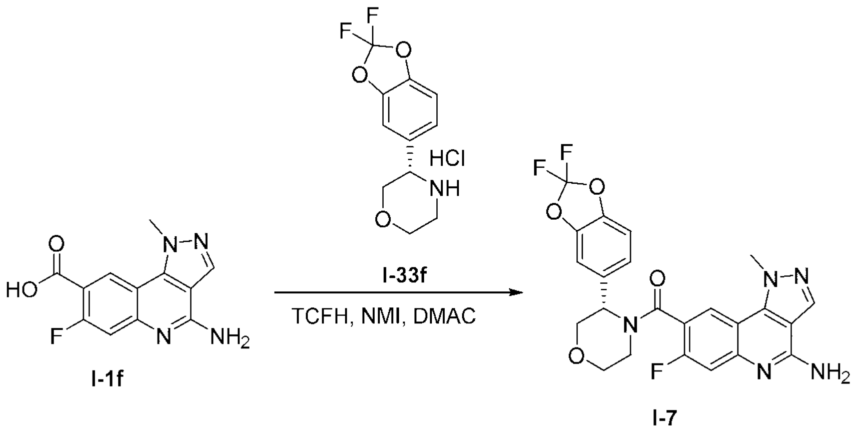


I-33f (2.4 g, 9.22 mmol, HCl), **I-1f** (2.58 g, 9.22 mmol), TCFH (3.88 g, 13.83 mmol), and NMI (2.27 g, 27.66 mmol) were dissolved in DMAC (50 mL), and the mixture was stirred at 45 °C for 16 hours for reaction. After the reaction was completed, water (200 mL) was added to quench the reaction, and the resulting mixture was extracted with ethyl acetate (300 mL × 2). The ethyl acetate phase was washed with saturated brine (100 mL × 3), dried, filtered, and concentrated. The residue was purified by column chromatography to give **I-7** (1.33 g, yield: 30%). $^1H$ NMR (400 MHz, DMSO-$d_6$) δ 8.25 (s, 2H), 7.63 - 7.23 (m, 6H), 5.71 (s, 1H), 4.44 (d, J = 39.6 Hz, 4H), 4.16 - 3.49 (m, 4H), 3.31 - 3.01 (m, 1H). LCMS (ESI) $[M+1]^+$ = 486.2.

**Example I-34:**

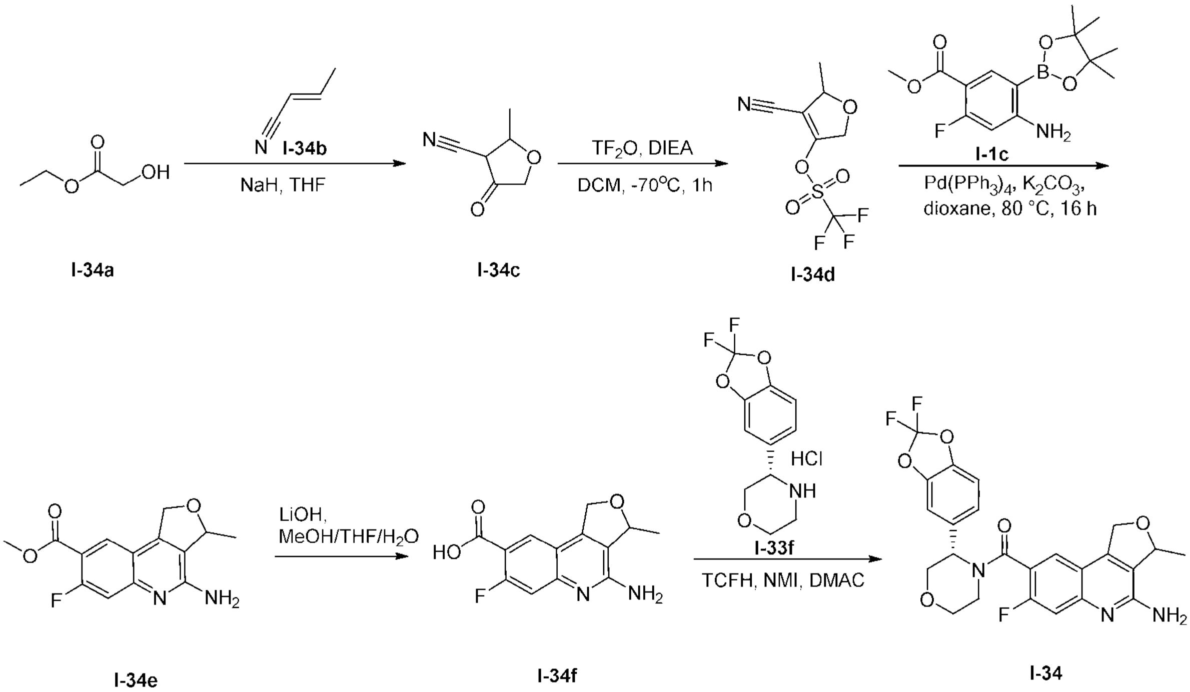


**Step 1**

Sodium hydride (14.9 g, 372.6 mmol, 60%) was dissolved in tetrahydrofuran (1 L), and **I-34a** (77.6 g, 745.3 mmol) was added at room temperature under a nitrogen atmosphere. The reaction liquid was heated to 65 °C, and **I-34b** (50 g,

745.3 mmol) was added. The reaction liquid was stirred at 65 °C for another 2 hours for reaction. After the reaction was completed, the reaction mixture was cooled to room temperature, quenched by adding a 2 N sodium hydroxide solution, and extracted with diethyl ether. The aqueous phase was adjusted to pH 1 with concentrated hydrochloric acid and extracted with dichloromethane. The dichloromethane phase was dried, filtered, and concentrated. The residue was purified by column chromatography to give **I-34c** (19 g, 20%). LCMS (ESI) $[M+H]^+$ = 126.0.

Step **2**

**I-34c** (5 g, 40.0 mmol) was dissolved in dichloromethane (100 mL). Nitrogen purging was performed before DIPEA (10.3 g, 80.0 mmol) and trifluoromethanesulfonic anhydride (15.8 g, 55.9 mmol) were added at - 70 °C. The reaction liquid was stirred at -70 °C for another 30 min for reaction. After the reaction was completed, water (50 mL) was added to quench the reaction, and the resulting mixture was extracted with dichloromethane (100 mL × 2). The dichloromethane phase was dried, filtered, and concentrated to give **I-34d** (9 g, 88%). $^1$HNMR (400 MHz, $CDCl_3$) δ 5.14 - 5.05 (m, 1H), 4.94 - 4.78 (m, 2H), 1.49 (d, J = 6.3 Hz, 3H). LCMS (ESI) $[M+H]^+$ = 258.0.

Step 3

**I-34d** (27 g, 105.0 mmol), **I-1c** (24.8 g, 84.0 mmol), and tetrakis(triphenylphosphine)palladium(0) (4.9 g, 4.2 mmol) were dissolved in a mixed solution of dioxane (360 mL)/water (36 mL), and potassium carbonate (43.5 g, 314.9 mmol) was added. The reaction liquid was stirred at 80 °C for 16 hours for reaction under a nitrogen atmosphere. After the reaction was completed, the reaction mixture was concentrated. The residue was purified by column chromatography to give **I-34e** (5.5 g, 19%). $^1$HNMR (400 MHz, DMSO-$d_6$) δ 8.04 (d, J = 8.1 Hz, 1H), 7.26 (d, J = 13.4 Hz, 1H), 7.03 (s, 2H), 5.46 - 5.21 (m, 3H), 3.85 (s, 3H), 1.39 (d, J = 6.0 Hz, 3H). LCMS (ESI) $[M+H]^+$ = 277.2.

Step **4**

**I-34e** (300 mg, 1.1 mmol) was dissolved in a mixed solution of methanol (5 mL)/tetrahydrofuran (5 mL)/water (3 mL), and lithium hydroxide (78 mg, 3.3 mmol) was added. The reaction liquid was heated to 50 °C and stirred for 20 hours for reaction. After the reaction was completed, the reaction mixture was diluted by adding water and adjusted to pH 5 with a 1 N hydrochloric acid solution. A solid was precipitated, and after filtration, the solid was dried to give intermediate **I-34f** (240 mg, 84%). $^1$H NMR (400 MHz, DMSO-$d_6$) δ 13.64 (s, 1H), 9.00 (s, 2H), 8.21 (d, J = 7.5 Hz, 1H), 7.68 (d, J = 11.6 Hz, 1H), 5.57 (ddd, J = 6.0, 3.7, 2.2 Hz, 1H), 5.51 - 5.35 (m, 2H), 1.42 (d, J = 6.1 Hz, 3H).

**Step 5**

**I-34f** (60 mg, 0.23 mmol), **I-33f** (92 mg, 0.33 mmol), TCFH (97 mg, 0.35 mmol), and NMI (57 mg, 0.69 mmol) were dissolved in DMAC (5 mL), and the mixture was reacted at 45 °C for 16 hours. After the reaction was completed, the reaction liquid was added dropwise to water, and the resulting mixture was extracted with ethyl acetate. The ethyl acetate phase was washed with saturated brine, dried over anhydrous sodium sulfate, filtered, and concentrated. The residue was purified by column chromatography to give **I-34** (48 mg, 42%). $^1$H NMR (400 MHz, DMSO-$d_6$) δ 7.65 (s, 1H), 7.58 - 7.03 (m, 4H), 6.77 (s, 2H), 5.68 (s, 1H), 5.49 - 5.17 (m, 3H), 4.82 - 4.22 (m, 2H), 3.95 - 3.70 (m, 2H), 3.51 (s, 1H), 3.27 (s, 1H), 1.39 (d, *J* = 6.1 Hz, 3H). LCMS (ESI) $[M+H]^+$ = 488.2.

**Example I-35:**

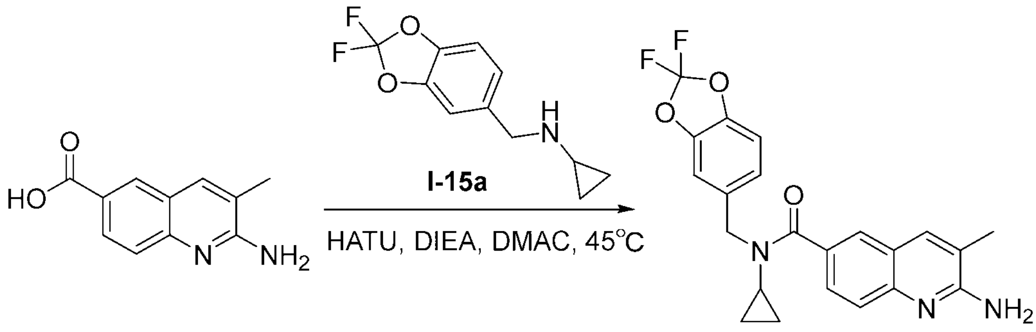


I-28e I-35

**I-28e** (60 mg, 0.30 mmol), **I-15a** (85 mg, 0.37 mmol), HATU (141 mg, 0.37 mmol), and DIPEA (117 mg, 0.91 mmol) were dissolved in DMAC (1.5 mL), and the mixture was reacted at 45 °C for 16 hours. After the reaction was completed, the reaction liquid was filtered and concentrated. The residue was purified by column chromatography to give **I-35** (79 mg, 59%). $^1$H NMR (400 MHz, DMSO-$d_6$) δ7.91 - 7.83 (m, 2H), 7.64 (dd, *J*= 8.6, 1.9 Hz, 1H), 7.48 (d, *J* = 8.6 Hz, 1H), 7.44 - 7.36 (m, 2H), 7.20 (d, *J* = 8.2 Hz, 1H), 6.78 (s, 2H), 4.70 (s, 2H), 2.84 (d, *J* = 9.5 Hz, 1H), 2.22 (s, 3H), 0.56 - 0.36 (m, 4H). LCMS (ESI) $[M+H]^+$ = 412.2.

**Example I-36:**

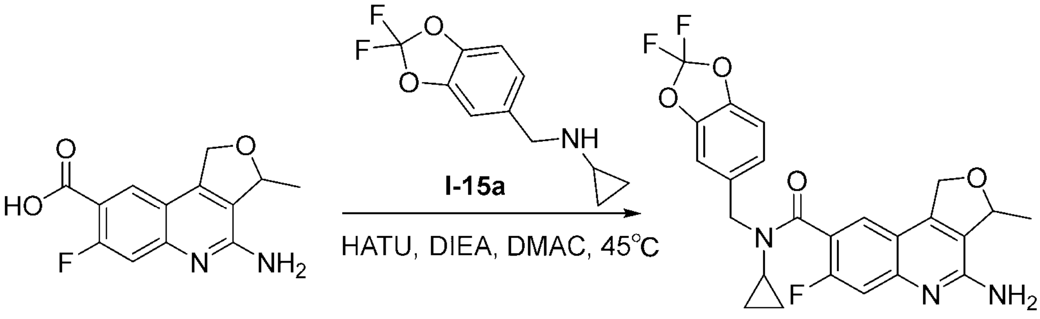


I-34f I-36

**I-34f** (60 mg, 0.23 mmol), **I-15a** (65 mg, 0.29 mmol), HATU (110 mg, 0.29 mmol), and DIPEA (90 mg, 0.70 mmol) were dissolved in DMAC (1.5 mL), and the mixture was reacted at 45 °C for 16 hours. After the reaction was completed, the reaction liquid was filtered, and the filtrate was concentrated. The residue was purified by column chromatography to give **I-36** (47 mg, 41%). $^1$H NMR (400 MHz, DMSO-$d_6$) δ 7.63 (d, *J* = 7.6 Hz, 1H), 7.43 *(d, J=* 8.2 Hz, 1H), 7.37 (s, 1H), 7.28 *(d, J=* 12.1 Hz, 1H), 7.21 *(d, J=* 8.2 Hz, 1H), 6.80 (s, 2H), 5.46 - 5.19 (m, 3H), 4.73 (s, 2H), 2.67 (s, 1H), 2.08 (s, 0H), 1.40 (d, *J* = 6.1 Hz, 3H), 0.54 - 0.33 (m, 4H). LCMS (ESI) $[M+H]^+$ = 472.2.

**Example I-37:**

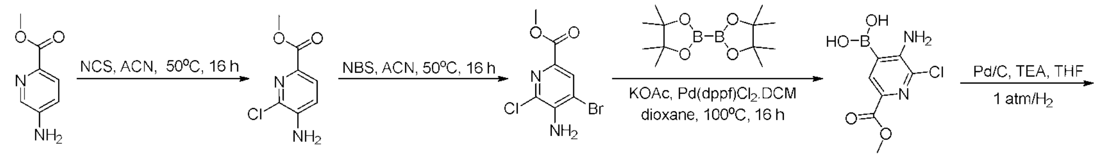


I-37a I-37b I-37c I-37d

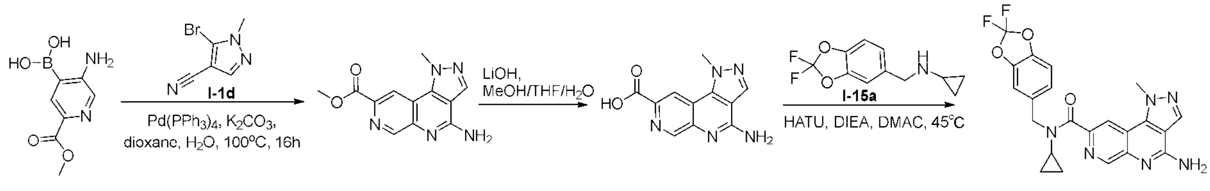


I-37e I-37f I-37g I-37

**Step 1**

**I-37a** (84 g, 552.1 mmol) was dissolved in acetonitrile (1.5 L), and a solution of NCS (73.7 g, 552.1 mmol) in acetonitrile (500 mL) was added. The reaction liquid was heated to 50 °C and stirred for 16 hours for reaction. After the reaction was completed, the reaction liquid was concentrated, and the residue was purified by column chromatography to give **I-37b** (73 g, 71%).

**Step 2**

**I-37b** (74 g, 396.6 mmol) was dissolved in acetonitrile (1.5 L), and a solution of NBS (70.6 g, 396.6 mmol) in acetonitrile (500 mL) was added. The reaction liquid was heated to 50 °C and stirred for 16 hours for reaction. After the reaction was completed, the reaction mixture was filtered, and the filtrate was concentrated. The residue was purified by column chromatography to give **I-37c** (86.0 g, 82%). LCMS (ESI) $[M+H]^+$ = 264.9.

**Step 3**

**I-37c** (47 g, 177 mmol) and bis(pinacolato)diboron (89.9 g, 354 mmol) were dissolved in DMF (750 mL), and potassium acetate (42.3 g, 354 mmol) and Pd(dppf)$Cl_2$ dichloromethane complex (15.9 g, 177 mmol) were added. The reaction liquid was heated to 100 °C and stirred for 16 hours for reaction under a nitrogen atmosphere. After the reaction was completed, the reaction mixture was filtered, and the filtrate was concentrated. The residue was purified by column chromatography to give **I-37d** (33 g, 47%). LCMS (ESI) $[M+H]^+$ = 230.9.

**Step 4**

**I-37d** (36.7 g, 117 mmol) was dissolved in tetrahydrofuran (400 mL), and triethylamine (35.6 g, 352 mmol) and 10% Pd/C (12.5 g, 11.7 mmol) were added. Hydrogen purging was performed before the mixture was stirred at 40 °C for 1 hour for reaction. After the reaction was completed, the reaction mixture was filtered, and the filtrate was concentrated to give **I-37e** (27.5 g, crude product), which was directly used in the next step. LCMS (ESI) $[M+H]^+$ = 196.9.

**Step 5**

**I-37e** (27.3 g, 139 mmol) and **I-1d** (25.9 g, 139 mmol) were dissolved in a mixed solution of dioxane (350 mL) and water (35 mL), and potassium carbonate (56.7 g, 417.2 mmol) and $Pd(PPh_3)_4$ (8 g, 6.95 mmol) were added. The reaction liquid was stirred at 100 °C for 16 hours for reaction under a nitrogen atmosphere. After the reaction was completed, the reaction liquid was filtered, and the filtrate was concentrated. The residue was purified by column chromatography to give **I-37f** (10.9 g, 31%). $^1$H NMR (400 MHz, DMSO-$d_6$) $\delta$ 8.90 (s, 1H), 8.74 (s, 1H), 8.37 (s, 1H), 7.67 (s, 2H), 4.46 (s, 3H), 3.91 (s, 3H). LCMS (ESI) $[M+H]^+$ = 257.9.

**Step 6**

**I-37f** (200 mg, 0.78 mmol) was dissolved in a mixed solution of methanol (5 mL)/tetrahydrofuran (5 mL)/water (3 mL), and lithium hydroxide (56 mg, 2.34 mmol) was added. The reaction liquid was stirred at 50 °C for 20 hours for reaction. After the reaction was completed, the reaction mixture was diluted by adding water and adjusted to pH 5 with a 1 N

hydrochloric acid solution. A solid was precipitated, and after filtration, the solid was dried to give **I-37g** (180 mg, 95%).$^1$H NMR (400 MHz, DMSO-$d_6$) δ 10.61 (s, 1H), 9.23 (s, 1H), 9.14 (s, 1H), 8.97 (s, 1H), 8.77 (s, 1H), 4.52 (s, 3H).

**Step 7**

**I-37g** (60 mg, 0.25 mmol), **I-15a** (70 mg, 0.30 mmol), HATU (114 mg, 0.30 mmol), and DIPEA (97 mg, 0.75 mmol) were dissolved in DMAC (2 mL), and the reaction liquid was stirred at 45 °C for 16 hours for reaction. After the reaction was completed, the reaction mixture was filtered, and the filtrate was concentrated. The residue was purified by column chromatography to give **I-37** (12 mg, 10%).$^1$H NMR (400 MHz, DMSO-$d_6$) δ 8.85 (s, 1H), 8.35 (s, 1H), 8.29 (s, 1H), 7.43 (d, $J$ = 8.1 Hz, 4H), 7.26 (s, 1H), 4.76 (s, 2H), 4.43 (s, 3H), 2.92 (s, 1H), 0.44 (s, 2H), 0.39 (d, $J$ = 8.3 Hz, 2H). LCMS (ESI) $[M+H]^+$ = 453.2.

**Example I-38:**

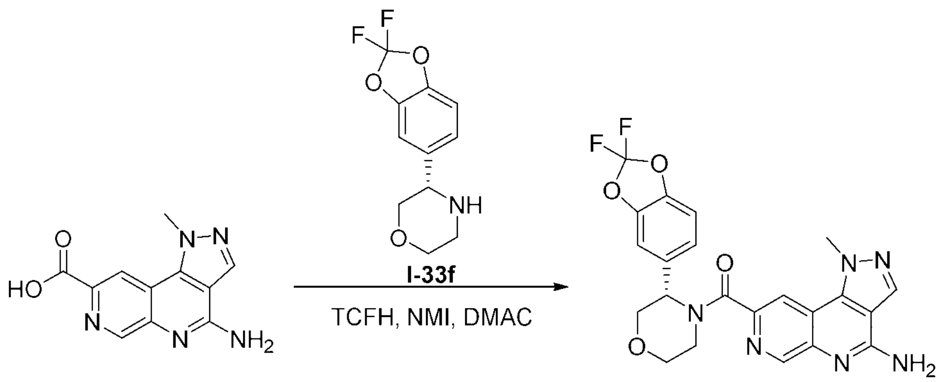


I-37g I-38

**I-37g** (60 mg, 0.25 mmol), **I-33f** (70 mg, 0.25 mmol), TCFH (110 mg, 0.38 mmol), and NMI (62 mg, 0.75 mmol) were dissolved in DMAC (5 mL). The reaction liquid was stirred at 45 °C for 16 hours for reaction. After the reaction was completed, the reaction liquid was poured into water. The resulting mixture was extracted with ethyl acetate (30 mL × 3). The ethyl acetate phase was washed with saturated brine, dried over anhydrous sodium sulfate, filtered, and concentrated. The residue was purified by column chromatography to give **I-38** (80 mg, 69%).$^1$H NMR (400 MHz, DMSO-$d_6$) δ 8.92 (s, 1H), 8.45 (d, $J$ = 4.2 Hz, 2H), 8.08 (s, 2H), 7.71 - 7.20 (m, 3H), 5.58 (d, $J$ = 93.5 Hz, 1H), 4.46 (s, 4H), 3.99 - 3.54 (m, 4H), 2.98 (d, $J$ = 29.0 Hz, 1H). LCMS (ESI) $[M+H]^+$ = 469.2.

**Example I-39:**

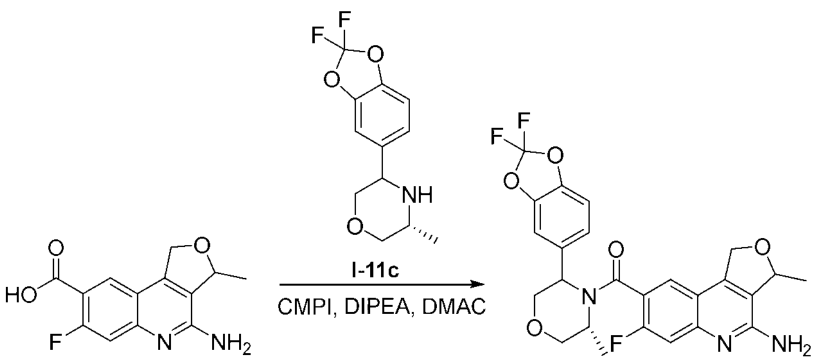


I-34f I-39

**I-34f** (60 mg, 0.23 mmol), **I-11c** (70 mg, 0.24 mmol), DIPEA (90 mg, 0.7 mmol), and CMPI (71 mg, 0.28 mmol) were dissolved in DMAC (5 mL). The reaction liquid was stirred at 45 °C for 16 hours for reaction. After the reaction was

completed, the reaction liquid was poured into water. The resulting mixture was extracted with ethyl acetate (30 mL × 3). The ethyl acetate phase was washed with saturated brine, dried over anhydrous sodium sulfate, filtered, and concentrated. The residue was purified by column chromatography to give **I-39** (20 mg, 17%).$^1$H NMR (400 MHz, DMSO-$d_6$) δ 7.67 (d, $J$ = 7.6 Hz, 1H), 7.53 (s, 1H), 7.43 (s, 2H), 7.32 (d, $J$ = 11.8 Hz, 1H), 6.74 (s, 2H), 5.68 - 5.37 (m, 2H), 5.36 - 5.19 (m, 2H), 4.69 (d, $J$ = 12.7 Hz, 1H), 3.73 (d, $J$ = 40.1 Hz, 4H), 1.39 (d, $J$ = 6.2 Hz, 3H), 0.77 (s, 3H). LCMS (ESI) [M+H]$^+$ = 502.2.

**Example I-40:**

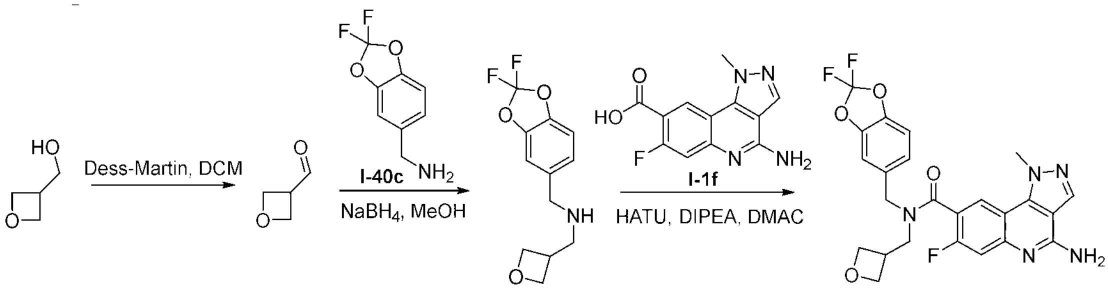


I-40a I-40b I-40d I-40

**Step 1**

**I-40a** (100 mg, 1.13 mmol) was dissolved in DCM (5 mL), and Dess-Martin reagent (480 mg, 1.13 mmol) was added. The reaction liquid was stirred at room temperature for 1 hour for reaction. After the reaction was completed, the reaction liquid was diluted by adding petroleum ether (15 mL). After stirring for another 10 min, the mixture was filtered, and the filtrate was concentrated. The residue was purified by column chromatography to give **I-40b** (90 mg, 94%).$^1$H NMR (400 MHz, $CDCl_3$) δ 9.96 (d, $J$ = 2.4 Hz, 1H), 4.92 - 4.83 (m, 4H), 3.82 (ttd, $J$ = 8.4, 6.1, 2.4 Hz, 1H).

**Step 2**

**I-40b** (78 mg, 0.9 mmol) and **I-40c** (100 mg, 0.53 mmol) were dissolved in methanol (5 mL). After the mixture was stirred at room temperature for 1 hour for reaction, sodium borohydride (20 mg, 0.53 mmol) was added at 0 °C. The reaction liquid was stirred at room temperature for another 1 hour for reaction. After the reaction was completed, the reaction liquid was quenched by adding water, and the resulting mixture was extracted with ethyl acetate (20 mL × 3). The ethyl acetate phase was washed with saturated brine, dried over anhydrous sodium sulfate, filtered, and concentrated. The residue was purified by column chromatography to give **I-40d** (100 mg, 72%). LCMS (ESI) [M+H]$^+$ = 258.1.

**Step 3**

**I-40d** (59 mg, 0.23 mmol), **I-1f** (60 mg, 0.23 mmol), and DIPEA (80 mg, 0.7 mmol) were dissolved in DMAC (5 mL), and HATU (100 mg, 0.28 mmol) was added. The reaction liquid was stirred at 45 °C for 16 hours for reaction. After the reaction was completed, water (20 mL) was added to quench the reaction. The resulting mixture was extracted with ethyl acetate (30 mL × 2). The ethyl acetate phase was washed with saturated brine (10 mL × 3), dried, filtered, and concentrated. The residue was purified by column chromatography to **give I-40** (26 mg, 21%).$^1$H NMR (400 MHz, DMSO-$d_6$) δ 8.31 - 8.11 (m, 2H), 7.50 - 7.01 (m, 6H), 4.85 - 4.56 (m, 2H), 4.48 (s, 1H), 4.43 - 4.19 (m, 5H), 3.99 (t, $J$ = 6.3 Hz, 1H), 3.65 (dd, $J$ = 74.2, 13.0 Hz, 3H). LCMS (ESI) [M+H]$^+$ = 500.2.

**Example I-41:**

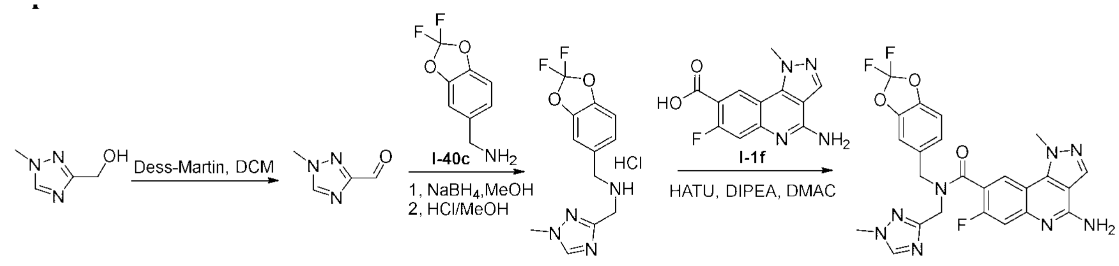


I-41a I-41b I-41c I-41

**Step 1**

**I-41a** (300 mg, 2.7 mmol) was dissolved in DCM (10 mL), and Dess-Martin reagent (1.24 g, 2.9 mmol) was added. The reaction liquid was stirred at room temperature for 1 hour for reaction. After the reaction was completed, the reaction liquid was diluted by adding petroleum ether (15 mL). After stirring for another 10 min, the mixture was filtered, and the filtrate was concentrated. The residue was purified by column chromatography to give **I-41b** (255 mg, 86%). $^1$H NMR (400 MHz, DMSO-$d_6$) δ 9.89 (d, *J* = 0.6 Hz, 1H), 8.72 (s, 1H), 4.00 (s, 3H).

**Step 2**

**I-41b** (100 mg, 0.9 mmol) and **I-40c** (100 mg, 0.5 mmol) were dissolved in methanol (5 mL). After the mixture was stirred at room temperature for 1 hour, sodium borohydride (20 mg, 0.5 mmol) was slowly added at 0 °C. The mixture was stirred at room temperature for another 1 hour for reaction. After the reaction was completed, water was added to quench the reaction, and the resulting mixture was extracted with ethyl acetate (30 mL × 3). The ethyl acetate phase was washed with saturated brine, dried over anhydrous sodium sulfate, filtered, and concentrated. The residue was purified by column chromatography to give a crude product, which was then dissolved in a hydrogen chloride/methanol (5 mL) solution. The mixture was stirred for 30 min and then concentrated to give **I-41c** (150 mg, 88%). $^1$H NMR (400 MHz, DMSO-$d_6$) δ 9.93 (s, 2H), 8.60 (s, 1H), 7.65 (d, *J* = 1.6 Hz, 1H), 7.48 (d, *J* = 8.3 Hz, 1H), 7.40 (dd, *J* = 8.3, 1.7 Hz, 1H), 4.30 - 4.23 (m, 2H), 4.19 - 4.12 (m, 2H), 3.89 (s, 3H).

**Step 3**

**I-41c** (88 mg, 0.3 mmol), **I-1f** (60 mg, 0.23 mmol), and DIPEA (80 mg, 0.7 mmol) were dissolved in DMAC (5 mL), and HATU (100 mg, 0.28 mmol) was added. The reaction liquid was stirred at 45 °C for 16 hours for reaction. After the reaction was completed, water (20 mL) was added to quench the reaction, and the resulting mixture was extracted with ethyl acetate (30 mL × 2). The ethyl acetate phase was washed with saturated brine (10 mL × 3), dried over anhydrous sodium sulfate, filtered, and concentrated. The residue was purified by column chromatography to give **I-41** (64 mg, 50%). $^1$H NMR (400 MHz, DMSO-$d_6$) δ 8.48 (s, 1H), 8.46 - 8.39 (m, 1H), 8.25 (s, 1H), 7.42 (d, *J* = 8.2 Hz, 1H), 7.38 - 7.24 (m, 4H), 7.22 - 6.97 (m, 1H), 4.65 (d, *J* = 37.6 Hz, 2H), 4.45 - 4.17 (m, 5H), 3.84 (d, *J* = 13.8 Hz, 3H). LCMS (ESI) $[M+H]^+$ = 525.2.

**Example I-34 (PEAK1) and I-34 (PEAK2):**

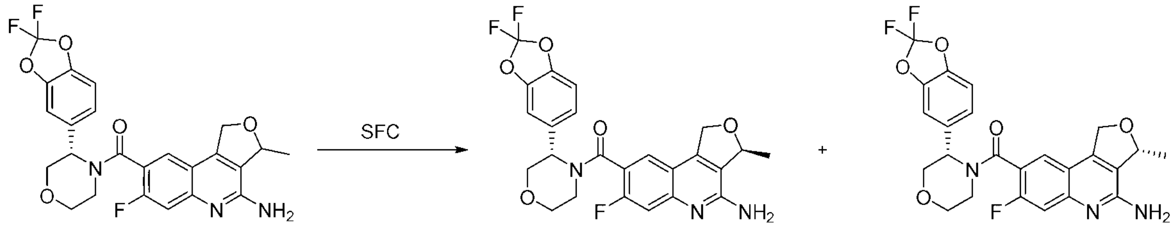


I-34

**I-34** was purified by preparative SFC (Shimadzu E-UC, IA-E-D-30-8MIN, mobile phase A: $CO_2$, mobile phase B: EtOH (0.05% DEA)) to give **I-34** (PEAK 1, Rt = 3.106 min) and **I-34** (PEAK 2, Rt = 4.205 min).

**I-34** (PEAK 1): $^1$H NMR (400 MHz, DMSO-$d_6$) δ 7.66 (s, 1H), 7.56 - 7.37 (m, 2H), 7.37 - 7.20 (m, 2H), 6.78 (s, 2H),

5.68 (s, 1H), 5.45 - 5.20 (m, 3H), 4.77 - 4.27 (m, 2H), 3.92 - 3.70 (m, 2H), 3.56 - 3.45 (m, 1H), 3.26 (s, 1H), 1.39 (d, *J* = 6.1 Hz, 3H). LCMS (ESI) $[M+H]^+$ = 488.2

**I-34** (PEAK 2): $^1$H NMR (400 MHz, DMSO-$d_6$) δ 7.66 (s, 1H), 7.56 - 7.37 (m, 2H), 7.37 - 7.20 (m, 2H), 6.78 (s, 2H), 5.68 (s, 1H), 5.45 - 5.20 (m, 3H), 4.77 - 4.27 (m, 2H), 3.92 - 3.70 (m, 2H), 3.56 - 3.45 (m, 1H), 3.26 (s, 1H), 1.39 (d, *J* = 6.1 Hz, 3H). LCMS (ESI) $[M+H]^+$ = 488.2.

**Example I-53**

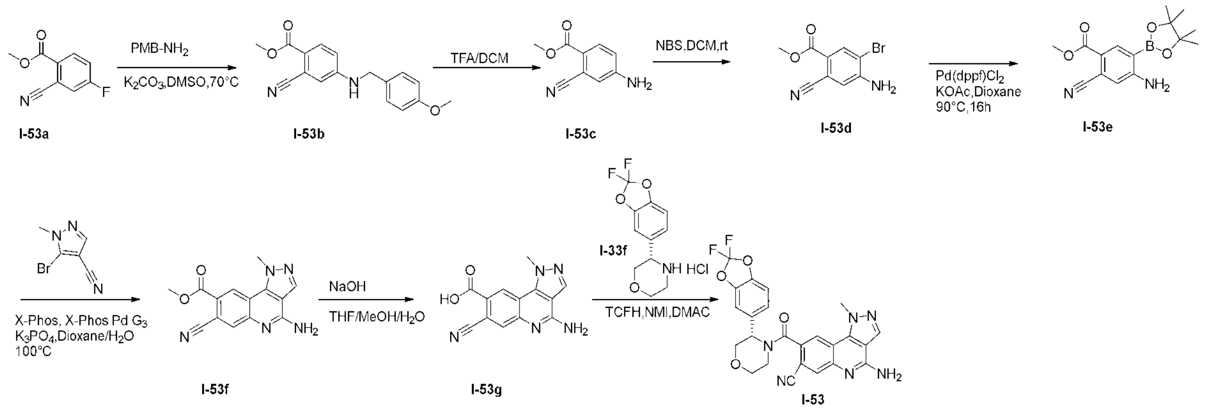


**Step 1**

I-53a (5 g, 27.91 mmol) was added to dry DMSO (30 mL), and 4-methoxybenzylamine (4.59 g, 33.49 mmol) and potassium carbonate (7.71 g, 55.82 mmol) were added. The mixture was reacted at 70 °C for 16 hours. After the reaction was completed as monitored by TLC, the reaction liquid was added to water, and the resulting mixture was extracted with ethyl acetate. The organic phase was washed 5 times with saturated brine, dried over anhydrous sodium sulfate, and separated by column chromatography to give I-53b (5 g, 60%) as a white solid. $^1$H NMR (400 MHz, DMSO-$d_6$) δ 7.80 (d, *J* = 8.9 Hz, 1H), 7.51 (t, *J* = 5.8 Hz, 1H), 7.30 - 7.24 (m, 2H), 7.02 (d, *J* = 2.4 Hz, 1H), 6.96 - 6.83 (m, 3H), 4.31 (d, *J* = 5.8 Hz, 2H), 3.79 (s, 3H), 3.73 (s, 3H).

**Step 2**

I-53b (4000 mg, 13.55 mmol) was dissolved in DCM (20 mL), and TFA (20 mL) was added dropwise. The mixture was reacted at 25 °C for 2 hours. After the reaction was completed as monitored by TLC, the reaction liquid was added dropwise to a 1 N aqueous NaOH solution, and the resulting mixture was extracted 2 times with dichloromethane. The organic phases were combined, dried over anhydrous sodium sulfate, and separated by column chromatography to give I-53c (2.2 g, 93%) as a white solid. $^1$H NMR (400 MHz, DMSO-$d_6$) δ 7.78 (d, *J* = 8.8 Hz, 1H), 6.96 (d, *J* = 2.3 Hz, 1H), 6.81 (dd, *J* = 8.8, 2.4 Hz, 1H), 6.47 (s, 2H), 3.79 (s, 3H).

**Step 3**

I-53c (2.7 g, 15.33 mmol) was dissolved in dichloromethane (30 mL), and NBS (2730 mg, 15.33 mmol) was added. The mixture was reacted at 25 °C for 2 hours. After the reaction was completed as monitored by TLC, an aqueous sodium thiosulfate solution was added to quench the reaction, and the resulting mixture was extracted with dichloromethane. The organic phase was washed with saturated brine, dried over anhydrous sodium sulfate, and separated by column chromatography to give I-53d (530 mg, 35%) as an off-white solid. $^1$H NMR (400 MHz, DMSO-$d_6$) δ 8.04 (s, 1H), 7.16 (s, 1H), 6.69 (s, 2H), 3.81 (s, 3H).

**Step 4**

I-53d (100 mg, 0.39 mmol), bis(pinacolato)diboron (200 mg, 0.78 mmol), potassium acetate (110 mg, 1.17 mmol), and Pd(dppf)$Cl_2$ (29 mg, 0.039 mmol) were dissolved in dry dioxane (5 mL), and the mixture was purged with nitrogen 3 times and reacted at 90 °C for 16 hours. After the reaction was completed as monitored by TLC, the reaction mixture was diluted by adding water, and the resulting mixture was extracted with ethyl acetate. The organic phase was

washed with saturated brine, dried over anhydrous sodium sulfate, and concentrated to dryness by rotary evaporation to give I-53e (110 mg, 93%) as a brown oil.

**Step 5**

I-53e (110 mg, 0.36 mmol), 5-bromo-1-methyl-1H-pyrazole-4-carbonitrile (67 mg, 24.49 mmol), potassium phosphate (230 mg, 1.08 mmol), X-Phos (18 mg, 0.036 mmol), and X-Phos Pd G3 (31 mg, 0.036 mmol) were dissolved in dioxane (4 mL) and water (1 mL), and the mixture was purged with nitrogen 3 times and reacted at 100 °C for 16 hours. After the reaction was completed as monitored by LCMS, the reaction mixture was diluted by adding water, and the resulting mixture was extracted with ethyl acetate. The organic phase was washed with saturated brine, dried over anhydrous sodium sulfate, and separated by column chromatography to give I-53f (100 mg, yield: 98%) as a yellowish-green solid. $^1$H NMR (400 MHz, DMSO-$d_6$) δ 8.19 (s, 1H), 7.80 (s, 1H), 7.25 (s, 1H), 6.66 (s, 2H), 3.81 (s, 3H), 3.67 (s, 3H). LCMS ESI (m/z) =282.2 $[M+H]^+$.

**Step 6**

I-53f (110 mg, 0.39 mmol) was dissolved in methanol (5 mL) and tetrahydrofuran (5 mL), and water (2 mL) and sodium hydroxide (49 mg, 1.17 mmol) were added. The mixture was reacted at 50 °C for 20 hours. After the reaction was completed as monitored by LCMS, the reaction mixture was adjusted to pH 5 with 1 N hydrochloric acid and concentrated to dryness by rotary evaporation to remove the organic phase. A large amount of solid was precipitated. The resulting mixture was filtered, and the filter cake was dried to give I-53g (45 mg, 43%) as a gray solid. LCMS ESI (m/z) = 268.1 $[M+H]^+$.

**Step 7**

I-53g (40 mg, 0.15 mmol), I-33f (42 mg, 0.15 mmol), and NMI (37 mg, 0.45 mmol) were dissolved in DMAC (1 mL). The mixture was stirred and cooled to 0 °C, and TCFH (63 mg, 0.22 mmol) was added. After the addition was completed, the mixture was reacted at room temperature for 16 hours. After the reaction was completed as monitored by LCMS, the reaction mixture was separated by reversed-phase preparative chromatography to give I-53 (41 mg, 56%). ESI (m/z) = 493.2 $[M+H]^+$. $^1$H NMR (400 MHz, DMSO-$d_6$) δ 8.37 - 8.25 (m, 2H), 8.02 (s, 1H), 7.62 - 7.36 (m, 5H), 5.83 - 4.82 (m, -1H), 4.58 - 3.58 (m, 8H), 3.27 - 3.14 (m, 1H).

**Example I-54**

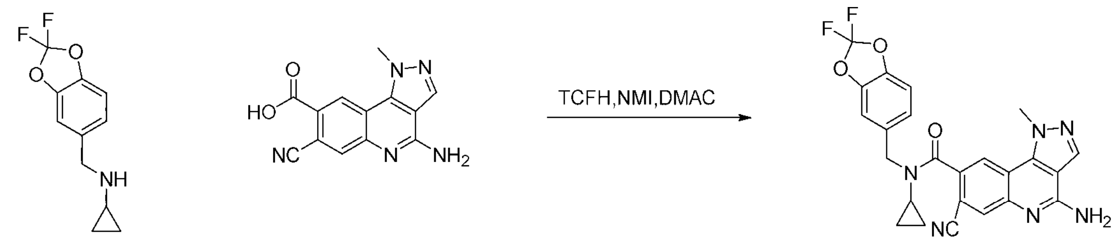


I-15a I-53g I-54

I-15a (34 mg, 0.15 mmol), I-53g (40 mg, 0.15 mmol), TCFH (63 mg, 0.22 mmol), NMI (37 mg, 0.45 mmol), and DMAC (1 mL) were mixed, and the mixture was reacted at 45 °C for 3 hours. After the reaction was completed as monitored by LCMS, the reaction mixture was separated by reversed-phase preparative chromatography to give I-54 (18.8 mg, 25%).$^1$H NMR (400 MHz, DMSO-$d_6$) δ 8.38 (s, 1H), 8.33 (s, 1H), 8.01 (s, 1H), 7.46 (s, 3H), 7.41 (d, *J* = 8.2 Hz, 1H), 7.29 (s, 1H), 4.76 (s, 2H), 4.41 (s, 3H), 2.79 (s, 1H), 2.54 (s, 0H), 0.49 (d, *J* = 25.2 Hz, 4H). LCMS (ESI) [M+1]+ = 477.2.

**Example I-80**

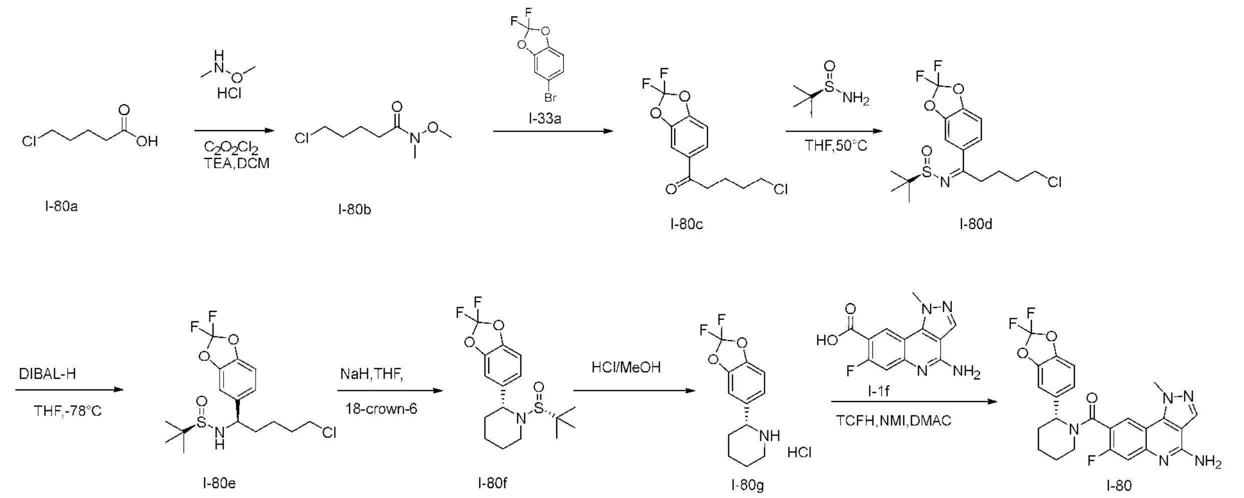


**Step 1**

I-80a (5 g, 36.61 mmol) and dry DCM (20 mL) were added to a 100 mL single-necked flask and cooled to 0 °C, and oxalyl chloride (9.29 g, 73.22 mmol) was added, followed by dropwise addition of DMF (0.5 mL). After the addition was completed, the mixture was reacted at room temperature for 3 hours. After the reaction was completed as detected by TLC, the reaction liquid was concentrated to dryness by rotary evaporation, and then diluted by adding 100 mL of dry DCM. The resulting mixture was designated as solution 1. Dimethylhydroxylamine hydrochloride (3.57 g, 36.61 mmol) and dry DCM (10 mL) were added to another 250 mL single-necked flask, and triethylamine (11.11 g, 109.83 mmol) was added under rapid stirring. After the dropwise addition was completed, solution 1 was added dropwise while the temperature was controlled at 0 °C. After the dropwise addition was completed, the mixture was warmed to room temperature and reacted for 1 hour. After the reaction was completed, the reaction liquid was poured into a saturated aqueous sodium bicarbonate solution (100 mL). The organic phase was washed with saturated brine (100 mL × 3), dried, and concentrated to give an oil. The oil was added to 50 mL of tetrahydrofuran, and the resulting mixture was quickly stirred for 30 min and filtered. The filtrate was concentrated to dryness by rotary evaporation to give **I-80b** (4.5 g, yield: 68%) as an oil. LCMS (ESI) $[M+1]^+$ = 180.1.

**Step 2**

I-33a (5 g, 21.1 mmol) and THF (20 mL) were added to a 100 mL three-necked flask, and the mixture was purged with nitrogen three times. Isopropylmagnesium chloride (12.66 mL, 25.31 mmol) was slowly added dropwise while the temperature was controlled at 0 °C. After the dropwise addition was completed, the resulting mixture was warmed to room temperature and reacted for 3 hours. A solution of I-80b (4 g, 22.16 mmol) in THF (5 mL) was then slowly added dropwise. After the dropwise addition was completed, the mixture was stirred at room temperature for 1 hour for reaction. After the reaction was completed, the reaction liquid was poured into a saturated aqueous ammonium chloride solution (100 mL), and the resulting mixture was extracted with ethyl acetate (50 mL × 2). The ethyl acetate phase was washed with saturated brine (50 mL × 3), dried, filtered, and concentrated. The residue was purified by column chromatography to give I-80c (3.5 g, yield: 60%). $^1$H NMR (400 MHz, $CDCl_3$) δ 7.77 (dd, *J* = 8.4, 1.7 Hz, 1H), 7.69 (d, *J* = 1.6 Hz, 1H), 7.13 (d, *J* = 8.4 Hz, 1H), 3.58 (t, *J* = 6.0 Hz, 2H), 2.98 (t, *J* = 6.7 Hz, 2H), 1.96 - 1.82 (m, 4H).

**Step 3**

I-80c (3 g, 10.84 mmol), THF (50 mL), and R-tert-butylsulfinamide (1.71 g, 14.09 mmol) were added to a 100 mL single-necked flask, and tetraethyl titanate (7.42 g, 32.52 mmol) was added under stirring. The mixture was stirred at 50 °C for 16 hours for reaction. After the reaction was completed, the reaction liquid was diluted by adding THF (200 mL), and saturated brine (10 mL) was added dropwise under rapid stirring to quench the reaction. After the dropwise addition was completed, the resulting mixture was stirred for 30 min and filtered. The filtrate was separated and rinsed with THF (100 mL). The organic phase was dried, filtered, and concentrated. The residue was purified by column chromatography to give I-80d (3 g, yield: 72.8%). $^1$H NMR (400 MHz, DMSO-$d_6$) δ 7.88 (s, 1H), 7.80 (d, *J* = 8.6 Hz, 1H), 7.53 (d, *J* = 8.5 Hz, 1H), 3.64 (t, *J* = 6.5 Hz, 2H), 3.30 - 3.13 (m, 2H), 1.79 (ddd, *J* = 13.4, 7.5, 4.3 Hz, 2H), 1.64 (p, *J* = 7.6 Hz, 2H), 1.23 (s, 9H). LCMS (ESI) $[M+1]^+$ = 380.2.

**Step 4**

A 100 mL three-necked flask was sealed and purged with nitrogen three times, and DIBAL-H (19.75 mL, 19.75 mmol) was added. The system was cooled to -78 °C, and a solution of I-80d (3 g, 7.9 mmol) in THF (20 mL) was slowly added dropwise. After the dropwise addition was completed, the mixture was stirred for 1 hour for reaction while the temperature was kept at -78 °C. After the reaction was completed, methanol (5 mL) was added dropwise to quench the reaction. The reaction liquid was poured into an aqueous potassium sodium tartrate (14.5 g, 69.13 mmol) solution (100 mL), and ethyl acetate (100 mL) was added. The resulting mixture was stirred rapidly for 3 hours, and liquid separation was performed. The ethyl acetate phase was washed with saturated brine (50 mL × 3), dried, filtered, and concentrated to give I-80e (3 g, crude product). $^1$H NMR (400 MHz, DMSO-$d_6$) δ 7.49 (d, *J* = 1.6 Hz, 1H), 7.35 (d, *J* = 8.3 Hz, 1H), 7.23 (dd, *J* = 8.3, 1.6 Hz, 1H), 5.65 (d, *J* = 9.1 Hz, 1H), 4.20 (td, *J* = 8.6, 6.1 Hz, 1H), 3.62 (t, *J* = 6.5 Hz, 2H), 1.86 - 1.59 (m, 4H), 1.50 (td, *J* = 14.9, 14.2, 6.3 Hz, 1H), 1.31 (ddd, *J* = 14.4, 7.3, 3.1 Hz, 1H), 1.14 (s, 9H). LCMS (ESI) [M+1]$^+$ = 382.2.

**Step 5**

I-80e (3 g, 7.82 mmol), THF (30 mL), and 18-crown-6 (1.03 g, 3.91 mmol) were added to a 100 mL three-necked flask. The mixture was purged with nitrogen three times, and sodium hydride (950 mg, 23.69 mmol, 60%) was added batchwise while the temperature was controlled at 0 °C. After the addition was completed, the resulting mixture was warmed to room temperature and reacted for 2 hours. After the reaction was completed, the reaction liquid was poured into a saturated aqueous ammonium chloride solution (100 mL), and the resulting mixture was extracted with ethyl acetate (50 mL × 2). The ethyl acetate phase was washed with saturated brine (50 mL × 3), dried, filtered, and concentrated to give I-80f (2.8 g, crude product). LCMS (ESI) [M+1]$^+$ = 346.1.

**Step 6**

I-80f (2.8 g, 8.11 mmol) and methanol (10 mL) were added to a 100 mL single-necked flask, and a hydrochloric acid/methanol solution (6 mL, 4 mol/L) was added. The mixture was stirred at room temperature for 1 hour for reaction. After the reaction was completed, the reaction liquid was concentrated to dryness by rotary evaporation, and ethyl acetate (10 mL) and petroleum ether (20 mL) were added. The resulting mixture was stirred for 30 min and filtered, and the filter cake was dried to give I-80g (1.8 g, three-step yield: 82%, HCl salt). $^1$H NMR (400 MHz, DMSO-$d_6$) δ 9.75 (s, 1H), 9.37 (s, 1H), 7.79 (d, *J* = 1.5 Hz, 1H), 7.53 - 7.39 (m, 2H), 4.26 (d, *J* = 10.6 Hz, 1H), 3.34 - 3.27 (m, 1H), 2.99 (t, *J* = 12.8 Hz, 1H), 1.96 - 1.82 (m, 4H), 1.81 - 1.54 (m, 2H). LCMS (ESI) [M+1]$^+$ = 242.2.

**Step 7**

I-1f (60 mg, 0.23 mmol), I-80g (72 mg, 0.30 mmol, HCl), NMI (57 mg, 0.69 mmol), and DMAC (3 mL) were mixed and cooled to 0 °C, and TCFH (97 mg, 0.35 mmol) was added. After the addition was completed, the mixture was reacted at 25 °C for 1 hour. After the reaction was completed as monitored by LCMS, the reaction mixture was separated by reversed-phase preparative chromatography to give I-80 (51 mg, 46%). $^1$H NMR (400 MHz, DMSO-$d_6$) δ 8.46 (s, 1H), 8.31 (s, 1H), 8.22 (s, 1H), 7.54 (d, *J* = 11.1 Hz, 1H), 7.44 - 7.34 (m, 2H), 7.21 (d, *J* = 8.5 Hz, 1H), 5.90 (s, 1H), 4.38 (s, 3H), 2.95 (s, 2H), 2.41 (d, *J* = 13.9 Hz, 1H), 1.97 (d, *J* = 10.1 Hz, 1H), 1.73 - 1.37 (m, 4H). LCMS (ESI) [M+1]$^+$ = 484.3.

**Example I-109**

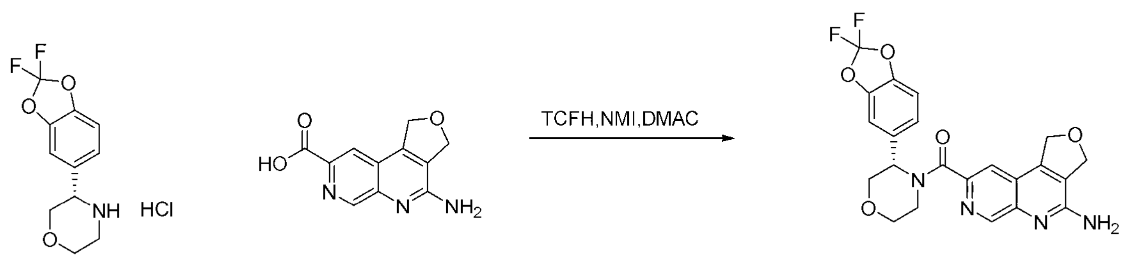


I-33f I-109a I-109

1-109a (50 mg, 0.22 mmol), 1-33f (62 mg, 0.22 mmol), NMI (54 mg, 0.45 mmol), and DMAC (3 mL) were mixed

and cooled to 0 °C, and TCFH (74 mg, 0.26 mmol) was added. After the addition was completed, the mixture was reacted at 25 °C for 1 hour. After the reaction was completed as monitored by LCMS, the reaction mixture was separated by reversed-phase preparative chromatography to give 1-109 (44 mg, 44%). $^1$H NMR (400 MHz, DMSO-$d_6$) δ 8.86 (s, 1H), 7.84 (s, 1H), 7.53 (s, 1H), 7.40 (d, $J$ = 26.1 Hz, 2H), 7.07 (s, 2H), 5.65 (s, 1H), 5.37 (t, $J$ = 3.5 Hz, 2H), 5.04 (t, $J$ = 3.6 Hz, 2H), 4.47 (s, 1H), 3.98 - 3.69 (m, 3H), 3.56 (t, $J$ = 11.7 Hz, 1H), 3.25 - 2.84 (m, 1H). LCMS (ESI) $[M+1]^+$ = 457.3.

**Example I-111**

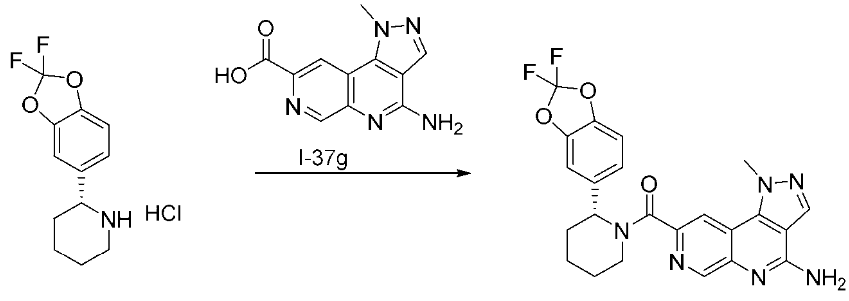


I-80g I-111

I-37g (200 mg, 0.66 mmol), I-80g (185 mg, 0.67 mmol), and TEA (240 mg, 2.38 mmol) were dissolved in DMAC (3 mL). The mixture was cooled to 0 °C, and TBTU (250 mg, 0.79 mmol) was added. After the addition was completed, the mixture was reacted at room temperature for 16 hours. After the reaction was completed as monitored by LCMS, the reaction liquid was diluted by adding water, and the resulting mixture was extracted with ethyl acetate, washed 5 times with saturated brine, dried over anhydrous sodium sulfate, and separated by column chromatography to give I-111 (31.6 mg, 10%). ESI(m/z) =467.3 $[M+H]^+$. $^1$H NMR (400 MHz, DMSO-$d_6$) δ 8.85 (s, 1H), 8.35 (d, $J$ = 1.4 Hz, 2H), 7.43 - 7.37 (m, 2H), 7.31 - 7.23 (m, 3H), 5.75 (s, 1H), 4.41 (s, 3H), 4.07 (s, 2H), 2.92 (s, 2H), 2.41 (d, $J$ = 14.4 Hz, 1H), 1.95 (d, $J$ = 15.3 Hz, 1H), 1.66 (d, $J$ = 14.6 Hz, 2H).

**Example I-114**

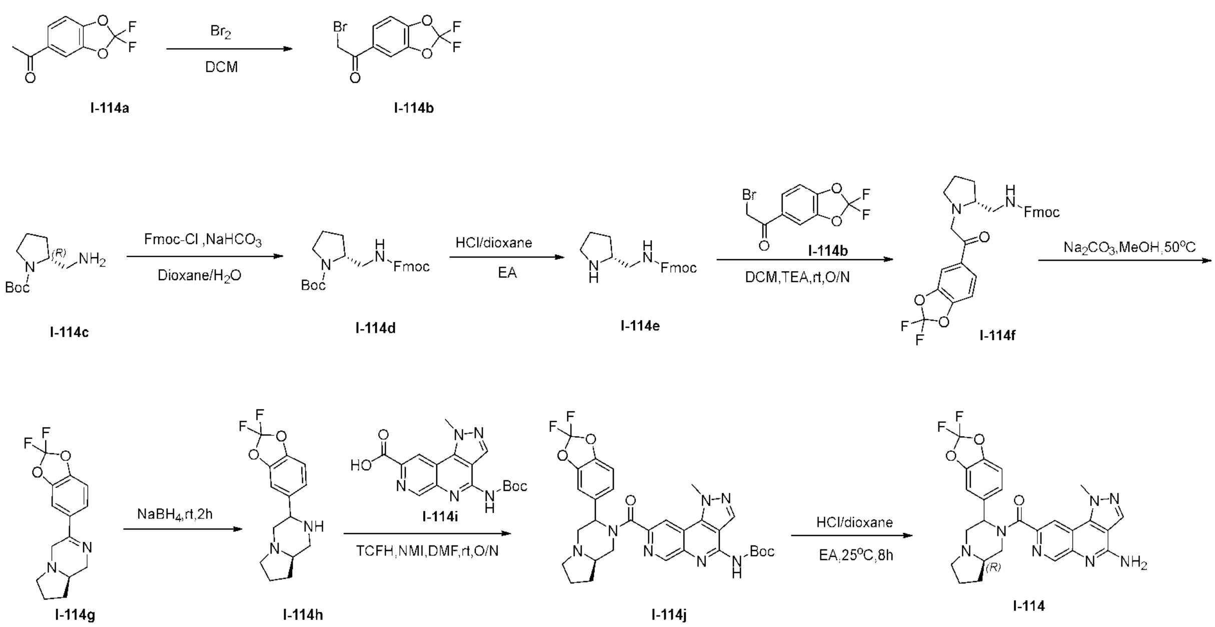

**Step 1**

Compound **I-114a** (5390 mg, 26.93 mmol) was added to dichloromethane (100 mL), and the mixture was cooled to 0 °C. A solution of liquid bromine (4303.89 mg, 26.93 mmol) in dichloromethane (10 mL) was slowly added dropwise, and the reaction liquid was stirred at room temperature for 16 hours for reaction under a nitrogen atmosphere. After the starting materials were consumed completely as detected by LC-MS, the reaction liquid was washed with a saturated aqueous sodium bicarbonate solution and extracted with dichloromethane. The organic phase was washed with saturated brine, dried over anhydrous sodium sulfate, and filtered. The filtrate was concentrated under reduced pressure, and the residue was purified by column chromatography (PE/EA = 90/10) to give **I-114b** (4500 mg, yield: 59.87%) as a white solid.

**Step 2**

Compound **I-114c** (1440 mg, 7.19 mmol) and sodium bicarbonate (1812.1 mg, 21.57 mmol) were added to dioxane/$H_2O$ (100 mL, 3/1). The mixture was cooled to 0 °C. Fmoc-Cl (2790.08 mg, 10.78 mmol) was slowly added, and the mixture was purged with nitrogen three times. The reaction liquid was stirred at room temperature for 16 hours for reaction under a nitrogen atmosphere. After the starting materials were consumed completely as detected by LC-MS. The reaction liquid was washed with water and extracted with ethyl acetate. The organic phase was washed with saturated brine, dried over anhydrous sodium sulfate, and filtered. The filtrate was concentrated under reduced pressure, and the residue was purified by column chromatography (PE/EA = 90/10) to give **I-114d** (2500 mg, yield: 82.29%) as a white solid. MS Calcd.: 422.1; MS Found: 323.0 (M+1-100).

**Step 3**

**I-114d** (2300 mg, 5.44 mmol) was added to a mixed solution of EA (20 mL) and dioxane/HCl (4 M) (10 mL). The mixture was stirred at 25 °C for 4 hours for reaction. After the starting materials were consumed completely as detected by LC-MS, the reaction liquid was concentrated under reduced pressure to give **I-114e** (1570 mg, yield: 80.36%) as a white solid. MS Calcd.: 322.1; MS Found: 323.1 (M+1).

**Step 4**

Compound **I-114e** (643.04 mg, 1.79 mmol), compound **I-114b** (500 mg, 1.79 mmol), and triethylamine (725.29 mg, 7.16 mmol) were added to dichloromethane (20 mL). The mixture was purged with nitrogen three times, and the reaction liquid was stirred for 16 hours for reaction under a nitrogen atmosphere. After the starting materials were consumed completely as detected by LC-MS, the reaction liquid was concentrated under reduced pressure, and the residue was purified by column chromatography (PE/EA = 50/50) to give **I-114f** (600 mg, yield: 64.32%) as a yellow solid. MS Calcd.: 520.1; MS Found: 521.0 (M+1).

**Step 5**

Compound **I-114f** (400 mg, 0.76 mmol) and sodium carbonate (244.36 mg, 2.30 mmol) were added to methanol (15 mL). The mixture was purged with nitrogen three times, and the reaction liquid was heated to 50 °C and stirred for 2 hours for reaction under a nitrogen atmosphere. After the starting materials were consumed completely as detected by LC-MS, the reaction liquid was cooled to room temperature and filtered. The filtrate was directly used in the next step.

**Step 6**

The filtrate in step 5 was cooled under an ice bath, and $NaBH_4$ (58.04 mg, 1.53 mmol) was added. The reaction liquid was stirred at 20 °C for 2 hours for reaction. After the starting materials were consumed completely as detected by LC-MS, the reaction liquid was quenched with water and concentrated under reduced pressure. The residue was purified by column chromatography (DCM/MeOH = 80/20, 0.5% $NH_3.H_2O$) to give **I-114h** (180 mg) as a yellow solid. MS Calcd.: 282.1; MS Found: 283.0 (M+1).

**Step 7**

Compound **I-114h** (180 mg, 0.63 mmol), compound **I-114i** (218.95 mg, 0.63 mmol), TCFH (214.73 mg, 0.76 mmol), and NMI (130.9 mg, 1.59 mmol) were added to DMF (15 mL). The mixture was purged with nitrogen. The reaction liquid was stirred at room temperature for 16 hours for reaction, quenched with $H_2O$ (30 mL), and extracted with EA (30 mL). The organic phase was washed with a saturated NaCl solution (30 mL), dried over anhydrous sodium sulfate, filtered,

and concentrated under reduced pressure. The residue was purified by column chromatography (PE/EA = 50/50) to give **I-114j** (280 mg, yield: 72.26%) as a yellow solid. MS Calcd.: 607.1; MS Found: 608.0 (M+1).

### Step 8

Compound **I-114j** (280 mg, 0.46 mmol) was added to a mixed solution of EA (10 mL) and dioxane/HCl (4 M) (10 mL). The mixture was stirred at 25 °C for 8 hours for reaction. After the starting materials were consumed completely as detected by LC-MS, the reaction liquid was cooled and concentrated under reduced pressure. The residue was purified by preparative high performance liquid chromatography to give **I-114** (130 mg, yield: 55.58%). $^1$H NMR (400 MHz, DMSO-$d_6$): δ 8.85 (s, 1 H), 8.34 (s, 1 H), 8.23 (s, 1 H), 7.46 (s, 3 H), 7.33-7.26 m, 2 H), 5.53-5.50 (m, 1 H), 4.36 (s, 3 H), 3.84-3.80 (m, 2 H), 3.15-3.05 (m, 3 H), 2.79-2.76 (m, 2 H), 1.81-1.75 (m, 2 H), 1.60-1.55 (m, 2 H). MS Calcd.: 507.1; MS Found: 508.1 (M+1).

### Example I-112

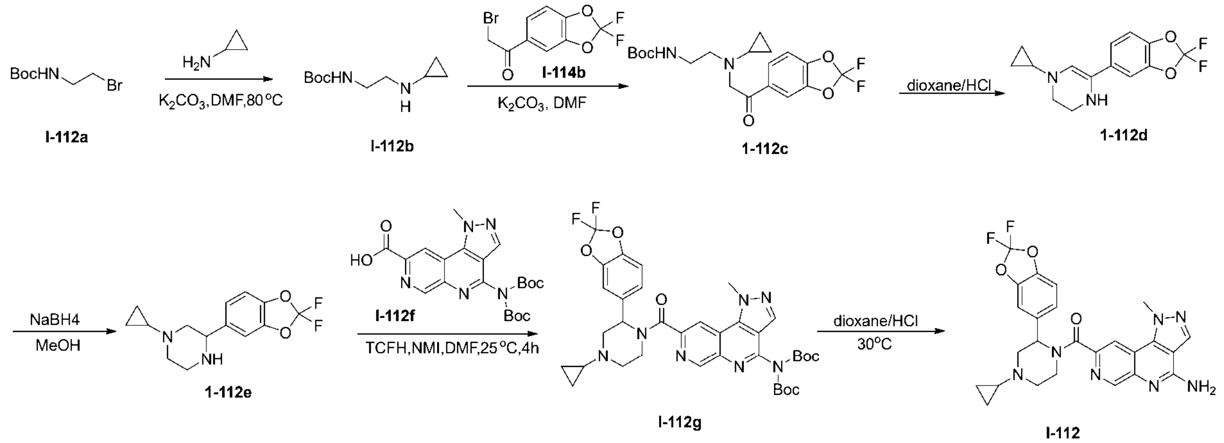


### Step 1

Compound **I-112a** (1.0 g, 4.46 mmol), cyclopropylamine (0.28 g, 4.91 mmol), and potassium carbonate (1.23 g, 8.92 mmol) were added to DMF (20 mL). The reaction liquid was stirred at 80 °C for 18 hours for reaction under a nitrogen atmosphere. After the starting materials were consumed completely as detected by LC-MS, the reaction liquid was cooled and concentrated under reduced pressure. The residue was purified by column chromatography (DCM/MeOH/NH3.H2O = 10/1/0.1%) to give **I-112b** (0.4 g, yield: 44.8%) as a yellow oil. $^1$H NMR (400 MHz, *DMSO_d$_6$*): δ 6.50 (brs, 1H), 2.83-2.78 (m, 2H), 2.42-2.39 (m, 2H), 1.88-1.85 (m, 2H), 1.11 (s, 9H), 0.17-0.13 (m, 2H), 0.01-(-0.03) (m, 2H).

### Step 2

Compound **I-112b** (400 mg, 1.99 mmol), compound **I-114b** (557.29 mg, 1.99 mmol), and potassium carbonate (549.24 mg, 3.98 mmol) were added to DMF (15 mL), and the reaction liquid was stirred at 20 °C for 4 hours for reaction under a nitrogen atmosphere. After the starting materials were consumed completely as detected by TLC, water was added to quench the reaction, and the resulting mixture was extracted with EA (30 mL × 3). The organic phase was washed with a saturated NaCl solution (50 mL), dried over anhydrous sodium sulfate, filtered, and concentrated under reduced pressure. The residue was purified by column chromatography (PE:EA = 4:1) to give **I-112c** (280 mg, yield: 35.17%) as a yellow oil. MS Calcd.: 398.17; MS Found: 399.5 (M+1).

### Step 3

Compound **I-112c** (280 mg, 0.70 mmol) was added to a mixed solution of dioxane (10 mL) and dioxane/HCl (4 M) (10 mL). The mixture was stirred at room temperature for 4 hours for reaction. After the starting materials were consumed completely as detected by LC-MS, the reaction liquid was cooled and concentrated under reduced pressure to give **I-112d** (260 mg, crude product) as an off-white solid. MS Calcd.: 280.1; MS Found: 281.0 (M+1).

### Step 4

Compound **I-112d** (322 mg, 1.15 mmol) was added to MeOH (20 mL), and $NaBH_4$ (130.4 mg, 3.45 mmol) was added under an ice bath. The reaction liquid was stirred at 20 °C for 2 hours for reaction. After the starting materials were consumed completely as detected by LC-MS, the reaction liquid was concentrated under reduced pressure, and the residue was extracted with water and DCM. The organic phase was washed with a saturated NaCl solution (50 mL), dried over anhydrous sodium sulfate, filtered, and concentrated under reduced pressure to give compound **I-112e** (180 mg, yield: 55.5%) as a yellow solid. MS Calcd.: 282.1; MS Found: 283.0 (M+1)

### Step 5

Compound **I-112e** (160 mg, 0.57 mmol), compound **I-112f** (251.35 mg, 0.57 mmol), TCFH (190.85 mg, 0.68 mmol), and NMI (160.19 mg, 1.42 mmol) were added to DMF (10 mL). The mixture was purged with nitrogen, and the reaction liquid was stirred at room temperature for 4 hours for reaction. The reaction mixture was quenched with $H_2O$ (30 mL) and extracted with EA (10 mL × 3). The organic phase was washed with a saturated NaCl solution (30 mL), dried over anhydrous sodium sulfate, filtered, and concentrated under reduced pressure. The residue was purified by column chromatography (DCM/MeOH = 50/1) to give **I-112g** (350 mg, yield: 87.25%) as a yellow solid. MS Calcd.: 707.2; MS Found: 708.0 (M+1)

### Step 6

Compound **I-112g** (300 mg, 0.42 mmol) was added to a mixed solution of dioxane (10 mL) and dioxane/HCl (4 M) (10 mL). The mixture was stirred at 30 °C for 5 hours for reaction. After the starting materials were consumed completely as detected by LC-MS, the reaction liquid was cooled and concentrated under reduced pressure, and the residue was purified by high performance liquid chromatography-mass spectrometry to give **I-112** (65 mg, yield: 30.2%). $^1H$ NMR (400 MHz, DMSO_*$d_6$*): δ 8.85 (s, 1 H), 8.39 (s, 1 H), 8.35 (s, 1 H), 7.48-7.36 (m, 3 H), 7.21-7.20 (m, 2 H), 5.83 (brs, 1 H), 4.48 (brs, 3 H), 3.48-3.34 (m, 2 H), 2.82-2.77 (m, 2 H), 2.74-2.67 (m, 1 H), 2.40-2.33 (m, 1 H), 1.71-1.66 (m, 1 H), 0.49-0.44 (m, 2 H), 0.42-0.34 (m, 2 H). MS Calcd.: 507.18; MS Found: 508.1 (M+1).

### Example I-113

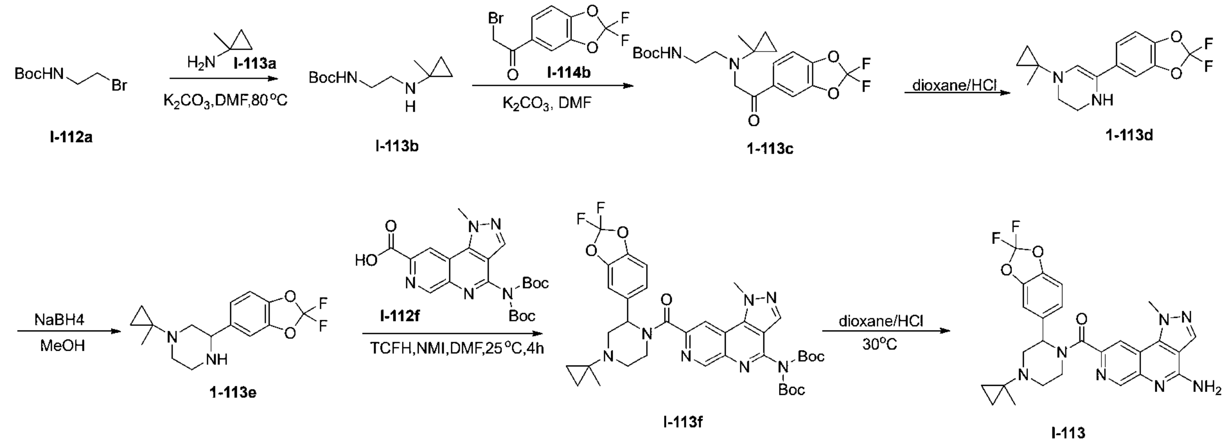

I-112a I-113b 1-113c 1-113d 1-113e I-113f I-113

With reference to the synthesis method of Example 1-112, cyclopropylamine was replaced with I-113a in Step 1 to synthesize and obtain I-113. $^1H$ NMR (400 MHz, DMSO-*$d_6$*): δ 8.85 (s, 1 H), 8.36 (d, *J*=10.8 Hz, 2 H), 7.48-7.15 (m, 5 H), 5.81-5.84 (m, 1 H), 4.44 (brs, 3 H), 3.86-3.39 (m, 2 H), 3.11-2.59 (m, 4 H), 1.23-0.98 (m, 3 H), 0.46-0.33 (m, 4 H). MS Calcd.: 521.2; MS Found: 522.1 (M+1).

### Example I-112 (PEAK1) and I-112 (PEAK2)

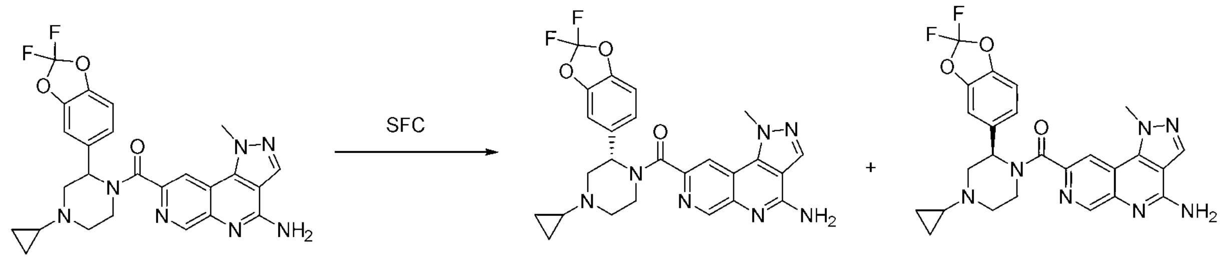


I-112

**I-112** was purified by preparative SFC (IG, Hex:EtOH:DEA = 50:50:0.3, 25 mL/min, 254 nm) to give **I-112** (PEAK 1, Rt = 19.53 min) and **I-112** (PEAK 2, Rt = 25.859 min).

**I-112** (PEAK 1): $^1$H NMR (400 MHz, DMSO_*d$_6$*): δ 8.85 (s, 1 H), 8.39 (s, 1 H), 8.35 (s, 1 H), 7.48-7.36 (m, 3 H), 7.21-7.20 (m, 2 H), 5.83 (brs, 1 H), 4.48 (brs, 3 H), 3.48-3.34 (m, 2 H), 2.82-2.77 (m, 2 H), 2.74-2.67 (m, 1 H), 2.40-2.33 (m, 1 H), 1.71-1.66 (m, 1 H), 0.49-0.44 (m, 2 H), 0.42-0.34 (m, 2 H). MS Calcd.: 507.18; MS Found: 508.1 (M+1).

**I-112** (PEAK 2): $^1$H NMR (400 MHz, DMSO_*d$_6$*): δ 8.85 (s, 1 H), 8.39 (s, 1 H), 8.35 (s, 1 H), 7.48-7.36 (m, 3 H), 7.21-7.20 (m, 2 H), 5.83 (brs, 1 H), 4.48 (brs, 3 H), 3.48-3.34 (m, 2 H), 2.82-2.77 (m, 2 H), 2.74-2.67 (m, 1 H), 2.40-2.33 (m, 1 H), 1.71-1.66 (m, 1 H), 0.49-0.44 (m, 2 H), 0.42-0.34 (m, 2 H). MS Calcd.: 507.18; MS Found: 508.1 (M+1).

**Example I-113 (PEAK1) and I-113 (PEAK2)**

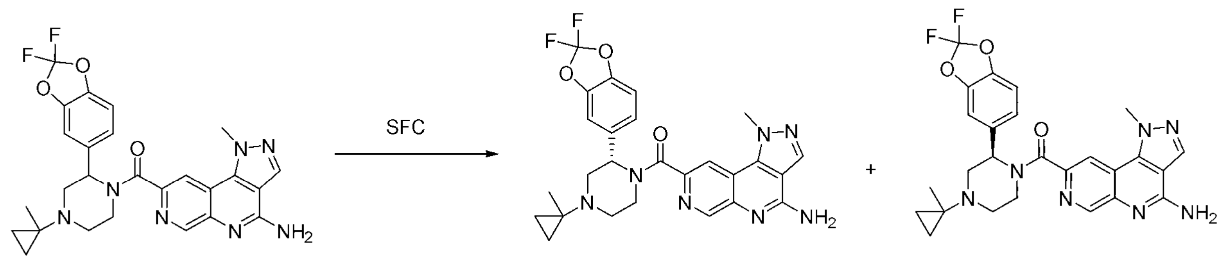


I-113

**I-113** was purified by preparative SFC (IE, Hex:IPA:DEA = 40:60:0.3, 25 mL/min, 254 nm) to give **I-113** (PEAK 1, Rt = 16.56 min) and **I-113** (PEAK 2, Rt = 26.21 min).

**I-113** (PEAK 1): $^1$H NMR (400 MHz, DMSO-*d$_6$*): δ 8.85 (s, 1 H), 8.36 (d, J=10.8 Hz, 2 H), 7.48-7.15 (m, 5 H), 5.81-5.84 (m, 1 H), 4.44 (brs, 3 H), 3.86-3.39 (m, 2 H), 3.11-2.59 (m, 4 H), 1.23-0.98 (m, 3 H), 0.46-0.33 (m, 4 H). MS Calcd.: 521.2; MS Found: 522.1 (M+1).

**I-113** (PEAK 2): $^1$H NMR (400 MHz, DMSO-*d$_6$*): δ 8.85 (s, 1 H), 8.36 (d, J=10.8 Hz, 2 H), 7.48-7.15 (m, 5 H), 5.81-5.84 (m, 1 H), 4.44 (brs, 3 H), 3.86-3.39 (m, 2 H), 3.11-2.59 (m, 4 H), 1.23-0.98 (m, 3 H), 0.46-0.33 (m, 4 H). MS Calcd.: 521.2; MS Found: 522.1 (M+1).

**Biological Assays**

**Experimental Example 1. Compound Activity Assay**

The compounds were prepared into 10 mM stock solutions using DMSO, and then 9 concentration gradients (with an initial concentration of 10 μM followed by 1:5 dilution) were prepared using a buffer (50 mM Tris-HCl pH 8.5, 5 mM $MgCl_2$, 4 mM DTT, 0.01% Triton X-100, 0.1 M NaCl, 0.5% DMSO), and each well received 5 μL of the diluted compound solutions. SAM, a polypeptide, and MTA were prepared using a buffer to final concentrations of 1 μM, 1 μM, and 2 μM, respectively, and were added to each well in a total of 5 μL. Meanwhile, an MTA-free group was set as a control. The PRMT5 protein had a final concentration of 25 nM and was added at 5 μL/well, followed by incubation at 37 °C for 2 hours. Subsequently, a mixture of 2.5 μL of stop buffer A, 2.5 μL of an 8× MT assay mixture (final concentration: 1×), and 24 μg/mL AMP2/GMP2 antibodies (final concentration: 3 μg/mL) was added. The resulting mixture was incubated at room temperature for 1.5 hours, and fluorescence polarization (FP) was measured (Ex 633, Em 647). Meanwhile, negative control wells without protein (Blank) and positive control wells without compound (DMSO) were set. Each experiment was independently repeated three times.

Data processing: The numerical values of each group were recorded for statistical analysis, and the inhibition

rate was calculated according to the following formula: inhibition percentage of compound wells (% inh) = [1 - (ave Blank - Cpd well)/(ave Blank - ave DMSO control)] × 100. Note: ave represents the average value, and Cpd represents the numerical value of the wells containing the compound. A sigmoidal dose-inhibition rate curve was plotted and fitted using a nonlinear regression model in the GraphPad Prism 7.0 software, and the $IC_{50}$ value was calculated. The results are shown in Table 1.

Table 1. Inhibitory activity of compounds against enzymatic activity of PRMT5

| Compound No. | $IC_{50}$ (nM) |
|---|---|
| **I-1** | 48.5 |
| **I-7** | 28.4 |
| **I-16** | 34.6 |

**Experimental Example 2. Compound Activity Assay at the Cellular Level**

**Proliferation inhibition**

CCK8 was used to evaluate the anti-proliferative activity of the compounds in the present application against human colon cancer cell strains HCT-116 and HCT-116-$MTAP^{-/-}$ (MTAP-deficient) and human lung adenocarcinoma cells A549. The above normally growing cells were digested with a cell trypsinization solution, centrifuged, counted, and seeded into a 96-well plate at an appropriate cell density (2000 cells/well) at 100 μL/well. The next day after cell seeding, the compounds at different concentrations were added to the wells. Three replicate wells were set for each concentration point, and a corresponding DMSO negative control group was also set. CCK8 solution was added to each well 8 days after drug treatment. The plate was incubated at 37 °C for a period of time (OD450 of the vehicle group reached 1.0 or higher) before the absorbance at 450 nm was read using a microplate reader. The inhibition rates were calculated, and the $EC_{50}$ values were determined by fitting using the GraphPad Prism 7.0 software.

Table 2. $EC_{50}$ values of compounds at cellular level

| **Compound No.** | **$EC_{50}$ (nM)** | | |
|---|---|---|---|
| | **HCT-116** | **HCT-116-$MTAP^{-/-}$** | **A549** |
| **I-1** | 3228 | 4.2 | 41.8 |
| **I-2** | 6741 | 42.6 | |
| **I-3** | 2369 | 8.4 | |
| **I-4** | >10000 | 27.0 | |
| **I-5** | 2362 | 4.7 | |
| **I-6** | 388.6 | 2.4 | 27.4 |
| **I-7** | 1155 | 6.6 | 34.7 |
| **I-4 (PEAK1)** | 6405.5 | 47.7 | |
| **I-11** | | 0.9 | 2.5 |
| **I-12** | 286.6 | 2.7 | 8.9 |
| **I-13** | | 3.4 | 10.8 |
| **I-14** | 1197.8 | 7.9 | 36.9 |
| **I-15** | 5756.5 | 34 | |
| **I-16** | 927.5 | 3.8 | 24.6 |
| **I-18** | 3445 | 18.3 | |
| **I-19** | >10000 | 48.2 | |
| **I-20** | >10000 | 16.1 | |
| **I-21** | 2609 | 22.0 | |

(continued)

| Compound No. | EC50 (nM) | | |
|---|---|---|---|
| | HCT-116 | HCT-116-MTAP-/- | A549 |
| I-22 | 935.3 | 5.6 | 18.5 |
| I-23 | | 2.1 | 7.7 |
| I-24 | | 3.2 | 8.0 |
| I-25 | 1318 | 5.3 | 33.0 |
| I-26 | | 3.5 | 10.1 |
| I-27 | 510.2 | 4.2 | 24.4 |
| I-29 | 1214 | 6.1 | 50.6 |
| I-30 | 410.9 | 6.4 | 38.5 |
| I-31 | 2714 | 35.8 | |
| I-34 | 1634 | 9.0 | 70.8 |
| I-36 | >10000 | 24.2 | |
| I-37 | 544.7 | 10.1 | 72.7 |
| I-38 | 312.9 | 7.4 | 53.7 |
| I-39 | | 2.5 | 18.5 |
| I-41 | | 1.6 | 16.8 |
| I-34 (PEAK2) | 979.9 | 8.3 | 43.5 |
| I-53 | 210.5 | 5.3 | 15.5 |
| I-54 | 671.9 | 4.2 | 28.9 |
| I-80 | 201 | 4.7 | 12.8 |
| I-109 | 4848 | 19.2 | |
| I-111 | 2171 | 15 | 77 |
| I-112 | >10000 | 57 | |
| I-113 | >10000 | 50.5 | |
| 1-112 (PEAK2) | 3029 | 45 | |
| I-113 (PEAK2) | 4042 | 42 | |

**SDMA Inhibition Assay**

HCT-116, HCT-116-MTAP$^{-/-}$, and A549 cells at a logarithmic growth phase were digested with a cell trypsinization solution, centrifuged, counted, and seeded into a 96-well plate at an appropriate cell density (2000 cells/well) at 100 μL/well, and the surrounding wells were filled with an appropriate amount of PBS to prevent water evaporation. The next day, the cells were treated with the compounds at different concentrations (with an initial concentration of 10 μM, 5-fold dilution, 9 concentration gradients). After 96 hours, the culture medium was pipetted out and the cells were washed 2 times with 100 μL of PBS. The cells were then fixed with 4% paraformaldehyde and incubated at room temperature for 20 min. The fixative solution was discarded, and the cells were washed with PBS. 150 μL of specialized blocking solution (containing 1% Triton-100) was added to each well for blocking at room temperature for 2 hours, and the plate was washed with PBS to remove excess blocking solution. Anti-SDMA (CST) was diluted at a ratio of 1:2000 with a blocking solution containing 0.033% Triton-100, and a blank control group was set. The cells were incubated at 4 °C overnight. The next day, the cells were separately washed 2 times with 1× PBST. Anti-rabbit-IgG (H + L) (CST) and DRAQ5 (Thermofisher) were diluted to an appropriate ratio with a blocking solution containing 0.033% Triton-100 (secondary antibody: 1:2000; DRAQ5: 1:10000). The cells were incubated at room temperature for 2 hours. The cells were washed 2 times with 1× PBST and 1 time with PBS. Fluorescence signals at 700 nm and 800 nm were separately detected using a Li-COR Odyssey two-color near-infrared laser imager. The inhibition rates were calculated, and the $EC_{50}$ values were determined

by fitting using the GraphPad Prism 7.0 software.

Table 3. Inhibition of SDMA by test compounds

| Compound No. | $EC_{50}$ (nM) | | |
|---|---|---|---|
| | HCT-116 | HCT-116-MTAP$^{-/-}$ | A549 |
| I-1 | 220.4 | 1.5 | 1.4 |
| I-2 | 1008 | 8.3 | 5.1 |
| I-3 | 101.1 | 0.6 | 2.1 |
| I-4 | 380.7 | 7.4 | 7.0 |
| I-5 | 200.5 | 2.1 | 2.6 |
| I-6 | | 2.0 | 1.9 |
| I-7 | 181.4 | 2.0 | 1.2 |
| I-4(PEAK1) | 905.7 | 9.1 | 6.1 |
| I-11 | | 0.4 | 0.3 |
| I-12 | | 0.5 | 0.3 |
| I-13 | | 3.5 | 2.1 |
| I-14 | 373.8 | 5.3 | 2.0 |
| I-15 | 627.6 | 10.3 | 7.6 |
| I-16 | 200.8 | 1.2 | 0.8 |
| I-18 | 1020 | 3.1 | 3.1 |
| I-19 | 827.7 | 6.6 | 7.2 |
| I-20 | 862.1 | 5.5 | 9.2 |
| I-21 | 292.2 | 3.0 | 1.9 |
| I-22 | | 1.1 | 0.6 |
| I-23 | | 1.3 | 0.8 |
| I-24 | | 1.7 | 1.0 |
| I-25 | 122.8 | 1.4 | 0.8 |
| I-26 | | 1.7 | 0.6 |
| I-27 | 120.4 | 1.0 | 0.8 |
| I-29 | 291.6 | 2.6 | 2.7 |
| I-30 | 124.9 | 2.0 | 1.9 |
| I-31 | 1124 | 8.7 | 6.7 |
| I-34 | 293.1 | 3.1 | 2.8 |
| I-37 | 205.1 | 1.9 | 2.7 |
| I-38 | 146.6 | 1.5 | 1.7 |
| I-39 | | 0.7 | 0.9 |
| I-41 | | 0.7 | 1.1 |
| I-34 (PEAK2) | 60.7 | 1.1 | 1.3 |
| I-53 | 68.7 | 0.7 | 1.0 |
| I-54 | 61.7 | 1.8 | 1.3 |
| I-80 | 65 | 0.9 | 2.2 |
| I-109 | 938 | 15 | 2.8 |

(continued)

| Compound No. | EC$_{50}$ (nM) | | |
|---|---|---|---|
| | HCT-116 | HCT-116-MTAP$^{-/-}$ | A549 |
| **I-110** | >1000 | 36 | 19 |
| **I-111** | 254 | 1.0 | 1.6 |
| **I-112** | 285 | 2.9 | |
| **I-113** | 403 | 4.1 | |
| **I-112 (PEAK2)** | 281 | 1.9 | |
| **I-113 (PEAK2)** | 492 | 1.6 | |

**Experimental Example 3. Pharmacokinetic Study of Compounds**

**Pharmacokinetic assay in mice**

Eighteen male Balb/c mice aged 6 to 8 weeks were randomly divided into 2 groups (IV group and PO group). The corresponding compounds were administered to the animals in the IV group by tail intravenous injection at 2 mg/kg, and the corresponding compounds were administered to the animals in the PO group by oral gavage at 10 mg/kg or 100 mg/kg. The animals in the IV group were allowed free access to food and water, and the animals in the PO group were fasted overnight before administration and were fed 4 h after administration. Throughout the experiment, the animals were allowed free access to water. Plasma samples were separately collected at 0.083 h (only group IV), 0.25 h, 0.5 h, 1 h, 2 h, 4 h, 8 h, and 24 h after administration. About 100 μL of blood was collected from the orbit of the animal, placed into a 1.5 mL anticoagulation centrifuge tube, and centrifuged at 8000 rpm and 4 °C for 10 min. The upper plasma sample was transferred to an EP tube. The plasma samples were stored in a refrigerator at -80 °C until sample analysis. Pharmacokinetic parameters of the drug were calculated based on the drug concentration-time data, including the peak concentration Cmax, peak time Tmax, clearance rate CL, steady state apparent volume of distribution Vss, area under drug-time curve AUC, elimination half-life t1/2, bioavailability, and the like. Data below the 80% lower limit of quantification were excluded from the calculation of pharmacokinetic parameters.

| Mouse PK | I-1 | | I-2 | | I-5 | | I-7 | | I-16 | |
|---|---|---|---|---|---|---|---|---|---|---|
| | IV 2mpk | PO 10mpk | IV 2mpk | PO 10mpk | IV 2mp k | PO 10mpk | IV 2mpk | PO 10mp k | IV 2mpk | PO 10mpk |
| AUC(0-t) (nM*h) | 5450 | 18075 | 3526 | 18104 | 3324 | 12420 | 4652 | 38012 | 6240 | 34383 |
| AUC(0-∞) (nM*h) | 5686 | 18386 | 4108 | 18800 | 3625 | 12433 | 4893 | 39131 | 6373 | 34520 |
| R_AUC(t/∞) (%) | 95.8 | 98.3 | 85.8 | 96.3 | 91.7 | 99.9 | 95.1 | 97.1 | 97.9 | 99.6 |
| MRT(0-∞) (h) | 2.3 | 5.5 | 4.0 | 7.1 | 2.9 | 4.8 | 2.7 | 6.1 | 4.9 | 5.2 |
| t1/2z (h) | 1.8 | 4.1 | 2.8 | 5.5 | 2.4 | 2.4 | 1.9 | 4.9 | 4.4 | 3.1 |
| Vz (L/kg) | 2.0 | \ | 4.7 | \ | 4.4 | \ | 2.3 | \ | 4.3 | \ |
| CLz (L/h/kg) | 0.8 | \ | 1.1 | \ | 1.3 | \ | 0.8 | \ | 0.7 | \ |
| $C_0$ (nM) | 3515 | \ | 1518 | \ | 1863 | \ | 2618 | \ | 1964 | \ |
| Tmax (h) | \ | 1 | \ | 1 | \ | 1 | \ | 2 | \ | 1 |
| Cmax (nM) | \ | 3240 | \ | 2193 | \ | 1928 | \ | 5012 | \ | 4761 |
| F (%) | \ | 64.7 | \ | 91.5 | \ | 68.6 | \ | 159.9 | \ | 108 |

| Compound No. | **I-38** | | **Control compound A** | |
|---|---|---|---|---|
| Compound structure | 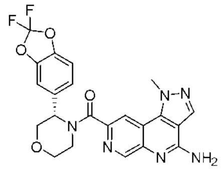 | | 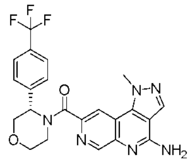 | |
| Administration route and dose of mouse PK | IV 2mpk | PO 100mpk | IV 2mpk | PO 100mpk |
| AUC(0-t) (nM*h) | 7872 | 709934 | 9601 | 454603 |
| Cmax (nM) | \ | 52121 | \ | 35743 |
| Compound No. | **I-109** | | **Control compound B** | |
| Compound structure | 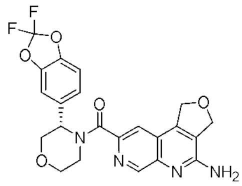 | | 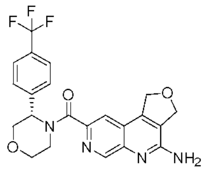 | |
| Administration route and dose of mouse PK | IV 2mpk | PO 10mpk | IV 2mpk | PO 10mpk |
| AUC(0-t) (nM*h) | 13367 | 44056 | 6899 | 26495 |
| Cmax (nM) | \ | 14736 | \ | 5759 |

Control compound A and control compound B were prepared and obtained according to the method described in the patent application WO 2022132914 A1.

**Pharmacokinetic assay in rats**

In this experimental example, the pharmacokinetic behavior of the test compounds after administration by intravenous injection (IV) and oral gavage (PO) in SD rats was tested.

On the day of administration, rats were weighed for actual body weight, and the administration volume was calculated. Each compound was tested in two groups with 3 rats in each group; one group was subjected to administration by single intravenous injection, and the other group was subjected to administration by single oral gavage. The whole blood samples were collected via the jugular vein at prescribed time points (0.25 h, 0.5 h, 1 h, 2 h, 4 h, 8 h, and 24 h after administration). After the blood sample collection, the samples were immediately transferred to labeled commercial sample tubes containing K2-EDTA (0.85-1.15 mg), followed by centrifugation (3200× g, 4 °C, 10 min) and plasma collection. The plasma was transferred to a pre-cooled centrifuge tube, frozen in dry ice, and then stored in an ultra-low temperature freezer at -60 °C or lower until the LC-MS/MS analysis was performed.

Plasma concentrations were determined by the LC-MS/MS method. Plasma drug concentration data for the compounds were processed using a non-compartmental model in the WinNonlin Version 6.3 (Pharsight, Mountain View, CA) pharmacokinetic software. The relevant pharmacokinetic parameters were calculated by a linear-log trapezoidal method.

| Rat | **I-2** | | **I-7** | | **I-16** | |
|---|---|---|---|---|---|---|
| | IV 2mpk | PO 10mpk | IV 0.2mpk | PO 10mpk | IV 2mpk | PO 10mpk |
| AUC(0-t) (nM*h) | 6424 | 14809 | 147.7 | 6578 | 2145 | 9061 |
| AUC(0-∞) (nM*h) | 6914 | 17243 | 220 | 9604 | 2427 | 13177 |
| R_AUC(t/∞) (%) | 91.6 | 86.6 | 67 | 76.9 | 88.8 | 71.5 |
| MRT(0-∞) (h) | 4.3 | 7.2 | 1.8 | 9.9 | 3.1 | 13.2 |
| t1/2z (h) | 3.5 | 4.7 | 1.3 | 8.1 | 2.3 | 9.3 |
| Vz (L/kg) | 3.4 | \ | 3.5 | \ | 5.6 | \ |

(continued)

| Rat | I-2 | | I-7 | | I-16 | |
|---|---|---|---|---|---|---|
| | IV 2mpk | PO 10mpk | IV 0.2mpk | PO 10mpk | IV 2mpk | PO 10mpk |
| CLz (L/h/kg) | 0.8 | \ | 1.9 | \ | 1.9 | \ |
| C0 (nM) | 2337 | \ | 176.5 | \ | 1140 | \ |
| Tmax (h) | \ | 2.3 | \ | 4.3 | \ | 4.7 |
| Cmax (nM) | \ | 1676 | \ | 781 | \ | 808 |
| F (%) | \ | 49.9 | \ | 89.1 | \ | 84 |

| Rat | I-38 | | Control compound A | |
|---|---|---|---|---|
| | IV 2 mpk | PO 100mpk | IV 2 mpk | PO 100mpk |
| AUC(0-t) (nM*h) | 3995 | 198545 | 4745 | 122067 |
| Cmax (nM) | \ | 10214 | \ | 5419 |
| F (%) | \ | 99% | \ | 51% |

**Experimental Example 4. Pharmacodynamic Study of Compounds**

Human colon cancer HCT-116-MTAP$^{-1-}$ cells were cultured in monolayer *in vitro* using the McCoy 5A culture medium (HyClone) containing 10% fetal bovine serum (BI) and 1% penicillin and streptomycin (Beyotime) in an incubator at 37 °C with 5% $CO_2$. The cells were digested with trypsin 2 to 3 times weekly for passaging. When the saturation degree of the cells was 80% to 90%, the cells were collected, counted, and inoculated. $5 \times 10^6$ (0.1 mL) HCT-116-MTAP$^{-/-}$ cells were subcutaneously inoculated on the right back of each mouse. On about day 15 after inoculation of the cells, the mean tumor volume reached 172 $mm^3$. The mice were randomly grouped for administration (the animals were divided into an administration group and a control group, with 6 animals in each group). The day of grouping was designated as d0, and intragastric administration was started on d1. The administration was performed once daily at an administration volume of 10 mL/kg. Vehicle control was administered to the control group. The tumor volume was measured 2 to 3 times weekly, and meanwhile, the mice were weighed and the data were recorded; daily observations were made to record the body surface signs of the mice. After the experiment was completed, the tumors were collected, weighed, and photographed for recording.

Calculation formula for tumor volume:

$$\text{Tumor volume (mm}^3\text{)} = 1/2 \times (a \times b^2)$$

(note: a represents long diameter and b represents short diameter)

Relative tumor growth inhibition rate (TGI%) = (1 - tumor weight in treatment group/tumor weight in control group) × 100%

| No. | Day 28 TGI (%) |
|---|---|
| **I-1** 60 mpk PO QD | 85 |
| **I-7** 10 mpk PO QD | 84 |
| **I-7** 30 mpk PO QD | 91 |
| **I-16** 10 mpk PO QD | 75 |
| **I-16** 30 mpk PO QD | 92 |

Human non-small cell lung cancer NCI-H838 cells were cultured in monolayer *in vitro* using the McCoy 5A culture medium (HyClone) containing 10% fetal bovine serum (BI) and 1% penicillin and streptomycin (Beyotime) in an incubator

at 37 °C with 5% $CO_2$. The cells were digested with trypsin 2 to 3 times weekly for passaging. When the saturation degree of the cells was 80% to 90%, the cells were collected, counted, and inoculated. $5 \times 10^6$ (0.1 mL) NCI-H838 cells were subcutaneously inoculated on the right back of each mouse. On about day 29 after inoculation of the cells, the mean tumor volume reached 156 $mm^3$. The mice were randomly grouped for administration (the animals were divided into an administration group and a control group, with 6 animals in each group). The day of grouping was designated as d0, and intragastric administration was started on d1. The administration was performed once daily at an administration volume of 10 mL/kg. Vehicle control was administered to the control group. The tumor volume was measured 2 to 3 times weekly, and meanwhile, the mice were weighed and the data were recorded; daily observations were made to record the body surface signs of the mice. After the experiment was completed, the tumors were collected, weighed, and photographed for recording.

Calculation formula for tumor volume:

$$\text{Tumor volume (mm}^3\text{)} = 1/2 \times (a \times b^2)$$

(note: a represents long diameter and b represents short diameter)

Relative tumor growth inhibition rate (TGI%) = (1 - tumor weight in treatment group/tumor weight in control group) × 100%

| No. | Day 31 TGI (%) |
|---|---|
| **I-16** 10 mpk PO QD | 97 |
| **I-37** 10 mpk PO QD | 58 |
| **I-37** 30 mpk PO QD | 71 |
| **I-37** 100 mpk PO QD | 104 |
| **I-38** 10 mpk PO QD | 75 |
| **I-38** 30 mpk PO QD | 105 |
| **I-38** 100 mpk PO QD | 107 |

**Experimental Example 5. Effect of Compounds on hERG Potassium Ion Channel**

HEK293 cells were cultured in a DMEM medium containing 10% fetal bovine serum and 0.8 mg/mL G418 at 37 °C with 5% $CO_2$. The cells were digested by TrypLE™ Express and then centrifuged. The cell density was adjusted to $2 \times 10^6$ cells/mL. The cells were then gently mixed for 15 to 20 min using a shaker equilibrated at room temperature, and subjected to a patch clamp assay on a machine. The culture medium of the prepared cells was replaced with extracellular fluid. The intracellular fluid and the extracellular fluid were taken from the fluid pool and added to the intracellular fluid pool and the cell and test substance pool of the QPlate chip, respectively. The voltage stimulation of the whole-cell hERG potassium current was recorded by the whole-cell patch clamp, and the experimental data were collected and stored by Qpatch. The compound was subjected to a 3-fold dilution starting from 30 μM to obtain 6 concentration points, each for two administrations over a period of at least 5 min. The current detected in compound-free extracellular fluid for each cell served as its own control group, and the detection was repeated twice independently using at least two cells per concentration. All electrophysiological experiments were performed at room temperature. For data analysis, the current after the action of each drug concentration and the current of the blank control were standardized $\left(\frac{\text{Peak tail current}_{\text{compound}}}{\text{Peak tail current}_{\text{vehicle}}}\right)$, and then the inhibition rate corresponding to each drug concentration was calculated $\left(1-\frac{\text{Peak tail current}_{\text{compound}}}{\text{Peak tail current}_{\text{vehicle}}}\right)$. The mean and standard error were calculated for each concentration, and the half maximal inhibitory concentration of each compound was calculated:

$$Y = \text{Bottom} + \frac{\text{Top} - \text{Bottom}}{1 + 10^{\wedge}((\text{LogIC}_{50} - C) \times \text{HillSlope})}$$

The dose-dependent effect was subjected to non-linear fitting using the above equation, where Y represents the inhibition

rate, C represents the concentration of the test substance, $IC_{50}$ is the half maximal inhibitory concentration, and HillSlope represents the Hill coefficient. Curve fitting and calculation of $IC_{50}$ were performed using Graphpad software.

| Compound No. | hERG $IC_{50}$ (μM) |
|---|---|
| **I-38** | 22 |
| **Control compound A** | 13 |

**Experimental Example 6. Study on Inhibitory Effect of Compounds on Human Liver Microsome CYP450 Enzyme**

1) Preparation of buffer:

100 mM K-Buffer: 9.5 mL of stock solution A was mixed with 40.5 mL of stock solution B, the total volume was adjusted to 500 mL with ultrapure water, and the buffer was titrated to pH 7.4 with KOH or $H_3PO_4$.

Starting material A (1 M potassium dihydrogen phosphate): 136.5 g of potassium dihydrogen phosphate in 1 L of water;
Stock solution B (1 M potassium dihydrogen phosphate): 174.2 g of potassium dihydrogen phosphate in 1 L of water.

2) Preparation of test substance

The test substance powder was prepared into a stock solution at a certain concentration with DMSO or another organic solvent, which was then further diluted with a suitable organic solvent.

*3) In vitro* incubation

The liver microsomal *in vitro* incubation system for the CYP450 enzyme metabolism phenotype study was a biochemical reaction of the prepared liver microsomes supplemented with a redox coenzyme and an enzyme-specific selective inhibitor under simulated physiological temperature and physiological environment conditions.

4) Detection of parent drug or metabolite

The concentration of the parent drug or a metabolite thereof in the incubation solution was determined by LC-MS/MS. and the $IC_{50}$ value was calculated.

| Compound No. | $IC_{50}$ (μM) | | | | |
|---|---|---|---|---|---|
| | **1A2** | **2C9** | **2C19** | **2D6** | **3A4** |
| **I-7** | >10 | >10 | >10 | >10 | >10 |
| **I-16** | >10 | >10 | >10 | >10 | >10 |
| **I-37** | | | | | >10 |
| **I-38** | | | | | >10 |

**Experimental Example 7: Mouse Brain-to-Blood Drug Concentration Ratio and Tumor Tissue-to-Blood Drug Concentration Ratio**

Human lung cancer PC-9 cells were cultured in monolayer *in vitro* using the 1640 culture medium (HyClone) containing 10% fetal bovine serum (BI) and 1% penicillin and streptomycin (Beyotime) in an incubator at 37 °C with 5% $CO_2$. The cells were digested with trypsin 2 to 3 times weekly for passaging. When the saturation degree of the cells was 80% to 90%, the cells were collected, counted, and inoculated. $5 \times 10^6$ (0.2 mL, 1:1 gel addition) PC-9 cells were subcutaneously inoculated into the right back of Balb/c-nude female mice, and the mice were used for subsequent PK and tissue distribution experiments after the mean tumor volume reached about 500 $mm^3$. On the day of administration, mice were weighed for actual body weight and the administration volume was calculated. In each group of 9 mice, the compound was administered by single intravenous injection or oral gavage. Whole blood samples were collected via the orbit at

prescribed time points. After the blood sample collection, the samples were immediately transferred to labeled commercial sample tubes containing K2-EDTA (0.85 to 1.15 mg), followed by centrifugation (3200× g, 4 °C, 10 min) and plasma collection. The plasma was transferred to a pre-cooled centrifuge tube, frozen in dry ice, and then stored in an ultra-low temperature freezer at -60 °C or lower until the LC-MS/MS analysis was performed. Tumor and brain tissues were collected after the animals were euthanized using $CO_2$ at prescribed time points, frozen in liquid nitrogen, and stored in an ultra-low temperature freezer at -60 °C or lower until the LC-MS/MS analysis was performed. 20 μL of the plasma sample or brain/tumor tissue homogenate was added to 80 μL of an internal standard solution (a 50 ng/mL solution of propafenone in acetonitrile). The plate was sealed using a film sealer at 165 °C, shaken on a micro oscillator (at the maximum vibration speed) for 10 min, and centrifuged at 4000 rpm for 20 min using a low-speed benchtop centrifuge; 10 μL of the supernatant was transferred and added to 90 μL of acetonitrile. The mixture was vortexed and mixed well, and then the plate was sealed using the film sealer at 165 °C. After the mixture was vortexed and mixed well, 1 μL of the supernatant was subjected to LC-MS/MS analysis.

Data processing:

Brain-to-blood drug concentration ratio of mice at the same time point = drug concentration in brain tissue/drug concentration in plasma
Tumor tissue-to-blood drug concentration ratio of mice at the same time point = drug concentration in tumor tissue/drug concentration in plasma

The obtained experimental results are shown in the table below:

| Compound No. | Administration dose | Time (h) | Drug concentration in brain (ng/mL) | Drug concentration in tumor tissue (ng/mL) | Drug concentration in blood (ng/mL) | **Brain-to-blood drug concentration ratio** | **Tumor tissue-to-blood drug concentration ratio** |
|---|---|---|---|---|---|---|---|
| **I-112** | 5 mg/kg PO | 1 | 258 | 346 | 220 | **1.17** | **1.57** |
| | | 4 | 31.3 | 93 | 10.3 | **3.05** | **9.05** |

As can be seen from the results in the table above, the compounds of the present application exhibit very high drug distribution in the brain and tumor tissues, and are applicable for the prevention and treatment of brain tumors.

Various modifications of the present disclosure in addition to those described herein will be apparent to those skilled in the art on the basis of the aforementioned description. Such modifications are also intended to fall within the scope of the appended claims. References (including all patents, patent applications, journal articles, books, and any other publications) cited in the present application are all incorporated herein by reference in their entirety.

## Claims

1. A compound, or a pharmaceutically acceptable salt, an ester, a stereoisomer, an atropisomer, a tautomer, a polymorph, a solvate, a metabolite, an isotopically labeled compound, or a prodrug thereof, wherein the compound has a structure of formula (I):

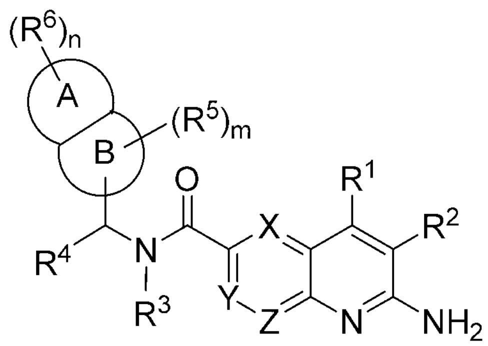

(I)

wherein:

ring A is a $C_{3-10}$ hydrocarbon ring, a 3- to 10-membered heterocyclic ring, a $C_{6-10}$ aromatic ring, or a 5- to 14-membered heteroaromatic ring;
ring B is a benzene ring;
X is $CR^X$ or N;
Y is $CR^Y$ or N;
Z is $CR^Z$ or N;
$R^X$, $R^Y$, $R^Z$, $R^1$, $R^2$, $R^3$, $R^4$, $R^5$, and $R^6$, at each occurrence, are each independently selected from H, halogen, -OH, $-NH_2$, -CN, $-NO_2$, $C_{1-6}$ alkyl, deuterated $C_{1-6}$ alkyl, $C_{2-6}$ alkenyl, $C_{2-6}$ alkynyl, $C_{3-6}$ cyclohydrocarbyl, 3-to 10-membered heterocyclyl, $C_{6-10}$ aryl, 5- to 14-membered heteroaryl, $C_{6-12}$ aralkyl, $-C(=O)R^a$, $-OC(=O)R^a$, $-C(=O)OR^a$, $-OR^a$, $-SR^a$, $-S(=O)R^a$, $-S(=O)_2R^a$, $-S(=O)_2NR^aR^b$, $-NR^aR^b$, $-C(=O)NR^aR^b$, $-NR^a-C(=O)R^b$, $-NR^a-C(=O)OR^b$, $-NR^a-S(=O)_2-R^b$, $-NR^a-C(=O)-NR^aR^b$, $-C_{1-6}$ alkylene-$OR^a$, $-C_{1-6}$ alkylene-$NR^aR^b$, and $-O-C_{1-6}$ alkylene-$NR^aR^b$; or
$R^1$ and $R^2$, together with the groups to which they are attached, optionally form a $C_{3-10}$ hydrocarbon ring, a 3- to 10-membered heterocyclic ring, a $C_{6-10}$ aromatic ring, or a 5- to 14-membered heteroaromatic ring; and/or, $R^3$ and $R^4$, together with the groups to which they are attached, optionally form a 3- to 10-membered heterocyclic ring;
$R^a$ and $R^b$, at each occurrence, are each independently selected from H, $C_{1-6}$ alkyl, $C_{3-10}$ cyclohydrocarbyl, 3-to 10-membered heterocyclyl, $C_{6-10}$ aryl, 5- to 14-membered heteroaryl, and $C_{6-12}$ aralkyl;
the above alkyl, alkylene, alkenyl, alkynyl, cyclohydrocarbyl, hydrocarbon ring, heterocyclyl, heterocyclic ring, aryl, aromatic ring, heteroaryl, heteroaromatic ring, and aralkyl, at each occurrence, are each optionally substituted with one or more substituents independently selected from the following: halogen, -OH, =O, $-NH_2$, -CN, $-NO_2$, $C_{1-6}$ alkyl, $C_{3-6}$ cyclohydrocarbyl, 3- to 10-membered heterocyclyl, $C_{6-10}$ aryl, 5- to 14-membered heteroaryl, $C_{6-12}$ aralkyl, $-C(=O)R^c$, $-OC(=O)R^c$, $-C(=O)OR^c$, $-OR^c$, $-SR^c$, $-S(=O)R^c$, $-S(=O)_2R^c$, $-S(=O)_2NR^cR^d$, $-NR^cR^d$, $-C(=O)NR^cR^d$, $-NR^c-C(=O)R^d$, $-NR^c-C(=O)OR^d$, $-NR^c-S(=O)_2-R^d$, $-NR^c-C(=O)-NR^cR^d$, $-C_{1-6}$ alkylene-$OR^c$, $-C_{1-6}$ alkylene-$NR^cR^d$, and $-O-C_{1-6}$ alkylene-$NR^cR^d$; the alkyl, cyclohydrocarbyl, heterocyclyl, aryl, heteroaryl, and aralkyl are further optionally substituted with one or more substituents independently selected from the following: halogen, -OH, =O, -C(=O)O-tert-butyl, $-NH_2$, -CN, $-NO_2$, $C_{1-6}$ alkyl, halogenated $C_{1-6}$ alkyl, $C_{3-6}$ cyclohydrocarbyl, 3- to 10-membered heterocyclyl, $C_{6-10}$ aryl, 5- to 14-membered heteroaryl, $C_{6-12}$ aralkyl, $-O-C_{1-6}$ alkyl, and $-C_{1-6}$ alkylene-$O-C_{1-6}$ alkyl;

$R^c$ and $R^d$, at each occurrence, are each independently selected from: H, $C_{1-6}$ alkyl, $C_{3-10}$ cyclohydrocarbyl, 3- to 10-membered heterocyclyl, $C_{6-10}$ aryl, 5- to 14-membered heteroaryl, and $C_{6-12}$ aralkyl; the alkyl, cyclohydrocarbyl, heterocyclyl, aryl, heteroaryl, and aralkyl are further optionally substituted with one or more substituents independently selected from the following: halogen, -OH, =O, -C(=O)O-tert-butyl, $-NH_2$, -CN, $-NO_2$, $C_{1-6}$ alkyl, halogenated $C_{1-6}$ alkyl, $C_{3-6}$ cyclohydrocarbyl, 3- to 10-membered heterocyclyl, $C_{6-10}$ aryl, 5- to 14-membered heteroaryl, $C_{6-12}$ aralkyl, and $-C_{1-6}$ alkylene-O-$C_{1-6}$ alkyl; and
m and n are each independently an integer selected from 1, 2, 3, or 4, preferably an integer selected from 1, 2, or 3.

2. The compound, or the pharmaceutically acceptable salt, the ester, the stereoisomer, the atropisomer, the tautomer, the polymorph, the solvate, the metabolite, the isotopically labeled compound, or the prodrug thereof according to claim 1, wherein $R^X$, $R^Y$, and $R^Z$ are each independently selected from H, halogen, -CN, $C_{1-6}$ alkyl, halogenated $C_{1-6}$ alkyl, and -O-($C_{1-6}$ alkyl);
preferably, $R^X$, $R^Y$, and $R^Z$ are each independently selected from H, F, Cl, -CN, methyl, trifluoromethyl, and methoxy.

3. The compound, or the pharmaceutically acceptable salt, the ester, the stereoisomer, the atropisomer, the tautomer, the polymorph, the solvate, the metabolite, the isotopically labeled compound, or the prodrug thereof according to claim 1 or 2,
wherein:

X is CH or C-$CH_3$;
Y is $CR^Y$ or N, wherein $R^Y$ is selected from H, halogen, -CN, $C_{1-6}$ alkyl, halogenated $C_{1-6}$ alkyl, and -O-($C_{1-6}$ alkyl), preferably selected from H, F, Cl, -CN, methyl, trifluoromethyl, and methoxy; and
Z is CH or N.

4. The compound, or the pharmaceutically acceptable salt, the ester, the stereoisomer, the atropisomer, the tautomer, the polymorph, the solvate, the metabolite, the isotopically labeled compound, or the prodrug thereof according to any one of claims 1 to 3, wherein $R^1$ and $R^2$ are each independently selected from H, halogen, $C_{1-6}$ alkyl, and deuterated $C_{1-6}$ alkyl; preferably, $R^1$ and $R^2$ are each independently selected from H, Cl, Br, I, methyl, $-CD_3$, and ethyl; or $R^1$ and $R^2$, together with the groups to which they are attached, optionally form a $C_{5-6}$ hydrocarbon ring, a 5-to 6-membered heterocyclic ring, or a 5- to 6-membered heteroaromatic ring; preferably, $R^1$ and $R^2$, together with the groups to which they are attached, optionally form

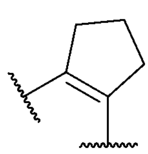 , 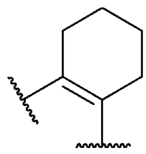 , 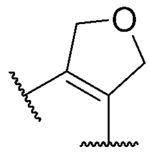 ,

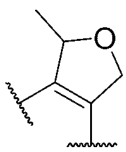 , 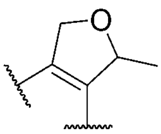 , 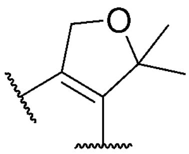 , 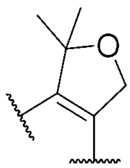 , 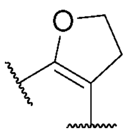 , 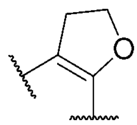 , 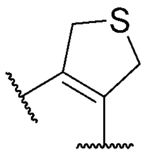 ,

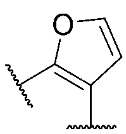 , 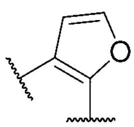 , 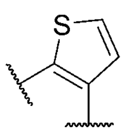 , 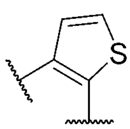 , 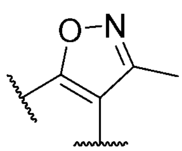 , 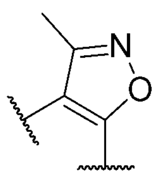 , 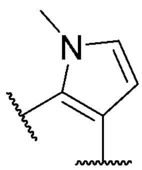 ,

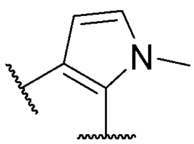 , 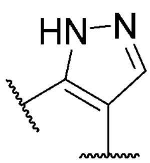 , 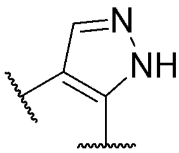 , 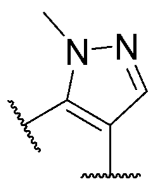 , 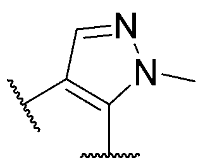 , 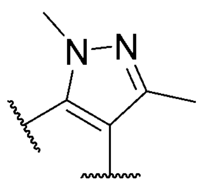 ,

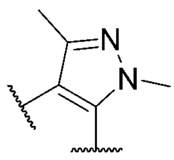, 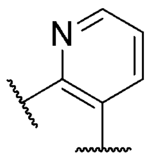, 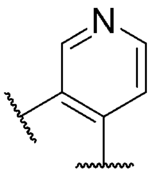, 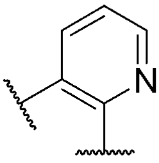, 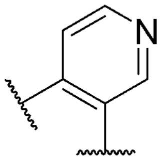, 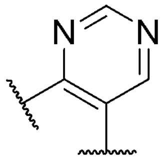,

or

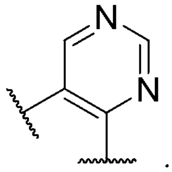.

**5.** The compound, or the pharmaceutically acceptable salt, the ester, the stereoisomer, the atropisomer, the tautomer, the polymorph, the solvate, the metabolite, the isotopically labeled compound, or the prodrug thereof according to any one of claims 1 to 4, wherein

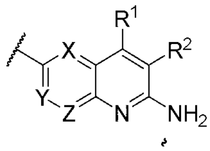

is selected from

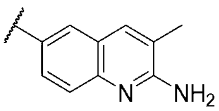, 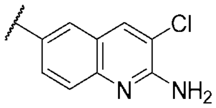, 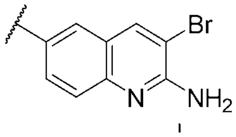, 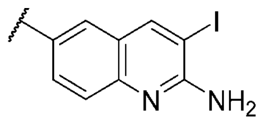,

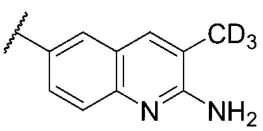, 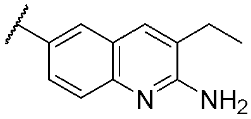, 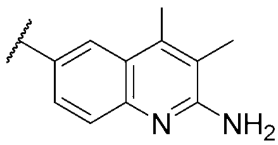, 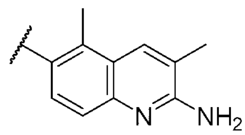,

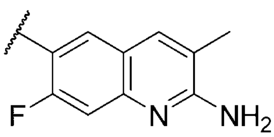, 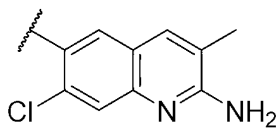, 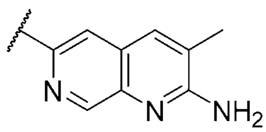, 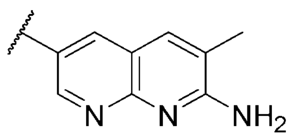,

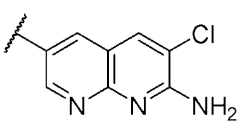, 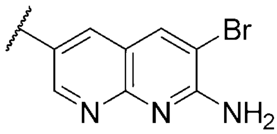, 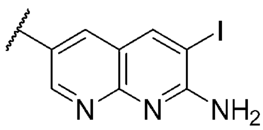, 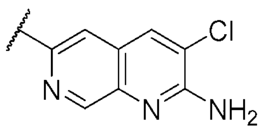,

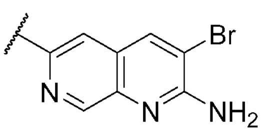, 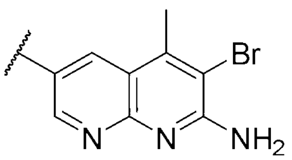, 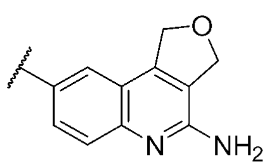, 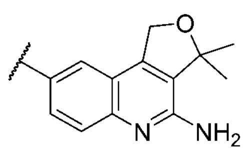,

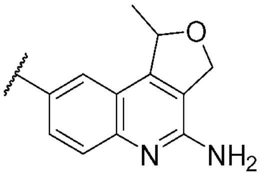 , 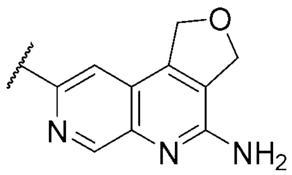 , 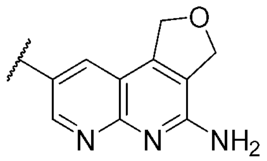 , 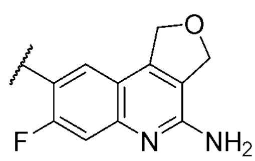 ,

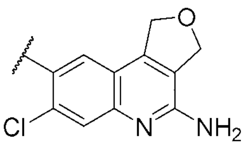 , 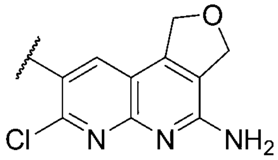 , 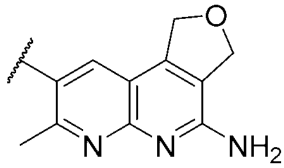 , 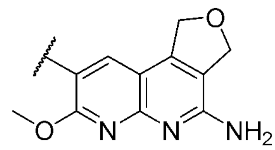 ,

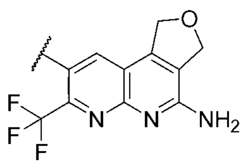 , 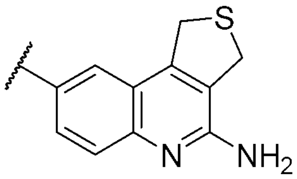 , 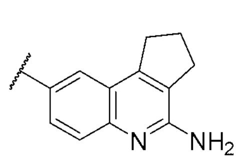 , 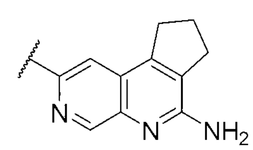 ,

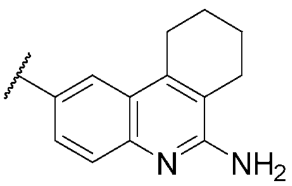 , 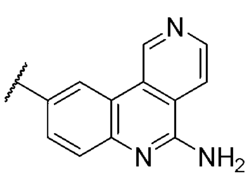 , 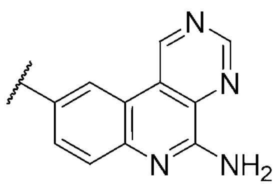 , 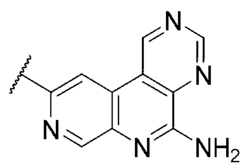 ,

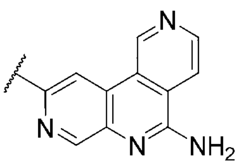 , 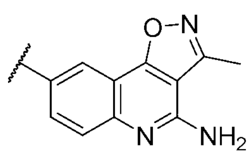 , 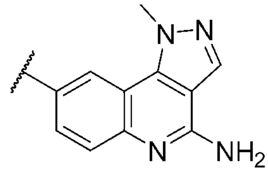 , 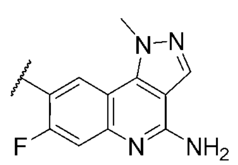 ,

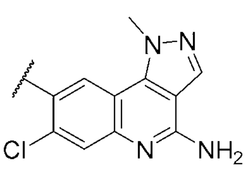 , 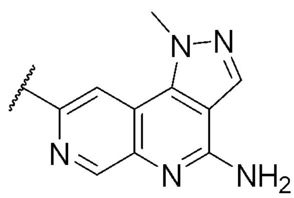 , 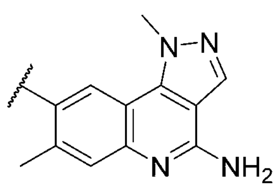 , 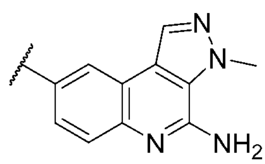 ,

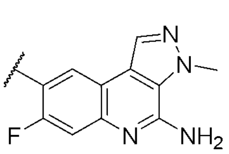 , 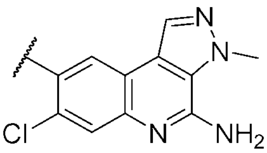 , 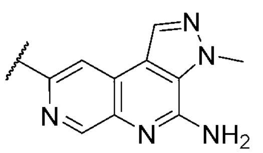 , 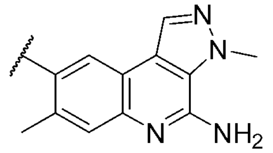 ,

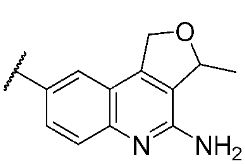 , 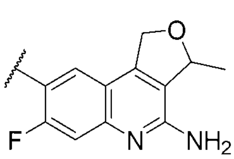 , 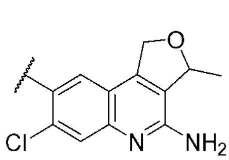 , 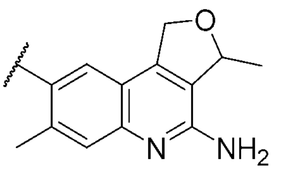 ,

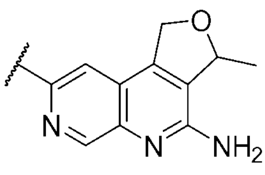, 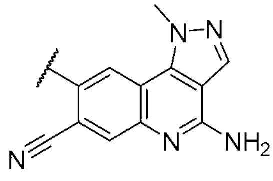, 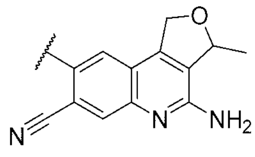, 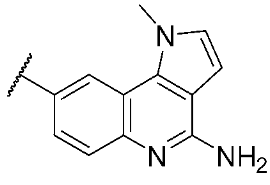,

and

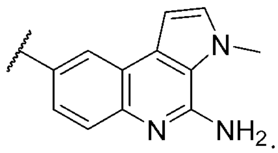.

6. The compound, or the pharmaceutically acceptable salt, the ester, the stereoisomer, the atropisomer, the tautomer, the polymorph, the solvate, the metabolite, the isotopically labeled compound, or the prodrug thereof according to any one of claims 1 to 5, wherein ring A is a 5- to 6-membered heterocyclic ring or a 5-to 6-membered heteroaromatic ring; preferably, ring A is an oxygen-containing 5-membered heterocyclic ring (e.g., a 5-membered heterocyclic ring containing one or two oxygen atoms); preferably, ring A is

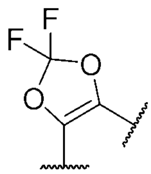, 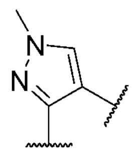, or 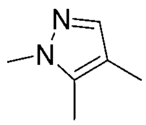;

preferably,

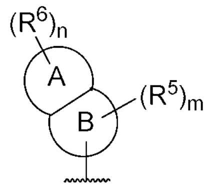

is

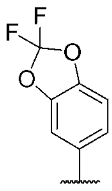, 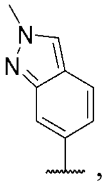, 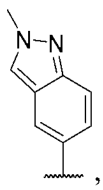, 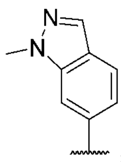, or 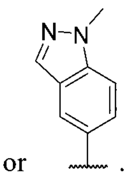.

7. The compound, or the pharmaceutically acceptable salt, the ester, the stereoisomer, the atropisomer, the tautomer, the polymorph, the solvate, the metabolite, the isotopically labeled compound, or the prodrug thereof according to any one of claims 1 to 6, wherein $R^3$ and $R^4$ are each independently selected from H, $C_{1-6}$ alkyl, $C_{3-6}$ cyclohydrocarbyl, 3- to 10-membered heterocyclyl, -$C_{1-6}$ alkylene-OH, and -$C_{1-6}$ alkylene-O-$C_{1-6}$ alkyl; the alkylene, alkyl, cyclohydrocarbyl, and heterocyclyl are optionally further substituted with $C_{3-6}$ cyclohydrocarbyl, 3- to 10-membered heterocyclyl, or 5- to 6-membered heteroaryl; the cyclohydrocarbyl, heterocyclyl, or heteroaryl is further optionally substituted with one or more $C_{1-6}$ alkyl groups;

preferably, $R^3$ and $R^4$ are each independently selected from H, methyl, ethyl, isopropyl, cyclopropyl,

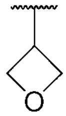,

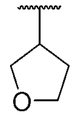, 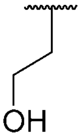, 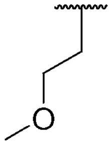, 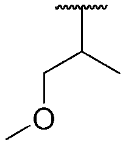, 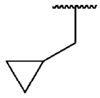, 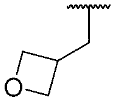, 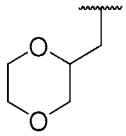, 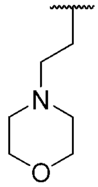, 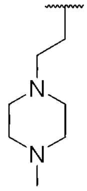, 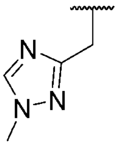,

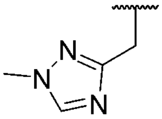, 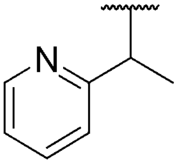, 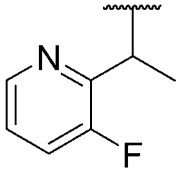, 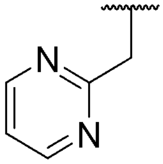, and 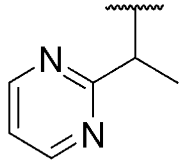;

or

$R^3$ and $R^4$, together with the groups to which they are attached, optionally form a group selected from the following:

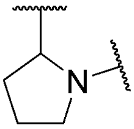, 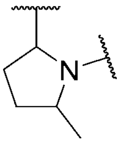, 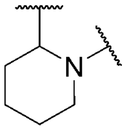, 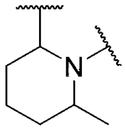, 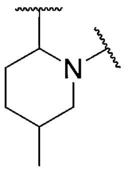, 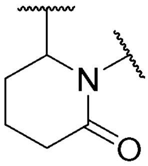, 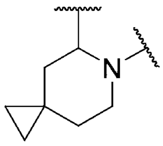,

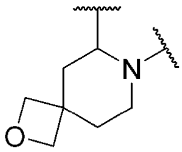, 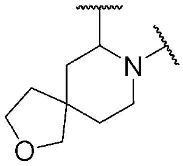, 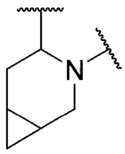, 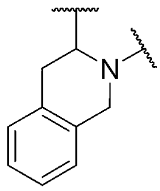, 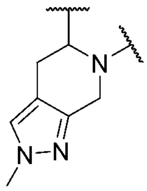, 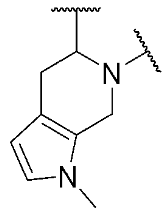,

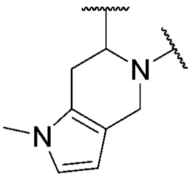, 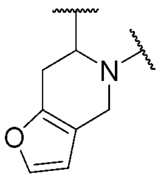, 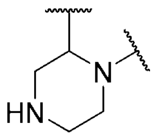, 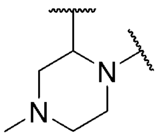, 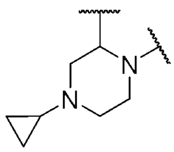, 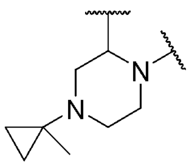,

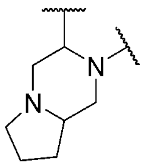, 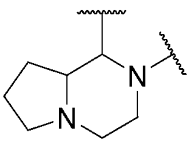, 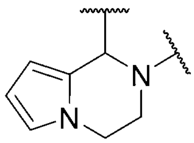, 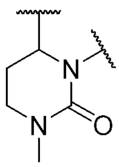, 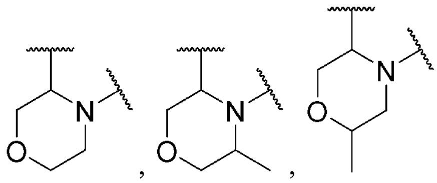, , ,

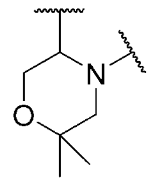, 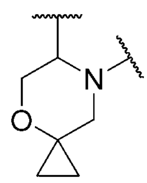, 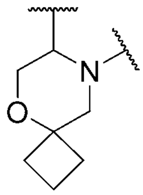, 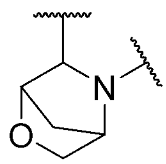, 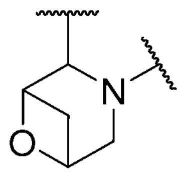, and 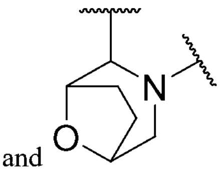.

**8.** The compound, or the pharmaceutically acceptable salt, the ester, the stereoisomer, the atropisomer, the tautomer, the polymorph, the solvate, the metabolite, the isotopically labeled compound, or the prodrug thereof according to any one of claims 1 to 7, wherein

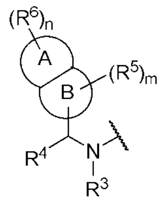

is selected from

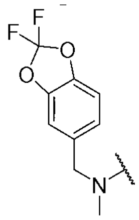,

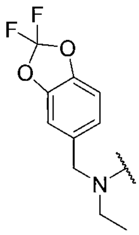, 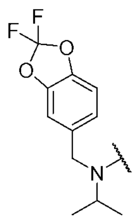, 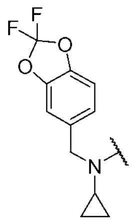, 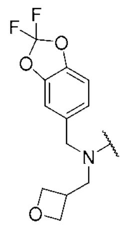, 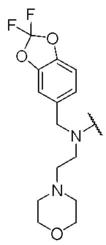, 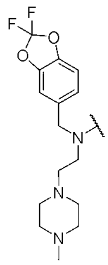, 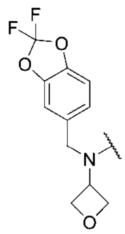, 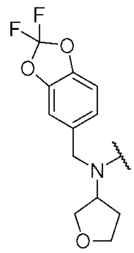,

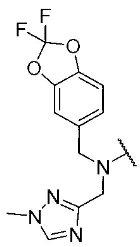, 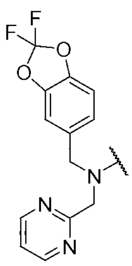, 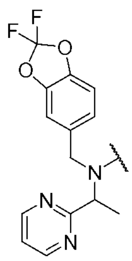, 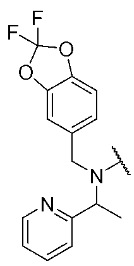, 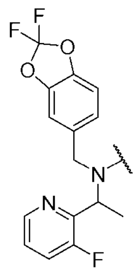, 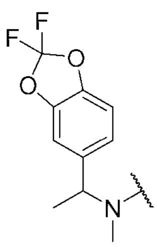, 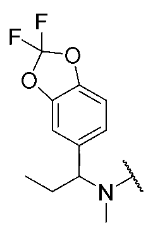, 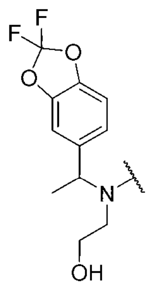,

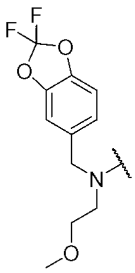, 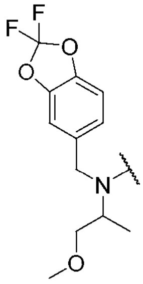, 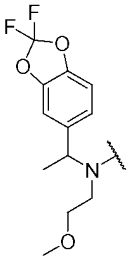, 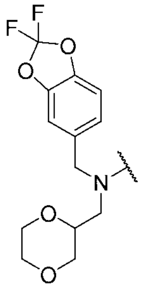, 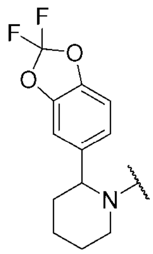, 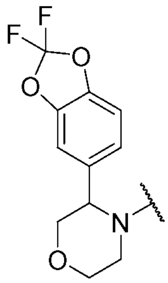, 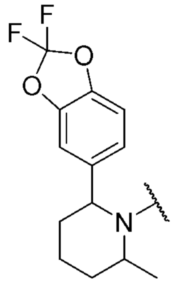,

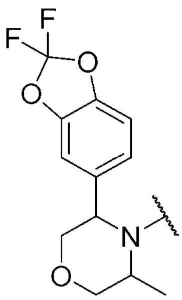, 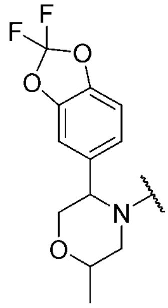, 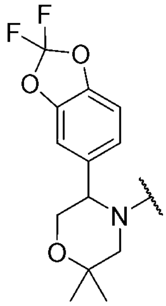, 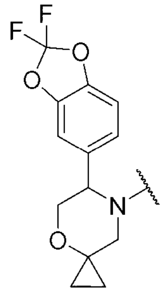, 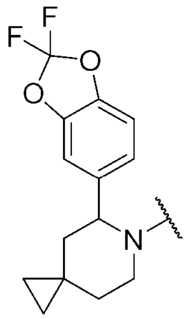, 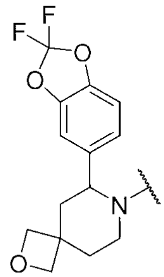,

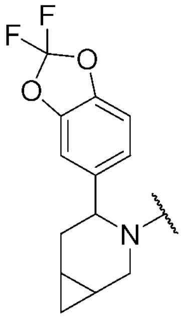, 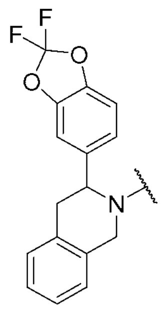, 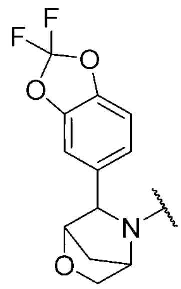, 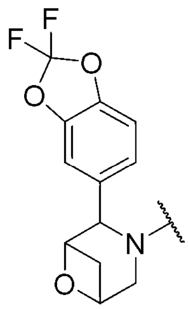, 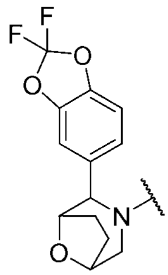, 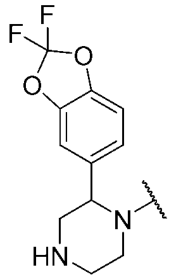,

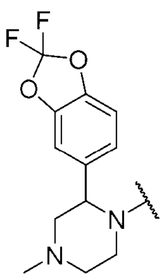, 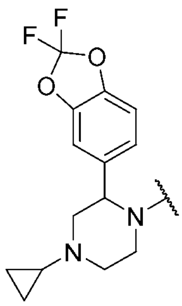, 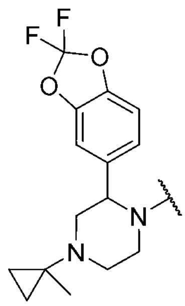, 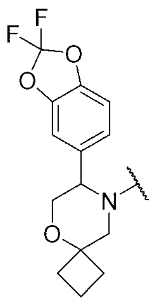, 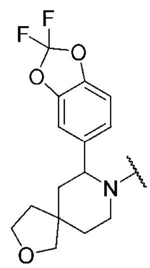, 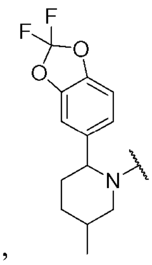, 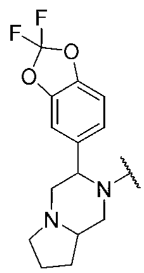,

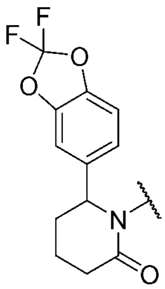, 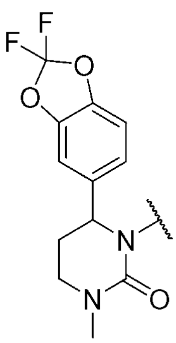, 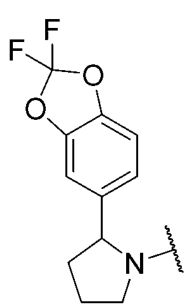, 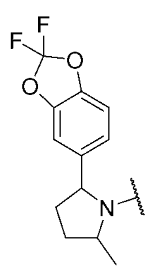, 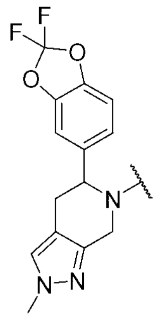, 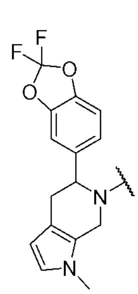, 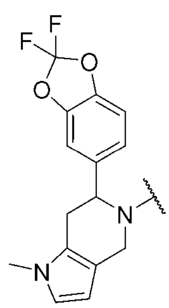,

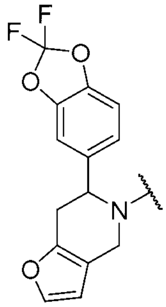 , 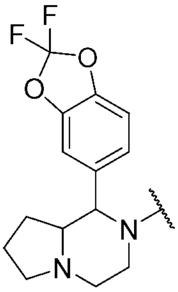 , 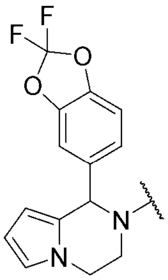 , 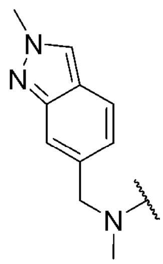 , 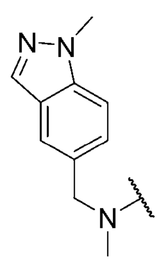 , and 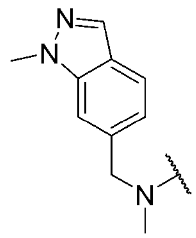 .

9. The compound, or the pharmaceutically acceptable salt, the ester, the stereoisomer, the atropisomer, the tautomer, the polymorph, the solvate, the metabolite, the isotopically labeled compound, or the prodrug thereof according to any one of claims 1 to 8, wherein the compound has the following structures:

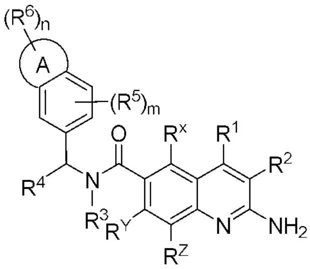
(II)

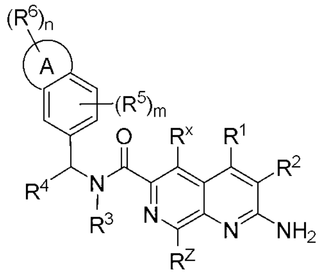
(II)'

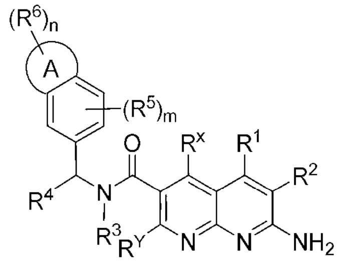
(II)"

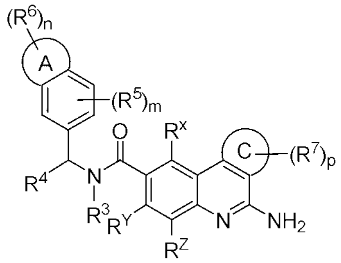
(III)

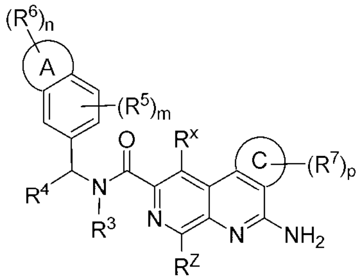
(III)'

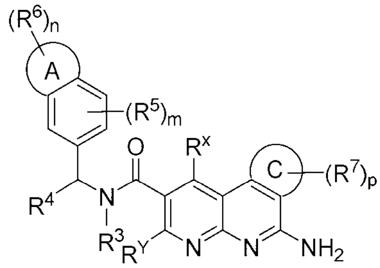
(III)"

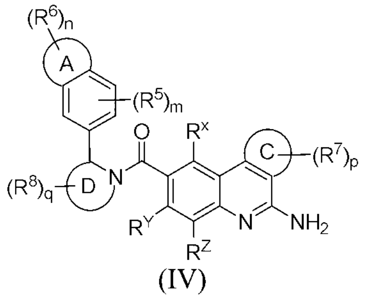
(IV)

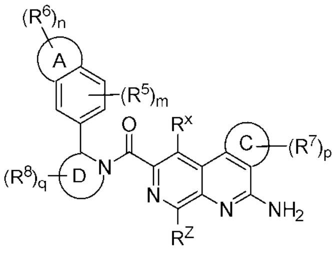
(IV)'

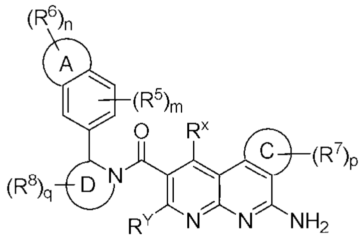
(IV)"

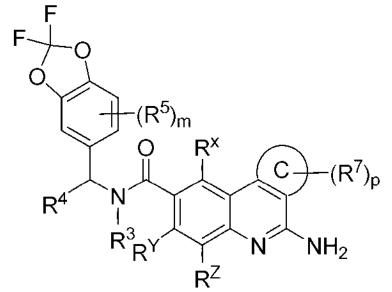
(V)

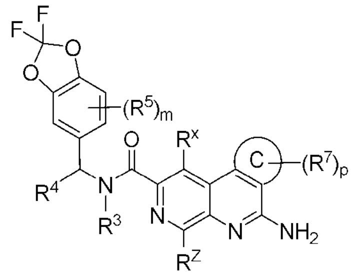
(V)'

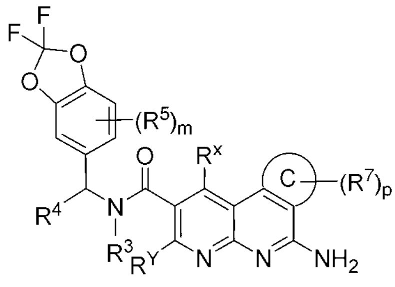
(V)''

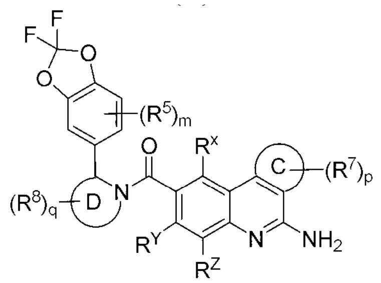
(VI)

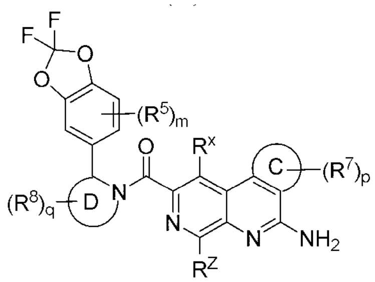
(VI)'

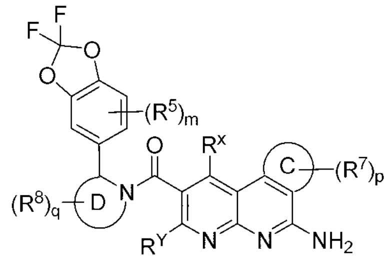
(VI)''

wherein:

ring C is selected from a $C_{5-6}$ hydrocarbon ring, a 5- to 6-membered heterocyclic ring, and a 5- to 6-membered heteroaromatic ring;
ring D is a 3- to 10-membered heterocyclic ring;
$R^7$ and $R^8$, at each occurrence, are each independently selected from H, $C_{1-6}$ alkyl, and $C_{3-6}$ cyclohydrocarbyl;
p and q are each independently an integer selected from 1 or 2; and the other groups are as defined in any one of claims 1 to 8.

**10.** The compound, or the pharmaceutically acceptable salt, the ester, the stereoisomer, the atropisomer, the tautomer, the polymorph, the solvate, the metabolite, the isotopically labeled compound, or the prodrug thereof according to any one of claims 1 to 9, wherein the compound is selected from:

| | | | | | |
|---|---|---|---|---|---|
| **I-1** | 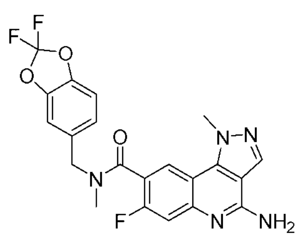 | **I-2** | 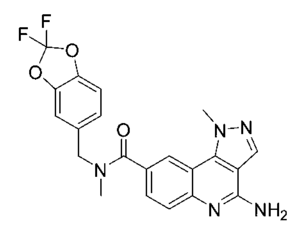 | **I-3** | 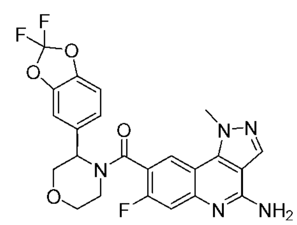 |
| **I-4** | 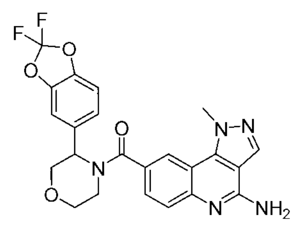 | **I-5** | 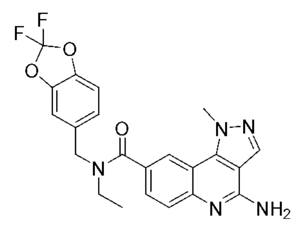 | **I-6** | 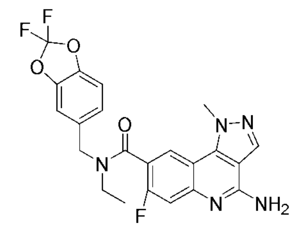 |

(continued)

| | | | | | |
|---|---|---|---|---|---|
| **I-7** | 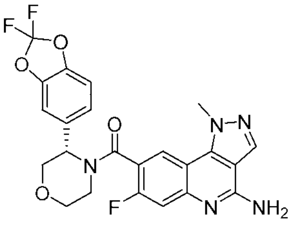 | **I-8** | 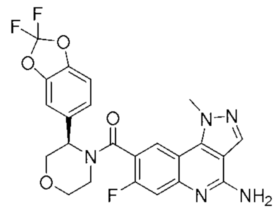 | **I-9** | 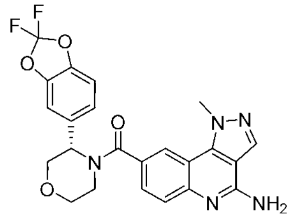 |
| **I-10** | 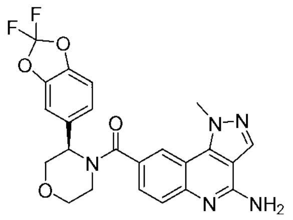 | **I-11** | 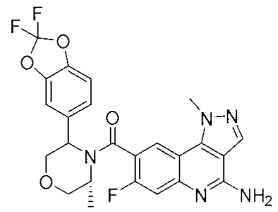 | **I-12** | 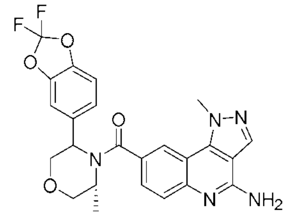 |
| **I-13** | 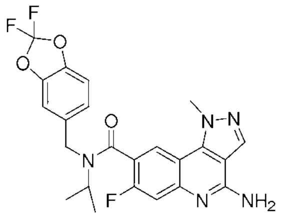 | **I-14** | 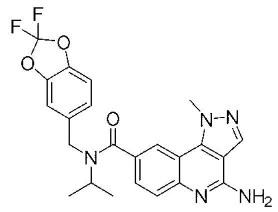 | **I-15** | 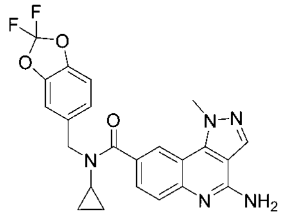 |
| **I-16** | 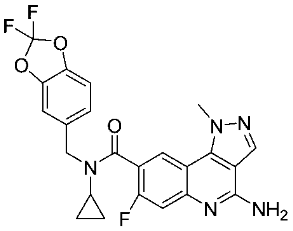 | **I-17** | 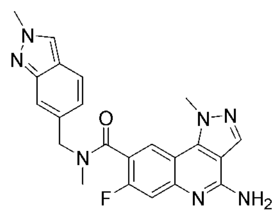 | **I-18** | 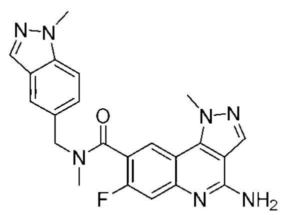 |
| **I-19** | 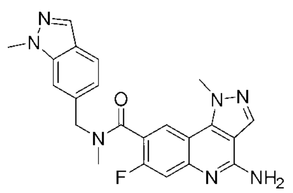 | **I-20** | 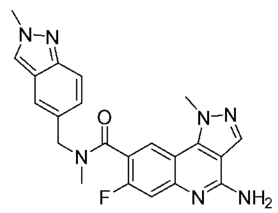 | **I-21** | 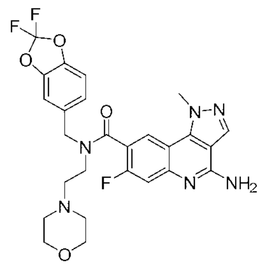 |
| **I-22** | 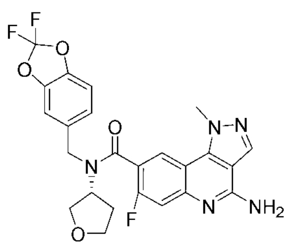 | **I-23** | 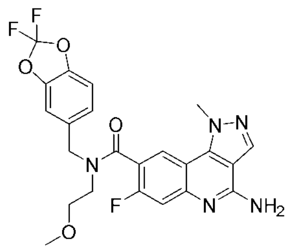 | **I-24** | 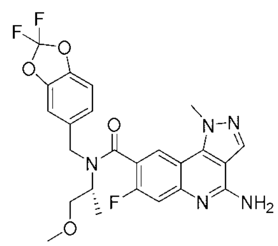 |
| **I-25** | 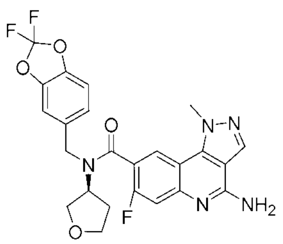 | **I-26** | 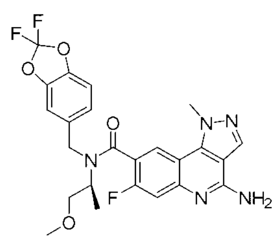 | **I-27** | 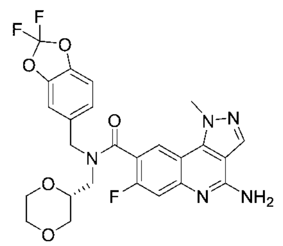 |

(continued)

| | | | | | |
|---|---|---|---|---|---|
| **I-28** | 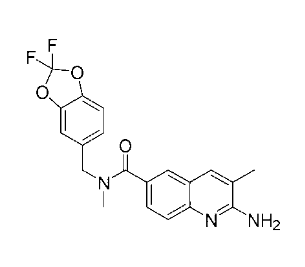 | **I-29** | 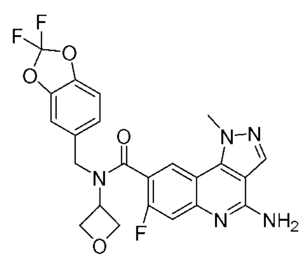 | **I-30** | 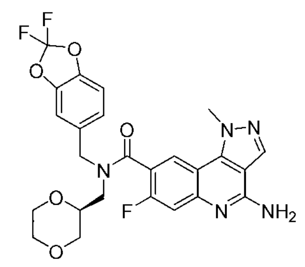 |
| **I-31** | 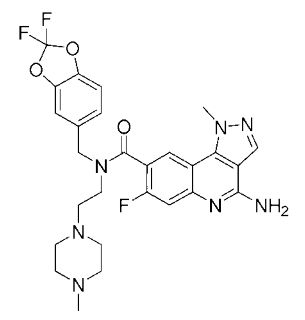 | **I-32** | 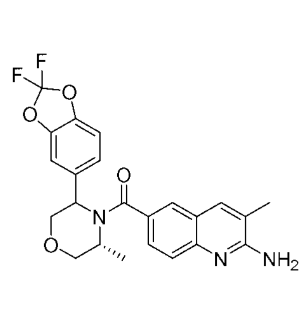 | **I-33** | 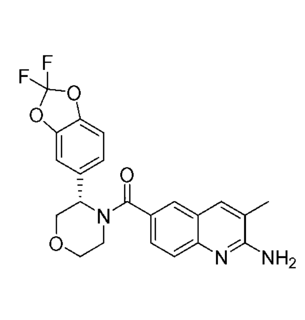 |
| **I-34** | 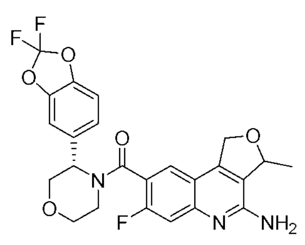 | **I-35** | 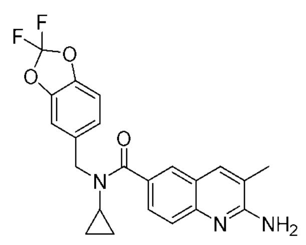 | **I-36** | 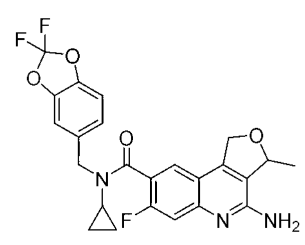 |
| **I-37** | 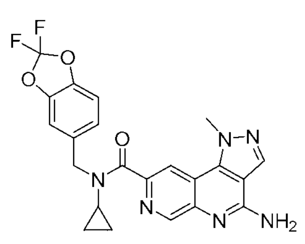 | **I-38** | 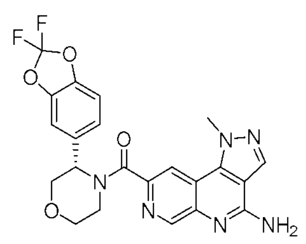 | **I-39** | 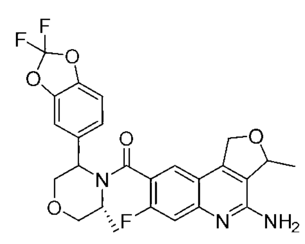 |
| **I-40** | 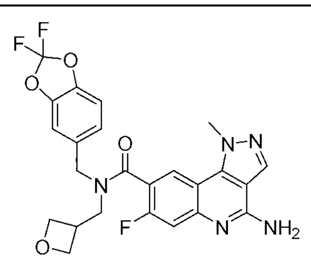 | **I-41** | 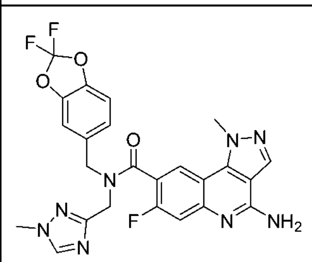 | **I-42** | 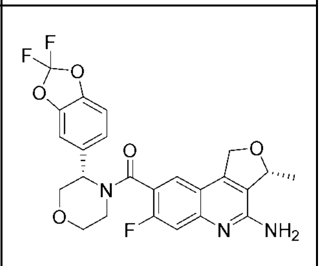 |
| **I-43** | 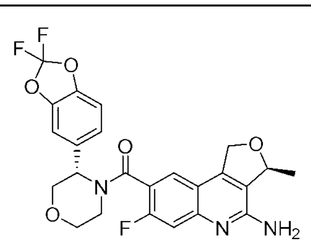 | **I-44** | 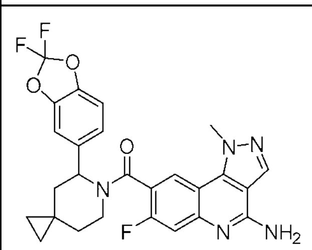 | **I-45** | 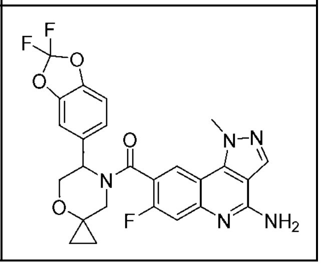 |

(continued)

| | | | | | |
|---|---|---|---|---|---|
| **I-46** | 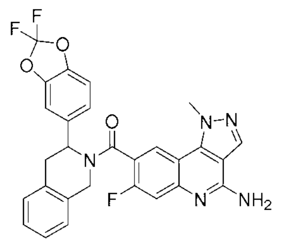 | **I-47** | 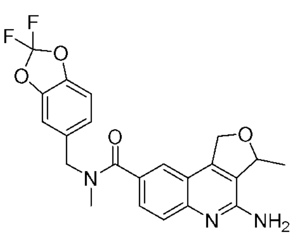 | **I-48** | 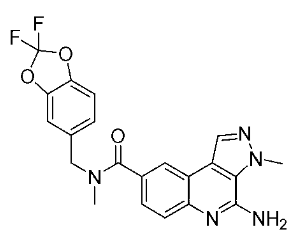 |
| **I-49** | 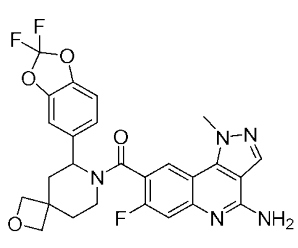 | **I-50** | 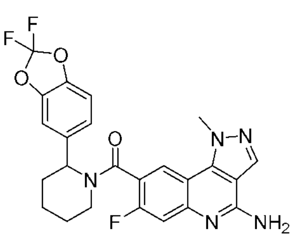 | **I-51** | 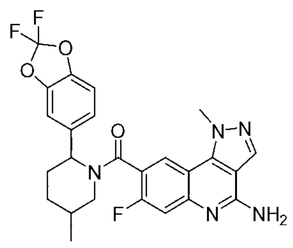 |
| **I-52** | 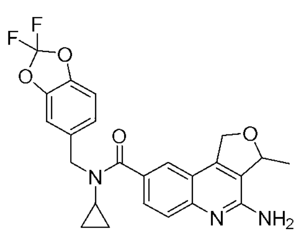 | **I-53** | 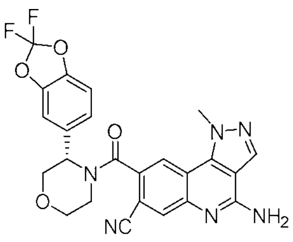 | **I-54** | 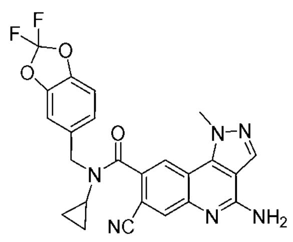 |
| **I-55** | 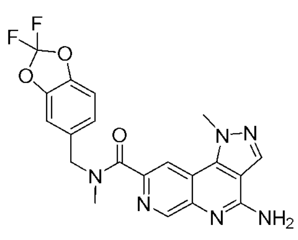 | **I-56** | 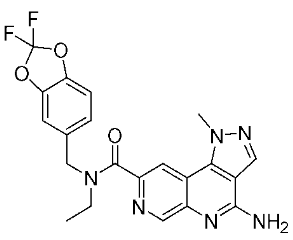 | **I-57** | 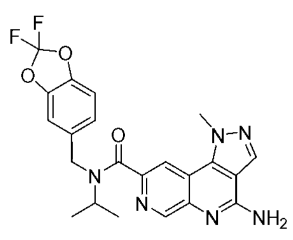 |
| I-58 | 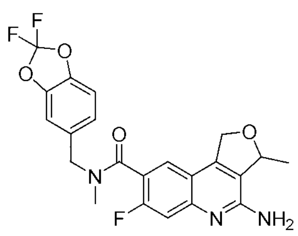 | **I-59** | 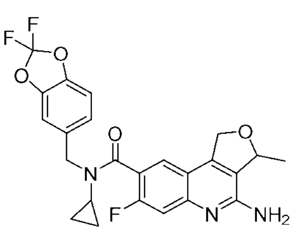 | **I-60** | 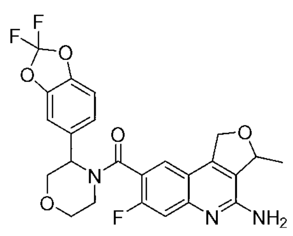 |
| **I-61** | 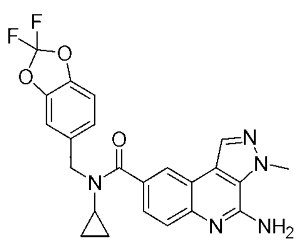 | **I-62** | 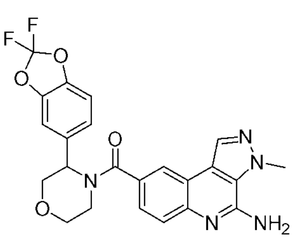 | **I-63** | 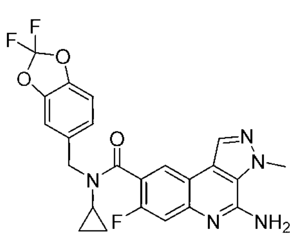 |
| **I-64** | 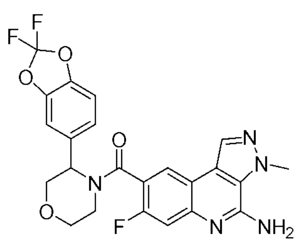 | **I-65** | 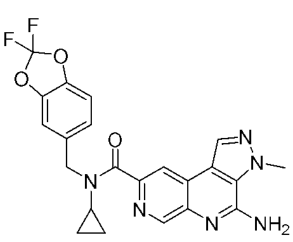 | **I-66** | 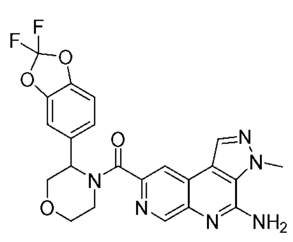 |

(continued)

| | | | | | |
|---|---|---|---|---|---|
| **I-67** | 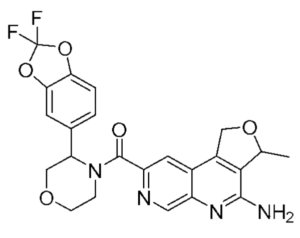 | **I-68** | 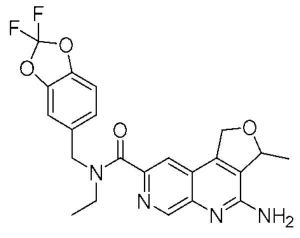 | **I-69** | 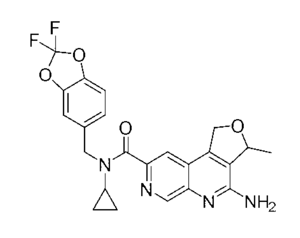 |
| **I-70** | 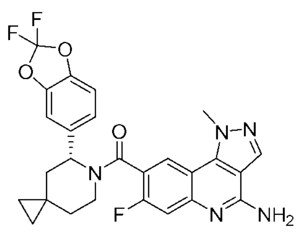 | **I-71** | 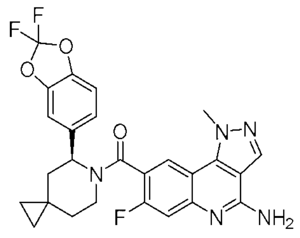 | **I-72** | 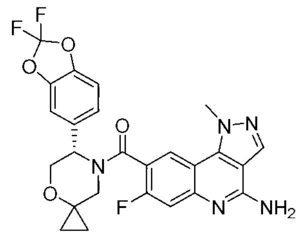 |
| **I-73** | 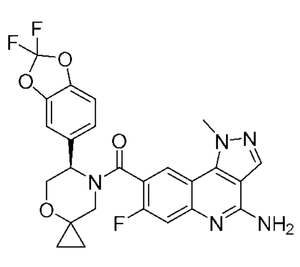 | **I-74** | 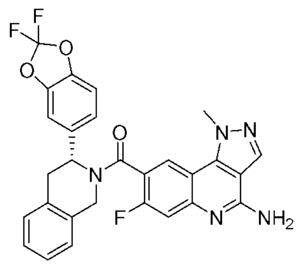 | **I-75** | 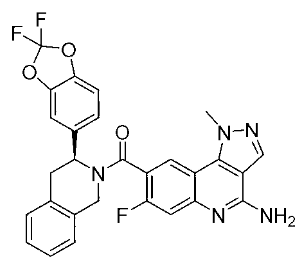 |
| **I-76** | 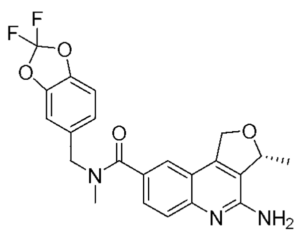 | **I-77** | 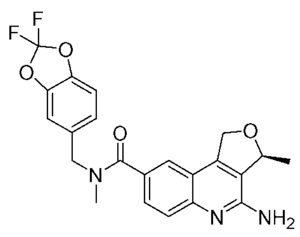 | **I-78** | 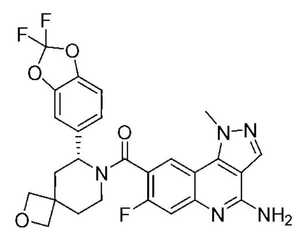 |
| **I-79** | 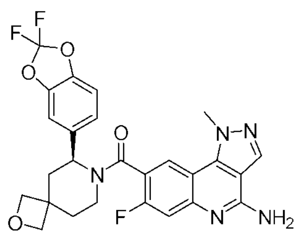 | **I-80** | 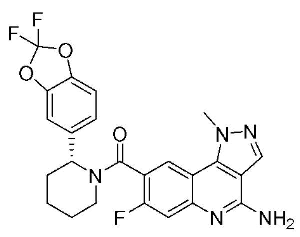 | **I-81** | 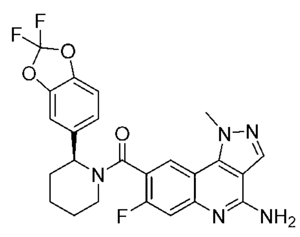 |
| **I-82** | 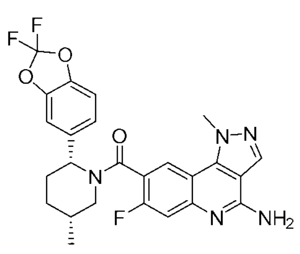 | **I-83** | 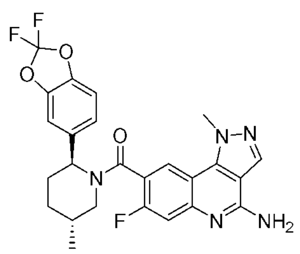 | **I-84** | 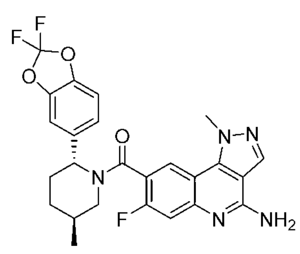 |

(continued)

| | | | | | |
|---|---|---|---|---|---|
| **I-85** | 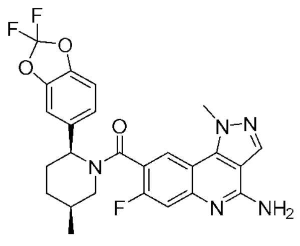 | **I-86** | 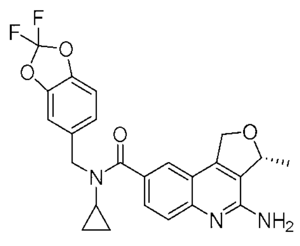 | **I-87** | 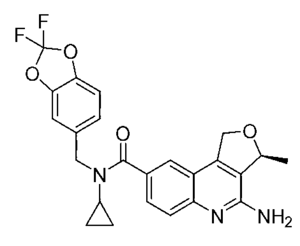 |
| **I-88** | 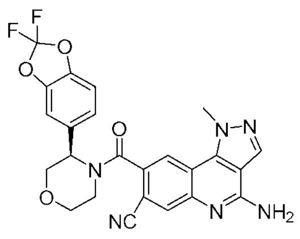 | **I-89** | 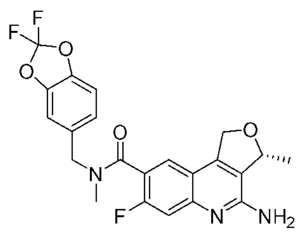 | **I-90** | 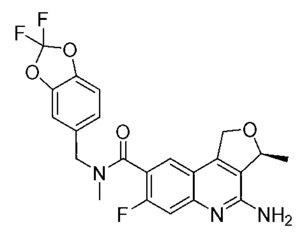 |
| **I-91** | 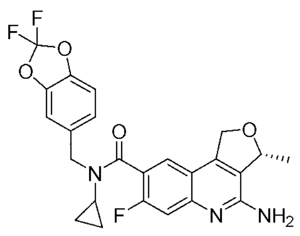 | **I-92** | 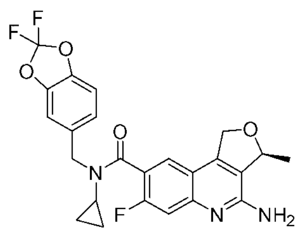 | **I-93** | 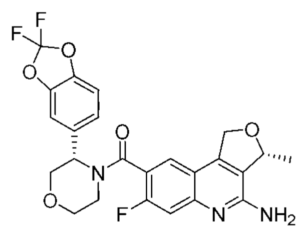 |
| **I-94** | 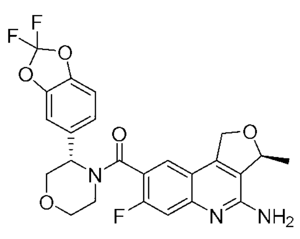 | **I-95** | 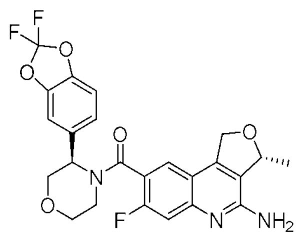 | **I-96** | 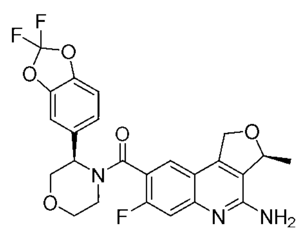 |
| **I-97** | 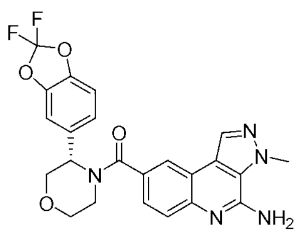 | **I-98** | 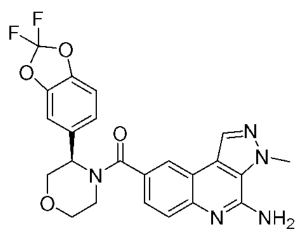 | **I-99** | 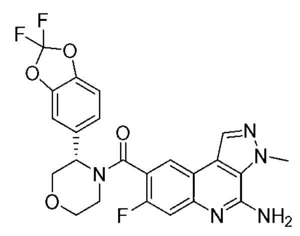 |
| **I-100** | 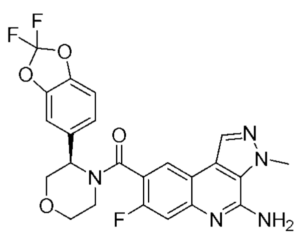 | **I-101** | 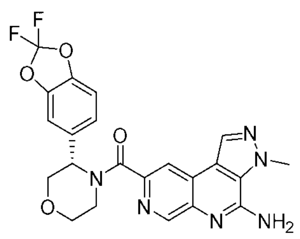 | **I-102** | 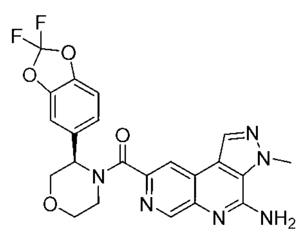 |
| **I-103** | 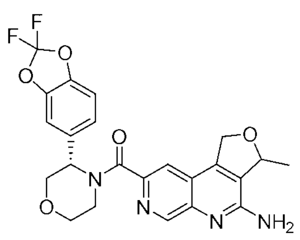 | **I-104** | 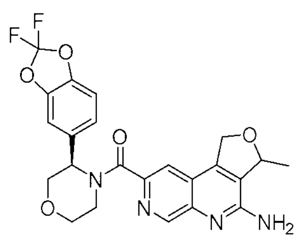 | **I-105** | 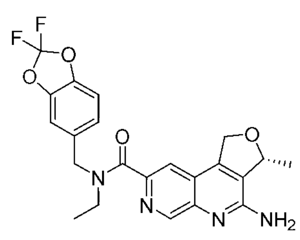 |

(continued)

| | | | | | |
|---|---|---|---|---|---|
| **I-106** | 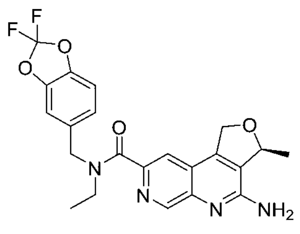 | **I-107** | 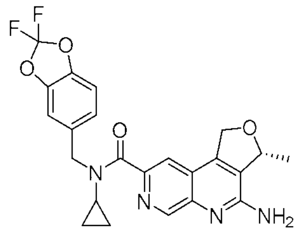 | **I-108** | 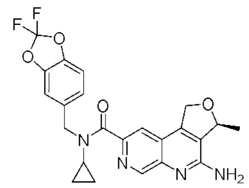 |
| **I-109** | 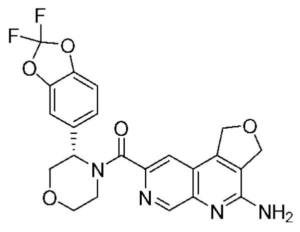 | **I-110** | 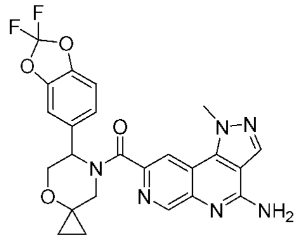 | **I-111** | 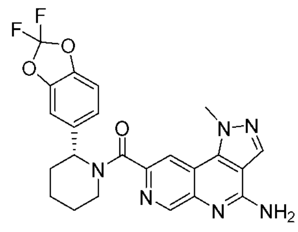 |
| **I-112** | 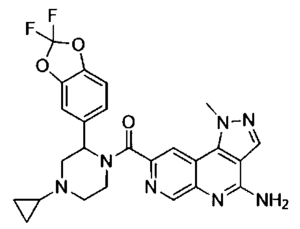 | **I-113** | 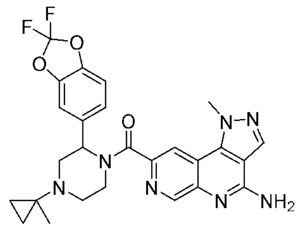 | **I-114** | 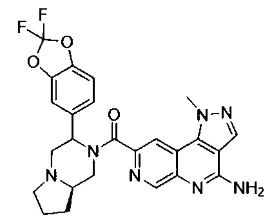 |
| **I-115** | 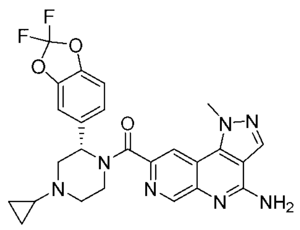 | **I-116** | 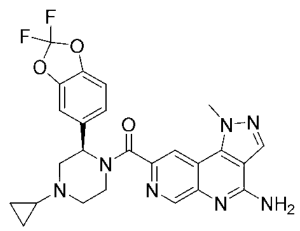 | **I-117** | 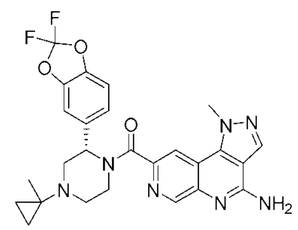 |
| **I-118** | 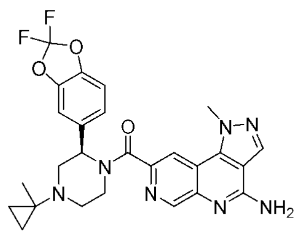 | . | | | |

**11.** A pharmaceutical composition, comprising a prophylactically or therapeutically effective amount of the compound, or the pharmaceutically acceptable salt, the ester, the stereoisomer, the tautomer, the polymorph, the solvate, the metabolite, the isotopically labeled compound, or the prodrug thereof according to any one of claims 1 to 10, and a pharmaceutically acceptable carrier.

**12.** Use of the compound, or the pharmaceutically acceptable salt, the ester, the stereoisomer, the tautomer, the polymorph, the solvate, the metabolite, the isotopically labeled compound, or the prodrug thereof according to any one of claims 1 to 10, or of the pharmaceutical composition according to claim 11 in preparing a medicament as a PRMT5 inhibitor, wherein preferably, the medicament is used in preventing or treating a cancer (preferably an MTAP-deficient cancer, such as pancreatic cancer, lung cancer, colorectal cancer, cholangiocarcinoma, multiple myeloma, melanoma, uterine cancer, endometrial cancer, thyroid cancer, acute myeloid leukemia, bladder cancer, urothelial cancer, gastric cancer, cervical cancer, head and neck cancer, head and neck squamous cell carcinoma, lymphoma, diffuse large B-cell lymphoma, esophageal cancer, chronic lymphocytic leukemia, hepatocellular carcinoma, skin cancer, breast cancer, ovarian cancer, prostate cancer, glioblastoma, renal cancer, and sarcoma).

## INTERNATIONAL SEARCH REPORT

International application No.
**PCT/CN2024/089253**

### A. CLASSIFICATION OF SUBJECT MATTER

C07D471/04(2006.01)i; C07D401/12(2006.01)i; C07D413/14(2006.01)i; C07D405/14(2006.01)i; A61K31/407(2006.01)i; A61P35/00(2006.01)i; A61P35/02(2006.01)i

According to International Patent Classification (IPC) or to both national classification and IPC

### B. FIELDS SEARCHED

Minimum documentation searched (classification system followed by classification symbols)

C07D471/-; C07D401/-; C07D413/-; C07D405/-; A61K31/-; A61P35/-

Documentation searched other than minimum documentation to the extent that such documents are included in the fields searched

Electronic data base consulted during the international search (name of data base and, where practicable, search terms used)

CNABS: CNTXT; VEN; WOTXT; USTXT; EPTXT; GBTXT; CATXT; CNKI; 万方, WANFANG; STN; 勤浩医药（苏州）有限公司; 张贵平; 李家鹏; 王奎锋; 郑计岳; 张涛; 法里东; 徐浩杰; 董雪; PRMT5; 式(I)结构, structure of Formula (I); 具体化合物结构, the specific compound structure.

### C. DOCUMENTS CONSIDERED TO BE RELEVANT

| Category* | Citation of document, with indication, where appropriate, of the relevant passages | Relevant to claim No. |
|---|---|---|
| PX | CN 117362323 A (SHANGHAI SAILAN BIOTECHNOLOGY CO., LTD.) 09 January 2024 (2024-01-09)<br>claim 10 | 1-7, 9, 11-12 |
| X | WO 2021163344 A1 (AMGEN INC.) 19 August 2021 (2021-08-19)<br>compounds 569, 570, 588, 589, 616, 618-620, 622-623 and 625, and description, paragraphs [0004]-[0022] and [0071]-[0072] | 1-12 |
| A | CN 102414180 A (SIRTRIS PHARMACEUTICALS INC.) 11 April 2012 (2012-04-11)<br>entire document | 1-12 |
| A | CN 110963997 A (SICHUAN KELUN-BIOTECH BIOPHARMACEUTICAL CO., LTD.) 07 April 2020 (2020-04-07)<br>entire document | 1-12 |
| A | CN 115260180 A (CHANGCHUN GENESCIENCE PHARMACEUTICALS CO., LTD.) 01 November 2022 (2022-11-01)<br>entire document | 1-12 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 July 2024** | **05 July 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/089253**

| Patent document cited in search report | | | Publication date (day/month/year) | Patent family member(s) | | | Publication date (day/month/year) |
|---|---|---|---|---|---|---|---|
| CN | 117362323 | A | 09 January 2024 | None | | | |
| WO | 2021163344 | A1 | 19 August 2021 | AU | 2021219730 | A1 | 25 August 2022 |
| | | | | JP | 2023513580 | A | 31 March 2023 |
| | | | | EP | 4103558 | A1 | 21 December 2022 |
| | | | | MX | 2022010011 | A | 18 October 2022 |
| | | | | US | 2023159510 | A1 | 25 May 2023 |
| | | | | CA | 3170321 | A1 | 19 August 2021 |
| CN | 102414180 | A | 11 April 2012 | AU | 2010221417 | A1 | 22 September 2011 |
| | | | | BRPI | 1011477 | A2 | 22 March 2016 |
| | | | | EA | 201171098 | A1 | 30 April 2012 |
| | | | | CA | 2754058 | A1 | 10 September 2010 |
| | | | | JP | 2012519211 | A | 23 August 2012 |
| | | | | WO | 2010101949 | A1 | 10 September 2010 |
| | | | | KR | 20110128908 | A | 30 November 2011 |
| | | | | IL | 214943 | A0 | 30 November 2011 |
| | | | | MX | 2011009213 | A | 14 December 2011 |
| | | | | EP | 2403833 | A1 | 11 January 2012 |
| | | | | EP | 2403833 | A4 | 29 August 2012 |
| CN | 110963997 | A | 07 April 2020 | None | | | |
| CN | 115260180 | A | 01 November 2022 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022132914 A1


### Non-patent literature cited in the description


- **T. HIGUCHI** ; **V. STELLA**. Pro-drugs as Novel Delivery Systems. *ACS Symposium Series*, vol. 14
- Bioreversible Carriers in Drug Design. American Pharmaceutical Association. Pergamon Press, 1987
- **H. BUNDGAARD**. Design of Prodrugs. Elsevier, 1985
- Protective Groups in Organic Chemistry. Plenum Press, 1973
- **T.W. GREENE** ; **P.G.M. WUTS**. Protective Groups in Organic Synthesis. John Wiley & Sons, 1991